# EUROPEAN PATENT APPLICATION

(11) **EP 1 905 762 A1**
(43) Date of publication of application: **02.04.2008**
(21) Application number: 06756677.8
(22) Date of filing: 29.05.2006
(51) Int. Cl.: C07D 231/20, A61K 31/4152, A61K 31/4155, A61K 31/4178, A61K 31/4192, A61K 31/4196, A61K 31/422, A61K 31/427, A61K 31/4439, A61K 31/454, A61K 31/4545, A61K 31/496, A61K 31/498, A61P 11/00, A61P 25/28, A61P 29/00, A61P 35/00, A61P 37/08, A61P 43/00, C07D 401/04, C07D 401/06

(54) **PYRAZOLONE DERIVATIVE**

(30) Priority: 30.05.2005 JP 2005158217
(71) Applicant: Genecare Research Institute Co., Ltd, Kamakura-shi, Kanagawa 247-0063 (JP)
(72) Inventor: FUJIWARA, Hideyasu,, 19-2 Kajiwara, Kamakura-shi, Kanagawa 247-0063 (JP); NAKAJIMA, Hiroto, 19-2 Kajiwara, Kamakura-shi, Kanagawa 247-0063 (JP); MASUDA, Takanobu, 19-2 Kajiwara, Kamakura-shi, Kanagawa 247-0063 (JP); FURUICHI, Yasuhiro, 19-2 Kajiwara, Kamakura-shi, Kanagawa 247-0063 (JP); SHIMBARA, Naoki, 19-2 Kajiwara, Kamakura-shi, Kanagawa 247-0063 (JP)
(74) Representative: Grünecker, Kinkeldey, Stockmair & Schwanhäusser Anwaltssozietät
(86) International application number: PCT/JP2006/310632
(87) International publication number: WO 2006/129583

(57) **Abstract**

The present invention relates to compounds represented by formula (I), or pharmaceutically acceptable prodrugs or salts thereof, or hydrates or solvates thereof, 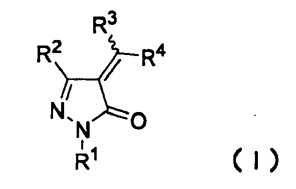 (wherein,
R¹, R², R³, and R⁴ are the same as R¹, R², R³, and R⁴ in the description of the present invention).

## Description

### Technical Field

The present invention relates to novel pyrazolone derivatives.

### Background Art

The ubiquitin-proteasome system, which is an intracellular ATP energy-dependent proteolysis system, plays an important role in a variety of higher biological functions (cell cycle signal transduction, transcription, metabolic regulation, apoptosis, immune response, and such) and maintenance of homeostasis in response to environmental stress (stress response and control of protein quality) (Non-Patent Documents 1 and 2).

Ubiquitin is a conserved eukaryotic protein composed of 76 amino acids. By covalently binding to a specific target protein, it modifies the protein and serves as a degradation signal that leads to degradation of the protein. Binding of ubiquitin to a target protein leads to modification of the protein through consecutive actions of the multi-enzyme ubiquitin system constituted of an ubiquitin-activating enzyme (E1), ubiquitin-binding enzyme (E2), and ubiquitin ligase (E3). Repetition of the E1-E2-E3 cascade reactions causes ubiquitins to bind to a target protein, and leads to the formation of a polyubiquitin chain. It is identified by intracellular proteases (proteasomes) and then the target protein is degraded (Non-Patent Document 3).

While only one type of E 1 is involved in the ubiquitin system, there is molecular diversity in E2 and E3. It is predicted that the number of types of E2 is on the order of tens, and more than 1,000 types of E3 are involved in the differentiation of target proteins. E3 is the most important molecule for determining the specificity of the ubiquitin system in catalyzing the reaction of transferring ubiquitin to a target protein, or to an ubiquitin chain already formed on the target protein, by direct or indirect interaction with the target protein. The primary types of E3 reported so far are the HECT type and the RING finger type (Non-Patent Document 4). Abnormalities of the ubiquitin system are suggested to be relevant to cancers, neurodegenerative diseases (for example, Parkinson's disease and Alzheimer's disease), and immune-related diseases (Non-Patent Document 5).

Meanwhile, IκB forms a complex with NFκB, a transcription factor involved in inflammation, to regulate its activity. IκBα, which is a type of IκB, is ubiquitinated as a result of binding to the SCF^{Fbw1} complex (ROC1/Cullin1/Skp1/Fbw1 (βTrCP1)), which is one type of E3 enzyme, and is then degraded by proteasomes (Non-Patent Documents 6 and 7). Therefore, substances that inhibit the ubiquitination of IκBα inhibit IκBα degradation via the ubiquitin-proteasome system, and suppress the activation of the transcription factor NFκB by stabilizing IκBα.

Genes activated by NFκB are cytokines, chemokines, adhesion molecules, growth factors, acute-phase proteins, inflammation-related enzymes, transcription factors, and such. Specific examples include inflammatory cytokines such as TNFα, interleukin (IL) 1-β, IL-2, IL-6, and IL-12; chemokines such as IL-8 and RANTES; adhesion factors such as ICAM-1 and VCAM-1; growth factors such as IL-3, GM-CSF, G-CSF, and M-CSF; and inflammation-related enzymes such as cyclooxygenase (COX)-2, 12-lipooxygenase phospholipase A2, and inducible nitric oxide synthase (iNOS) (Non-Patent Document 8).

Activation of NFκB has been observed in various inflammatory diseases, cancers, and neurodegenerative diseases. For example, activation of NFκB has been reported in chronic inflammatory diseases such as asthma, rheumatism, and inflammatory bowel disease (Non-Patent Document 9), and in diseases such as arteriosclerosis (Non-Patent Document 10), Alzheimer's disease (Non-Patent Document 11), and cancer (Non-Patent Document 12).

Therefore, suppression of NFκB activation will be effective for treating diseases including various inflammatory diseases, neurodegenerative diseases, and cancers (Non-Patent Documents 13 and 14).

There is an invention of pyrazolone derivatives as kinase inhibitors (Patent Document 1). However, the structures are different from those of the compounds of the present invention. Furthermore, there has been no report on compounds that suppress the NFκB activation by inhibiting IκBα ubiquitination, and development of such novel compounds is desired.
[Non-Patent Document 1] Annu. Rev. Biochem., 67, 425-479, 1998
[Non-Patent Document 2] Annu. Rev. Biochem., 70, 503-533, 2001
[Non-Patent Document 3] Annu. Rev. Biochem., 65, 801-847, 1996
[Non-Patent Document 4] Cell, 116, 11-190, 2004
[Non-Patent Document 5] Biochim. Biophys. Acta, 1695, 3-17, 2004
[Non-Patent Document 6] Nature, 396, 590-594, 1998
[Non-Patent Document 7] Annu. Rev. Immunol., 18, 621-663, 2000
[Non-Patent Document 8] J. Clin. Pharmacol., 38, 981-993, 1998
[Non-Patent Document 9] J. Clin. Invest., 107, 7-11, 2001
[Non-Patent Document 10] J. Clin. Invest., 107, 255-264, 2001
[Non-Patent Document 11] J. Clin. Invest., 107, 247-253, 2001
[Non-Patent Document 12] J. Clin. Invest., 107, 241-246, 2001
[Non-Patent Document 13] J. Clinical Chemistry, 45, 7-17, 1999
[Non-Patent Document 14] J. Clin. Invest., 107, 135-141, 2001
[Patent Document 1] International Patent Application Publication No. WO01/32653

### Disclosure of the Invention

### [Problems to be Solved by the Invention]

An objective of the present invention is to provide pharmaceuticals comprising as an active ingredient, a novel substance that suppresses NFκB action based on the inhibition of an IκBα-ubiquitination enzyme.

### [Means for Solving the Problems]

Upon dedicated research to solve the above-mentioned objective, the present inventors discovered that compounds represented by formula (I) shown below or salts thereof have an activity of inhibiting the ubiquitination of IκBα and are useful as an active ingredient in pharmaceuticals, and thus completed the present invention.

More specifically, the present invention comprises:
[1] a compound represented by formula (I), or a pharmaceutically acceptable prodrug or salt thereof, or a hydrate or solvate thereof, 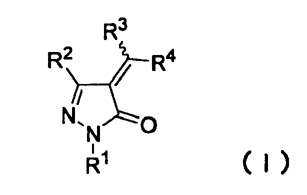 (wherein,
   R¹ represents a C₁₋₆ alkyl group, a C₃₋₈ cycloalkyl group, a C₁₋₆ alkoxycarbonyl group, a C₁₋₆ alkoxycarbonyl C₁₋₃ alkyl group, a C₆₋₁₀ aryl group, a C₆₋₁₀ aryl C₁₋₆ alkyl group, or a 5- to 10-membered heteroaryl group;
   the aforementioned C₁₋₆ alkyl group may comprise a substituent selected from a halogen atom, a carboxyl group, a C₃₋₈ cycloalkyl group, a C₁₋₆ alkoxycarbonyl group, a 5- to 10-membered heteroaryl group, a 5- to 8-membered heterocyclic group, and -NR^{1d}R^{2d} (wherein R^{1d} and R^{2d} each independently represent a hydrogen atom or a C₁₋₆ alkyl group);
   the aforementioned aryl group and the aryl moiety of the C₆₋₁₀ aryl C₁₋₆ alkyl group may each independently comprise 1 to 3 substituents selected from the group consisting of a halogen atom, a hydroxy group, a cyano group, a nitro group, a carboxyl group, a C₁₋₆ alkyl group, a C₁₋₆ alkoxy group, -NR^{1a}R^{2a} (wherein R^{1a} and R^{2a} each independently represent a hydrogen atom or a C₁₋₆ alkyl group), and -SO₂R^{b} (wherein R^{b} represents a hydroxy group, a piperidyl group, a hydroxypiperidyl group, or NR^{1b}R^{2b} (wherein, R^{1b} and R^{2b} each independently represent a hydrogen atom or a C₁₋₆ alkyl group));
   alternatively, R¹ represents a group represented by formula (II), 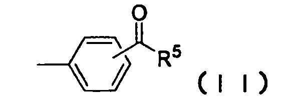 (wherein,
   R⁵ represents a 5- to 10-membered heteroaryl group, a 5- to 8-membered heterocyclic group, or a group represented by -NR^{1c}R^{2c} (wherein, R^{1c} and R^{2c} each independently represent a hydrogen atom, or a C₁₋₆ alkyl group or benzodioxoylmethyl group that may comprise substituents selected from Group A described below; and the 5- to 10-membered heteroaryl group and the 5- to 8-membered heterocyclic group may each independently comprise 1 to 3 substituents selected from the group consisting of a hydroxy group, a carboxyl group, a C₁₋₆ alkyl group, a hydroxy C₁₋₆ alkyl group, a C₆₋₁₀ aryl group, and a hydroxy C₆₋₁₀ aryl group;
   Group A: a halogen atom, a hydroxy group, a carboxyl group, a C₁₋₆ alkoxy group, a C₃₋₈ cycloalkyl group, a C₁₋₆ alkoxycarbonyl group, a C₁₋₆ alkylcarbonyloxy group, a 5- to 10-membered heteroaryl group, a 5- to 8-membered heterocyclic group, and NR^{1d}R^{2d} (wherein, R^{1d} and R^{2d} each independently represent a hydrogen atom or a C₁₋₆ alkyl group);
   R² represents a C₁₋₆ alkyl group, C₃₋₈ cycloalkyl group, R^{e}CO- that may comprise substituents selected from Group A mentioned above (wherein, R^{e} represents a heterocyclic group or -NR^{1e}R^{2e} (wherein, R^{1e} and R^{2e} each independently represent a hydrogen atom or a C₁₋₆ alkyl group), and the heterocyclic group may comprise a C₁₋₆ alkoxycarbonyl group as a substituent), a 5- to 10-membered heteroaryl group, a C₆₋₁₀ aryl group, or a C₆₋₁₀ aryl C₁₋₆ alkyl group (the aforementioned aryl group and the aryl moiety of the C₆₋₁₀ aryl C₁₋₆ alkyl group may comprise 1 to 3 substituents selected from the group consisting of a halogen, a hydroxy group, and a C₁₋₆ alkoxy group);
   R³ represents a hydrogen atom or a C₁₋₆ alkyl group, R³ and R⁴ may be either an E or Z isomer;
   R⁴ represents a C₆₋₁₀ aryl group, a 5- to 10-membered heteroaryl group, or a 5- to 8-membered heterocyclic group, and said C₆₋₁₀ aryl group, 5- to 10-membered heteroaryl group, and 5- to 8-membered heterocyclic group may each independently comprise 1 to 3 substituents selected from Group B described below;
   Group B: a halogen atom, a nitro group, a C₁₋₆ alkoxy group, a carboxyl group, an oxo group, a C₁₋₆ alkyl group which may comprise a substituent selected from Group A described above (when said alkyl group comprises an isoxazolyl group as a substituent, the isoxazolyl group may comprise a C₁₋₃ alkyl group as a substituent), a C₁₋₆ alkylenedioxy group, C₆₋₁₀ aryl group, C₆₋₁₀ aryl C₁₋₆ alkyl group, -SO₃H, -SO₂NR^{1f}R^{2f} (wherein, R^{1f} and R^{2f} each independently represent a hydrogen atom or a C₁₋₆ alkyl group), a C₃₋₈ cycloalkylcarbonyl group, a C₆₋₁₀ arylcarbonyl group, a 5- to 10-membered heteroarylcarbonyl group, a 5- to 10-membered heteroaryl group, and a 5- to 8-membered heterocyclic group;
   In Group B mentioned above, the C₆₋₁₀ aryl group, 5- to 10-membered heteroaryl group, and 5- to 8-membered heterocyclic group may each independently comprise 1 to 4 substituents selected from Group C described below;
   Group C: a halogen atom, a C₁₋₆ alkyl group that may comprise a substituent selected from Group A described above, a C₁₋₆ alkoxy group, a hydroxy group, a carboxyl group, a C₁₋₆ alkylcarbonyl group, a C₁₋₆ alkoxycarbonyl group, a C₆₋₁₀ arylcarbonyl group, a C₆₋₁₀ aryl C₁₋₃ alkoxycarbonyl group, a C₆₋₁₀ aryl group, a C₆₋₁₀aryl C₁₋₆ alkyl group, a halogenated C₆₋₁₀ aryl group, a methanesulfonyl group, a 5- to 8-membered heterocyclic group, and a 5- to 10-membered heteroarylcarbonyl group; alternatively, R⁴ may represent a group represented by formula (III),

   -Ar¹-O-R⁶ (III)

   (wherein,
   Ar¹ represents a phenyl group or a C₁₋₃ alkoxyphenyl group;
   R⁶ represents a hydrogen atom, a C₁₋₆ alkyl group that may comprise a substituent selected from Group A described above (when said alkyl group comprises a 5- to 10-membered heteroaryl group as a substituent, said 5- to 10-membered heteroaryl group may comprise 1 to 3 substituents selected from the group consisting of a C₁₋₆ alkyl group and a C₁₋₆ alkoxycarbonyl group), a methanesulfonyl group, a phenylsulfonyl group, a *p*-toluenesulfonyl group, a *p*-(*t*-butyl)phenylsulfonyl group, a C₆₋₁₀ aryl group, a C₆₋₁₀ aryl C₁₋₆ alkyl group (in R⁶, the aforementioned aryl group and the aryl moiety of the C₆₋₁₀ aryl C₁₋₆ alkyl group may comprise 1 to 3 substituents selected from a nitro group, a C₁₋₆ alkyl group, and a C₁₋₃ alkoxycarbonyl group), and R^{g}CO-CH₂- (wherein, R^{g} represents a heterocyclic group or -NR^{1g}R^{2g} (wherein, R^{1g} and R^{2g} each independently represent a hydrogen atom, a C₁₋₆ alkyl group, or a C₆₋₁₀ aryl group));
   alternatively, R⁴ may represent a group represented by formula (IV); 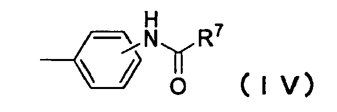 (wherein,
   k⁷ represents a C₂₋₆ alkenyl group, a C₆₋₁₀ aryl group, a 5- to 10-membered heteroaryl group, or a phenylethylene group;
   the aforementioned C₆₋₁₀ aryl group, 5- to 10-membered heteroaryl group, and phenylethylene group may each independently comprise 1 to 3 substituents selected from the group consisting of a halogen atom, a hydroxy group, a C₁₋₃ alkoxycarbonyl group, and a C₁₋₃ alkoxy group));
[2] the compound of [1], or a pharmaceutically acceptable prodrug or salt thereof, or a hydrate or solvate thereof, wherein the aforementioned R¹ represents a C₁₋₃ alkyl group, a C₃₋₈ cycloalkyl group, a C₁₋₆ alkoxycarbonyl C₁₋₃ alkyl group, a C₆₋₁₀ aryl C₁₋₃ alkyl group, a C₆₋₁₀ aryl group, a C₁₋₆ alkoxycarbonyl group, or a pyridyl group;
   the aforementioned aryl group and the aryl moiety of the C₆₋₁₀ aryl C₁₋₃ alkyl group may each independently comprise 1 to 3 substituents selected from the group consisting of a halogen atom, a hydroxy group, a cyano group, a nitro group, a carboxyl group, a C₁₋₆ alkyl group, a C₁₋₆ alkoxy group, -SO₂R^{a} (wherein, R^{a} represents a hydroxy group, NH₂, a piperidyl group, or a hydroxypiperidyl group), and -NR^{1b}R^{2b} (wherein, R^{1b} and R^{2b} each independently represent a hydrogen atom or a C₁₋₆ alkyl group);
   alternatively, the aforementioned R¹ represents a group represented by formula (II) 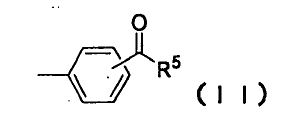 (wherein,
   R⁵ represents -NH₂, a benzodioxoylmethylamino group, a mono C₁₋₆ alkylamino group, a di C₁₋₆ alkylamino group, a mono (C₃₋₈ cycloalkyl C₁₋₃ alkyl) amino group, a hydroxy C₁₋₆ alkylamino group, an *N*-(hydroxy C₁₋₆ alkyl)-*N*-C₁₋₆ alkylamino group, a C₁₋₆ alkoxy C₁₋₆ alkylamino group, a pyrrolidinyl group, a piperidyl group, a piperazinyl group, a tetrahydropyridyl group, or a morpholinyl group;
   said pyrrolidinyl group, piperidyl group, piperazinyl group, and tetrahydropyridyl group may each independently comprise 1 to 3 substituents selected from the group consisting of a hydroxy group, a carboxyl group, a C₁₋₆ alkyl group, a hydroxy C₁₋₆ alkyl group, a C₆₋₁₀ aryl group, and a hydroxy C₆₋₁₀ aryl group);
[3] the compound of [1], or a pharmaceutically acceptable prodrug or salt thereof, or a hydrate or solvate thereof, wherein the aforementioned R¹ represents a C₆₋₁₀ aryl C₁₋₃ alkyl group, a C₆₋₁₀ aryl group, a cyclohexyl group, or a pyridyl group;
   the aforementioned C₆₋₁₀ aryl group and the aryl moiety of the C₆₋₁₀ aryl C₁₋₃ alkyl group may each independently comprise 1 to 3 substituents selected from the group consisting of a halogen atom, a hydroxy group, a cyano group, a nitro group, a carboxyl group, a C₁₋₆ alkyl group, a C₁₋₆ alkoxy group, -SO₂R^{a} (wherein, R^{a} represents a hydroxy group, -NH₂, a piperidyl group, or a hydroxypiperidyl group), and -NR^{1b}R^{2b} (wherein, R^{1b} and R^{2b} each independently represent a hydrogen atom or a C₁₋₆ alkyl group);
   alternatively, the aforementioned R¹ represents a group represented by formula (II) 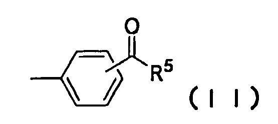 (wherein,
   R⁵ represents -NH₂, a benzodioxoylmethylamino group, a mono C₁₋₆ alkylamino group, a di C₁₋₆ alkylamino group, a mono (C₃₋₈ cycloalkyl C₁₋₃ alkyl) amino group, a hydroxy C₁₋₆ alkylamino group, an *N*-(hydroxy C₁₋₆ alkyl)-*N*-C₁₋₆ alkylamino group, a C₁₋₆ alkoxy C₁₋₆ alkylamino group, a pyrrolidinyl group, a piperidyl group, a piperazinyl group, a tetrahydropyridyl group, or a morpholinyl group;
   said pyrrolidinyl group, piperidyl group, piperazinyl group, and tetrahydropyridyl group may each independently comprise 1 to 3 substituents selected from the group consisting of a hydroxy group, a carboxyl group, a C₁₋₆ alkyl group, a hydroxy C₁₋₆ alkyl group, a C₆₋₁₀ aryl group, and a hydroxy C₆₋₁₀ aryl group);
[4] the compound of any one of [1] to [3], or a pharmaceutically acceptable prodrug or salt thereof, or a hydrate or solvate thereof, wherein R² represents a C₁₋₆ alkyl group, a C₃₋₈ cycloalkyl group, a halogenated C₁₋₆ alkyl group, a C₁₋₆ alkoxy C₁₋₆ alkyl group, R^{e}CO- (wherein R^{e} represents a heterocyclic group or -NR^{1e}R^{2e} (wherein, R^{1e} and R^{2e} each independently represent a hydrogen atom or a C₁₋₆ alkyl group), and the heterocyclic group may comprise a C₁₋₆ alkoxycarbonyl group as a substituent), a furyl group, a pyridyl group, a C₆₋₁₀ aryl group, or a C₆₋₁₀ aryl C₁₋₆ alkyl group (the aforementioned aryl group and the aryl moiety of the C₆₋₁₀ aryl C₁₋₆ alkyl group may comprise 1 to 3 substituents selected from the group consisting of a halogen, a hydroxy group, and a C₁₋₆ alkoxy group);
[5] the compound of any one of [1] to [4], or a pharmaceutically acceptable prodrug or salt thereof, or a hydrate or solvate thereof, wherein R³ is a hydrogen atom;
[6] the compound of any one of [1] to [5], or a pharmaceutically acceptable prodrug or salt thereof, or a hydrate or solvate thereof, wherein
   R⁴ represents a phenyl group, a naphthyl group, an indolyl group, a furyl group, a thienyl group, a thiazolyl group, a pyrrolyl group, a benzofuranyl group, a dimethylpyrrolyl group, a pyridyl group, a quinoxalinyl group, a pyrazolinyl group, or a pyrazolyl group;
   said phenyl group, naphthyl group, indolyl group, furyl group, thienyl group, thiazolyl group, pyrrolyl group, benzofuranyl group, dimethylpyrrolyl group, pyridyl group, quinoxalinyl group, pyrazolinyl group, and pyrazolyl group may each independently comprise 1 to 3 substituents selected from Group D described below;
   Group D: a halogen atom, a nitro group, a C₁₋₃ alkoxy group, a carboxyl group, an oxo group, a C₁₋₆ alkyl group, a C₃₋₈ cycloalkyl C₁₋₃ alkyl group, a hydroxy C₁₋₃ alkyl group, a C₆₋₁₀ aryl C₁₋₃ alkyl group, an isoxazolyl C₁₋₃ alkyl group (the aforementioned isoxazolyl ring may comprise a C₁₋₃ alkyl group as a substituent), a C₁₋₃ alkylcarbonyloxy C₁₋₃ alkyl group, a C₁₋₆ alkylenedioxy group, a phenyl group, -SO₃H, a C₆₋₁₀ arylcarbonyl group, a furylcarbonyl group, a C₃₋₈ cycloalkylcarbonyl group, an *N*-methylpyrrolylcarbonyl group, a morpholinyl group, a pyrrolyl group, a triazolyl group, a thiazolyl group, an imidazolyl group, a pyrazolyl group, a pyrrolidinyl group, a piperidyl group, and a piperazinyl group;
   in the aforementioned Group D, a phenyl group, a morpholinyl group, a pyrrolyl group, a triazolyl group, a thiazolyl group, an imidazolyl group, a pyrazolyl group, a pyrrolidinyl group, a piperidyl group, and a piperazinyl group may each independently comprise 1 to 4 substituents selected from Group E described below;
   Group E: a halogen atom, a C₁₋₃ alkyl group, a hydroxy C₁₋₃ alkyl group, a C₁₋₃ alkoxy group, a C₁₋₆ alkoxycarbonyl C₁₋₆ alkyl group, a hydroxy group, a carboxyl group, a C₁₋₆ alkylcarbonyl group, a C₁₋₆ alkoxycarbonyl group, a C₆₋₁₀ arylcarbonyl group, a C₆₋₁₀ aryl C₁₋₃ alkoxycarbonyl group, a phenyl group, a halophenyl group, a benzyl group, a methanesulfonyl group, a pyrrolidinyl group, a furylcarbonyl group, and an isoxazolylcarbonyl group;
   alternatively, R⁴ may represent a group represented by formula (III);

   -Ar¹-O-R⁶ (III)

   (wherein,
   Ar¹ represents a phenyl group or a C₁₋₃ alkoxyphenyl group;
   R⁶ represents a hydrogen atom, a C₁₋₆ alkyl group (said alkyl group may comprise an isoxazolyl group, a furyl group, or a benzotriazole group as a substituent, and said isoxazolyl group, furyl group, and benzotriazole group may each independently comprise 1 to 3 substituents selected from the group consisting of a C₁₋₃ alkyl group and a C₁₋₃ alkoxycarbonyl group), a C₃₋₈ cycloalkylmethyl group, a hydroxycarbonyl C₁₋₃ alkyl group, a C₁₋₆ alkoxycarbonyl C₁₋₃ alkyl group, a tetrahydrofuranylmethyl group, an amino C₁₋₆ alkyl group, a mono C₁₋₃ alkylamino C₁₋₆ alkyl group, a di C₁₋₃ alkylamino C₁₋₆ alkyl group, a methanesulfonyl group, a phenylsulfonyl group, a *p*-toluenesulfonyl group, ap-(t-butyl)phenylsulfonyl group, a C₆₋₁₀ aryl group, a C₆₋₁₀ aryl C₁₋₆ alkyl group (said C₆₋₁₀ aryl group and the aryl moiety of the C₆₋₁₀ aryl C₁₋₆ alkyl group may be substituted with 1 to 3 groups selected from a nitro group, a C₁₋₆ alkyl group, a carboxyl group, and a C₁₋₃ alkoxycarbonyl group); or
   R^{g}CO-CH₂- (wherein, R^{g} represents a piperidyl group, a morpholinyl group, or -NR^{1g}R^{2g} (wherein, R^{1g} and R^{2g} each independently represent a hydrogen atom, a C₁₋₆ alkyl group, or a C₆₋₁₀ aryl group)); and
   alternatively, R⁴ may represent a group represented by formula (IV); 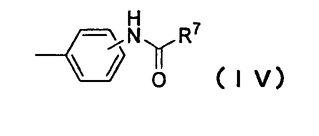 (wherein, R⁷ represents a C₂₋₆ alkenyl group, a phenyl group, a furyl group, a naphthyl group, or a phenylethylene group;
   the aforementioned phenyl group, furyl group, naphthyl group, phenylethylene group may each independently comprise 1 to 3 substituents selected from the group consisting of a halogen atom, a hydroxy group, a C₁₋₃ alkoxycarbonyl group, and a C₁₋₃ alkoxy group));
[7] the compound of any one of [1] to [5], or a pharmaceutically acceptable prodrug or salt thereof, or a hydrate or solvate thereof, wherein
   R⁴ represents a phenyl group, a naphthyl group, a quinoxalinyl group, a thienyl group, a thiazolyl group, a benzofuranyl group, an indolyl group, or a furyl group;
   the aforementioned phenyl group may be substituted with a group selected from a halogen atom, a nitro group, a carboxyl group, a morpholinyl group, a pyrrolyl group, a pyrazolyl group, a pyrazolinyl group, a triazolyl group, an imidazolyl group, a thiazolyl group, a phenyl group, a C₁₋₆ alkyl group, a C₁₋₆ alkylenedioxy group, a C₁₋₃ alkylimidazolyl group, a
   C₆₋₁₀ aryl C₁₋₃ alkylimidazolyl group, -SO₂NR^{1f}R^{2f} (wherein, R^{1f} and R^{2f} each independently represent a hydrogen atom or a C₁₋₆ alkyl group), a pyrrolidinyl group (the pyrrolidinyl group may comprise a hydroxy group or a hydroxymethyl group as a substituent), a piperidyl group (the piperidyl group may be substituted with a group selected from a hydroxy group, a pyrrolidinyl group, a carboxyl group, a fluorophenyl group, and a hydroxymethyl group), and a piperazinyl group (the piperazinyl group may be substituted with a group selected from a phenyl group, a fluorophenyl group, a C₁₋₃ alkyl group, a C₁₋₆ alkylcarbonyl group, a furylcarbonyl group, an isoxazolylcarbonyl group, a C₁₋₆ alkoxycarbonyl group, a C₆₋₁₀ arylcarbonyl group, and a methanesulfonyl group);
   the aforementioned indolyl group may be substituted with a group selected from a halogen atom, a benzyl group, a C₁₋₃ alkyl group, a C₃₋₈ cycloalkylmethyl group, a methylisoxazolylmethyl group, a dimethylisoxazolylmethyl group, a methylthiazolylmethyl group, a C₃₋₈ cycloalkylcarbonyl group, a furylcarbonyl group, a methylpyrrolylcarbonyl group, and a C₆₋₁₀ arylcarbonyl group (the aryl moiety may be substituted with a C₁₋₆ alkyl group);
   the aforementioned furyl group may be substituted with a group selected from a hydroxymethyl group, a nitro group, a hydroxysulfonyl group, an *N*-methylpyrazolyl group, an *N*-benzylpyrazolyl group, and a phenyl group (the phenyl group may comprise a substituent selected from a halogen atom, a C₁₋₃ alkoxy group, a C₁₋₃ alkoxycarbonyl group, and a C₁₋₃ alkylcarbonyl group);
   alternatively, the aforementioned R⁴ represents formula (III);

   -Ar¹-O-R⁶ (III)

   (wherein,
   Ar¹ represents a phenyl group, a methoxyphenyl group, or an ethoxyphenyl group;
   R⁶ represents a hydrogen atom, a methyl group, a cyclopropylmethyl group, a cyclohexylmethyl group, an ethoxycarbonylmethyl group, a *t*-butoxycarbonylmethyl group, an ethoxycarbonylfurylmethyl group, -CH₂CH₂CH₂N(CH₃)₂, a benzotriazolmethyl group, a *p*-(*t*-butylphenyl)sulfonyl group, a *p*-toluenesulfonyl group, -CH₂CO₂H, R^{g}CO-CH₂- (wherein, R^{g} represents -NH₂, -N(CH₃)₂, an *n*-propylamino group, a phenylamino group, a piperidyl group, or a morpholinyl group), a phenyl group, a benzyl group, an isoxazolylmethyl group, an oxazolyl group, a triazolyl group, or a furyl group; said phenyl group, benzyl group, isoxazolylmethyl group, oxazolyl group, triazolyl group, and furyl group may comprise 1 to 3 substituents selected from a methyl group, an ethyl group, a nitro group, -CO₂Me, and -CO₂Et); or
   alternatively, the aforementioned R⁴ represents formula (IV) 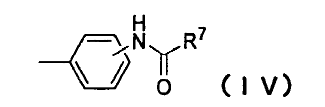 (wherein, R⁷ represents a -CH=C(CH₃)₂, -CH=CHPh, a furyl group, a naphthyl group, or a phenyl group;
   said phenyl group may comprise 1 to 3 substituents selected from a halogen atom, a hydroxy group, and a C₁₋₃ alkoxy group;
[8] the compound of any one of [1] to [5], or a pharmaceutically acceptable prodrug or salt thereof, or a hydrate or solvate thereof, wherein
   R⁴ represents a naphthyl group, a quinoxalinyl group, a thienyl group, a thiazolyl group, a benzofuranyl group, a 4-chloroindol-3-yl group, a 1-benzylindol-5-yl group, a 1-(3,5-dimethylisoxazol-4-ylmethyl)indol-5-yl group, a 1-benzylindol-5-yl group, a 1-methylindol-5-yl group, a 1-methylindol-2-yl group, a 1-cyclopropylmethylindol-5-yl group, a 1-cyclohexylmethylindol-5-yl group, a 1-cyclopropylcarbonylindol-5-yl group, a 1-benzoylindol-5-yl group, a 1-(furan-2-carbonyl)indol-5-yl group, a 1-(1-methylpyrrol-2-carbonyl)indol-5-yl group, a 5-chloroindol-3-yl group, an indol-4-yl group, an indol-5-yl group, an indol-6-yl group, an indol-7-yl group, a 1-methylindol-2-yl group, a 1-(5-methylisoxazol-3-ylmethyl)indol-5-yl group, a 1-(2-methylthiazol-4-ylmethyl)indol-5-yl group, a phenyl group, or a furyl group;
   the aforementioned phenyl group may be substituted with a group selected from a fluorine atom, a nitro group, a carboxyl group, a morpholinyl group, a pyrrolyl group, a pyrazolyl group, a pyrazolinyl group, a triazolyl group, a thiazolyl group, a phenyl group, a *t*-butyl group, a C₁₋₆ alkylenedioxy group, an imidazolyl group, a C₁₋₃ alkylimidazolyl group, a C₆₋₁₀ aryl C₁₋₃ alkylimidazolyl group, -SO₂NHCH₃, -SO₂N(CH₃)₂, -SO₂NHPh, or SO₂NPh₂,
   a pyrrolidinyl group (the pyrrolidinyl group may comprise a hydroxy group or a hydroxymethyl group as a substituent),
   a piperidyl group (said piperidyl group may comprise as a substituent a group selected from a hydroxy group, a hydroxymethyl group, a pyrrolidinyl group, a carboxyl group, and a fluorophenyl group), and
   a piperazinyl group (said piperazinyl group may comprise as a substituent a group selected from a methyl group, a methoxycarbonyl group, an ethoxycarbonyl group, a *t*-butoxycarbonyl, an acetyl group, a phenyl group, a fluorophenyl group, a benzoyl group, a furan-3-carbonyl group, an isoxazol-4-carbonyl group, a methylsulfonyl group, and a tosyl group);
   the aforementioned furyl group may comprise a substituent selected from a nitro group, a hydroxymethyl group, a hydroxysulfonyl group, a phenyl group (said phenyl group may comprise as a substituent a group selected from a fluorine atom, a chlorine atom, a methoxy group, a methoxycarbonyl group, an ethoxycarbonyl group, and a methylcarbonyl group), an *N*-methylpyrazolyl group, and an *N*-benzylpyrazolyl group;
   alternatively, the aforementioned R⁴ represents formula (III);

   -Ar¹-O-R⁶ (III)

   (wherein,
   Ar¹ represents a phenyl group, a methoxyphenyl group, or an ethoxyphenyl group;
   R⁶ represents a hydrogen atom, a methyl group, a cyclopropylmethyl group, a cyclohexylmethyl group, an ethoxycarbonylmethyl group, a *t*-butoxycarbonylmethyl group, an ethoxycarbonylfurylmethyl group, -CH₂CH₂CH₂N(CH₃)₂, a benzotriazolmethyl group, a *p*-(*t*-butylphenyl)sulfonyl group, a *p*-toluenesulfonyl group, -CH₂CO₂H, R^{g}CO-CH₂- (wherein, R^{g} represents NH₂, -N(CH₃)₂, an *n*-propylamino group, a phenylamino group, a piperidyl group, or a morpholinyl group), a phenyl group, a benzyl group, an isoxazolylmethyl group, an oxazolyl group, a triazolyl group, or a furyl group, said phenyl group, benzyl group, isoxazolylmethyl group, oxazolyl group, triazolyl group, and furyl group may comprise 1 to 3 substituents selected from a methyl group, an ethyl group, a nitro group, -CO₂Me, and -CO₂Et); or
   alternatively, the aforementioned R⁴ represents formula (IV) 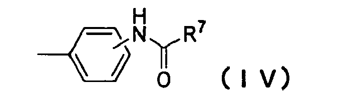 (wherein, R⁷ represents -CH=C(CH₃)₂, -CH=CHPh, a furyl group, a naphthyl group, or a phenyl group;
   said phenyl group may comprise 1 to 3 substituents selected from a halogen atom, a hydroxy group, and a C₁₋₃ alkoxy group;
[9] a pharmaceutical composition comprising as an active ingredient, the compound of any one of [1] to [8], or a pharmaceutically acceptable prodrug or salt thereof, or a hydrate or solvate thereof;
[10] a pharmaceutical composition comprising, the compound of any one of [1] to [8], or a pharmaceutically acceptable prodrug or salt thereof, or a hydrate or solvate thereof, and a pharmaceutically acceptable excipient;
[11] a protein modification inhibitor for inhibiting ubiquitination, comprising as an active ingredient, the compound of any one of [1] to [8], or a pharmaceutically acceptable prodrug or salt thereof, or a hydrate or solvate thereof;
[12] a preventive and/or therapeutic agent for an inflammatory disease, comprising the protein modification inhibitor of [11] for inhibiting ubiquitination;
[13] a therapeutic agent for malignant tumor, comprising the protein modification inhibitor of [11] for inhibiting ubiquitination;
[14] a therapeutic agent for chronic rheumatoid arthritis, juvenile rheumatoid arthritis, osteoarthrosis, osteoarthritis, gout, ulcerative colitis, Crohn's disease, peptic ulcer, gastritis, systemic lupus erythematosus, scleroderma, polymyositis, dermatomyosis, vasculitis syndrome, Bechet's disease, connective tissue disease, nephrotic syndrome, polymyositis, dermatomyosis, Sjoegren's syndrome, insulin-dependent diabetes mellitus, multiple sclerosis, bronchial asthma, bronchitis, emphysema, allergic rhinitis, atopic dermatitis, urticaria, glomerulonephritis, pulmonary fibrosis, solid tumor cancer, angina, myocardial infarction, or Alzheimer's disease, comprising the protein modification inhibitor of [11] for inhibiting ubiquitination;
[15] a preventive and/or therapeutic agent for an inflammatory disease, comprising as an active ingredient, the compound of any one of [1] to [8], or a pharmaceutically acceptable prodrug or salt thereof, or a hydrate or solvate thereof;
[16] a therapeutic agent for malignant tumor, comprising as an active ingredient, the compound of any one of [1] to [8], or a pharmaceutically acceptable prodrug or salt thereof, or a hydrate or solvate thereof;
[17] a therapeutic agent for chronic rheumatoid arthritis, juvenile rheumatoid arthritis, osteoarthrosis, osteoarthritis, gout, ulcerative colitis, Crohn's disease, peptic ulcer, gastritis, systemic lupus erythematosus, scleroderma, polymyositis, dermatomyosis, vasculitis syndrome, Bechet's disease, connective tissue disease, nephrotic syndrome, polymyositis, dermatomyosis, Sjoegren's syndrome, insulin-dependent diabetes mellitus, multiple sclerosis, bronchial asthma, bronchitis, emphysema, allergic rhinitis, atopic dermatitis, urticaria, glomerulonephritis, pulmonary fibrosis, solid tumor cancer, angina, myocardial infarction, or Alzheimer's disease, comprising as an active ingredient, the compound of any one of [1] to [8], or a pharmaceutically acceptable prodrug or salt thereof, or a hydrate or solvate thereof;
[18] a method for treating a disease associated with abnormal ubiquitination, wherein a pharmaceutically effective dose of the compound of any one of [1] to [8], or a pharmaceutically acceptable prodrug or salt thereof, or a hydrate or solvate thereof is administered to a patient to inhibit ubiquitination;
[19] a method for treating an inflammatory disease, comprising administering to a patient a pharmaceutically effective dose of the compound of any one of [1] to [8], or a pharmaceutically acceptable prodrug or salt thereof, or a hydrate or solvate thereof;
[20] a method for treating malignant tumor, comprising administering to a patient a pharmaceutically effective dose of the compound of any one of [1] to [8], or a pharmaceutically acceptable prodrug or salt thereof, or a hydrate or solvate thereof;
[21] a method for treating a disease selected from the group consisting of chronic rheumatoid arthritis, juvenile rheumatoid arthritis, osteoarthrosis, osteoarthritis, gout, ulcerative colitis, Crohn's disease, peptic ulcer, gastritis, systemic lupus erythematosus, scleroderma, polymyositis, dermatomyosis, vasculitis syndrome, Bechet's disease, connective tissue disease, nephrotic syndrome, Sjoegren's syndrome, insulin-dependent diabetes mellitus, multiple sclerosis, bronchial asthma, bronchitis, emphysema, allergic rhinitis, atopic dermatitis, urticaria, glomerulonephritis, pulmonary fibrosis, angina, myocardial infarction, and Alzheimer's disease, comprising administering a pharmaceutically effective dose of the compound of any one of [1] to [8], or a pharmaceutically acceptable prodrug or salt thereof, or a hydrate or solvate thereof;
[22] use of the compound of any one of [1] to [8], or a pharmaceutically acceptable prodrug or salt thereof, or a hydrate or solvate thereof for producing a therapeutic agent for a disease associated with abnormal ubiquitination;
[23] use of the compound of any one of [1] to [8], or a pharmaceutically acceptable prodrug or salt thereof, or a hydrate or solvate thereof for producing a therapeutic agent for an inflammatory disease;
[24] use of the compound of any one of [1] to [8], or a pharmaceutically acceptable prodrug or salt thereof, or a hydrate or solvate thereof for producing a therapeutic agent for malignant tumor; and
[25] use of the compound of any one of [1] to [8], or a pharmaceutically acceptable prodrug or salt thereof, or a hydrate or solvate thereof for producing a therapeutic agent for a disease selected from the group consisting of chronic rheumatoid arthritis, juvenile rheumatoid arthritis, osteoarthrosis, osteoarthritis, gout, ulcerative colitis, Crohn's disease, peptic ulcer, gastritis, systemic lupus erythematosus, scleroderma, polymyositis, dermatomyosis, vasculitis syndrome, Bechet's disease, connective tissue disease, nephrotic syndrome, Sjoegren's syndrome, insulin-dependent diabetes mellitus, multiple sclerosis, bronchial asthma, bronchitis, emphysema, allergic rhinitis, atopic dermatitis, urticaria, glomerulonephritis, pulmonary fibrosis, angina, myocardial infarction, and Alzheimer's disease.

The present invention will be described in detail below by explaining the meaning of the terms, symbols, and such mentioned in the present description.

The "C₁₋₆ alkyl group" in the present description refers to a straight chain or branched chain alkyl group of 1 to 6 carbons, which is a monovalent group derived by removing any one hydrogen atom from an aliphatic hydrocarbon of 1 to 6 carbons. "C₁₋₃ alkyl group" means those among the aforementioned C₁₋₆ alkyl group having 1 to 3 carbons. Specific examples include a methyl group, an ethyl group, a 1-propyl group, a 2-propyl group, a 2-methyl-1-propyl group, a 2-methyl-2-propyl group, a 1-butyl group, a 2-butyl group, a 1-pentyl group, a 2-pentyl group, a 3-pentyl group, a 2-methyl-1-butyl group, a 3-methyl-1-butyl group, a 2-methyl-2-butyl group, a 3-methyl-2-butyl group, a 2,2-dimethyl-1-propyl group, a 1-hexyl group, a 2-hexyl group, a 3-hexyl group, a 2-methyl-1-pentyl group, a 3-methyl-1-pentyl group, a 4-methyl-1-pentyl group, a 2-methyl-2-pentyl group, a 3-methyl-2-pentyl group, a 4-methyl-2-pentyl group, a 2-methyl-3-pentyl group, a 3-methyl-3-pentyl group, a 2,3 -dimethyl-1-butyl group, a 3,3-dimethyl-1-butyl group, a 2,2-dimethyl-1-butyl group, a 2-ethyl-1-butyl group, a 3,3-dimethyl-2-butyl group, and a 2,3-dimethyl-2-butyl group.

Herein, for example, when the expression "C₁₋₆ alkyl" is changed to the expression "C₁₋₃ alkyl", this means that it is an alkyl that has 1 to 3 carbons but is otherwise the same.

Herein, "C₂₋₆ alkenyl group" refers to a straight chain or branched chain alkenyl group of 2 to 6 carbons, and specific examples include a vinyl group, an allyl group, a 1-propenyl group, a 2-propenyl group, a 2-methyl-1-propenyl group, a 1-butenyl group, a 2-butenyl group, a 3-butenyl group, a pentenyl group, and a hexenyl group.

Herein, "C₃₋₈ cycloalkyl group" refers to a cyclic aliphatic hydrocarbon group of 3 to 8 carbons, and specific examples include a cyclopropyl group, a cyclobutyl group, a cyclopentyl group, a cyclohexyl group, a cycloheptyl group, and a cyclooctyl group.

Herein, "C₁₋₆ alkylene group" refers to a divalent group derived by further removing any one hydrogen atom from the "C₁₋₆ alkyl group" defined above, and specific examples include a methylene group, a 1,2-ethylene group, a 1,1-ethylene group, a 1,3-propylene group, a tetramethylene group, a pentamethylene group, and a hexamethylene group.

Herein, "C₁₋₆ alkylenedioxy group" refers to a divalent group represented by -O-R-O-(R represents an alkylene group of 1 to 6 carbon atoms, preferably 1 to 4, and more preferably 1 to 2). Examples of a C₁₋₆ alkylenedioxy group include methylenedioxy (-O-CH₂-O-), ethylenedioxy (-O-CH₂CH₂-O-), trimethylenedioxy, tetramethylenedioxy, -O-CH(CH₃)-O-, and -O-C(CH₃)₂-O-, and among them, a methylenedioxy group and an ethylenedioxy group are preferred.

A group in which a methylenedioxy group is bound to a phenyl group is a 1,3-benzodioxol group, and a group in which an ethylenedioxy group is bound to a phenyl group is a 1,4-benzodioxane group.

Herein, "C₁₋₆ alkoxy group" refers to an oxy group bound to the "C₁₋₆ alkyl group" defined above. Specific examples include a methoxy group, an ethoxy group, a 1-propyloxy group, a 2-propyloxy group, a 2-methyl-1-propyloxy group, a 2-methyl-2-propyloxy group, a 1-butyloxy group, a 2-butyloxy group, a 1-pentyloxy group, a 2-pentyloxy group, a 3-pentyloxy group, a 2-methyl-1-butyloxy group, a 3-methyl-1-butyloxy group, a 2-methyl-2-butyloxy group, a 3-methyl-2-butyloxy group, a 2,2-dimethyl-1-propyloxy group, a 1-hexyloxy group, a 2-hexyloxy group, a 3-hexyloxy group, a 2-methyl-1-pentyloxy group, a 3-methyl-1-pentyloxy group, a 4-methyl-1-pentyloxy group, a 2-methyl-2-pentyloxy group, a 3-methyl-2-pentyloxy group, a 4-methyl-2-pentyloxy group, a 2-methyl-3-pentyloxy group, a 3-methyl-3-pentyloxy group, a 2,3-dimethyl-1-butyloxy group, a 3,3-dimethyl-1-butyloxy group, a 2,2-dimethyl-1-butyloxy group, a 2-ethyl-1-butyloxy group, a 3,3-dimethyl-2-butyloxy group, and a 2,3-dimethyl-2-butyloxy group.

Herein, "C₆₋₁₀ aryl group" refers to an aromatic cyclic hydrocarbon group of 6 to 10 carbons and specific examples include a phenyl group, a 1-naphthyl group, and a 2-naphthyl group.

Herein, "C₆₋₁₀ aryl C₁₋₆ alkyl group" refers to a group in which any hydrogen atom in the "C₁₋₆ alkyl group" defined above is substituted with the "C₆₋₁₀ aryl group" defined above. Specific examples include phenylalkyl groups and naphthylalkyl groups such as a benzyl group, a 1-phenethyl group, a 2-phenethyl group, an α-naphthylmethyl group, and a β-naphthylmethyl group.

Herein, "halogen atom" refers to a fluorine atom, a chlorine atom, a bromine atom, and an iodine atom.

Herein, "heteroatom" refers to a sulfur atom, an oxygen atom, or a nitrogen atom.

Herein, "5- to 10-membered heteroaryl ring" refers to an aromatic ring in which the number of atoms constituting the ring is 5 to 10, and the atoms constituting the ring include one or more heteroatoms. Specific examples include a pyridine ring, a thiophene ring, a furan ring, a pyrrole ring, an oxazole ring, an isoxazole ring, a thiazole ring, a thiadiazole ring, an isothiazole ring, an imidazole ring, a triazole ring, a tetrazole ring, a pyrazole ring, a furazan ring, a thiadiazole ring, an oxadiazole ring, a pyridazine ring, a pyrimidine ring, a pyrazine ring, a triazine ring, an indole ring, an isoindole ring, an indazole ring, a chromene ring, a quinoline ring, an isoquinoline ring, a cinnoline ring, a quinazoline ring, a quinoxaline ring, a naphthyridine ring, a phthalazine ring, a purine ring, a pteridine ring, a thienofuran ring, an imidazothiazole ring, a benzofuran ring, a benzothiophene ring, a benzotriazole ring, a benzoxazole ring, a benzthiazole ring, a benzthiadiazole ring, a benzimidazole ring, an imidazopyridine ring, a pyrrolopyridine ring, a pyrrolopyrimidine ring, and a pyridopyrimidine ring.

Preferred examples of the "5- to 10-membered heteroaryl ring" include a pyridine ring, a thiophene ring, a furan ring, a pyrrole ring, an oxazole ring, an isoxazole ring, a thiazole ring, an imidazole ring, a triazole ring, a tetrazole ring, a pyrazole ring, an indole ring, an isoindole ring, a quinoline ring, a quinoxaline ring, a benzofuran ring, and a benzotriazole ring.

Herein, the "5- to 10-membered heteroaryl group" refers to a monovalent or divalent group derived by removing 1 or 2 hydrogen atoms from any position of the "5- to 10-membered heteroaryl ring" described above.

Specifically, preferred examples include a pyridyl group, a thienyl group, a furyl group, a pyrrolyl group, an oxazolyl group, an isoxazolyl group, a thiazolyl group, an imidazolyl group, a triazolyl group, a tetrazolyl group, a pyrazolyl group, a pyrazolinyl group, an indolyl group, an isoindolyl group, a quinolyl group, a quinoxalinyl group, a benzofuryl group, and a benzotriazolyl group.

Herein, "5- to 10-membered heteroarylcarbonyl group" refers to a carbonyl group bound to the "5- to 10-membered heteroaryl group" defined above.

Herein, "5- to 8-membered heterocycle" refers to a ring in which
(1) the number of atoms constituting the ring is 5 to 8,
(2) the atoms constituting the ring include 1 to 2 heteroatoms,
(3) the ring may include 1 to 2 double bonds,
(4) the ring may include 1 to 3 carbonyl groups, and
(5) the ring is monocyclic and non-aromatic.

Specific examples of the 5- to 8-membered heterocycle include an azetidine ring, a pyrrolidine ring, a piperidine ring, an azepane ring, an azocane ring, a tetrahydrofuran ring, a tetrahydropyran ring, a tetrahydropyridine ring, a morpholine ring, a thiomorpholine ring, a piperazine ring, a thiazolidine ring, a dioxane ring, an imidazoline ring, and a thiazoline ring.

Preferred examples of the "5- to 8-membered heterocycle" include a pyrrolidine ring, a piperidine ring, a piperazine ring, a morpholine ring, and a tetrahydropyridine ring.

Herein, "5- to 8-membered heterocyclic group" refers to a monovalent or divalent group derived by removing 1 or 2 hydrogen atoms from any position of the "5- to 8-membered heterocycle" described above. The "5- to 8-membered heterocyclic group" preferably refers to a pyrrolidyl group, a piperidyl group, a piperazyl group, a morpholinyl group, a tetrahydropyridyl group, and a tetrahydrofuryl group, and more preferably refers to a pyrrolidin-1-yl group, a piperidin-1-yl group, a piperazin-1-yl group, a morpholin-4-yl group, a tetrahydropyridin-1-yl group, and a tetrahydrofuran-2-yl group.

Herein, "C₃₋₈ cycloalkyl C₁₋₃ alky group" refers to a group in which any hydrogen atom in the "C₁₋₃ alkyl group" defined above is substituted with the "C₃₋₈ cycloalkyl group" defined above. Specific examples include a cyclopropylmethyl group, a cyclobutylmethyl group, a cyclopentylmethyl group, a cyclohexylmethyl group, a cycloheptylmethyl group, a cyclooctylmethyl group, a cyclopropylethyl group, a cyclobutylethyl group, a cyclopentylethyl group, a cyclohexylethyl group, a cycloheptylethyl group, and a cyclooctylethyl group.

Furthermore, "C₃₋₈ cycloalkylmethyl group" refers to a group in which any hydrogen atom in a methyl group is substituted with the "C₃₋₈ cycloalkyl group" defined above.

Herein, "hydroxy C₁₋₆ alkyl group" refers to a group in which any hydrogen atom in the "C₁₋₆ alkyl group" defined above is substituted with a hydroxyl group (-OH).

Herein, "C₁₋₆ alkoxy C₁₋₆ alkyl group" refers to a group in which any hydrogen atom in the "C₁₋₆ alkyl group" defined above is substituted with the "C₁₋₆ alkoxy group" defined above. Specific examples include a methoxymethyl group, an ethoxymethyl group, a 1-propyloxymethyl group, a 2-propyloxymethyl group, a *t*-butoxymethyl group, a methoxyethyl group, an ethoxyethyl group, a 1-propyloxyethyl group, a 2-propyloxyethyl group, and a *t*-butoxyethyl group.

Herein, "halogenated C₁₋₆ alkyl group" refers to a group in which any hydrogen atom in the "C₁₋₆ alkyl group" defined above is substituted with a halogen atom.

Herein, "5- to 10-membered heteroaryl C₁₋₆ alkyl group" refers to a group in which any hydrogen atom in the "C₁₋₆ alkyl group" defined above is substituted with the "5- to 10-membered heteroaryl group" defined above.

Herein, "5- to 8-membered heterocyclic C₁₋₆ alkyl group" refers to a group in which any hydrogen atom in the "C₁₋₆ alkyl group" defined above is substituted with the "5- to 8-membered heterocyclic group" defined above.

Herein, "hydroxy C₆₋₁₀ aryl group" refers to a group in which any hydrogen atom in the "C₆₋₁₀ aryl group" defined above is substituted with a hydroxy group (-OH).

Herein, "halogenated C₆₋₁₀ aryl group" refers to a group in which any hydrogen atom in the "C₆₋₁₀ aryl group" defined above is substituted with a halogen atom.

Herein, "C₁₋₆ alkoxycarbonyl group" refers to a carbonyl group bound to the "C₁₋₆ alkoxy group" defined above, and specific examples include a methoxycarbonyl group, an ethoxycarbonyl group, a 1-propyloxycarbonyl group, a 2-propyloxycarbonyl group, and a *t*-butoxycarbonyl group.

Herein, "C₁₋₆ alkoxycarbonyl C₁₋₆ alkyl group" refers to a group in which any hydrogen atom in the "C₁₋₆ alkyl group" defined above is substituted with the "C₁₋₆ alkoxycarbonyl group" defined above. Furthermore, "C₁₋₆ alkoxycarbonyl C₁₋₃ alkyl group" is an alkyl group in which the number of carbons in the "C₁₋₆ alkyl group" moiety is 1 to 3.

Herein, "C₁₋₆ alkoxyphenyl group" refers to a phenyl group bound to the "C₁₋₆ alkoxy group" defined above, and specific examples include a methoxyphenyl group, an ethoxyphenyl group, and a 1-propyloxyphenyl group.

Herein, "C₁₋₆ alkylcarbonyl group" refers to a carbonyl group bound to the "C₁₋₆ alkyl group" defined above, and specific examples include a methylcarbonyl group, an ethylcarbonyl group, a 1-propylcarbonyl group, and a 2-propylcarbonyl group.

Herein, "C₃₋₈ cycloalkylcarbonyl group" refers to a carbonyl group bound to the "C₃₋₈ cycloalkyl group" defined above, and specific examples include a cyclopropylcarbonyl group, a cyclopentylcarbonyl group, a cyclohexylcarbonyl group, and a cycloheptylcarbonyl group.

Herein, "C₆₋₁₀ arylcarbonyl group" refers to a carbonyl group bound to the "C₆₋₁₀ aryl group" defined above, and specific examples include a phenylcarbonyl group and a naphthylcarbonyl group.

Herein, "C₁₋₆ alkylcarbonyloxy group" refers to an oxy group bound to the "C₁₋₆ alkylcarbonyl group" defined above.

Herein, "C₆₋₁₀ aryl C₁₋₆ alkoxy group" refers to an oxy group bound to the "C₆₋₁₀ aryl C₁₋₆ alkyl group" defined above.

Herein, "C₁₋₆ alkylcarbonyloxy C₁₋₃ alkyl group" refers to a group in which any hydrogen atom in the "C₁₋₆ alkyl group" defined above is substituted with the "C₁₋₆ alkylcarbonyloxy group" defined above.

Herein, "furylcarbonyl group" refers to a carbonyl group bound to a furyl group.

Herein, "*N*-methylpyrrolylcarbonyl group" refers to a carbonyl group bound to an *N*-methylpyrrolyl group.

Herein, "isoxazolylcarbonyl group" refers to a carbonyl group bound to an isoxazolyl group.

Herein, "C₆₋₁₀ aryl C₁₋₃ alkoxycarbonyl group" refers to a carbonyl group bound to the "C₆₋₁₀ aryl C₁₋₆ alkoxy group" defined above.

Herein, "C₆₋₁₀ aryl C₁₋₆ alkoxy group" refers to an oxy group bound to the "C₆₋₁₀ aryl C₁₋₆ alkyl group" defined above.

Herein, "C₆₋₁₀ aryl C₁₋₆ alkoxycarbonyl group" refers to a carbonyl group bound to the "C₆₋₁₀ aryl C₁₋₆ alkoxy group" defined above.

Herein, "mono C₁₋₆ alkylamino group" refers to a group in which one of the hydrogen atoms in an amino group (NH₂-) is substituted with the "C₁₋₆ alkyl group" defined above. Specific examples include a methylamino group, an ethylamino group, a 1-propylamino group, and a 2-propylamino group.

Herein, "di C₁₋₆ alkylamino group" refers to a group in which the 2 hydrogen atoms of an amino group (NH₂-) are each independently substituted with the "C₁₋₆ alkyl group" defined above. Specific examples include a dimethylamino group, a diethylamino group, a di-1-propylamino group, a di-2-propylamino group, and a methylethylamino group.

Herein, "mono(C₃₋₈ cycloalkyl C₁₋₃ alkyl)amino group" refers to a group in which 1 hydrogen atom in an amino group (NH₂-) is substituted with the "C₃₋₈ cycloalkyl C₁₋₃ alkyl group" defined above.

Herein, "hydroxy C₁₋₆ alkylamino group" refers to a group in which any hydrogen atom of the C₁₋₆ alkyl in the "mono C₁₋₆ alkylamino group" defined above is substituted with a hydroxy group (-OH).

Herein, "*N*-(hydroxy C₁₋₆ alkyl)-*N*-C₁₋₆ alkylamino group" refers to a group in which 2 hydrogen atoms of an amino group (NH₂-) are each substituted with the "hydroxy C₁₋₆ alkyl group" and "C₁₋₆ alkyl" defined above.

Herein, "C₁₋₆ alkoxy C₁₋₆ alkylamino group" refers to a group in which 1 hydrogen atom in an amino group (NH₂-) is substituted with the "C₁₋₆ alkoxy C₁₋₆ alkyl group" defined above.

Herein, "di C₁₋₆ alkylamino C₁₋₆ alkyl group" refers to a group in which any hydrogen atom in the "C₁₋₆ alkyl group" defined above is substituted with the "di C₁₋₆ alkylamino group" defined above.

Herein, "C₁₋₆ alkylimidazolyl group" refers to a group in which a hydrogen atom in an imidazolyl group is substituted with the "C₁₋₆ alkyl group" defined above.

Herein, C₆₋₁₀ aryl C₁₋₃ alkylimidazolyl group" refers to a group in which a hydrogen atom in an imidazolyl group is substituted with the "C₆₋₁₀ aryl C₁₋₃ alkyl group" defined above.

Herein, "oxo group" refers to a divalent group represented by "O=" (an oxygen atom having a double bond).

Herein, "prodrug" refers to a compound having a group that can be converted to NH₂, OH, CO₂H, or such of a compound of the present invention by solvolysis or under physiological conditions. Examples of a prodrug-forming group include groups described in Pro. Med., 2157-2161 (1985) and in "Development of Pharmaceuticals (Iyakuhin no Kaihatsu)" (Hirokawa Shoten, 1990) Vol. 7, Molecular Design 163-198. "Salt" in the present invention is not particularly limited so long as it is a pharmaceutically acceptable salt, and it forms a salt with a compound of the present invention, and examples include acid addition salts and base addition salts.

The acid addition salts are not particularly limited, and examples include hydrochloride, acetate, sulfate, nitrate, oxalate, maleate, tartarate, citrate, carbonate, succinate, benzoate, acetate, hydrobromide, hydroiodide, phosphate, fumarate, gluconate, *p*-toluenesulfonate, methanesulfonate, and ethanesulfonate.

The base addition salts are not particularly limited, and examples include alkali metal salts such as sodium salts and potassium salts; alkaline earth metal salts such as calcium salts and magnesium salts; organic amine salts such as ethanol amine salts, triethylamine salts, and methylamine salts; and ammonium salts.

Herein, "Me" refers to a methyl group, "Et" refers to an ethyl group, and "Ph" refers to a phenyl group.

Herein, "ubiquitination" means that multiple ubiquitins covalently bind to a target protein by the action of three types of enzymes, E1 (ubiquitin-activating enzyme), E2 (ubiquitin-binding enzyme), and E3 (ubiquitin ligase). To explain "ubiquitination" in more detail, a small ubiquitin protein composed of 76 amino acids uses its C-terminal glycine residue to bind ATP-dependently to the cysteine residue of E1 (ubiquitin-activating enzyme), and then it is transferred from E1 to E2 (ubiquitin-binding enzyme), and then forms an isopeptide linkage to the ε-amino group of the lysine side chain of the target protein recognized by E3 (ubiquitin ligase). Further, a polyubiquitin chain is formed by repeated isopeptide binding of the ubiquitin C terminus to the lysine residue of ubiquitin bound to the lysine residue of the target protein. This series of protein modification reactions is called "ubiquitination". "Protein modification inhibitor for inhibiting ubiquitination" refers to a pharmaceutical agent comprising a substance that inhibits protein modification by the above-mentioned "ubiquitination".

Herein, "diseases that involve abnormal ubiquitination" refers to diseases caused by abnormal degradation of proteins that are targets of ubiquitination as a result of abnormal protein modification by ubiquitination. Specific examples include immune-related diseases (including inflammation), malignant tumor, and neurodegenerative diseases.

Herein, examples of an "immune-related disease" include chronic rheumatoid arthritis, juvenile rheumatoid arthritis, osteoarthrosis, osteoarthritis, gout, ulcerative colitis, Crohn's disease, peptic gastric ulcer, gastritis, systemic erythematosus, scleroderma, polymyositis, dermatomyosis, vasculitis syndrome, Bechet's disease, connective tissue disease, nephrotic syndrome, Sjoegren's syndrome, insulin-dependent diabetes mellitus, multiple sclerosis, bronchial asthma, bronchitis, emphysema, allergic rhinitis, atopic dermatitis, urticaria, glomerulonephritis, pulmonary fibrosis, angina, and myocardial infarction.

Herein, "malignant tumor" refers to a disease (cancer) in which malignant cells proliferate in a body, and additionally metastasize to the lymph nodes and to other organs and cause death.

Specifically, examples of malignant tumor of the head and neck include glioma, meningioma, glioma, neuroblastoma, maxillary cancer, pharyngeal cancer, laryngeal cancer, tongue cancer, thyroid cancer, gingival cancer, nasal cancer, and nasal cavity cancer.

Examples of malignant tumor of the lungs include small cell lung cancer, nonsmall cell lung cancer (adenocarcinoma, squamous cell carcinoma, and large cell carcinoma), breast cancer, mammary Paget's disease, and mammary sarcoma.

Examples of malignant tumor of the digestive organs include esophageal cancer, gastric cancer, pancreatic/gall bladder cancer, duodenal cancer, colon cancer (adenocarcinoma), and primary liver cancer (hepatocellular carcinoma and cholangiocellular carcinoma).

Examples of malignant tumor of the urinary organs include renal cancer, ureter cancer, bladder cancer, prostate cancer, and Wilms' tumor.

Examples of malignant tumor of the reproductive organs include cervical cancer, uterine adenocarcinoma, uterine sarcoma, and ovarian cancer.

Examples of malignant tumor of the skin include skin squamous cell cancer, skin basal cell cancer, skin Bowen's disease, skin Paget's disease, and malignant melanoma.

Examples of malignant tumor of the blood include acute myeloid leukemia, acute promyelocytic leukemia, acute myelomonocytic leukemia, acute monocytic leukemia, acute lymphoblastic leukemia, acute undifferentiated leukemia, chronic myeloid leukemia, chronic lymphoblastic leukemia, adult T cell leukemia, malignant lymphoma (lymphosarcoma, reticulum cell sarcoma, Hodgkin's disease), and multiple myeloma.

Herein, examples of a "neurodegenerative disease" include Alzheimer's disease, Parkinson's disease, and polyglutamine disease.

Compounds of the present invention, and prodrugs and salts thereof may exist as hydrates or solvates. The hydrates or solvates may be used as an active ingredient of the pharmaceuticals of the present invention.

Furthermore, since the compounds of the present invention have an olefin in the molecule, stereoisomers which are geometric isomers based on such a double bond may exist, and since the compounds may have an asymmetric carbon depending on the type of substituents, stereoisomers which are optical isomers based on such an asymmetric carbon may exist. For an active ingredient of the pharmaceuticals of the present invention, any substance from pure-form stereoisomers, any mixture of stereoisomers, racemates, and such may be used.

Polymorphic crystals may exist, and similarly without limitation, any of the crystal forms may exist singularly or as a mixture of crystal forms. Furthermore, the so-called metabolites which are formed by degradation of the compounds of the present invention *in vivo* are also included in the Claims of the present invention.

Preferred embodiments of the compounds represented by formula (I) in the present invention are, for example, the following cases.

### (1) Preferred embodiments of R¹ are the following.

(i) A compound in which the aforementioned R¹ represents a C₁₋₃ alkyl group, a C₃₋₈ cycloalkyl group, a C₁₋₆ alkoxycarbonyl C₁₋₃ alkyl group, a C₆₋₁₀ aryl C₁₋₃ alkyl group, a C₆₋₁₀ aryl group, a C₁₋₆ alkoxycarbonyl group, or a pyridyl group;
   the aforementioned aryl group and the aryl moiety of the C₆₋₁₀ aryl C₁₋₃ alkyl group may each independently comprise 1 to 3 substituents selected from the group consisting of a halogen atom, a hydroxy group, a cyano group, a nitro group, a carboxyl group, a C₁₋₆ alkyl group, a C₁₋₆ alkoxy group, -SO₂R^{a} (wherein, R^{a} represents a hydroxy group, -NH₂, a piperidyl group, or a hydroxypiperidyl group), and -NR^{1b}R^{2b} (wherein, R^{1b} and R^{2b} each independently represent a hydrogen atom or a C₁₋₆ alkyl group);
   alternatively, the aforementioned R¹ represents a group represented by formula (II) 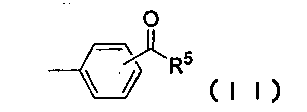 (wherein,
   R⁵ represents -NH₂, a benzodioxoylmethylamino group, a mono C₁₋₆ alkylamino group, a di C₁₋₆ alkylamino group, a mono(C₃₋₈ cycloalkyl C₁₋₃ alkyl) amino group, a hydroxy C₁₋₆ alkylamino group, an *N*-(hydroxy C₁₋₆ alkyl)-*N*-C₁₋₆ alkylamino group, a C₁₋₆ alkoxy C₁₋₆ alkylamino group, a pyrrolidinyl group, a piperidyl group, a piperazinyl group, a tetrahydropyridyl group, or a morpholinyl group;
   said pyrrolidinyl group, piperidyl group, piperazinyl group, and tetrahydropyridyl group may each independently comprise 1 to 3 substituents selected from the group consisting of a hydroxy group, a carboxyl group, a C₁₋₆ alkyl group, a hydroxy C₁₋₆ alkyl group, a C₆₋₁₀ aryl group, and a hydroxy C₆₋₁₀ aryl group).
(ii) A compound in which the aforementioned R¹ represents a C₆₋₁₀ aryl C₁₋₃ alkyl group, a C₆₋₁₀ aryl group, a cyclohexyl group, or a pyridyl group;
   the aforementioned C₆₋₁₀ aryl group and the aryl moiety of the C₆₋₁₀ aryl C₁₋₃ alkyl group may each independently comprise 1 to 3 substituents selected from the group consisting of a halogen atom, a hydroxy group, a cyano group, a nitro group, a carboxyl group, a C₁₋₆ alkyl group, a C₁₋₆ alkoxy group, -SO₂R^{a} (wherein, R^{a} represents a hydroxy group, NH₂, a piperidyl group, or a hydroxypiperidyl group), and -NR^{1b}R^{2b} (wherein, R^{1b} and R^{2b} each independently represent a hydrogen atom or a C₁₋₆ alkyl group);
   alternatively, the aforementioned R¹ represents a group represented by formula (II) 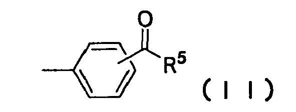 (wherein,
   R⁵ represents -NH₂, a benzodioxoylmethylamino group, a mono C₁₋₆ alkylamino group, a di C₁₋₆ alkylamino group, a mono (C₃₋₈ cycloalkyl C₁₋₃ alkyl) amino group, a hydroxy C₁₋₆ alkylamino group, an *N*-(hydroxy C₁₋₆ alkyl)-*N*-C₁₋₆ alkylamino group, a C₁₋₆ alkoxy C₁₋₆ alkylamino group, a pyrrolidinyl group, a piperidyl group, a piperazinyl group, a tetrahydropyridyl group, or a morpholinyl group;
   said pyrrolidinyl group, piperidyl group, piperazinyl group, and tetrahydropyridyl group may each independently comprise 1 to 3 substituents selected from the group consisting of a hydroxy group, a carboxyl group, a C₁₋₆ alkyl group, a hydroxy C₁₋₆ alkyl group, a C₆₋₁₀ aryl group, and a hydroxy C₆₋₁₀ aryl group).
(iii) A compound in which the aforementioned R¹ represents a naphthyl group, a benzyl group, a hydroxybenzyl group, a phenyl group (the phenyl group may comprise a substituent selected from the group consisting of a halogen atom, a carboxyl group, a *t*-butyl group, a hydroxy group, -NH₂, -SO₃H, and -SO₂NH₂ at position 3 or 4), a pyridyl group, a cyclohexyl group, or a group represented by formula (II) 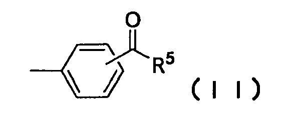 (wherein,
   R⁵ represents -NH₂, a hydroxy group, a cyclopropylmethylamino group, a dimethylamino group, a 2-hydroxypropylamino group, an *N*-methyl-*N*-(hydroxyethyl)amino group, a methoxyethylamino group, a benzodioxolylmethylamino group, a morpholinyl group, a pyrrolidinyl group, a piperidyl group, or a piperazinyl group; and the pyrrolidinyl group, piperidyl group, and piperazinyl group may comprise 1 to 3 substituents selected from a hydroxymethyl group, a hydroxy group, -CO₂H, a phenyl group, and a hydroxyphenyl group).
(iv) A compound in which the aforementioned R¹ represents a naphthyl group, a benzyl group, a hydroxybenzyl group, a phenyl group (the phenyl group may comprise a substituent selected from the group consisting of a carboxyl group, a *t*-butyl group, and -SO₂NH₂ at position 3 or 4), a cyclohexyl group, a pyridyl group, or a group represented by formula (II) 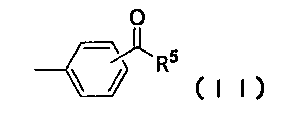 (wherein,
   R⁵ represents a morpholinyl group, a pyrrolidinyl group, a piperidyl group, or a piperazinyl group; and the pyrrolidinyl group, piperidyl group, and piperazinyl group may comprise 1 to 3 substituents selected from a hydroxymethyl group, a hydroxy group, -CO₂H, a phenyl group, and a hydroxyphenyl group).
(v) A compound in which the aforementioned R¹ represents an aryl C₁₋₃ alkyl group or a C₆₋₁₀ aryl group;
   the aforementioned aryl group and the aryl moiety of the C₆₋₁₀ aryl C₁₋₃ alkyl group may each independently comprise 1 to 3 substituents selected from the group consisting of a halogen atom, a hydroxy group, a cyano group, a nitro group, a carboxyl group, a C₁₋₆ alkyl group, a C₁₋₆ alkoxy group, -SO₂R^{a} (wherein, R^{a} represents a hydroxy group, NH₂, a piperidyl group, or a hydroxypiperidyl group), and -NR^{1b}R^{2b} (wherein, R^{1b} and R^{2b} each independently represent a hydrogen atom or a C₁₋₆ alkyl group);
   alternatively, the aforementioned R¹ represents a group represented by formula (II) 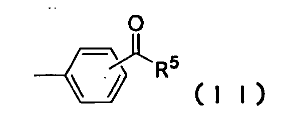 (wherein,
   R⁵ represents NH₂, a benzodioxoylmethylamino group, a mono C₁₋₆ alkylamino group, a di C₁₋₆ alkylamino group, a mono C₃₋₈ cycloalkyl C₁₋₃ alkylamino group (404), a hydroxy C₁₋₆ alkylamino group, an *N*-(hydroxy C₁₋₆ alkyl)-*N*-C₁₋₆ alkylamino group, a C₁₋₆ alkoxy C₁₋₆ alkylamino group, a pyrrolidinyl group, a piperidyl group, a piperazinyl group, a tetrahydropyridyl group, or a morpholinyl group;
   said pyrrolidinyl group, piperidyl group, piperazinyl group, and tetrahydropyridyl group may each independently comprise 1 to 3 substituents selected from the group consisting of a hydroxy group, a carboxyl group, a C₁₋₆ alkyl group, a hydroxy C₁₋₆ alkyl group, a C₆₋₁₀ aryl group, and a hydroxy C₆₋₁₀ aryl group).
(vi) A compound in which the aforementioned R¹ represents a naphthyl group, a benzyl group, a hydroxybenzyl group, a phenyl group (the phenyl group may comprise a substituent selected from the group consisting of a halogen atom, a carboxyl group, a *t*-butyl group, a hydroxy group, -NH₂, -SO₃H, and -SO₂NH₂ at position 3 or 4), or formula (II) 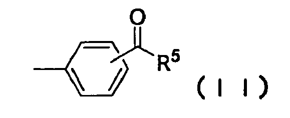 (wherein,
   R⁵ represents NH₂, a hydroxy group, a cyclopropylmethylamino group, a dimethylamino group, a 2-hydroxypropylamino group, a methoxyethylamino group, a benzodioxolylmethylamino group, a morpholinyl group, a pyrrolidinyl group, a piperidyl group, or a piperazinyl group; and the pyrrolidinyl group, piperidyl group, and piperazinyl group may comprise 1 to 3 substituents selected from a hydroxymethyl group, a hydroxy group, -CO₂H, a phenyl group, and a hydroxyphenyl group).
(vii) A compound in which the aforementioned R¹ represents a naphthyl group, a benzyl group, a hydroxybenzyl group, a phenyl group (the phenyl group may comprise a substituent selected from the group consisting of a carboxyl group, a *t*-butyl group, and -SO₂NH₂ at position 3 or 4), or formula (II) 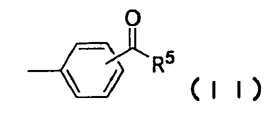 (wherein,
   R⁵ represents a morpholinyl group, a pyrrolidinyl group, a piperidyl group, or a piperazinyl group; and the pyrrolidinyl group, piperidyl group, and piperazinyl group may comprise 1 to 3 substituents selected from a hydroxymethyl group, a hydroxy group, -CO₂H, a phenyl group, and a hydroxyphenyl group).

### (2) Preferred embodiments of R² are the following.

(i) A compound in which R² represents a C₁₋₆ alkyl group, a C₃₋₈ cycloalkyl group, a halogenated C₁₋₆ alkyl group, a C₁₋₆ alkoxy C₁₋₆ alkyl group, R^{e}CO- (wherein, R^{e} represents a heterocyclic group or NR^{1e}R^{2e} (wherein, R^{1e} and R^{2e} each independently represent a hydrogen atom or a C₁₋₆ alkyl group), and the heterocyclic group may comprise a C₁₋₆ alkoxycarbonyl group as a substituent), a furyl group, a pyridyl group, a C₆₋₁₀ aryl group, or a C₆₋₁₀ aryl C₁₋₆ alkyl group (the aforementioned aryl group and the aryl moiety of the C₆₋₁₀ aryl C₁₋₆ alkyl group may comprise 1 to 3 substituents selected from the group consisting of a halogen, a hydroxy group, and a C₁₋₆ alkoxy group).
(ii) A compound in which R² represents a C₁₋₆ alkyl group, a C₃₋₈ cycloalkyl group, a C₁₋₆ alkoxy C₁₋₆ alkyl group, a furyl group, a pyridyl group, a C₆₋₁₀ aryl group, or a C₆₋₁₀ aryl C₁₋₆ alkyl group (the aforementioned C₆₋₁₀ aryl group and the aryl moiety of the C₆₋₁₀ aryl C₁₋₆ alkyl group may comprise 1 to 3 substituents selected from the group consisting of a halogen, a hydroxy group, and a C₁₋₆ alkoxy group).
(iii) A compound in which R² represents a methyl group, an ethyl group, an *n*-propyl group, an isopropyl group, an *n*-butyl group, a cyclopropyl group, a methoxymethyl group, a methoxyethyl group, a furyl group, a pyridyl group (334), a phenyl group, or a benzyl group; and the furyl group, phenyl group, and benzyl group may comprise 1 to 3 substituents selected from a halogen atom, a hydroxy group, and a C₁₋₃ alkoxy group.

### (3) Preferred embodiments of R³ are the following.

(i) A compound in which R³ is a hydrogen atom. R³ and R⁴ may be either an E or Z isomer.

### (4) Preferred embodiments of R⁴ are the following.

(i) A compound in which R⁴ represents a phenyl group, a naphthyl group, an indolyl group, a furyl group, a thienyl group, a thiazolyl group, a pyrrolyl group, a benzofuranyl group, a dimethylpyrrolyl group, a pyridyl group, a quinoxalinyl group, a pyrazolinyl group, or a pyrazolyl group;
   the phenyl group, naphthyl group, indolyl group, furyl group, thienyl group, thiazolyl group, pyrrolyl group, benzofuranyl group, dimethylpyrrolyl group, pyridyl group, quinoxalinyl group, pyrazolinyl group, and pyrazolyl group may each independently comprise 1 to 3 substituents selected from Group D described below;
   Group D: a halogen atom, a nitro group, a C₁₋₃alkoxy group, a carboxyl group, an oxo group, a C₁₋₆ alkyl group, a C₃₋₈ cycloalkyl C₁₋₃ alkyl group, a hydroxy C₁₋₃ alkyl group, a C₆₋₁₀ aryl C₁₋₃ alkyl group, an isoxazolyl C₁₋₃ alkyl group (the aforementioned isoxazolyl ring may comprise a C₁₋₃ alkyl group as a substituent), a C₁₋₃ alkylcarbonyloxy C₁₋₃ alkyl group,
   a C₁₋₆ alkylenedioxy group,
   a phenyl group,
   -SO₃H,
   a C₆₋₁₀ arylcarbonyl group, a furylcarbonyl group, a C₃₋₈ cycloalkylcarbonyl group, an *N*-methylpyrrolylcarbonyl group,
   a morpholinyl group, a pyrrolyl group, a triazolyl group, a thiazolyl group, an imidazolyl group, a pyrazolyl group, a pyrrolidinyl group, a piperidyl group, and a piperazinyl group;
   in the aforementioned Group D, a phenyl group, a morpholinyl group, a pyrrolyl group, a triazolyl group, a thiazolyl group, an imidazolyl group, a pyrazolyl group, a pyrrolidinyl group, a piperidyl group, and a piperazinyl group may each independently comprise 1 to 4 substituents selected from Group E described below;
   Group E: a halogen atom, a C₁₋₃ alkyl group, a hydroxy C₁₋₃ alkyl group, a C₁₋₃ alkoxy group, a C₁₋₆ alkoxycarbonyl C₁₋₆ alkyl group, a hydroxy group, a carboxyl group, a C₁₋₆ alkylcarbonyl group, a C₁₋₆ alkoxycarbonyl group, a C₆₋₁₀ arylcarbonyl group, a C₆₋₁₀ aryl C₁₋₃ alkoxycarbonyl group, a phenyl group, a halophenyl group, a benzyl group, a methanesulfonyl group, a pyrrolidinyl group, a furylcarbonyl group, and an isoxazolylcarbonyl group; alternatively, R⁴ may represent a group represented by formula (III)

   -Ar¹-O-R⁶ (III)

   (wherein,
   Ar¹ represents a phenyl group or a C₁₋₃ alkoxyphenyl group;
   R⁶ represents a hydrogen atom, a C₁₋₆ alkyl group (the alkyl group may comprise an isoxazolyl group, a furyl group, or a benzotriazolyl group as a substituent, and the isoxazolyl group, furyl group, and benzotriazolyl group may each independently comprise 1 to 3 substituents selected from the group consisting of a C₁₋₃ alkyl group and a C₁₋₃ alkoxycarbonyl group), a C₃₋₈ cycloalkylmethyl group, a hydroxycarbonyl C₁₋₃ alkyl group, a C₁₋₆ alkoxycarbonyl C₁₋₃ alkyl group, a tetrahydrofuranylmethyl group, an amino C₁₋₆ alkyl group, a mono C₁₋₃ alkylamino C₁₋₆ alkyl group, a di C₁₋₃ alkylamino C₁₋₆ alkyl group,
   a methanesulfonyl group, a phenylsulfonyl group, a *p*-toluenesulfonyl group, a *p*-(*t*-butyl)phenylsulfonyl group,
   a C₆₋₁₀ aryl group, a C₆₋₁₀ aryl C₁₋₆ alkyl group (the C₆₋₁₀ aryl group and the aryl moiety of the C₆₋₁₀ aryl C₁₋₆ alkyl group may be substituted with 1 to 3 groups selected from a nitro group, a C₁₋₆ alkyl group, a carboxyl group, and a C₁₋₃ alkoxycarbonyl group), or
   R^{g}CO-CH₂- (wherein, R^{g} represents a piperidyl group, a morpholinyl group, or -NR^{1g}R^{2g} (wherein, R^{1g} and R^{2g} each independently represent a hydrogen atom, a C₁₋₆ alkyl group, or a C₆₋₁₀ aryl group)); and
   alternatively, R⁴ may represent a group represented by formula (IV) below, 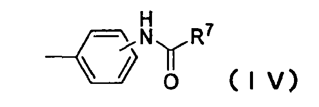 (wherein, R⁷ represents a C₂₋₆ alkenyl group, a phenyl group, a furyl group, a naphthyl group, or a phenylethylene group;
   the aforementioned phenyl group, furyl group, naphthyl group, and phenylethylene group may each independently comprise 1 to 3 substituents selected from the group consisting of a halogen atom, a hydroxy group, a C₁₋₃ alkoxycarbonyl group, and a C₁₋₃ alkoxy group).
(ii) A compound in which R⁴ represents a phenyl group, a naphthyl group, a quinoxalinyl group, a thienyl group, a thiazolyl group, a benzofuranyl group, an indolyl group, or a furyl group;
   the aforementioned phenyl group may be substituted with a group selected from a halogen atom, a nitro group, a carboxyl group, a morpholinyl group, a pyrrolyl group, a pyrazolyl group, a pyrazolinyl group, a triazolyl group, an imidazolyl group, a thiazolyl group, a phenyl group, a C₁₋₆ alkyl group, a C₁₋₆ alkylenedioxy group, a C₁₋₃ alkylimidazolyl group, a C₆₋₁₀ aryl C₁₋₃ alkylimidazolyl group, -SO₂NR^{1f}R^{2f} (wherein, R^{1f} and R^{2f} each independently represent a hydrogen atom or a C₁₋₆ alkyl group), a pyrrolidinyl group (the pyrrolidinyl group may comprise a hydroxy group or a hydroxymethyl group as a substituent), a piperidyl group (the piperidyl group may be substituted with a group selected from a hydroxy group, a pyrrolidinyl group, a carboxyl group, a fluorophenyl group, and a hydroxymethyl group), and a piperazinyl group (the piperazinyl group may be substituted with a group selected from a phenyl group, a fluorophenyl group, a C₁₋₃ alkyl group, a C₁₋₆ alkylcarbonyl group, a furylcarbonyl group, an isoxazolylcarbonyl group, a C₁₋₆ alkoxycarbonyl group, a C₆₋₁₀ arylcarbonyl group, and a methanesulfonyl group);
   the aforementioned indolyl group may be substituted with a group selected from a halogen atom, a benzyl group, a C₁₋₃ alkyl group, a C₃₋₈ cycloalkylmethyl group, a methylisoxazolylmethyl group, a dimethylisoxazolylmethyl group, a methylthiazolylmethyl group, a C₃₋₈ cycloalkylcarbonyl group, a furylcarbonyl group, a methylpyrrolylcarbonyl group, and a C₆₋₁₀ arylcarbonyl group (the aryl moiety may be substituted with a C₁₋₆ alkyl group);
   the aforementioned furyl group may be substituted with a group selected from a hydroxymethyl group, a nitro group, a hydroxysulfonyl group, an *N*-methylpyrazolyl group, an *N*-benzylpyrazolyl group, and a phenyl group (the phenyl group may comprise a substituent selected from a halogen atom, a C₁₋₃ alkoxy group, a C₁₋₃ alkoxycarbonyl group, and a C₁₋₃ alkylcarbonyl group);
   alternatively, the aforementioned R⁴ represents a group represented by formula (III)

   -Ar¹-O-R⁶ (III)

   (wherein,
   Ar¹ represents a phenyl group, a methoxyphenyl group, or an ethoxyphenyl group;
   R⁶ represents a hydrogen atom, a methyl group, a cyclopropylmethyl group, a cyclohexylmethyl group, an ethoxycarbonylmethyl group, a *t*-butoxycarbonylmethyl group, an ethoxycarbonylfurylmethyl group (154), -CH₂CH₂CH₂N(CH₃)₂, a benzotriazolmethyl group, a *p*-(*t*-butylphenyl)sulfonyl group, a *p*-toluenesulfonyl group, -CH₂CO₂H, R^{g}CO-CH₂- (wherein, R^{g} represents -NH₂, -N(CH₃)₂, an *n*-propylamino group, a phenylamino group, a piperidyl group, or a morpholinyl group), a phenyl group, a benzyl group, an isoxazolylmethyl group, an oxazolyl group, a triazolyl group, or a furyl group, and the phenyl group, benzyl group, isoxazolylmethyl group, oxazolyl group, triazolyl group, and furyl group may comprise 1 to 3 substituents selected from a methyl group, an ethyl group, a nitro group, -CO₂Me, and -CO₂Et); or
   alternatively, the aforementioned R⁴ represents a group represented by formula (IV) 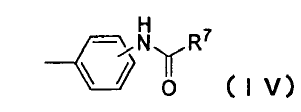 (wherein, R⁷ represents -CH=C(CH₃)₂, -CH=CHPh, a furyl group, a naphthyl group, or a phenyl group;
   the phenyl group may comprise 1 to 3 substituents selected from a halogen atom, a hydroxy group, and a C₁₋₃ alkoxy group.
(iii) A compound in which R⁴ represents a naphthyl group, a quinoxalinyl group, a thienyl group, a thiazolyl group, a benzofuranyl group,
   a 4-chloroindol-3-yl group, a 1-benzylindol-5-yl group, a 1-(3,5-dimethylisoxazol-4-ylmethyl)indol-5-yl group, a 1-benzylindol-5-yl group, a 1-methylindol-5-yl group, a 1-methylindol-2-yl group, a 1-cyclopropylmethylindol-5-yl group, a 1-cyclohexylmethylindol-5-yl group, a 1-cyclopropylcarbonylindol-5-yl group, a 1-benzoylindol-5-yl group, a 1-(furan-2-carbonyl)indol-5-yl group, a 1-(1-methylpyrrol-2-carbonyl)indol-5-yl group, a 5-chloroindol-3-yl group, an indol-4-yl group, an indol-5-yl group, an indol-6-yl group, an indol-7-yl group, a 1-methylindol-2-yl group, a 1-(5-methylisoxazol-3-ylmethyl)indol-5-yl group, a 1-(2-methylthiazol-4-ylmethyl)indol-5-yl group,
   a phenyl group, or a furyl group;
   the aforementioned phenyl group may be substituted with a group selected from a fluorine atom, a nitro group, a carboxyl group, a morpholinyl group, a pyrrolyl group, a pyrazolyl group, a pyrazolinyl group, a triazolyl group, a thiazolyl group, a phenyl group, a *t*-butyl group, a C₁₋₆ alkylenedioxy group, an imidazolyl group, a C₁₋₃ alkylimidazolyl group, a C₆₋₁₀ aryl C₁₋₃ alkylimidazolyl group, -SO₂NHCH₃, -SO₂N(CH₃)₂, -SO₂NHPh, or SO₂NPh₂,
   a pyrrolidinyl group (the pyrrolidinyl group may comprise a hydroxy group or a hydroxymethyl group as a substituent),
   a piperidyl group (the piperidyl group may comprise as a substituent a group selected from a hydroxy group, a hydroxymethyl group, a pyrrolidinyl group, a carboxyl group, and a fluorophenyl group), and
   a piperazinyl group (the piperazinyl group may comprise as a substituent a group selected from a methyl group, a methoxycarbonyl group, an ethoxycarbonyl group, a *t*-butoxycarbonyl, an acetyl group, a phenyl group, a fluorophenyl group, a benzoyl group, a furan-3-carbonyl group, an isoxazol-4-carbonyl group, a methylsulfonyl group, and a tosyl group);
   the aforementioned furyl group may comprise a substituent selected from a nitro group, a hydroxymethyl group, a hydroxysulfonyl group, a phenyl group (the phenyl group may comprise as a substituent a group selected from a fluorine atom, a chlorine atom, a methoxy group, a methoxycarbonyl group, an ethoxycarbonyl group, and a methylcarbonyl group), an *N*-methylpyrazolyl group, and an *N*-benzylpyrazolyl group;
   alternatively, the aforementioned R⁴ represents formula (III);

   -Ar¹-O-R⁶ (III)

   (wherein,
   Ar¹ represents a phenyl group, a methoxyphenyl group, or an ethoxyphenyl group;
   R⁶ represents a hydrogen atom, a methyl group, a cyclopropylmethyl group, a cyclohexylmethyl group, an ethoxycarbonylmethyl group, a *t*-butoxycarbonylmethyl group, an ethoxycarbonylfurylmethyl group, -CH₂CH₂CH₂N(CH₃)₂, a benzotriazolmethyl group, a *p*-(*t*-butylphenyl)sulfonyl group, a *p*-toluenesulfonyl group, -CH₂CO₂H, R^{g}CO-CH₂- (wherein, R^{g} represents -NH₂, -N(CH₃)₂, an *n*-propylamino group, a phenylamino group, a piperidyl group, or a morpholinyl group), a phenyl group, a benzyl group, an isoxazolylmethyl group, an oxazolyl group, a triazolyl group, or a furyl group. Herein, the phenyl group, benzyl group, isoxazolylmethyl group, oxazolyl group, triazolyl group, and furyl group may comprise 1 to 3 substituents selected from a methyl group, an ethyl group, a nitro group, -CO₂Me, and -CO₂Et); or
   alternatively, the aforementioned R⁴ represents formula (IV) 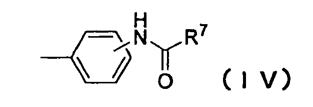 (wherein, R⁷ represents -CH=C(CH₃)₂, -CH=CHPh, a furyl group, a naphthyl group, or a phenyl group;
   said phenyl group may comprise 1 to 3 substituents selected from a halogen atom, a hydroxy group, and a C₁₋₃ alkoxy group.
(iv) A compound in which R⁴ represents a phenyl group, a naphthyl group, an indolyl group, a furyl group, a benzofuranyl group, or a quinoxalinyl group;
   the phenyl group, naphthyl group, indolyl group, furyl group, benzofuranyl group, or quinoxalinyl group may each independently comprise 1 to 3 substituents selected from Group D described below;
   Group D: a halogen atom, a nitro group, a C₁₋₃ alkoxy group, a C₁₋₆ alkyl group, a C₃₋₈ cycloalkyl C₁₋₃ alkyl group, a hydroxy C₁₋₃ alkyl group, a C₆₋₁₀ aryl C₁₋₃ alkyl group, an isoxazolyl C₁₋₃ alkyl group (the aforementioned isoxazolyl ring may comprise a C₁₋₃ alkyl group as a substituent), a C₁₋₆ alkylenedioxy group, a C₆₋₁₀ arylcarbonyl group, a furylcarbonyl group, a C₃₋₈ cycloalkylcarbonyl group, an *N*-methylpyrrolylcarbonyl group,
   a phenyl group, a morpholinyl group, a pyrrolyl group, a triazolyl group, a thiazolyl group, an imidazolyl group, a pyrazolyl group, a pyrrolidinyl group, a piperidyl group, and a piperazinyl group;
   in the aforementioned Group D, a phenyl group, a morpholinyl group, a pyrrolyl group, a triazolyl group, a thiazolyl group, an imidazolyl group, a pyrazolyl group, a pyrrolidinyl group, a piperidyl group, and a piperazinyl group may each independently comprise 1 to 4 substituents selected from Group E described below:
   Group E: a halogen atom, a C₁₋₃ alkyl group, a hydroxy C₁₋₃ alkyl group, a C₁₋₃ alkoxy group, a carboxyl group, a C₁₋₆ alkylcarbonyl group, a C₁₋₆ alkoxycarbonyl group, a C₆₋₁₀ arylcarbonyl group, a phenyl group, a halophenyl group, a benzyl group, a methanesulfonyl group, a furylcarbonyl group, and an isoxazolylcarbonyl group;
   alternatively, R⁴ may represent a group represented by formula (III);

   -Ar¹-O-R⁶ (III)

   (wherein,
   Ar¹ represents a phenyl group or a C₁₋₃ alkoxyphenyl group;
   R⁶ represents a hydrogen atom, a C₁₋₆ alkyl group (the alkyl group may comprise as a substituent an isoxazolyl group or a furyl group, in which the isoxazolyl group and furyl group may each independently comprise 1 to 3 substituents selected from the group consisting of a C₁₋₃ alkyl group and a C₁₋₃ alkoxycarbonyl group), a C₃₋₈ cycloalkylmethyl group, a C₁₋₆ alkoxycarbonyl C₁₋₃ alkyl group, a di C₁₋₃ alkylamino C₁₋₆ alkyl group, a *p*-toluenesulfonyl group, a C₆₋₁₀ aryl group, a C₆₋₁₀ aryl C₁₋₆ alkyl group (the C₆₋₁₀ aryl group and the aryl moiety of the C₆₋₁₀ aryl C₁₋₆ alkyl group may be substituted with 1 to 3 groups selected from a nitro group and a C₁₋₃ alkoxycarbonyl group); or
   R^{g}CO-CH₂- (wherein, R^{g} represents a piperidyl group, a morpholinyl group, or -NR^{1g}R^{2g} (wherein, R^{1g} and R^{2g} each independently represent a hydrogen atom, a C₁₋₆ alkyl group, or a C₆₋₁₀ aryl group)); and
   alternatively, R⁴ may represent a group represented by formula (IV); 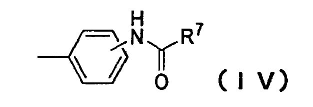 (wherein, R⁷ represents a C₂₋₆ alkenyl group, a phenyl group, -CH=CHPh, a furyl group, or a naphthyl group;
   the aforementioned phenyl group, furyl group, and naphthyl group may each independently comprise 1 to 3 substituents selected from the group consisting of a halogen atom and a C₁₋₃ alkoxy group)).
(v) A compound in which R⁴ represents a naphthyl group, a quinoxalinyl group, a benzofuranyl group,
   a 1-benzylindol-5-yl group, a 1-(3,5-dimethylisoxazol-4-ylmethyl)indol-5-yl group, a 1-benzylindol-5-yl group, a 1-methylindol-5-yl group, a 1-methylindol-2-yl group, a 1-cyclopropylmethylindol-5-yl group, a 1-cyclohexylmethylindol-5-yl group, a 1-cyclopropylcarbonylindol-5-yl group, a 1-benzoylindol-5-yl group, a 1-(furan-2-carbonyl)indol-5-yl group, a 1-(1-methylpyrrol-2-carbonyl)indol-5-yl group, a 5-chloroindol-3-yl group, an indol-4-yl group, an indol-5-yl group, an indol-6-yl group, an indol-7-yl group, a 1-methylindol-2-yl group, a 1-(5-methylisoxazol-3-ylmethyl)indol-5-yl group, a 1-(2-methylthiazol-4-ylmethyl)indol-5-yl group, a phenyl group, or a furyl group;
   the aforementioned phenyl group may be substituted with a group selected from a morpholinyl group, a pyrazolyl group, a pyrazolinyl group, a triazolyl group, a phenyl group, a *t*-butyl group, a C₁₋₆ alkylenedioxy group, an imidazolyl group,
   a pyrrolidinyl group,
   a piperidyl group (the piperidyl group may comprise as a substituent a group selected from a hydroxymethyl group and a carboxyl group), and
   a piperazinyl group (the piperazinyl group may comprise as a substituent a group selected from a methyl group, a methoxycarbonyl group, an acetyl group, a phenyl group, a fluorophenyl group, a benzoyl group, a furan-3-carbonyl group, an isoxazol-4-carbonyl group, a methylsulfonyl group, and a tosyl group);
   the aforementioned furyl group may comprise a substituent selected from a nitro group, a hydroxymethyl group, a phenyl group (the phenyl group may comprise as a substituent a group selected from a fluorine atom, a chlorine atom, a methoxy group, a methoxycarbonyl group, an ethoxycarbonyl group, and a methylcarbonyl group), an *N*-methylpyrazolyl group, and an *N*-benzylpyrazolyl group;
   alternatively, the aforementioned R⁴ represents formula (III);

   -Ar¹-O-R⁶ (III)

   (wherein,
   Ar¹ represents a phenyl group, a methoxyphenyl group, or an ethoxyphenyl group;
   R⁶ represents a hydrogen atom, a methyl group, a cyclopropylmethyl group, an ethoxycarbonylmethyl group, a *t*-butoxycarbonylmethyl group, an ethoxycarbonylfurylmethyl group, -CH₂CH₂CH₂N(CH₃)₂, a *p*-toluenesulfonyl group, R^{g}CO-CH₂- (wherein, R^{g} represents -NH₂, -N(CH₃)₂, a phenylamino group, a piperidyl group, or a morpholinyl group), a phenyl group, a benzyl group, or an isoxazolylmethyl group. Herein, the phenyl group, benzyl group, and isoxazolylmethyl group may comprise 1 to 3 substituents selected from a methyl group, an ethyl group, a nitro group, and -CO₂Me); or
   alternatively, the aforementioned R⁴ represents by formula (IV) 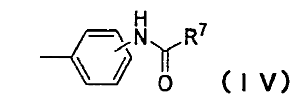 (wherein, R⁷ represents -CH=CHPh, a furyl group, a naphthyl group, or a phenyl group;
   said phenyl group may comprise 1 to 3 substituents selected from a halogen atom and a C₁₋₃ alkoxy group.

Each preferred embodiment of the above-mentioned R¹, R², R³, and R⁴, can be arbitrarily combined.

Hereinbelow, specific examples of the compounds represented by formula (I) will be presented in the following Table, but the present invention is not limited thereto.

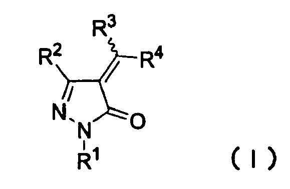

**Table 1**

| COMPOUND NO. | R¹ | R² | R³ | R⁴ |
|---|---|---|---|---|
| 1 | 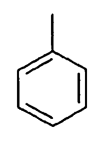 | 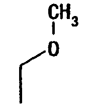 | -H | 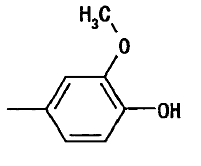 |
| 2 | 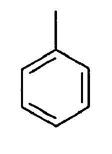 | 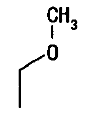 | -H | 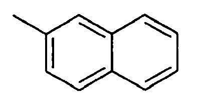 |
| 3 | 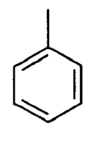 | 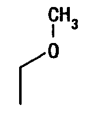 | -H | 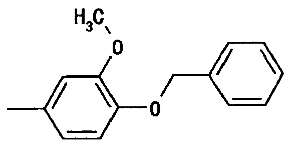 |
| 4 | 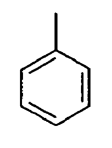 | 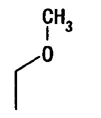 | -H | 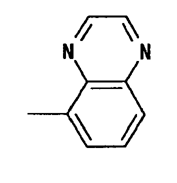 |
| 5 | 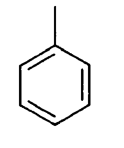 | 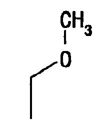 | -H | 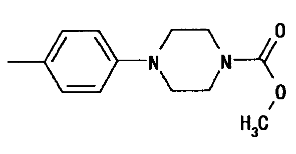 |
| 6 | 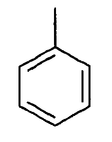 | 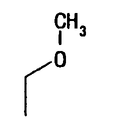 | -H | 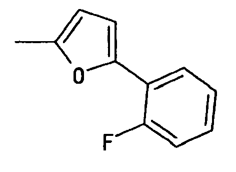 |
| 7 | 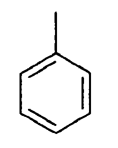 | 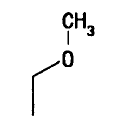 | -H | 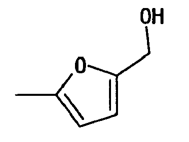 |
| 8 | 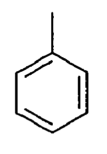 | 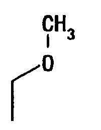 | -H | 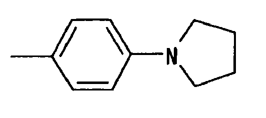 |
| 9 | 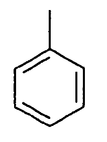 | 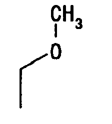 | -H | 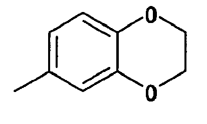 |
| 10 | 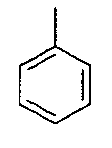 | 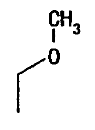 | -H | 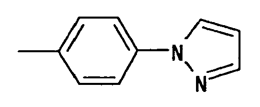 |
| 11 | 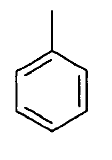 | 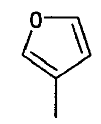 | -H | 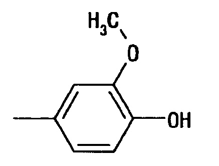 |
| 12 | 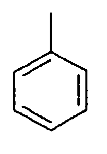 | 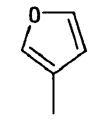 | -H | 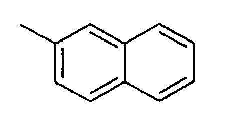 |
| 13 | 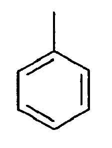 | 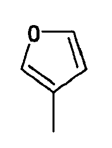 | -H | 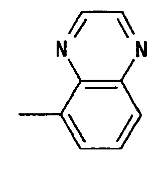 |
| 14 | 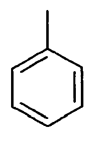 | 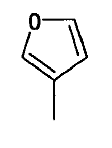 | -H | 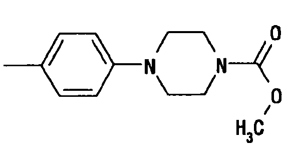 |
| 15 | 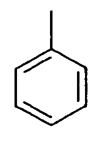 | 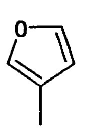 | -H | 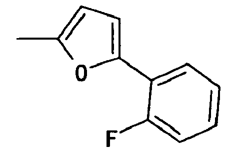 |
| 16 | 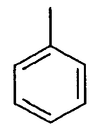 | 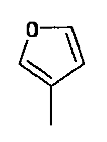 | -H | 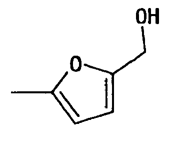 |
| 17 | 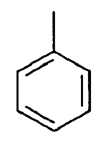 | 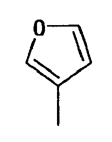 | -H | 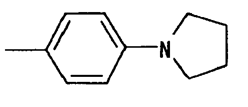 |
| 18 | 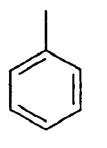 | -CH₃ | -H | 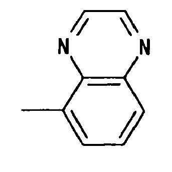 |
| 19 | 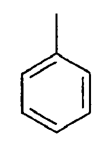 | -CH₃ | -H | 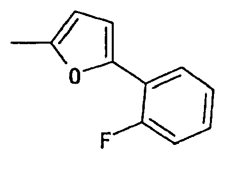 |
| 20 | 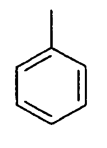 | -CH₃ | -H | 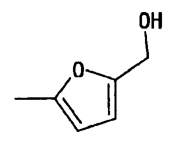 |
| 21 | 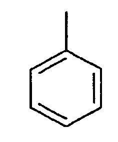 | -CH₃ | -H | 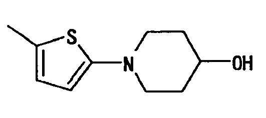 |
| 22 | 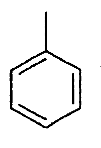 | 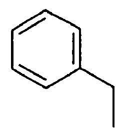 | -H | 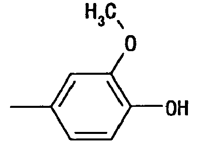 |
| 23 | 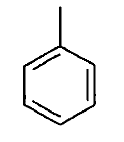 | 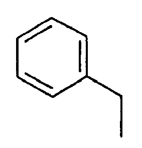 | -H | 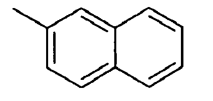 |
| 24 | 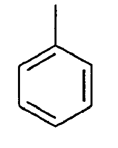 | 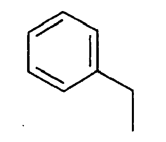 | -H | 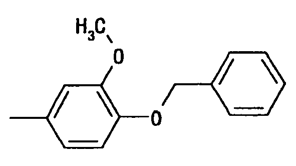 |
| 25 | 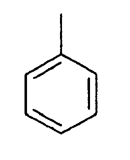 | 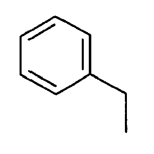 | -H | 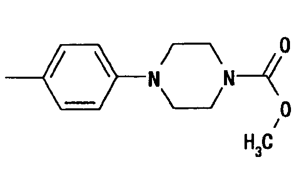 |
| 26 | 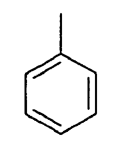 | 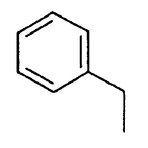 | -H | 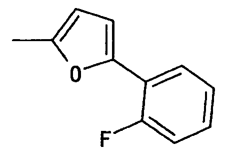 |
| 27 | 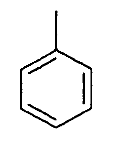 | 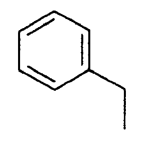 | -H | 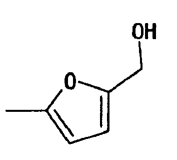 |
| 28 | 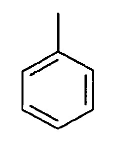 | 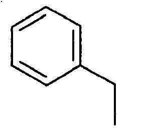 | -H | 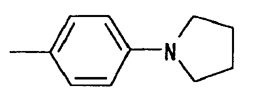 |
| 29 | 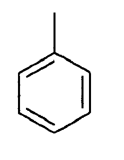 | 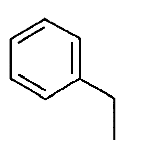 | -H | 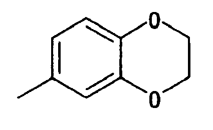 |
| 30 | 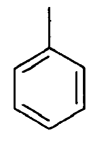 | 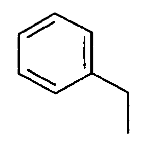 | -H | 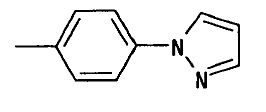 |
| 31 | 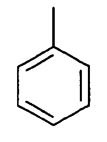 | 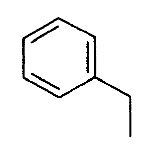 | -H | 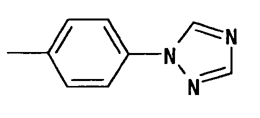 |
| 32 | 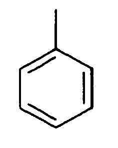 | 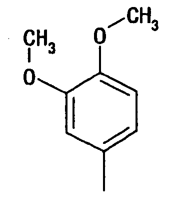 | -H | 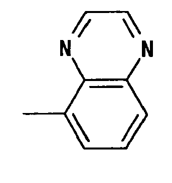 |
| 33 | 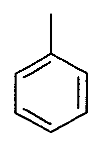 | 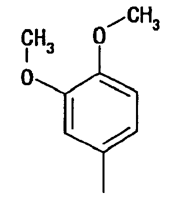 | -H | 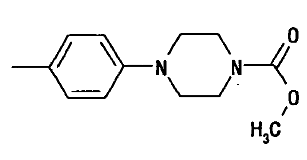 |
| 34 | 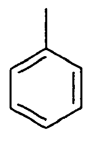 | 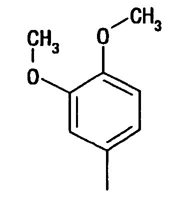 | -H | 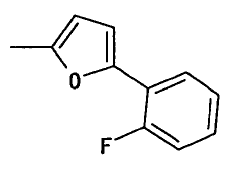 |
| 35 | 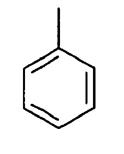 | 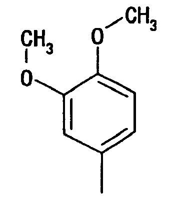 | -H | 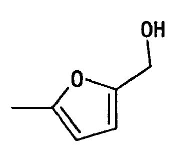 |
| 36 | 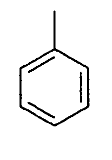 | 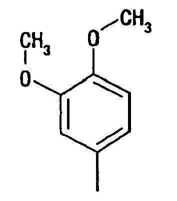 | -H | 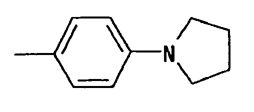 |
| 37 | 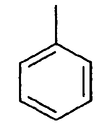 | 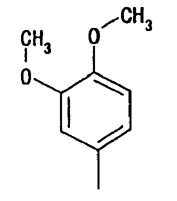 | -H | 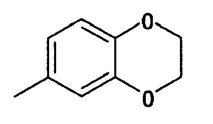 |
| 38 | 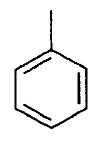 | 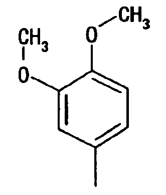 | -H | 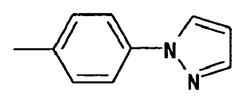 |
| 39 | 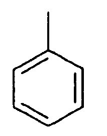 | 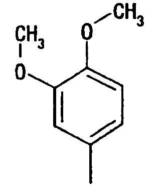 | -H | 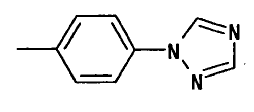 |
| 40 | 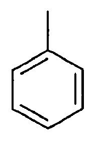 | 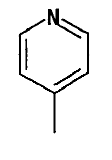 | -H | 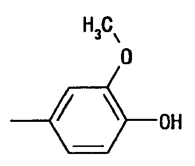 |
| 41 | 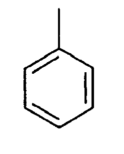 | 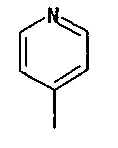 | -H | 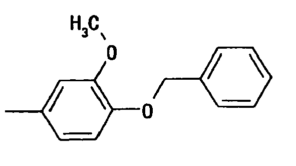 |
| 42 | 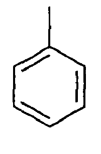 | 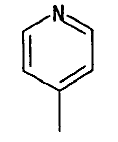 | -H | 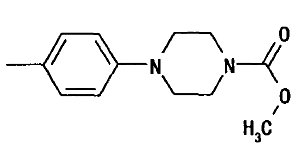 |
| 43 | 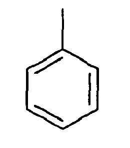 | 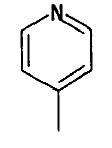 | -H | 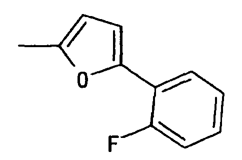 |
| 44 | 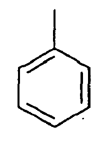 | 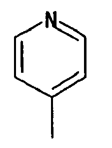 | -H | 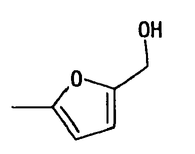 |
| 45 | 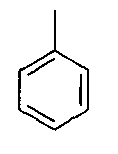 | 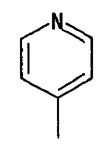 | -H | 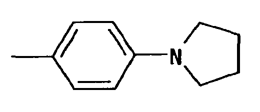 |
| 46 | 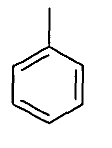 | 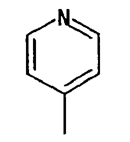 | -H | 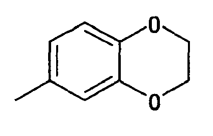 |
| 47 | 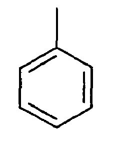 | 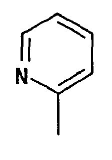 | -H | 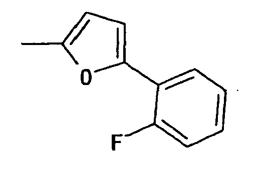 |
| 48 | 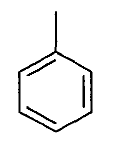 | 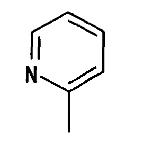 | -H | 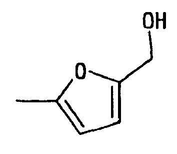 |
| 49 | 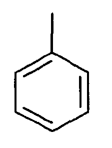 | 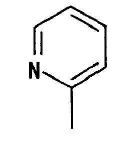 | -H | 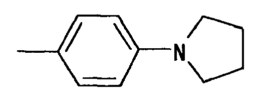 |
| 50 | 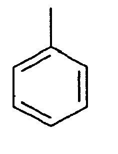 | 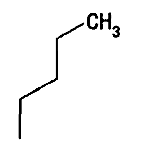 | -H | 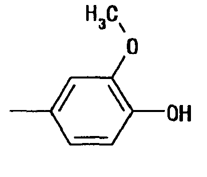 |
| 51 | 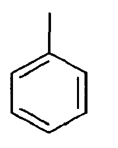 | 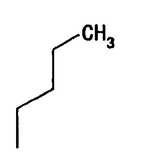 | -H | 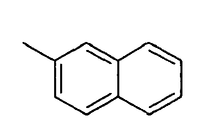 |
| 52 | 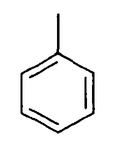 | 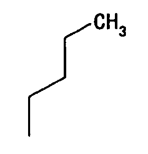 | -H | 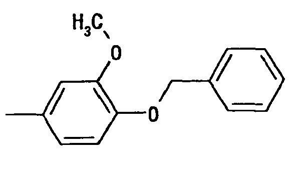 |
| 53 | 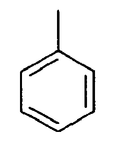 | 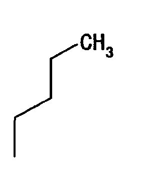 | -H | 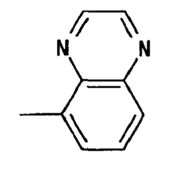 |
| 54 | 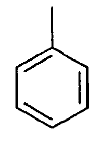 | 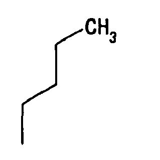 | -H | 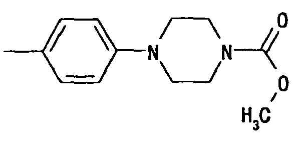 |
| 55 | 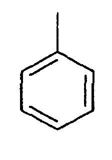 | 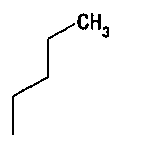 | -H | 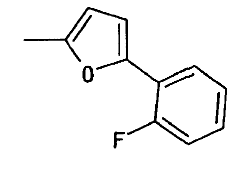 |
| 56 | 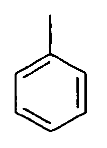 | 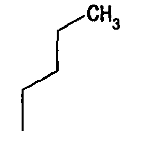 | -H | 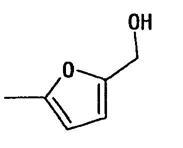 |
| 57 | 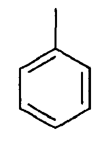 | 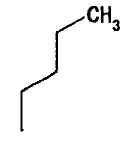 | -H | 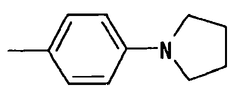 |
| 58 | 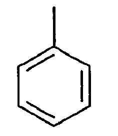 | 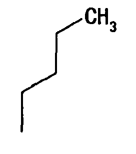 | -H | 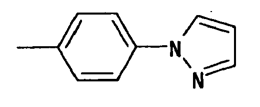 |
| 59 | 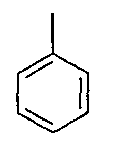 | 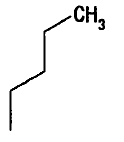 | -H | 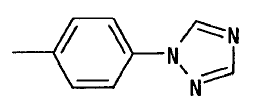 |
| 60 | 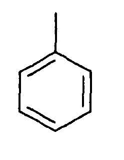 | 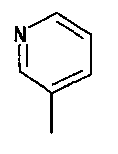 | -H | 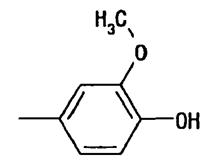 |
| 61 | 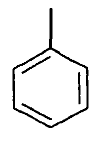 | 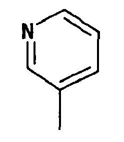 | -H | 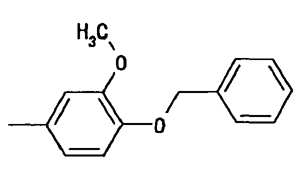 |
| 62 | 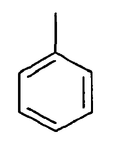 | 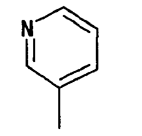 | -H | 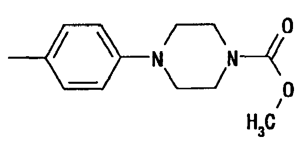 |
| 63 | 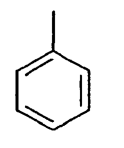 | 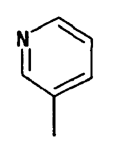 | -H | 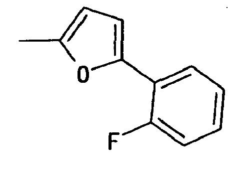 |
| 64 | 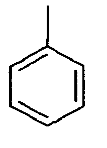 | 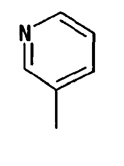 | -H | 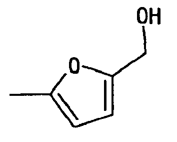 |
| 65 | 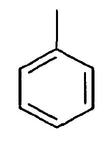 | 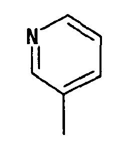 | -H | 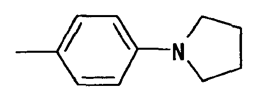 |
| 66 | 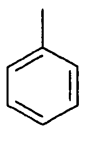 | 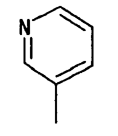 | -H | 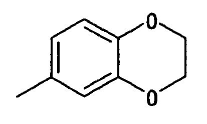 |
| 67 | 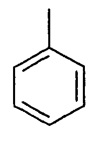 | 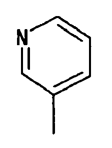 | -H | 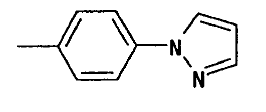 |
| 68 | 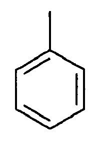 | 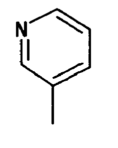 | -H | 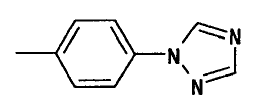 |
| 69 | 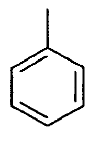 | 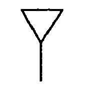 | -H | 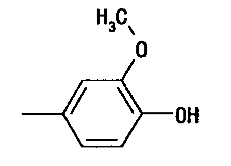 |
| 70 | 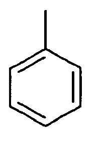 | 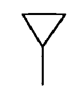 | -H | 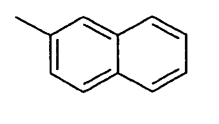 |
| 71 | 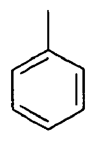 | 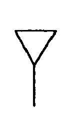 | -H | 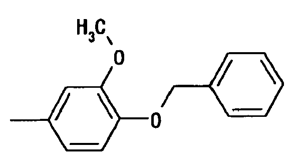 |
| 72 | 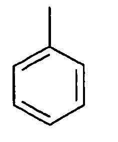 | 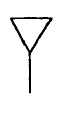 | -H | 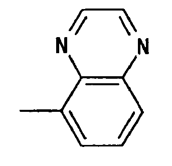 |
| 73 | 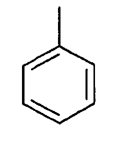 | 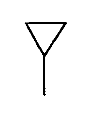 | -H | 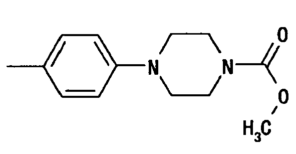 |
| 74 | 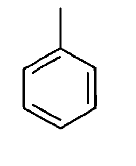 | 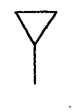 | -H | 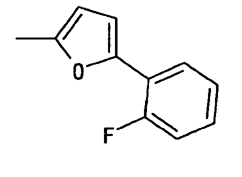 |
| 75 | 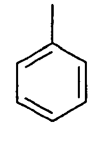 | 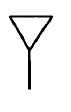 | -H | 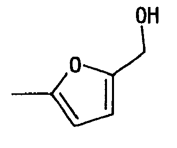 |
| 76 | 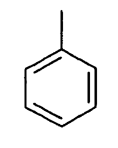 | 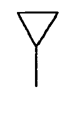 | -H | 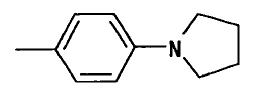 |
| 77 | 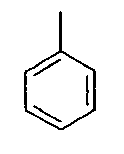 | 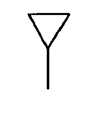 | -H | 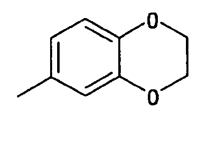 |
| 78 | 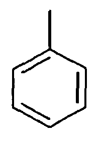 | 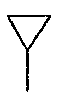 | -H | 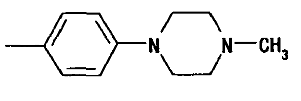 |
| 79 | 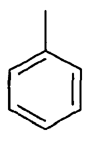 | 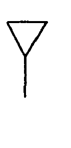 | -H | 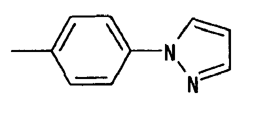 |
| 80 | 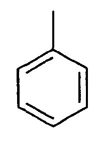 | 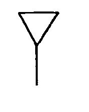 | -H | 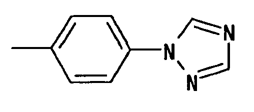 |
| 81 | 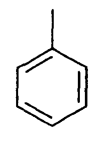 | 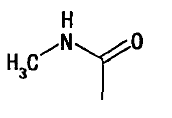 | -H | 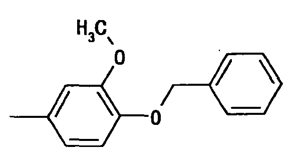 |
| 82 | 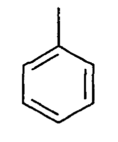 | 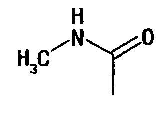 | -H | 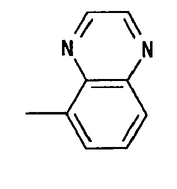 |
| 83 | 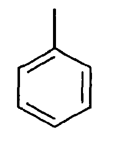 | 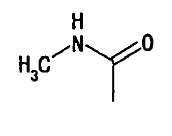 | -H | 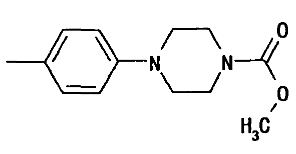 |
| 84 | 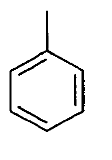 | 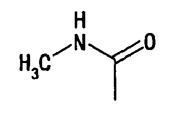 | -H | 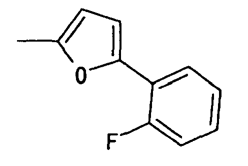 |
| 85 | 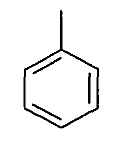 | 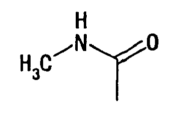 | -H | 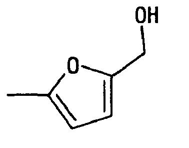 |
| 86 | 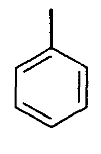 | 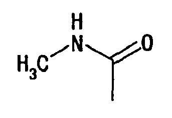 | -H | 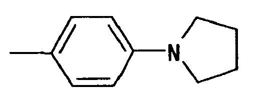 |
| 87 | 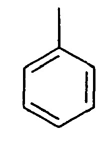 | 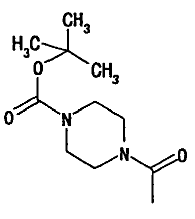 | -H | 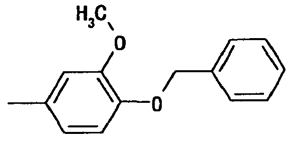 |
| 88 | 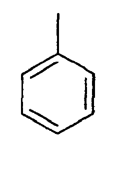 | 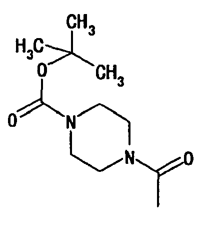 | -H | 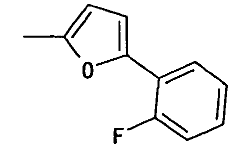 |
| 89 | 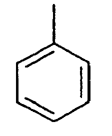 | 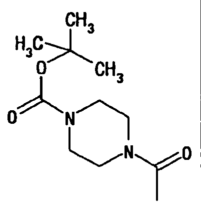 | -H | 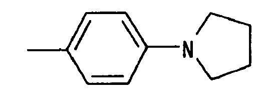 |
| 90 | 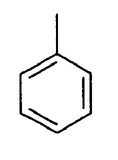 | 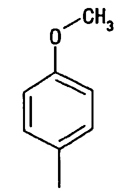 | -H | 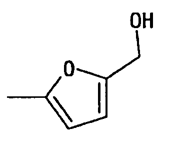 |
| 91 | 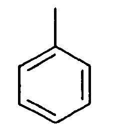 | 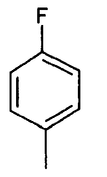 | -H | 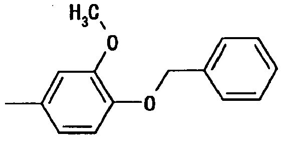 |
| 92 | 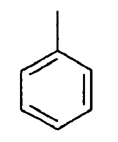 | 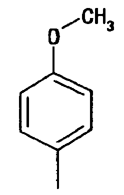 | -H | 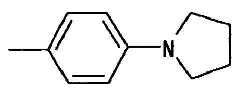 |
| 93 | 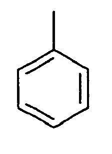 | 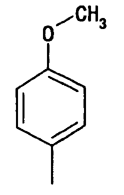 | -H | 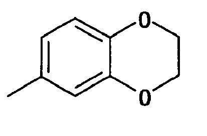 |
| 94 | 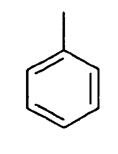 | 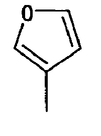 | -H | 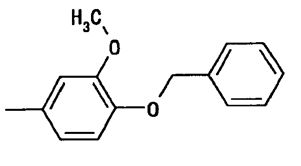 |
| 95 | 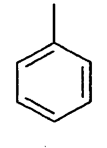 | -CH₃ | -H | 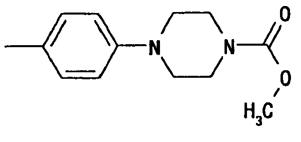 |
| 96 | 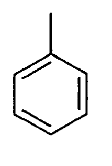 | 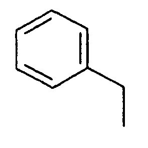 | -H | 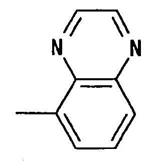 |
| 97 | 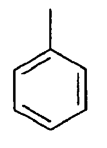 | 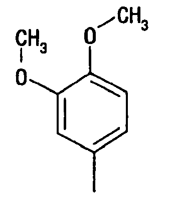 | -H | 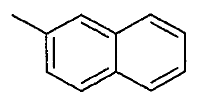 |
| 98 | 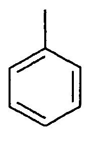 | 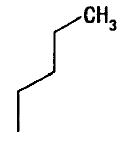 | -H | 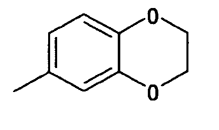 |
| 99 | 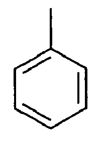 | -CH₃ | -H | 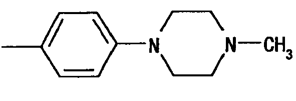 |
| 100 | 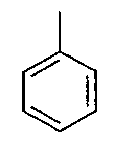 | 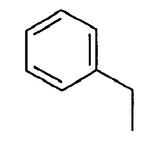 | -H | 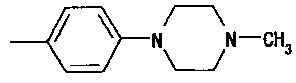 |
| 101 | 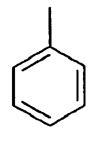 | 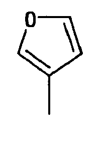 | -H | 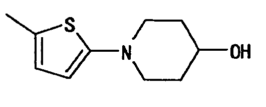 |
| 102 | 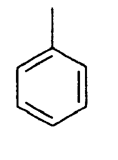 | 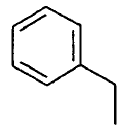 | -H | 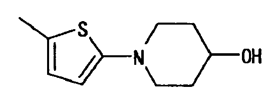 |
| 103 | 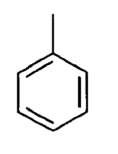 | 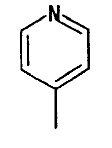 | -H | 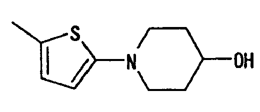 |
| 104 | 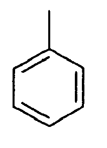 | 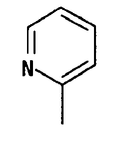 | -H | 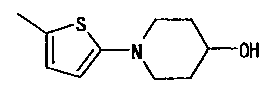 |
| 105 | 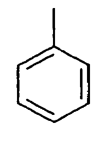 | 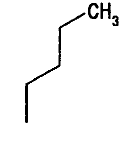 | -H | 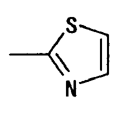 |
| 106 | 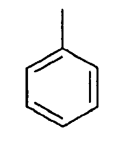 | 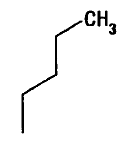 | -H | 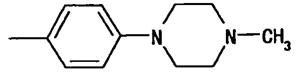 |
| 107 | 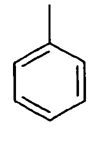 | 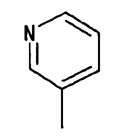 | -H | 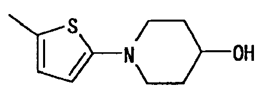 |
| 108 | 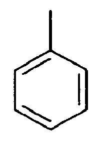 | -CH₃ | -H | 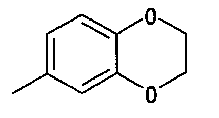 |
| 109 | 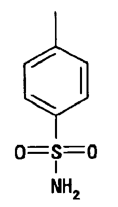 | -CH₃ | -H | 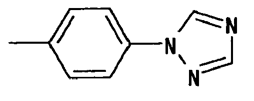 |
| 110 | 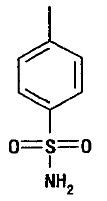 | 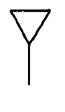 | -H | 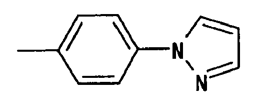 |
| 111 | 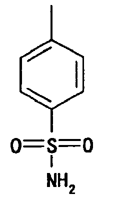 | 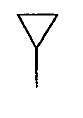 | -H | 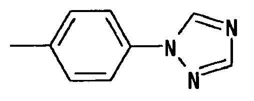 |
| 112 | 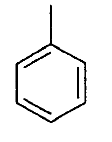 | 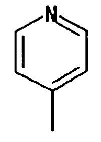 | -H | 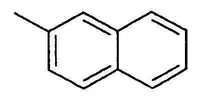 |
| 113 | 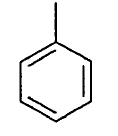 | 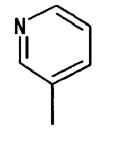 | -H | 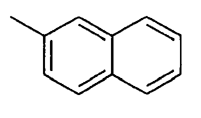 |
| 114 | 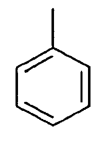 | 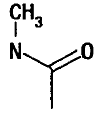 | -H | 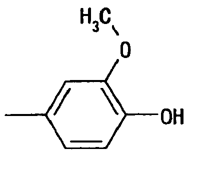 |
| 115 | 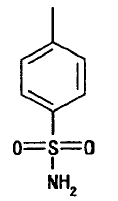 | -CH₃ | -H | 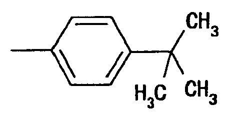 |
| 116 | 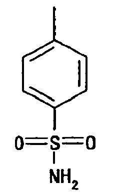 | -CH₃ | -H | 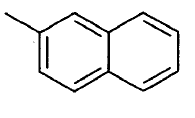 |
| 117 | 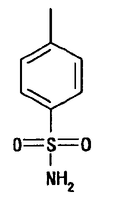 | -CH₃ | -H | 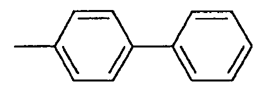 |
| 118 | 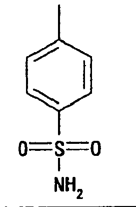 | -CH₃ | -H | 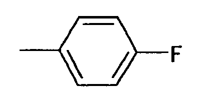 |
| 119 | 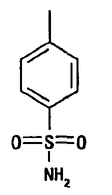 | -CH₃ | -H | 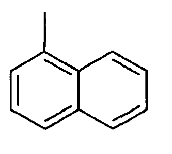 |
| 120 | 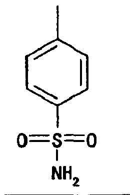 | -CH₃ | -H | 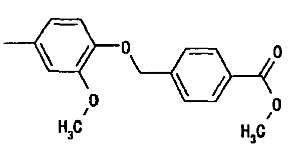 |
| 121 | 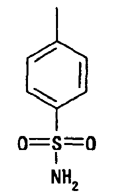 | -CH₃ | -H | 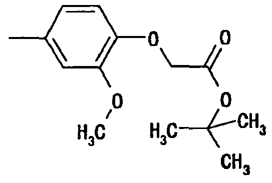 |
| 122 | 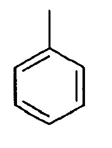 | -CH₃ | -H | 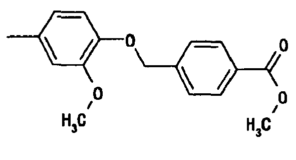 |
| 123 | 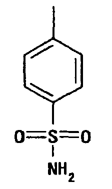 | -CH₃ | -H | 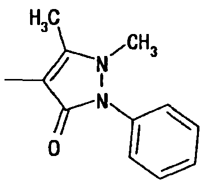 |
| 124 | 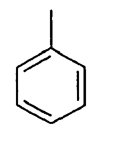 | -CH₃ | -H | 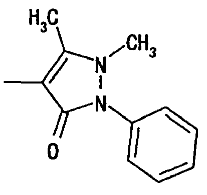 |
| 125 | 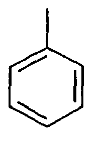 | -CH₃ | -H | 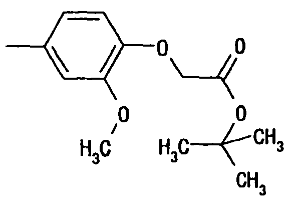 |
| 126 | 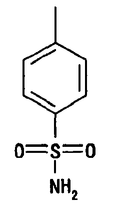 | -CH₃ | -H | 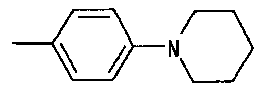 |
| 127 | 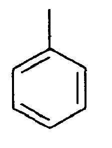 | -CH₃ | -H | 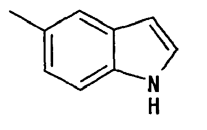 |
| 128 | 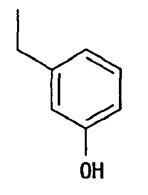 | -CH₃ | -H | 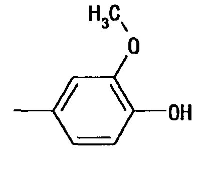 |
| 129 | 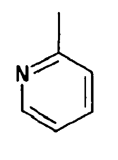 | -CH₃ | -H | 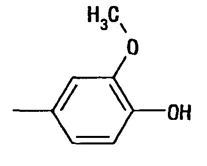 |
| 130 | 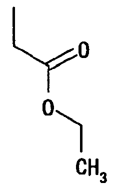 | -CH₃ | -H | 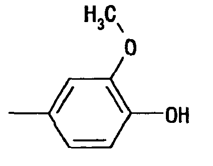 |
| 131 | 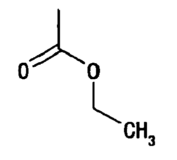 | -CH₃ | -H | 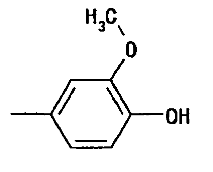 |
| 132 | -CH³ | -CH₃ | -H | 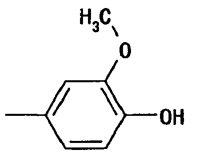 |
| 133 | 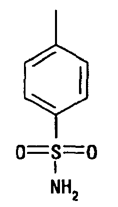 | -CH₃ | -H | 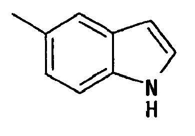 |
| 134 | 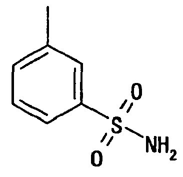 | -CH₃ | -H | 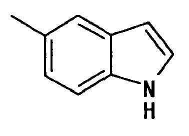 |
| 135 | 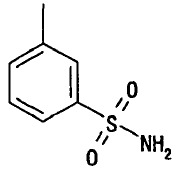 | -CH₃ | -H | 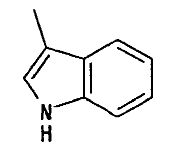 |
| 136 | 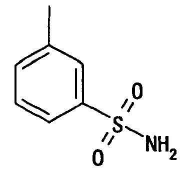 | -CH₃ | -H | 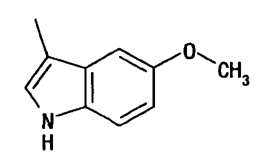 |
| 137 | 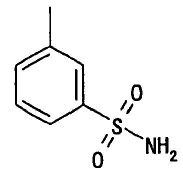 | -CH₃ | -H | 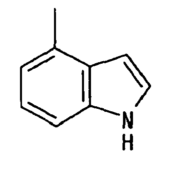 |
| 138 | 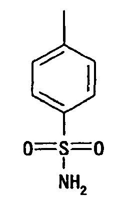 | -CH₃ | -H | 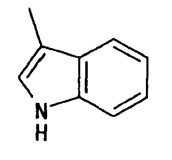 |
| 139 | 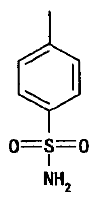 | -CH₃ | -H | 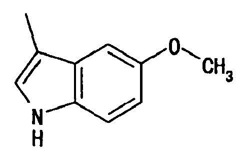 |
| 140 | 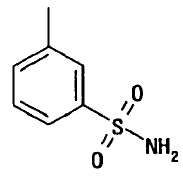 | -CH₃ | -H | 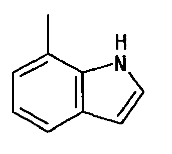 |
| 141 | 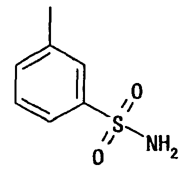 | -CH₃ | -H | 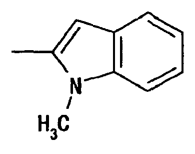 |
| 142 | 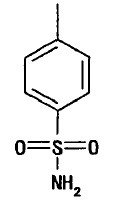 | -CH₃ | -H | 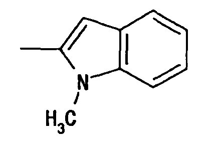 |
| 143 | 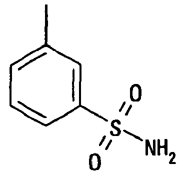 | -CH₃ | -H | 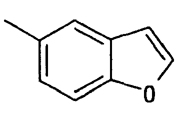 |
| 144 | 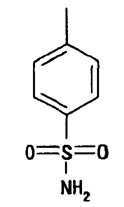 | -CH₃ | -H | 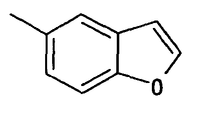 |
| 145 | 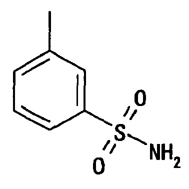 | -CH₃ | -H | 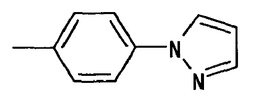 |
| 146 | 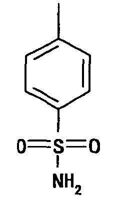 | -CH₃ | -H | 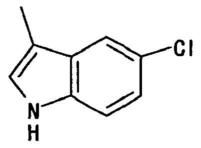 |
| 147 | 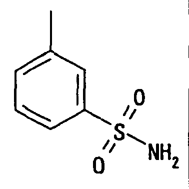 | -CH₃ | -H | 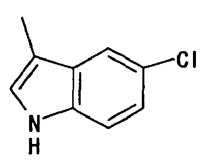 |
| 148 | 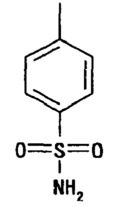 | -CH₃ | -H | 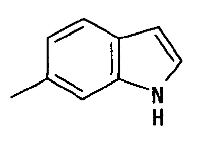 |
| 149 | 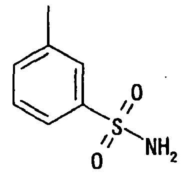 | -CH₃ | -H | 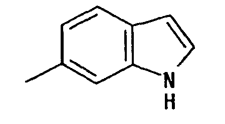 |
| 150 | 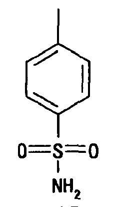 | -CH₃ | -H | 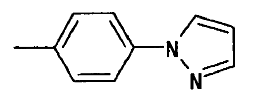 |
| 151 | 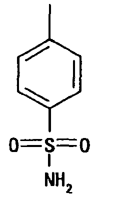 | -CH₃ | -H | 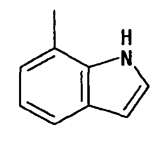 |
| 152 | 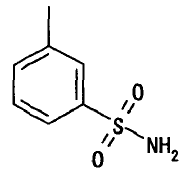 | -CH₃ | -H | 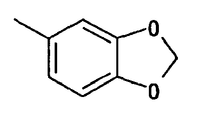 |
| 153 | 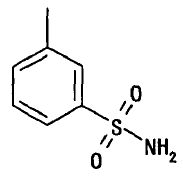 | -CH₃ | -H | 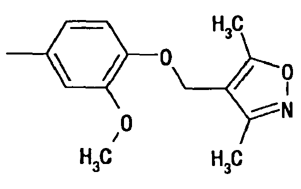 |
| 154 | 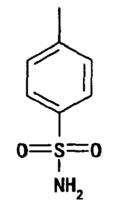 | -CH₃ | -H | 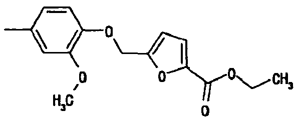 |
| 155 | 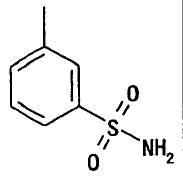 | -CH₃ | -H | 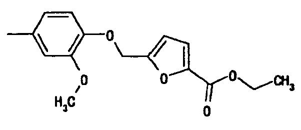 |
| 156 | 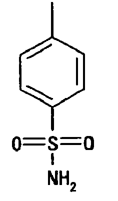 | -CH₃ | -H | 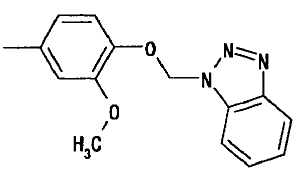 |
| 157 | 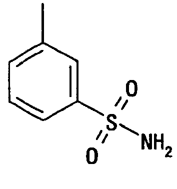 | -CH₃ | -H | 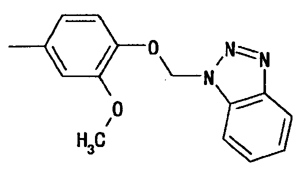 |
| 158 | 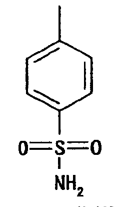 | -CH₃ | -H | 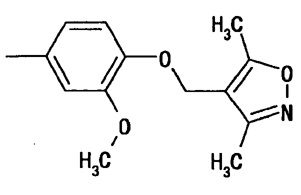 |
| 159 | 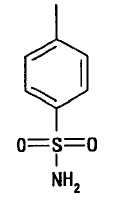 | -CH₃ | -H | 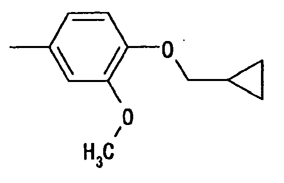 |
| 160 | 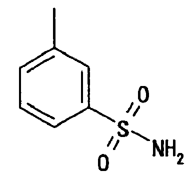 | -CH₃ | -H | 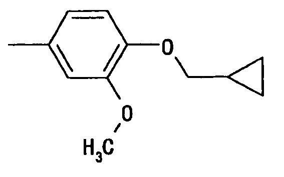 |
| 161 | 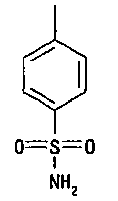 | -CH₃ | -H | 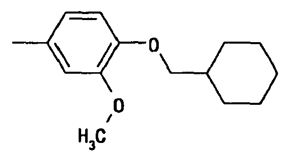 |
| 162 | 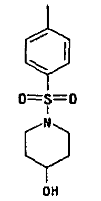 | -CH₃ | -H | 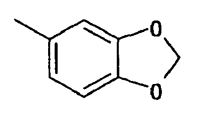 |
| 163 | 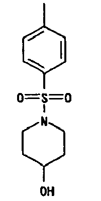 | -CH₃ | -H | 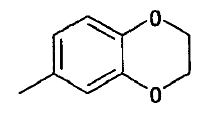 |
| 164 | 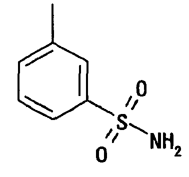 | -CH₃ | -H | 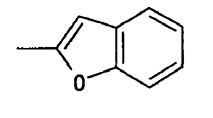 |
| 165 | 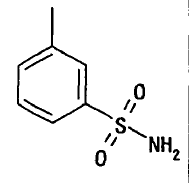 | -CH₃ | -H | 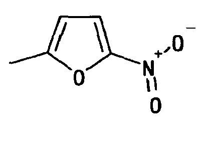 |
| 166 | 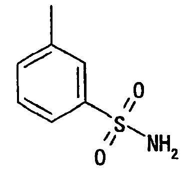 | -CH₃ | -H | 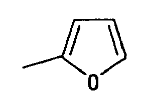 |
| 167 | 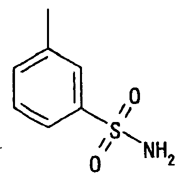 | -CH₃ | -H | 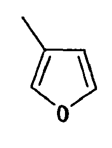 |
| 168 | 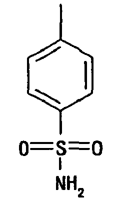 | -CH₃ | -H | 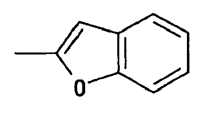 |
| 169 | 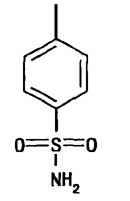 | -CH₃ | -H | 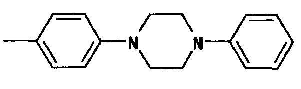 |
| 170 | 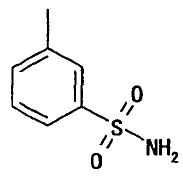 | -CH₃ | -H | 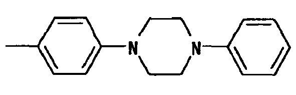 |
| 171 | 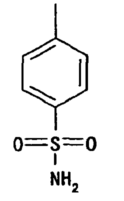 | -CH₃ | -H | 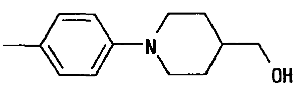 |
| 172 | 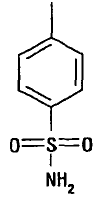 | -CH₃ | -H | 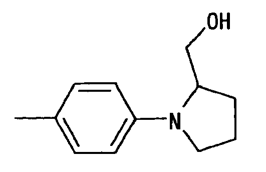 |
| 173 | 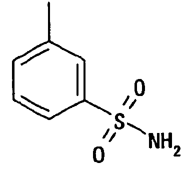 | -CH₃ | -H | 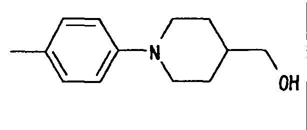 |
| 174 | 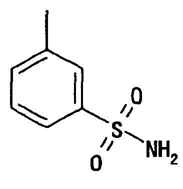 | -CH₃ | -H | 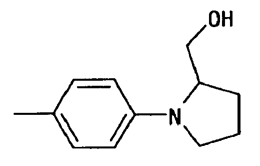 |
| 175 | 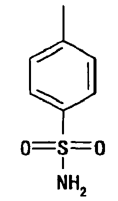 | -CH₃ | -H | 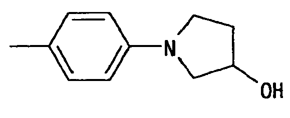 |
| 176 | 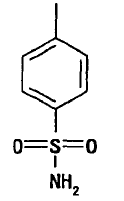 | -CH₃ | -H | 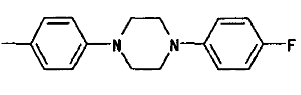 |
| 177 | 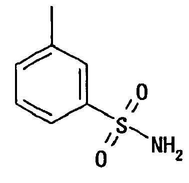 | -CH₃ | -H | 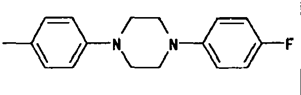 |
| 178 | 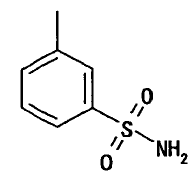 | -CH₃ | -H | 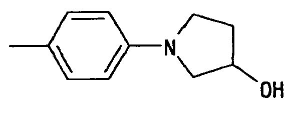 |
| 179 | 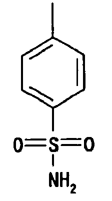 | -CH₃ | -H | 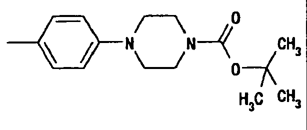 |
| 180 | 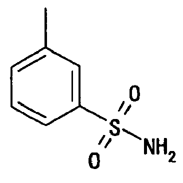 | -CH₃ | -H | 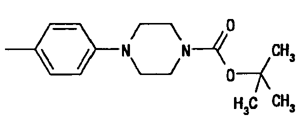 |
| 181 | 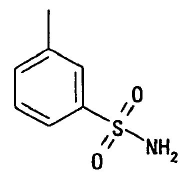 | -CH₃ | -H | 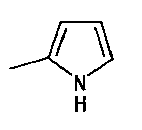 |
| 182 | 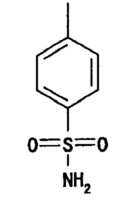 | -CH₃ | -H | 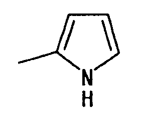 |
| 183 | 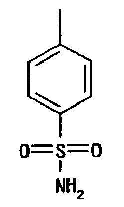 | -CH₃ | -H | 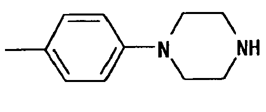 |
| 184 | 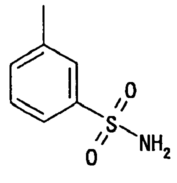 | -CH₃ | -H | 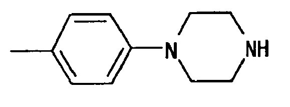 |
| 185 | 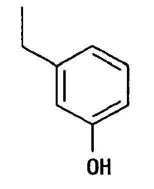 | -CH₃ | -H | 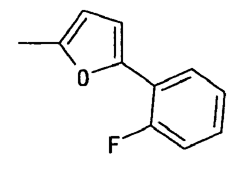 |
| 186 | 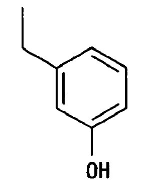 | 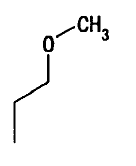 | -H | 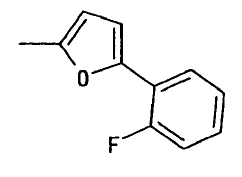 |
| 187 | 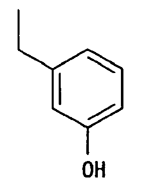 | 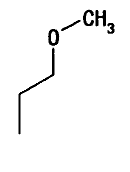 | -H | 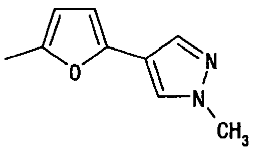 |
| 188 | 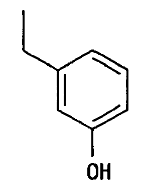 | 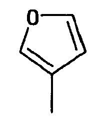 | -H | 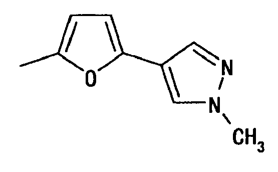 |
| 189 | 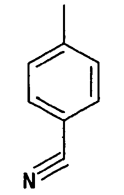 | -CH₃ | -H | 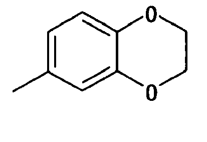 |
| 190 | 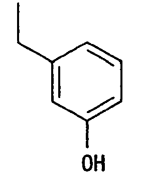 | 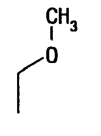 | -H | 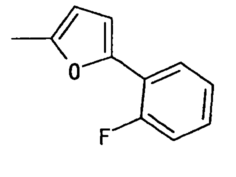 |
| 191 | 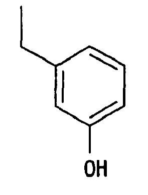 | 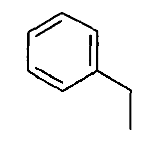 | -H | 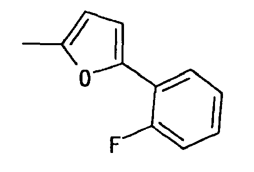 |
| 192 | 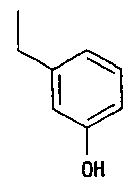 | 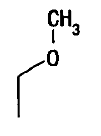 | -H | 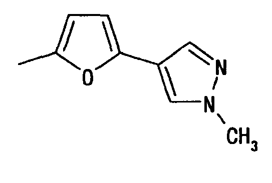 |
| 193 | 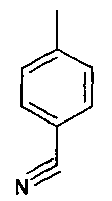 | -CH₃ | -H | 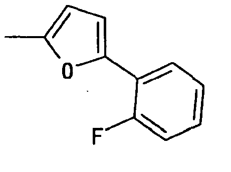 |
| 194 | 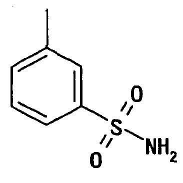 | -CH₃ | -H | 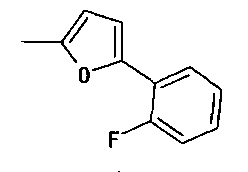 |
| 195 | 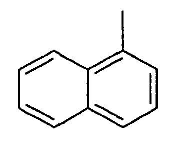 | -CH₃ | -H | 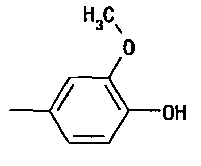 |
| 196 | 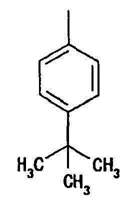 | -CH₃ | -H | 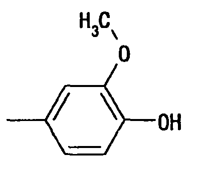 |
| 197 | 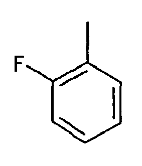 | -CH₃ | -H | 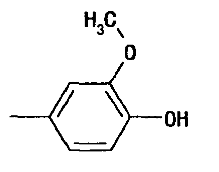 |
| 198 | 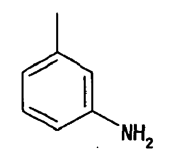 | -CH₃ | -H | 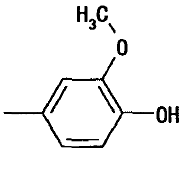 |
| 199 | 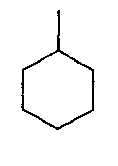 | -CH₃ | -H | 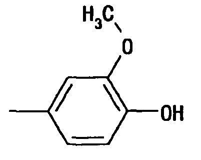 |
| 200 | 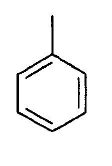 | -CH₃ | -H | 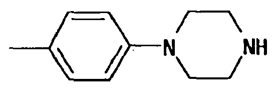 |
| 201 | 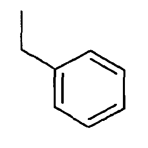 | -CH₃ | -H | 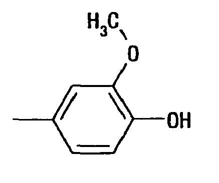 |
| 202 | 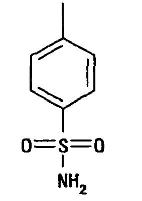 | -CH₃ | -H | 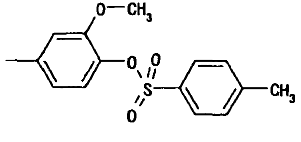 |
| 203 | 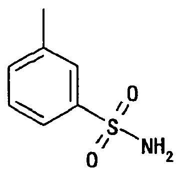 | -CH₃ | -H | 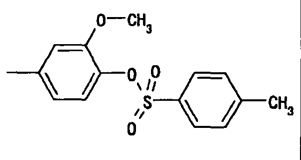 |
| 204 | 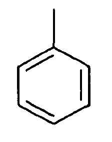 | -CH₃ | -H | 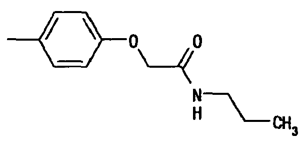 |
| 205 | 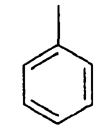 | -CH₃ | -H | 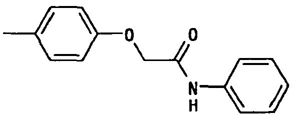 |
| 206 | 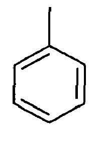 | -CH₃ | -H | 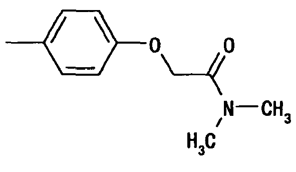 |
| 207 | 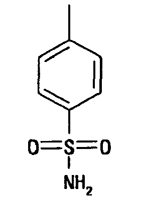 | -CH₃ | -H | 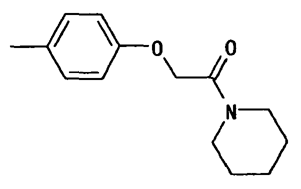 |
| 208 | 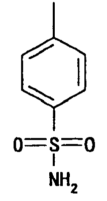 | -CH₃ | -H | 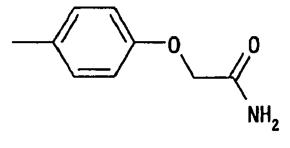 |
| 209 | 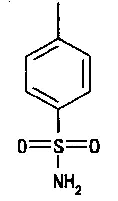 | -CH₃ | -H | 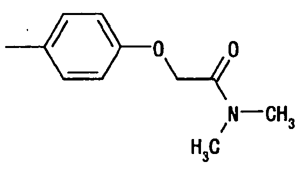 |
| 210 | 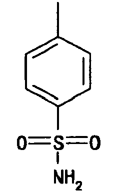 | -CH₃ | -H | 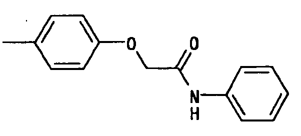 |
| 211 | 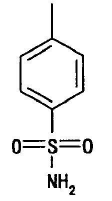 | -CH₃ | -H | 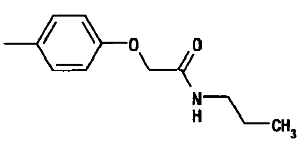 |
| 212 | 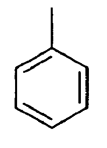 | -CH₃ | -H | 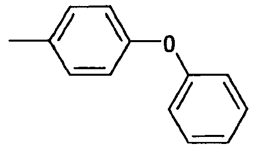 |
| 213 | 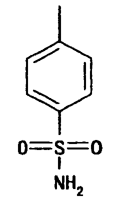 | -CH₃ | -H | 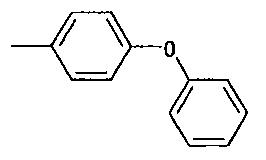 |
| 214 | 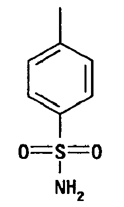 | -CH₃ | -H | 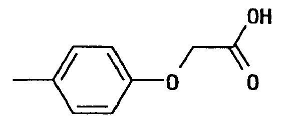 |
| 215 | 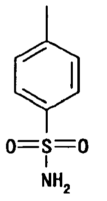 | -CH₃ | -H | 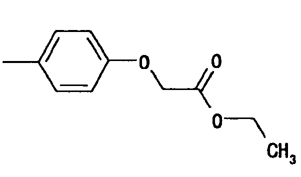 |
| 216 | 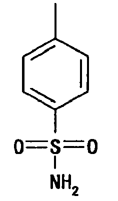 | -CH₃ | -H | 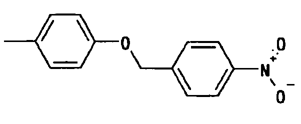 |
| 217 | 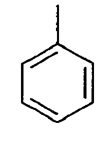 | -CH₃ | -H | 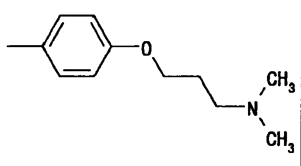 |
| 218 | 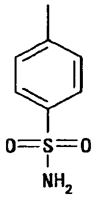 | -CH₃ | -H | 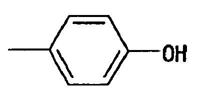 |
| 219 | 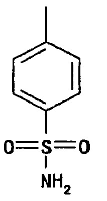 | -CH₃ | -H | 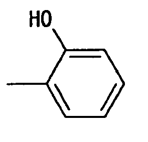 |
| 220 | 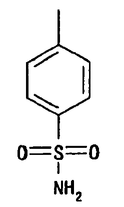 | -CH₃ | -H | 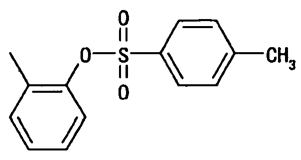 |
| 221 | 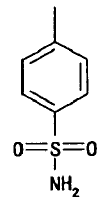 | -CH₃ | -H | 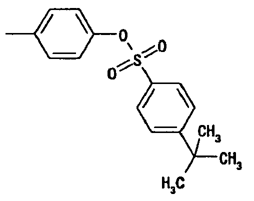 |
| 222 | 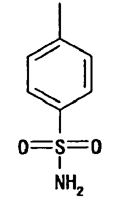 | -CH₃ | -H | 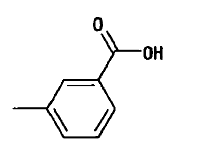 |
| 223 | 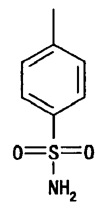 | -CH₃ | -H | 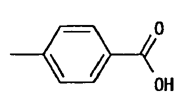 |
| 224 | 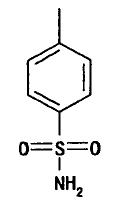 | -CH₃ | -H | 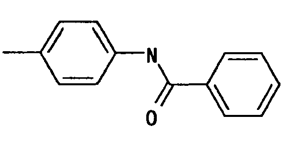 |
| 225 | 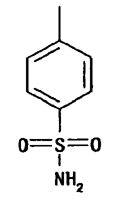 | -CH₃ | -H | 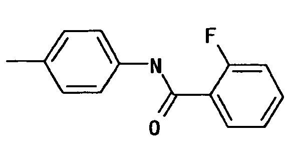 |
| 226 | 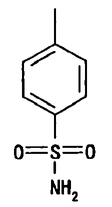 | -CH₃ | -H | 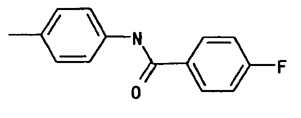 |
| 227 | 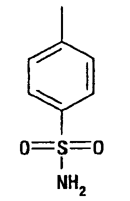 | -CH₃ | -H | 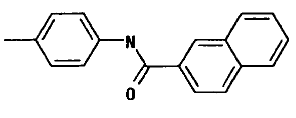 |
| 228 | 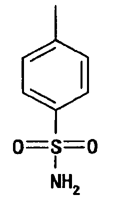 | -CH₃ | -H | 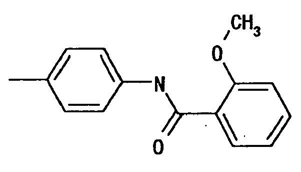 |
| 229 | 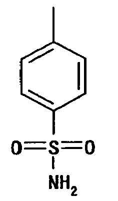 | -CH₃ | -H | 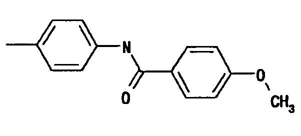 |
| 230 | 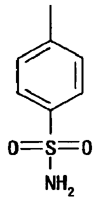 | -CH₃ | -H | 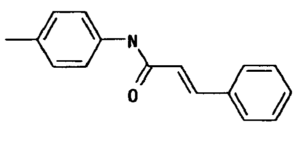 |
| 231 | 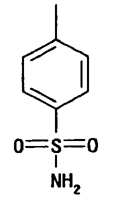 | -CH₃ | -H | 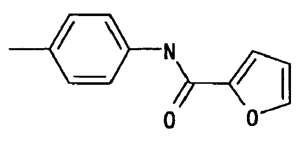 |
| 232 | 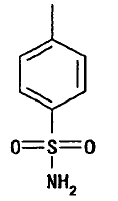 | -CH₃ | -H | 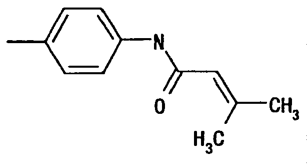 |
| 233 | 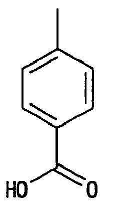 | 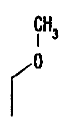 | -H | 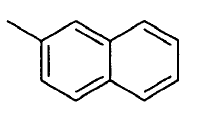 |
| 234 | 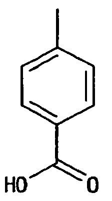 | 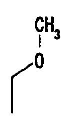 | -H | 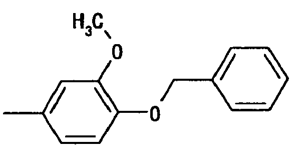 |
| 235 | 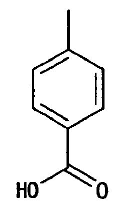 | 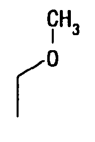 | -H | 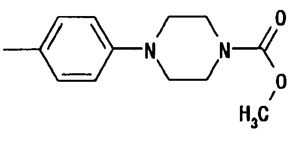 |
| 236 | 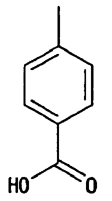 | 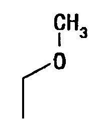 | -H | 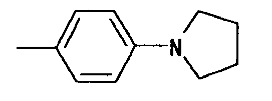 |
| 237 | 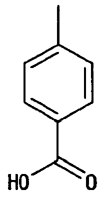 | 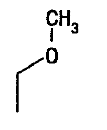 | -H | 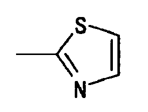 |
| 238 | 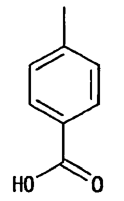 | 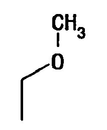 | -H | 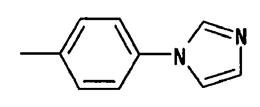 |
| 239 | 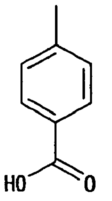 | 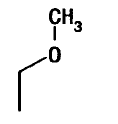 | -H | 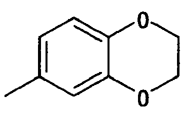 |
| 240 | 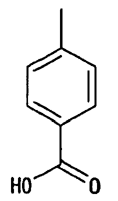 | 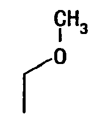 | -H | 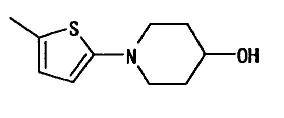 |
| 241 | 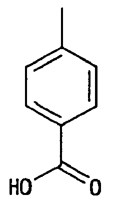 | 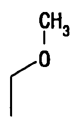 | -H | 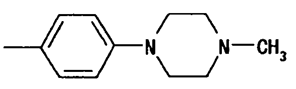 |
| 242 | 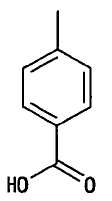 | 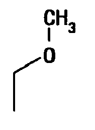 | -H | 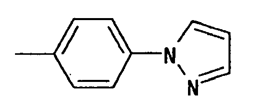 |
| 243 | 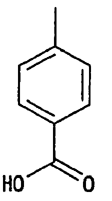 | 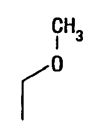 | -H | 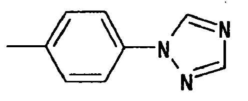 |
| 244 | 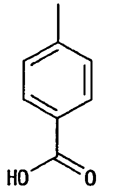 | 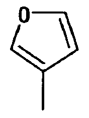 | -H | 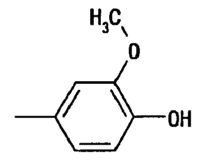 |
| 245 | 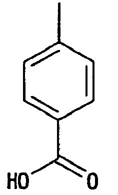 | 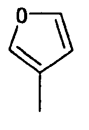 | -H | 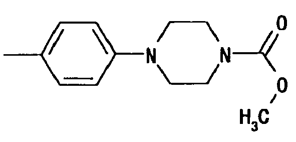 |
| 246 | 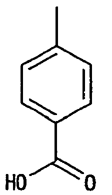 | 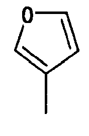 | -H | 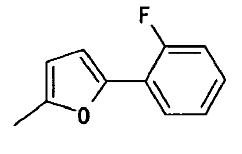 |
| 247 | 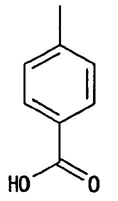 | 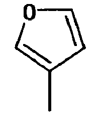 | -H | 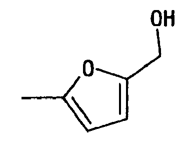 |
| 248 | 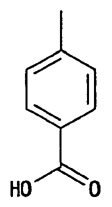 | 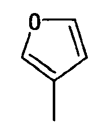 | -H | 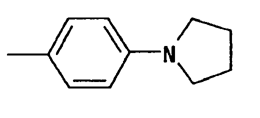 |
| 249 | 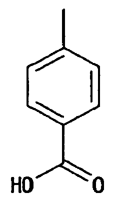 | 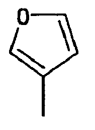 | -H | 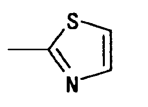 |
| 250 | 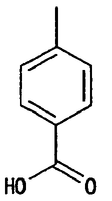 | 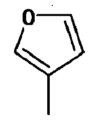 | -H | 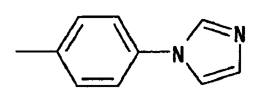 |
| 251 | 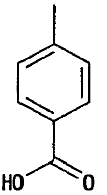 | 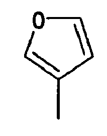 | -H | 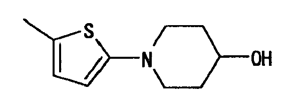 |
| 252 | 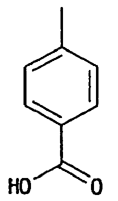 | 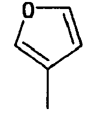 | -H | 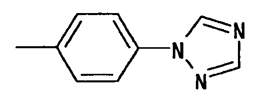 |
| 253 | 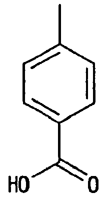 | -CH₃ | -H | 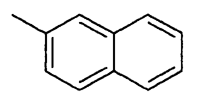 |
| 254 | 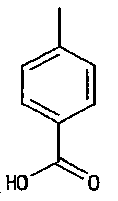 | -CH₃ | -H | 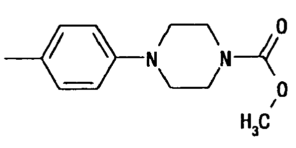 |
| 255 | 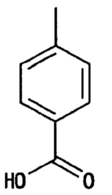 | -CH₃ | -H | 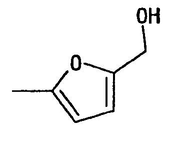 |
| 256 | 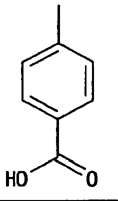 | -CH₃ | -H | 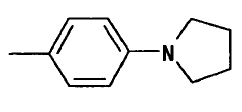 |
| 257 | 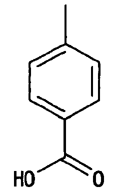 | -CH₃ | -H | 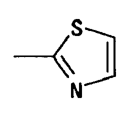 |
| 258 | 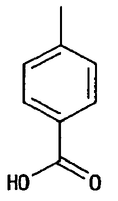 | -CH₃ | -H | 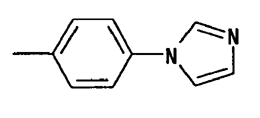 |
| 259 | 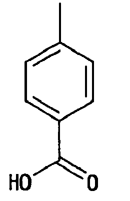 | -CH₃ | -H | 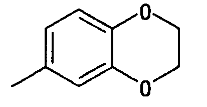 |
| 260 | 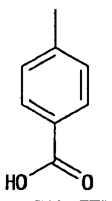 | -CH₃ | -H | 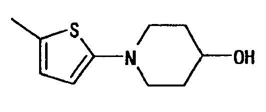 |
| 261 | 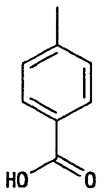 | -CH₃ | -H | 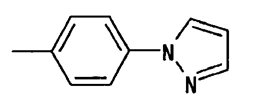 |
| 262 | 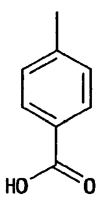 | -CH₃ | -H | 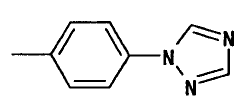 |
| 263 | 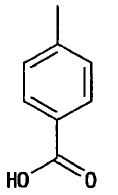 | 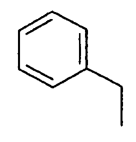 | -H | 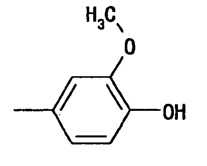 |
| 264 | 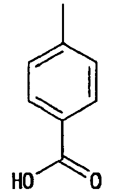 | 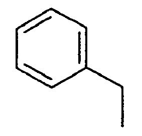 | -H | 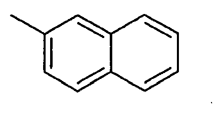 |
| 265 | 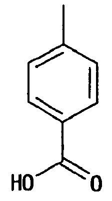 | 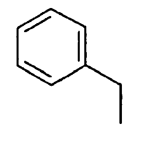 | -H | 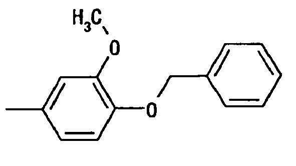 |
| 266 | 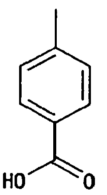 | 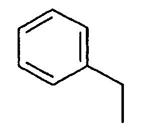 | -H | 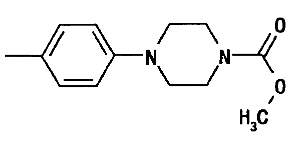 |
| 267 | 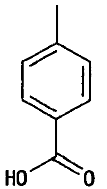 | 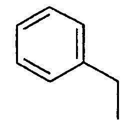 | -H | 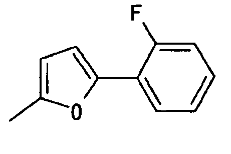 |
| 268 | 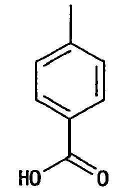 | 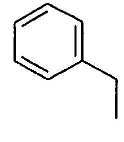 | -H | 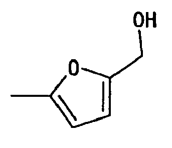 |
| 269 | 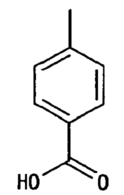 | 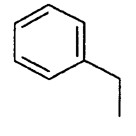 | -H | 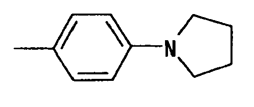 |
| 270 | 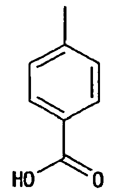 | 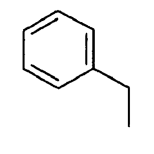 | -H | 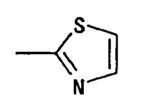 |
| 271 | 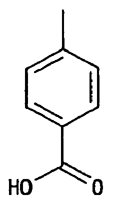 | 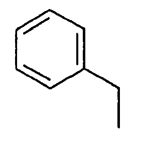 | -H | 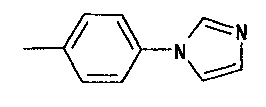 |
| 272 | 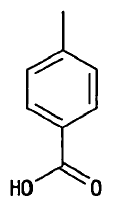 | 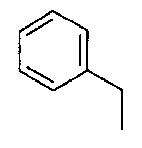 | -H | 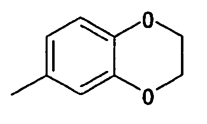 |
| 273 | 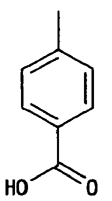 | 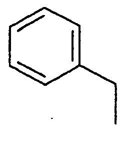 | -H | 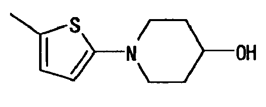 |
| 274 | 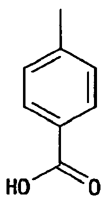 | 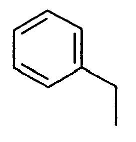 | -H | 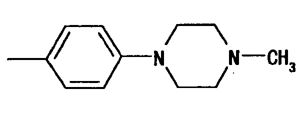 |
| 275 | 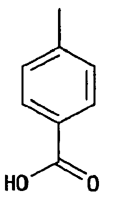 | 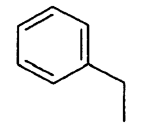 | -H | 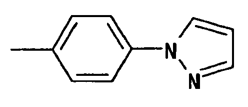 |
| 276 | 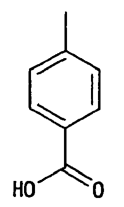 | 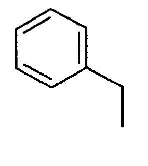 | -H | 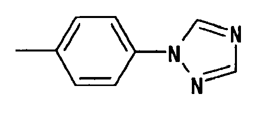 |
| 277 | 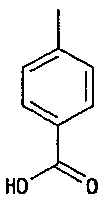 | 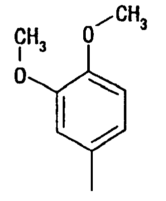 | -H | 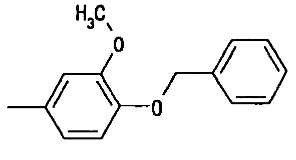 |
| 278 | 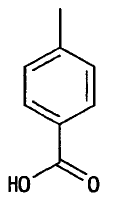 | 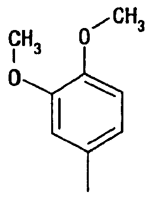 | -H | 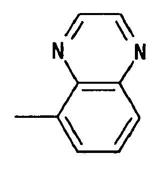 |
| 279 | 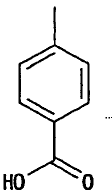 | 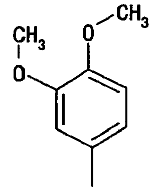 | -H | 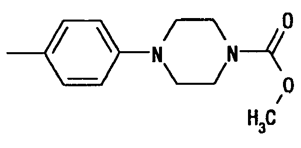 |
| 280 | 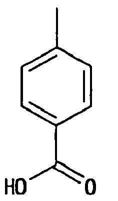 | 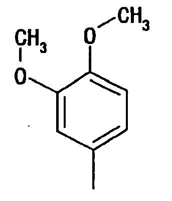 | -H | 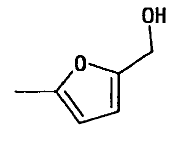 |
| 281 | 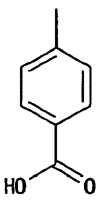 | 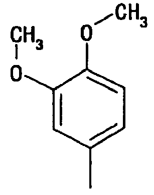 | -H | 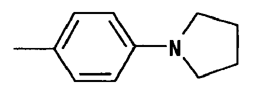 |
| 282 | 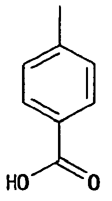 | 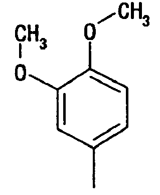 | -H | 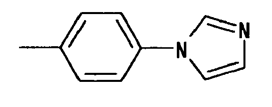 |
| 283 | 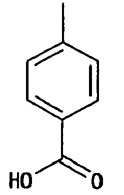 | 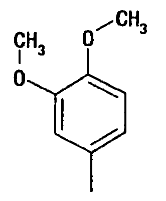 | -H | 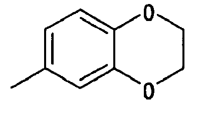 |
| 284 | 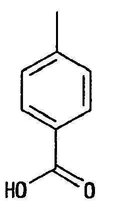 | 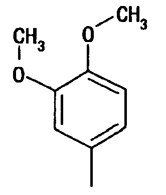 | -H | 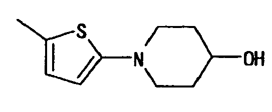 |
| 285 | 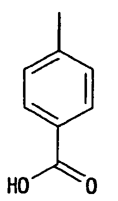 | 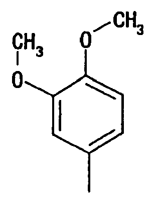 | -H | 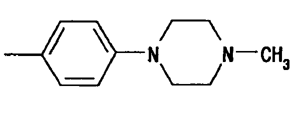 |
| 286 | 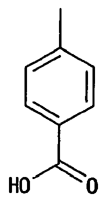 | 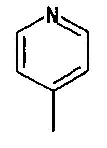 | -H | 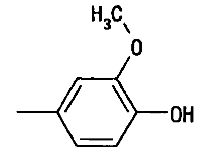 |
| 287 | 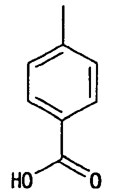 | 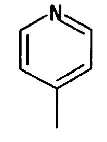 | -H | 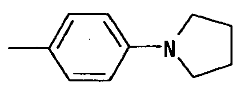 |
| 288 | 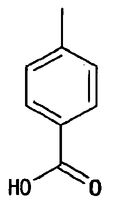 | 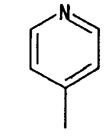 | -H | 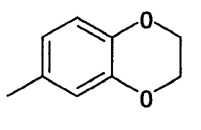 |
| 289 | 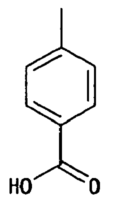 | 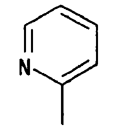 | -H | 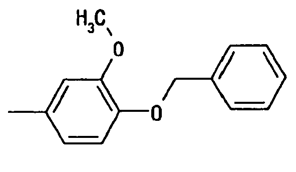 |
| 290 | 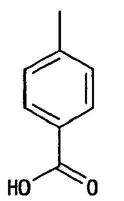 | 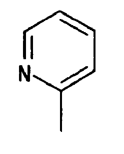 | -H | 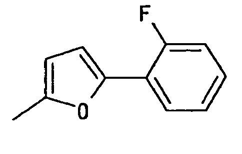 |
| 291 | 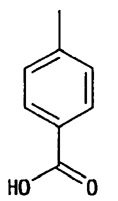 | 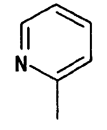 | -H | 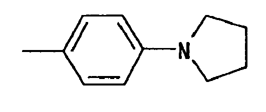 |
| 292 | 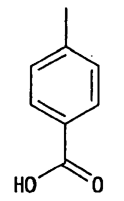 | 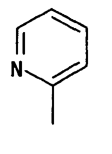 | -H | 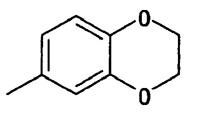 |
| 293 | 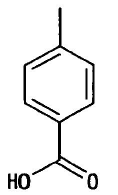 | 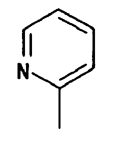 | -H | 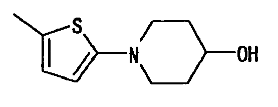 |
| 294 | 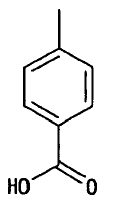 | 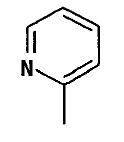 | -H | 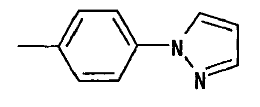 |
| 295 | 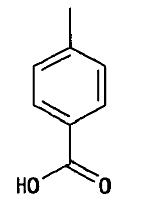 | 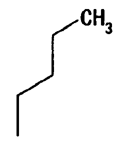 | -H | 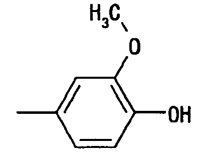 |
| 296 | 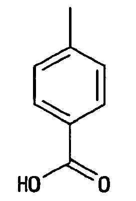 | 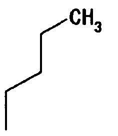 | -H | 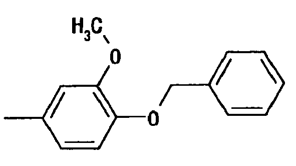 |
| 297 | 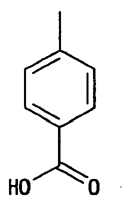 | 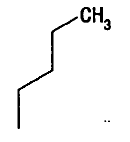 | -H | 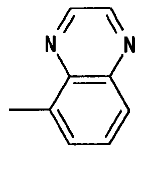 |
| 298 | 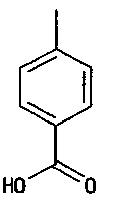 | 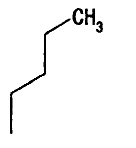 | -H | 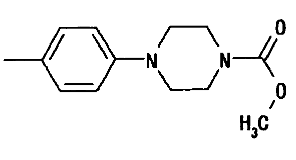 |
| 299 | 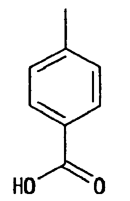 | 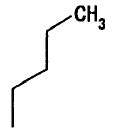 | -H | 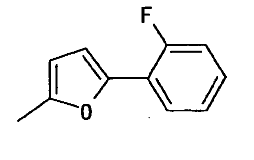 |
| 300 | 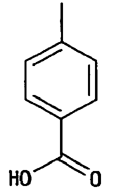 | 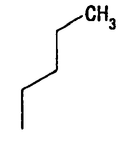 | -H | 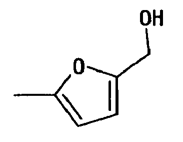 |
| 301 | 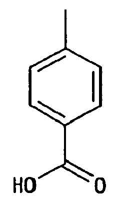 | 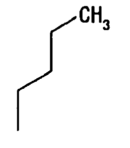 | -H | 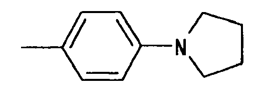 |
| 302 | 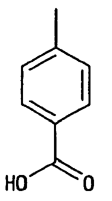 | 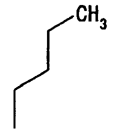 | -H | 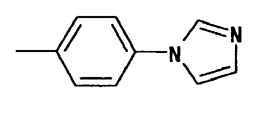 |
| 303 | 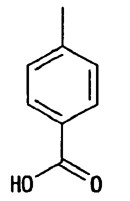 | 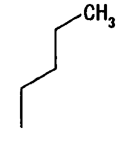 | -H | 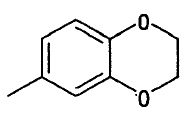 |
| 304 | 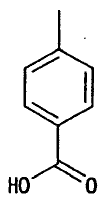 | 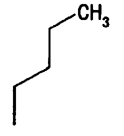 | -H | 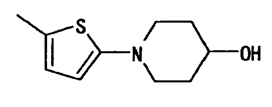 |
| 305 | 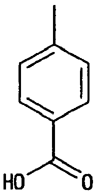 | 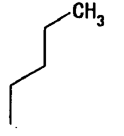 | -H | 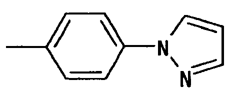 |
| 306 | 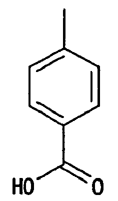 | 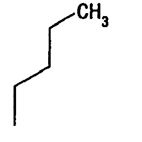 | -H | 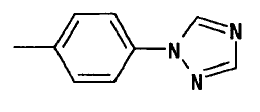 |
| 307 | 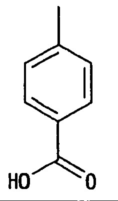 | 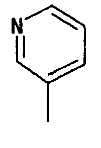 | -H | 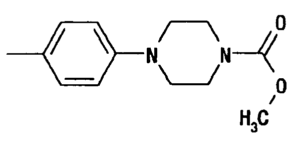 |
| 308 | 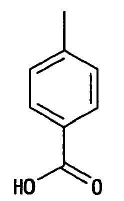 | 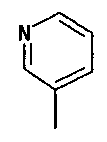 | -H | 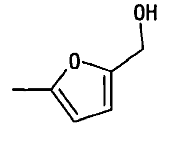 |
| 309 | 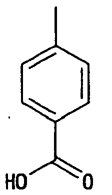 | 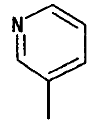 | -H | 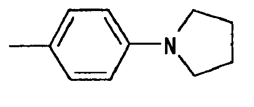 |
| 310 | 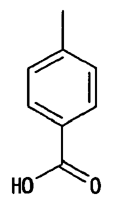 | 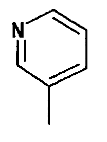 | -H | 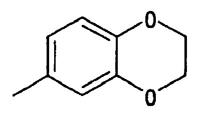 |
| 311 | 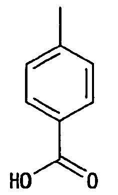 | 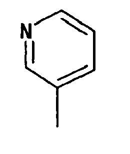 | -H | 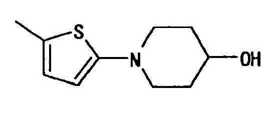 |
| 312 | 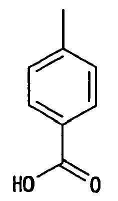 | 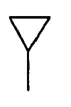 | -H | 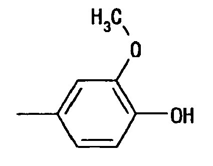 |
| 313 | 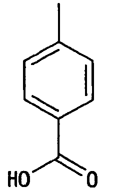 | 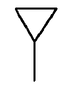 | -H | 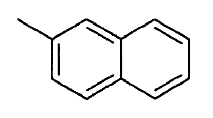 |
| 314 | 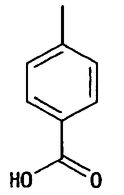 | 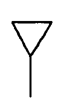 | -H | 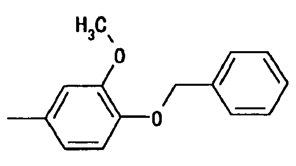 |
| 315 | 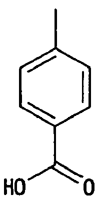 | 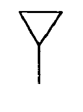 | -H | 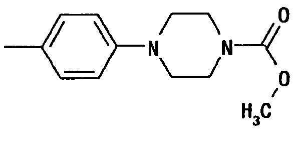 |
| 316 | 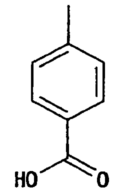 | 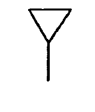 | -H | 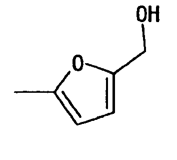 |
| 317 | 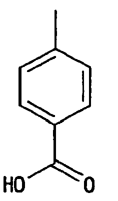 | 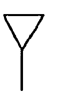 | -H | 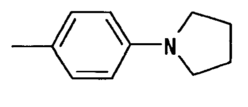 |
| 318 | 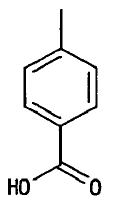 | 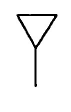 | -H | 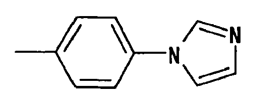 |
| 319 | 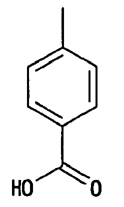 | 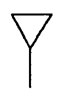 | -H | 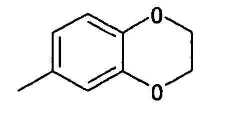 |
| 320 | 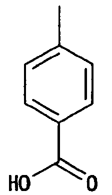 | 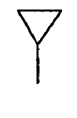 | -H | 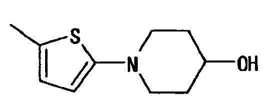 |
| 321 | 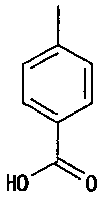 | 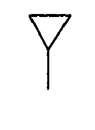 | -H | 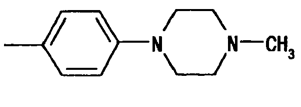 |
| 322 | 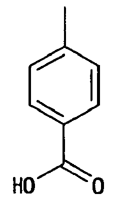 | 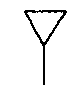 | -H | 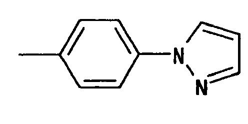 |
| 323 | 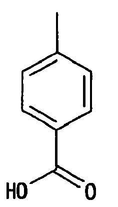 | 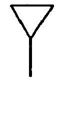 | -H | 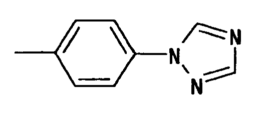 |
| 324 | 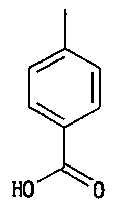 | 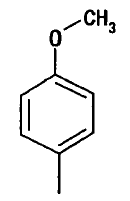 | -H | 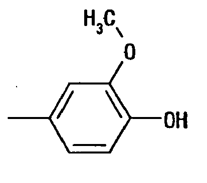 |
| 325 | 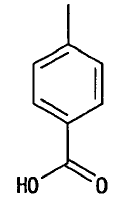 | 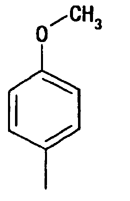 | -H | 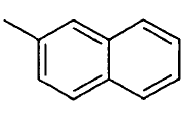 |
| 326 | 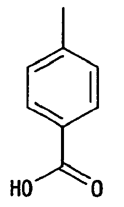 | 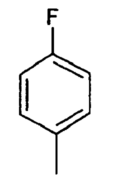 | -H | 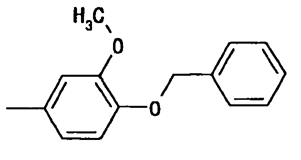 |
| 327 | 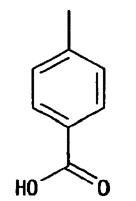 | 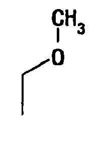 | -H | 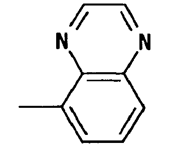 |
| 328 | 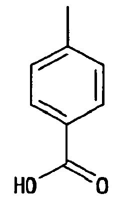 | 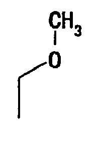 | -H | 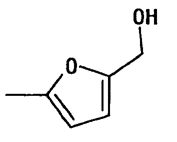 |
| 329 | 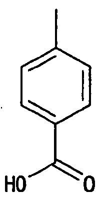 | 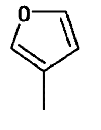 | -H | 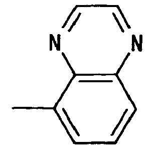 |
| 330 | 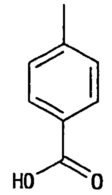 | 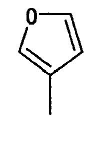 | -H | 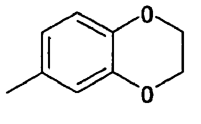 |
| 331 | 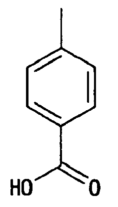 | -CH₃ | -H | 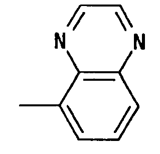 |
| 332 | 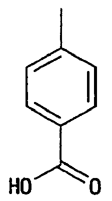 | 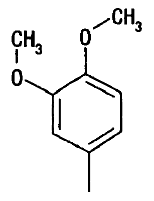 | -H | 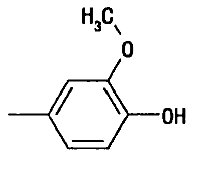 |
| 333 | 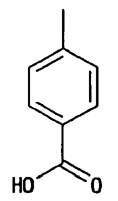 | 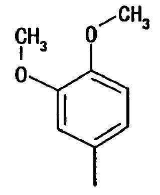 | -H | 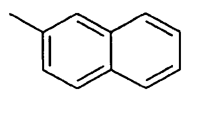 |
| 334 | 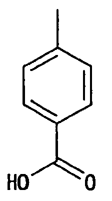 | 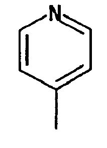 | -H | 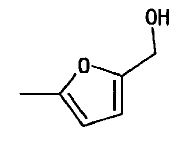 |
| 335 | 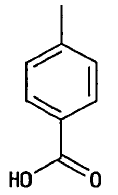 | 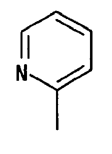 | -H | 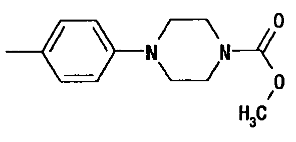 |
| 336 | 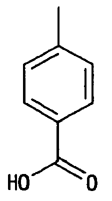 | 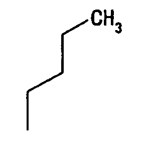 | -H | 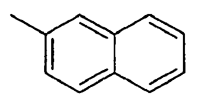 |
| 337 | 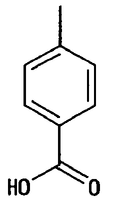 | 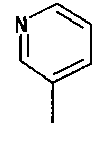 | -H | 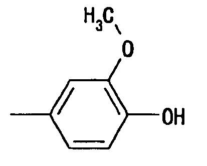 |
| 338 | 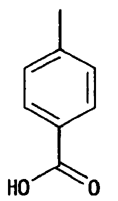 | 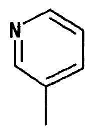 | -H | 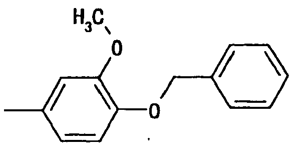 |
| 339 | 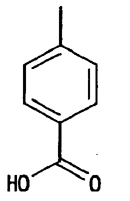 | 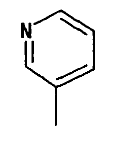 | -H | 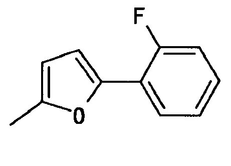 |
| 340 | 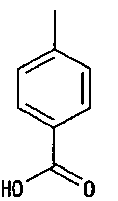 | 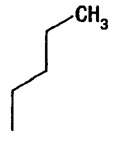 | -H | 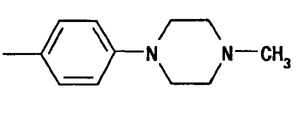 |
| 341 | 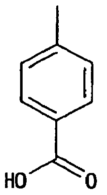 | 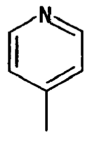 | -H | 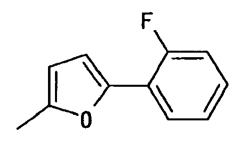 |
| 342 | 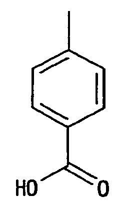 | 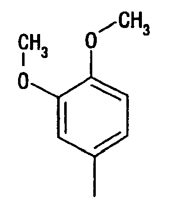 | -H | 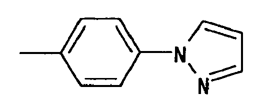 |
| 343 | 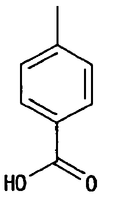 | 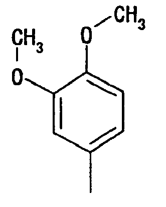 | -H | 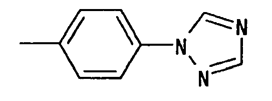 |
| 344 | 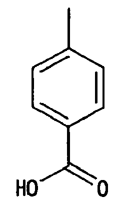 | 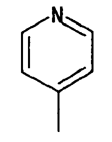 | -H | 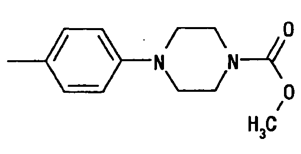 |
| 345 | 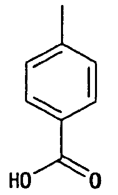 | 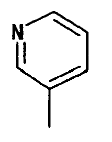 | -H | 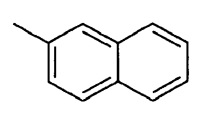 |
| 346 | 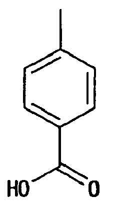 | -CH₃ | -H | 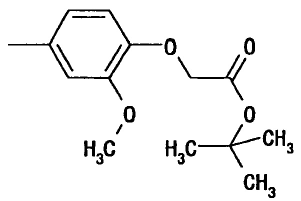 |
| 347 | 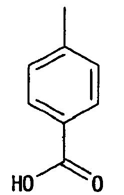 | -CH₃ | -H | 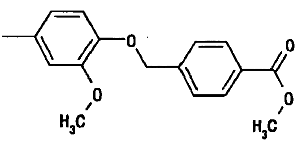 |
| 348 | 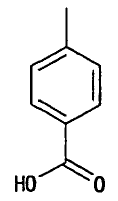 | -CH₃ | -H | 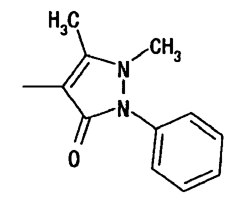 |
| 349 | 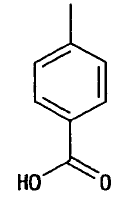 | -CH₃ | -H | 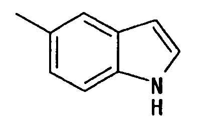 |
| 350 | 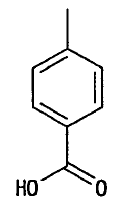 | -CH₃ | -H | 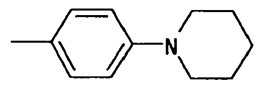 |
| 351 | 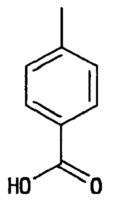 | -CH₃ | -H | 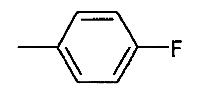 |
| 352 | 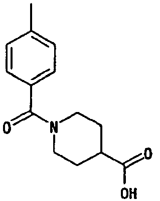 | -CH₃ | -H | 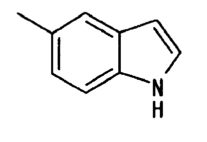 |
| 353 | 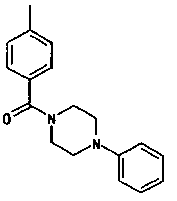 | -CH₃ | -H | 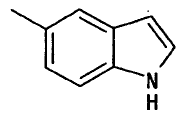 |
| 354 | 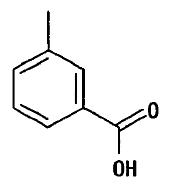 | -CH₃ | -H | 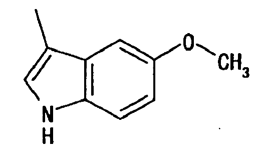 |
| 355 | 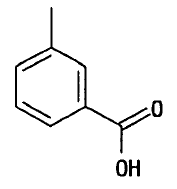 | -CH₃ | -H | 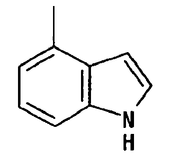 |
| 356 | 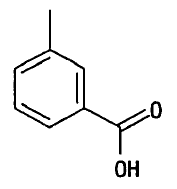 | -CH₃ | -H | 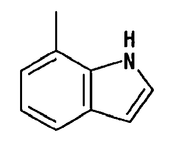 |
| 357 | 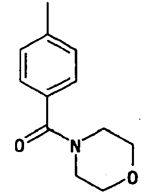 | -CH₃ | -H | 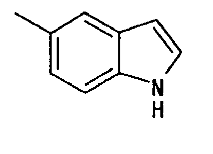 |
| 358 | 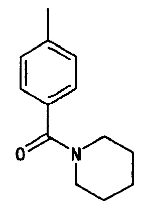 | -CH₃ | -H | 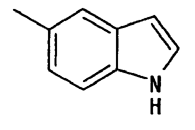 |
| 359 | 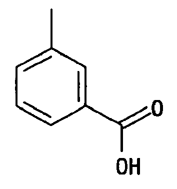 | -CH₃ | -H | 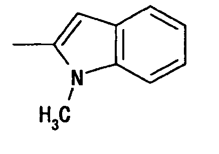 |
| 360 | 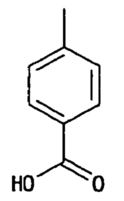 | -CH₃ | -H | 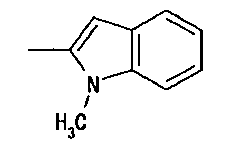 |
| 361 | 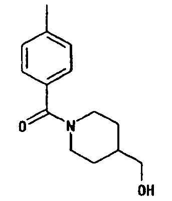 | -CH₃ | -H | 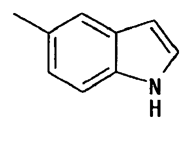 |
| 362 | 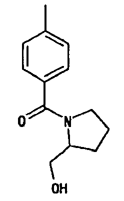 | -CH₃ | -H | 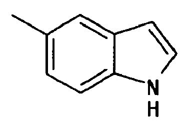 |
| 363 | 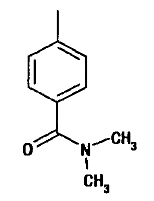 | -CH₃ | -H | 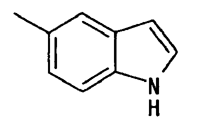 |
| 364 | 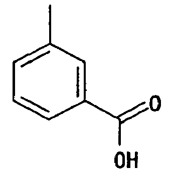 | -CH₃ | -H | 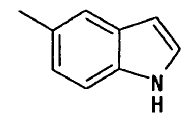 |
| 365 | 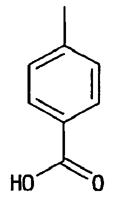 | -CH₃ | -H | 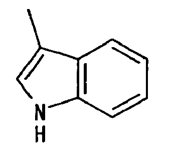 |
| 366 | 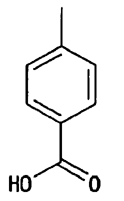 | -CH₃ | -H | 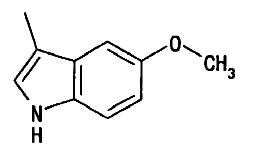 |
| 367 | 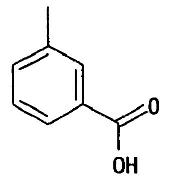 | -CH₃ | -H | 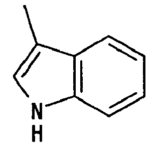 |
| 368 | 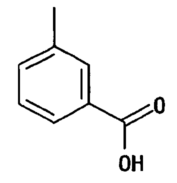 | -CH₃ | -H | 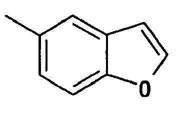 |
| 369 | 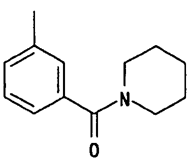 | -CH₃ | -H | 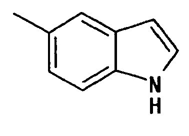 |
| 370 | 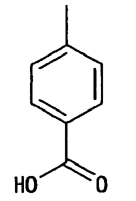 | -CH₃ | -H | 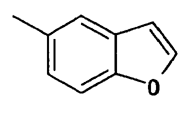 |
| 371 | 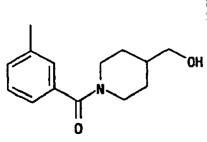 | -CH₃ | -H | 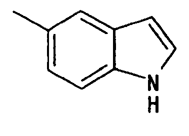 |
| 372 | 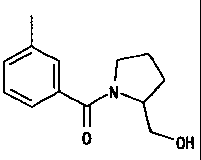 | -CH₃ | -H | 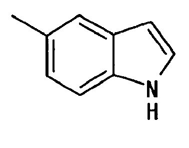 |
| 373 | 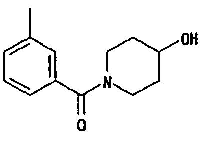 | -CH₃ | -H | 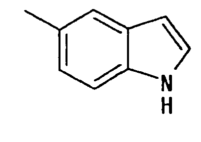 |
| 374 | 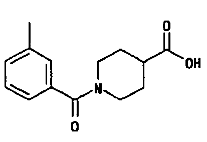 | -CH₃ | -H | 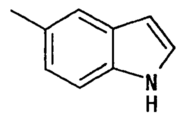 |
| 375 | 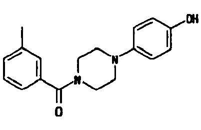 | -CH₃ | -H | 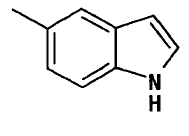 |
| 376 | 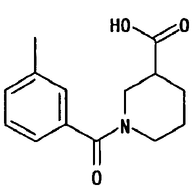 | -CH₃ | -H | 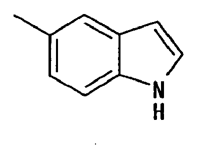 |
| 377 | 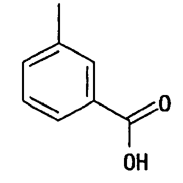 | -CH₃ | -H | 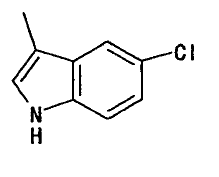 |
| 378 | 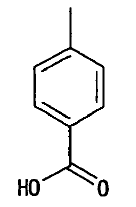 | -CH₃ | -H | 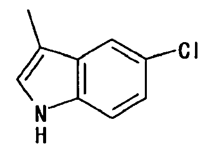 |
| 379 | 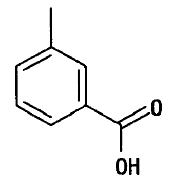 | -CH₃ | -H | 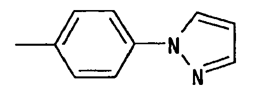 |
| 380 | 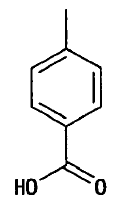 | -CH₃ | -H | 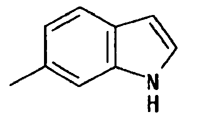 |
| 381 | 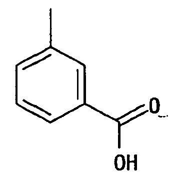 | -CH₃ | -H | 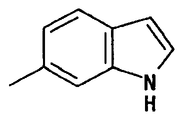 |
| 382 | 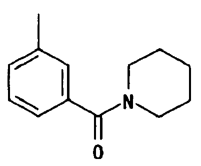 | -CH₃ | -H | 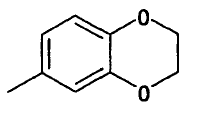 |
| 383 | 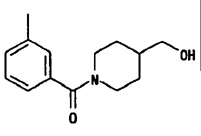 | -CH₃ | -H | 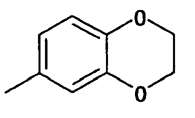 |
| 384 | 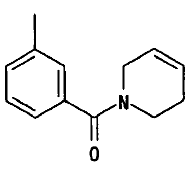 | -CH₃ | -H | 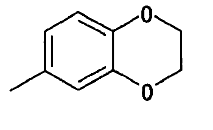 |
| 385 | 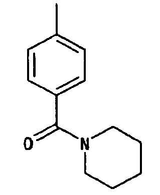 | -CH₃ | -H | 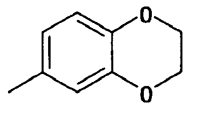 |
| 386 | 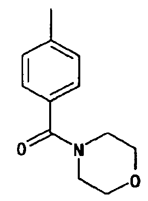 | -CH₃ | -H | 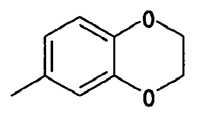 |
| 387 | 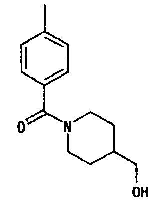 | -CH₃ | -H | 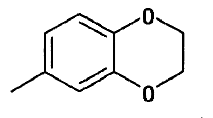 |
| 388 | 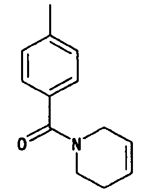 | -CH₃ | -H | 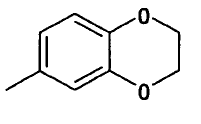 |
| 389 | 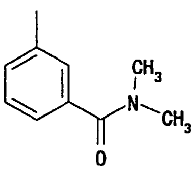 | -CH₃ | -H | 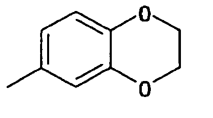 |
| 390 | 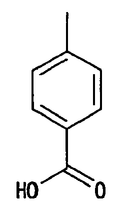 | -CH₃ | -H | 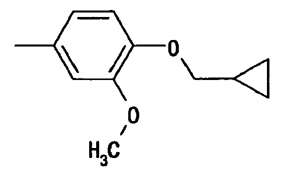 |
| 391 | 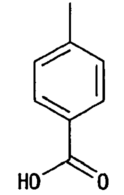 | -CH₃ | -H | 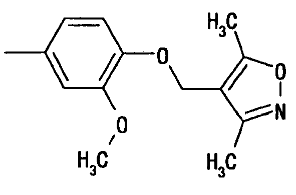 |
| 392 | 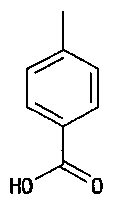 | -CH₃ | -H | 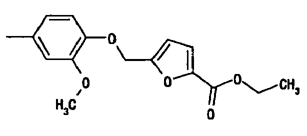 |
| 393 | 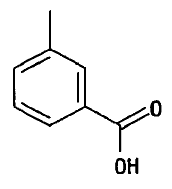 | -CH₃ | -H | 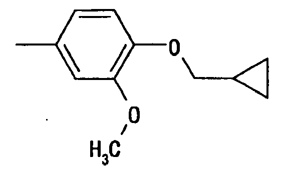 |
| 394 | 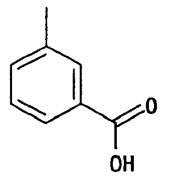 | -CH₃ | -H | 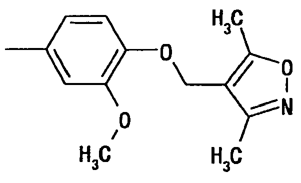 |
| 395 | 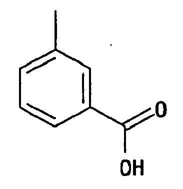 | -CH₃ | -H | 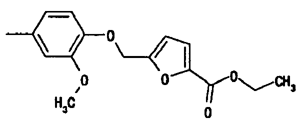 |
| 396 | 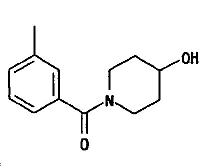 | -CH₃ | -H | 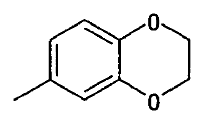 |
| 397 | 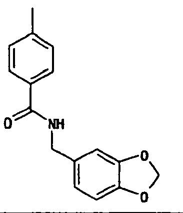 | -CH₃ | -H | 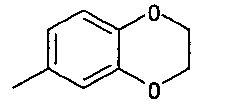 |
| 398 | 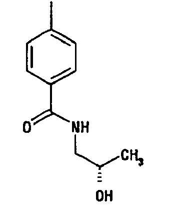 | -CH₃ | -H | 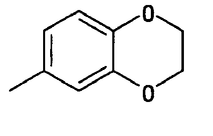 |
| 399 | 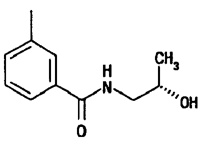 | -CH₃ | -H | 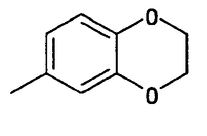 |
| 400 | 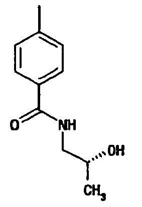 | -CH₃ | -H | 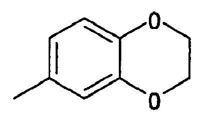 |
| 401 | 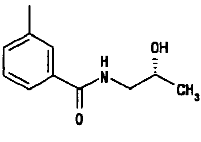 | -CH₃ | -H | 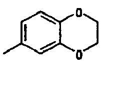 |
| 402 | 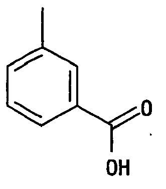 | -CH₃ | -H | 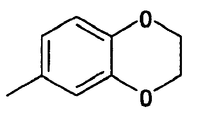 |
| 403 | 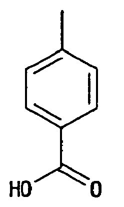 | -CH₃ | -H | 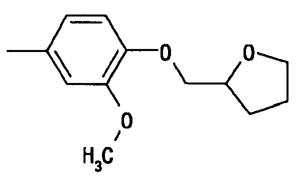 |
| 404 | 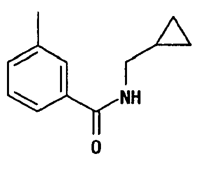 | -CH₃ | -H | 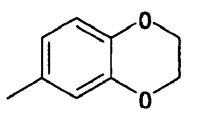 |
| 405 | 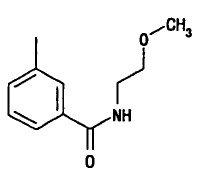 | -CH₃ | -H | 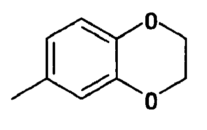 |
| 406 | 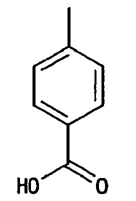 | -CH₃ | -H | 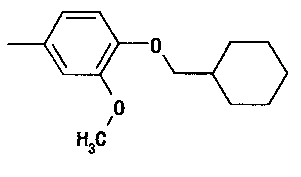 |
| 407 | 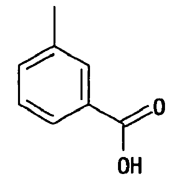 | -CH₃ | -H | 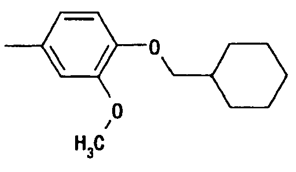 |
| 408 | 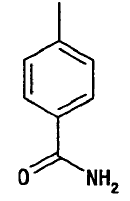 | -CH₃ | -H | 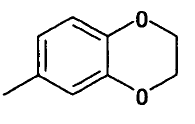 |
| 409 | 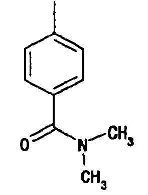 | -CH₃ | -H | 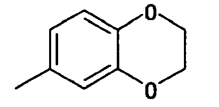 |
| 410 | 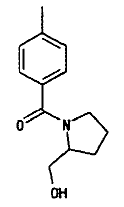 | -CH₃ | -H | 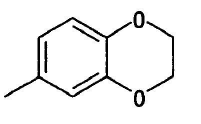 |
| 411 | 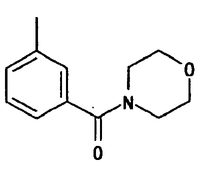 | -CH₃ | -H | 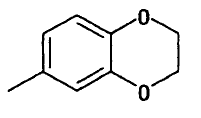 |
| 412 | 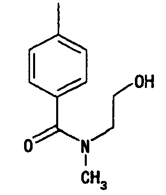 | -CH₃ | -H | 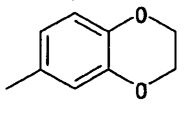 |
| 413 | 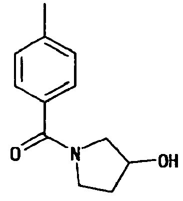 | -CH₃ | -H | 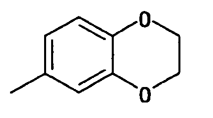 |
| 414 | 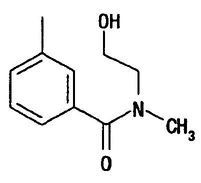 | -CH₃ | -H | 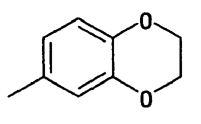 |
| 415 | 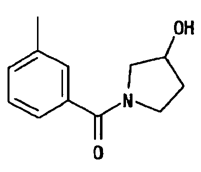 | -CH₃ | -H | 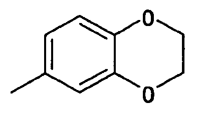 |
| 416 | 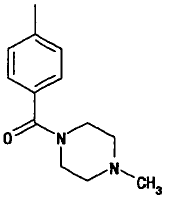 | -CH₃ | -H | 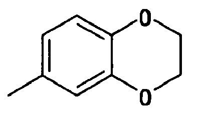 |
| 417 | 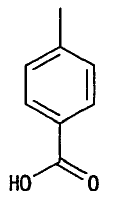 | -CH₃ | -H | 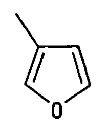 |
| 418 | 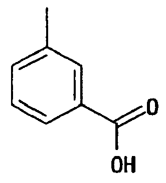 | -CH₃ | -H | 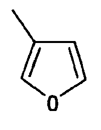 |
| 419 | 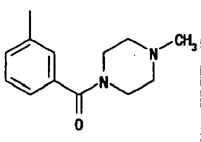 | -CH₃ | -H | 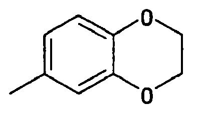 |
| 420 | 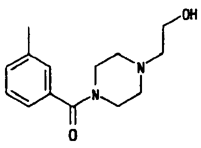 | -CH₃ | -H | 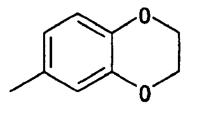 |
| 421 | 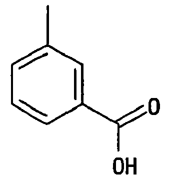 | -CH₃ | -H | 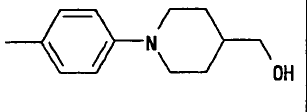 |
| 422 | 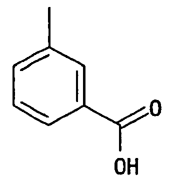 | -CH₃ | -H | 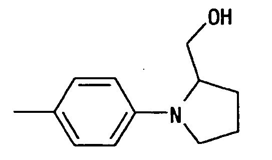 |
| 423 | 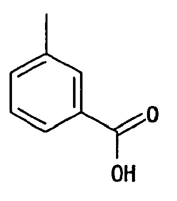 | -CH₃ | -H | 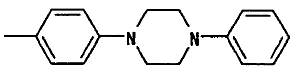 |
| 424 | 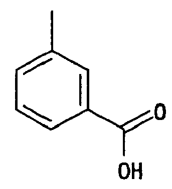 | -CH₃ | -H | 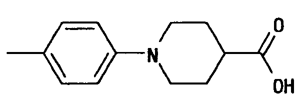 |
| 425 | 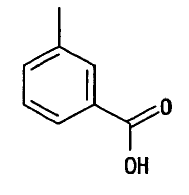 | -CH₃ | -H | 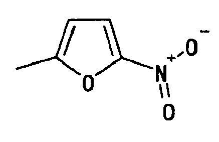 |
| 426 | 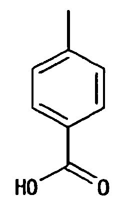 | -CH₃ | -H | 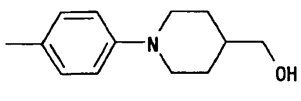 |
| 427 | 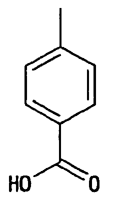 | -CH₃ | -H | 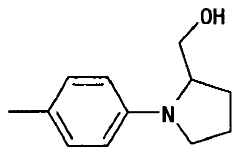 |
| 428 | 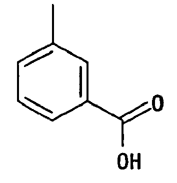 | -CH₃ | -H | 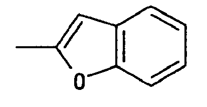 |
| 429 | 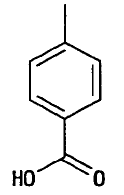 | -CH₃ | -H | 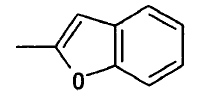 |
| 430 | 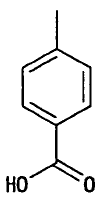 | -CH₃ | -H | 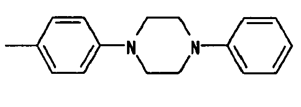 |
| 431 | 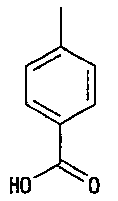 | -CH₃ | -H | 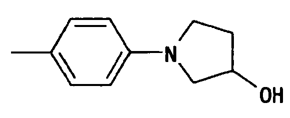 |
| 432 | 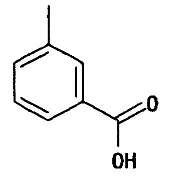 | -CH₃ | -H | 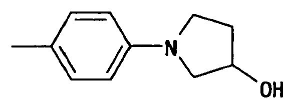 |
| 433 | 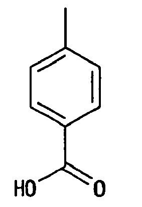 | 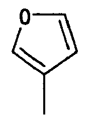 | -H | 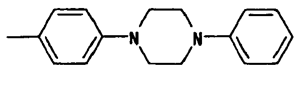 |
| 434 | 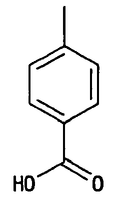 | 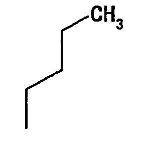 | -H | 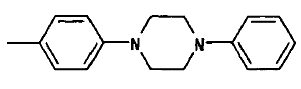 |
| 435 | 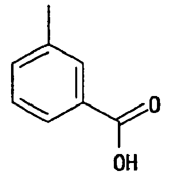 | -CH₃ | -H | 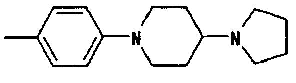 |
| 436 | 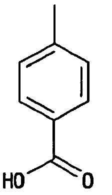 | -CH₃ | -H | 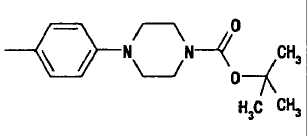 |
| 437 | 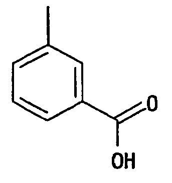 | -CH₃ | -H | 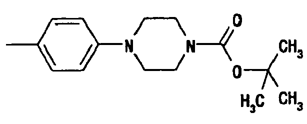 |
| 438 | 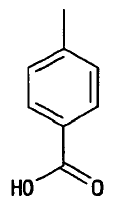 | 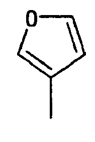 | -H | 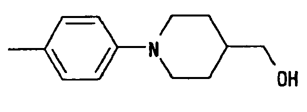 |
| 439 | 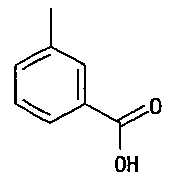 | -CH₃ | -H | 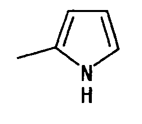 |
| 440 | 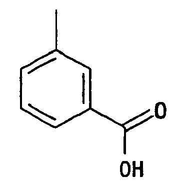 | -CH₃ | -H | 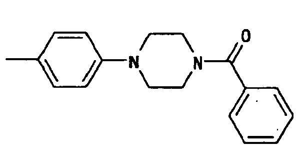 |
| 441 | 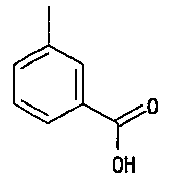 | -CH₃ | -H | 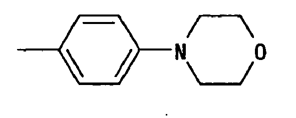 |
| 442 | 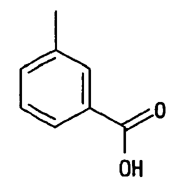 | -CH₃ | -H | 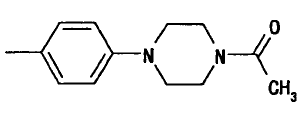 |
| 443 | 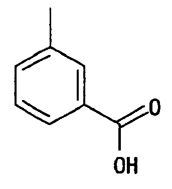 | 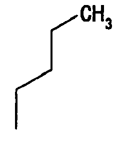 | -H | 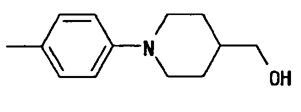 |
| 444 | 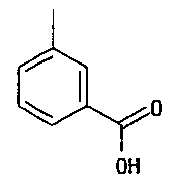 | -CH₃ | -H | 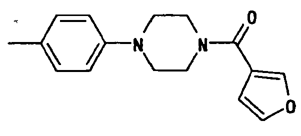 |
| 445 | 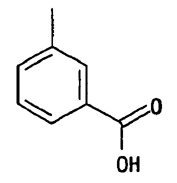 | -CH₃ | -H | 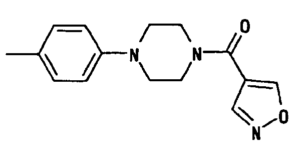 |
| 446 | 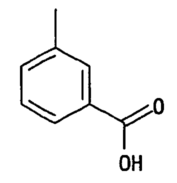 | -CH₃ | -H | 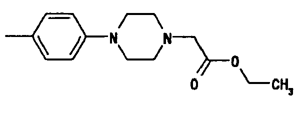 |
| 447 | 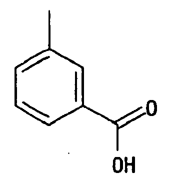 | -CH₃ | -H | 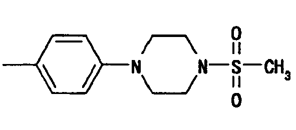 |
| 448 | 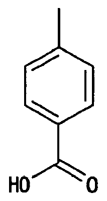 | -CH₃ | -H | 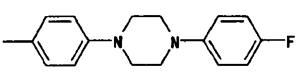 |
| 449 | 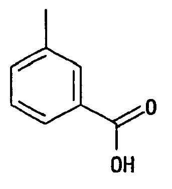 | -CH₃ | -H | 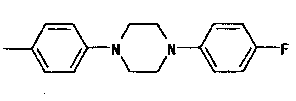 |
| 450 | 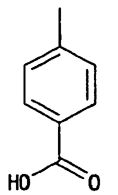 | -CH₃ | -H | 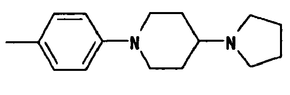 |
| 451 | 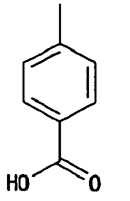 | -CH₃ | -H | 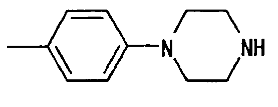 |
| 452 | 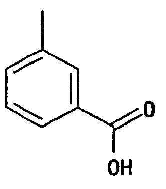 | -CH₃ | -H | 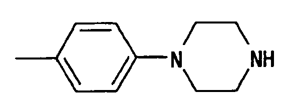 |
| 453 | 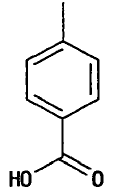 | -CH₃ | -H | 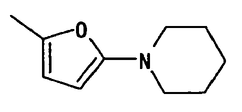 |
| 454 | 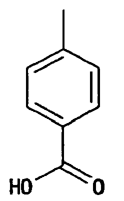 | -CH₃ | -H | 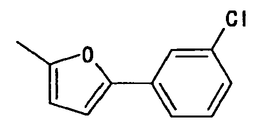 |
| 455 | 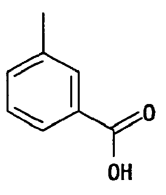 | -CH₃ | -H | 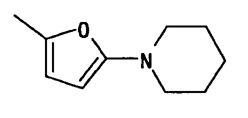 |
| 456 | 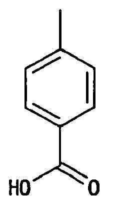 | -CH₃ | -H | 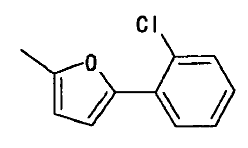 |
| 457 | 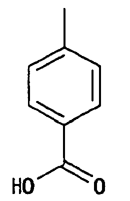 | -CH₃ | -H | 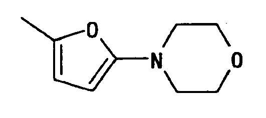 |
| 458 | 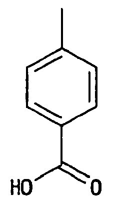 | -CH₃ | -H | 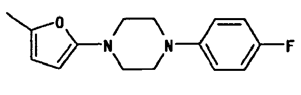 |
| 459 | 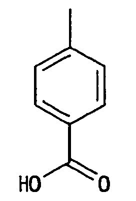 | -CH₃ | -H | 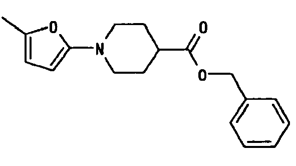 |
| 460 | 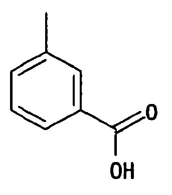 | -CH₃ | -H | 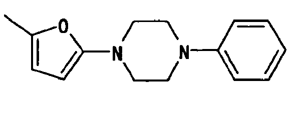 |
| 461 | 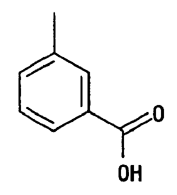 | -CH₃ | -H | 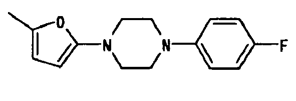 |
| 462 | 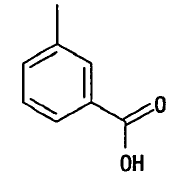 | -CH₃ | -H | 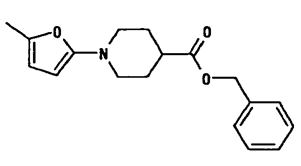 |
| 463 | 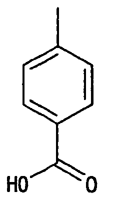 | -CH₃ | -H | 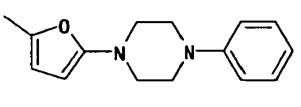 |
| 464 | 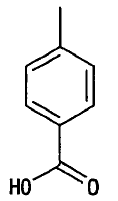 | 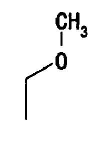 | -H | 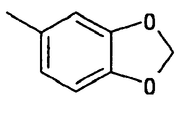 |
| 465 | 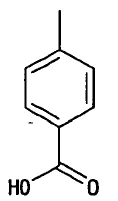 | 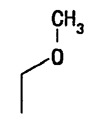 | -H | 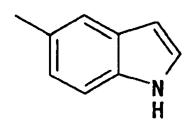 |
| 466 | 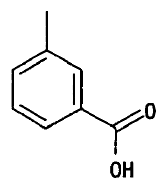 | -CH₃ | -H | 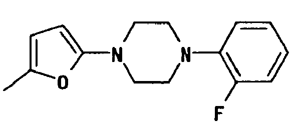 |
| 467 | 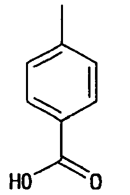 | -CH₃ | -H | 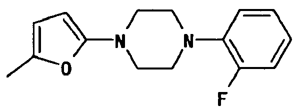 |
| 468 | 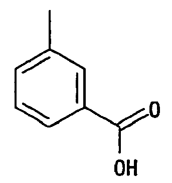 | 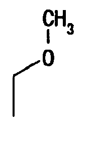 | -H | 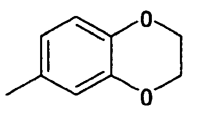 |
| 469 | 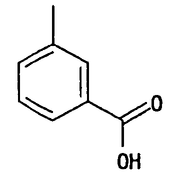 | 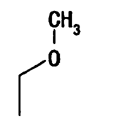 | -H | 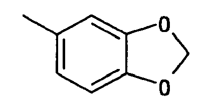 |
| 470 | 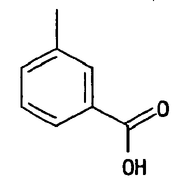 | 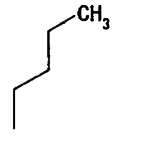 | -H | 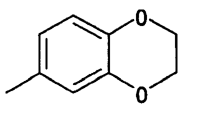 |
| 471 | 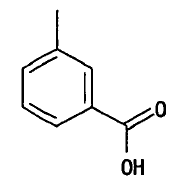 | 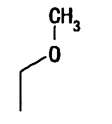 | -H | 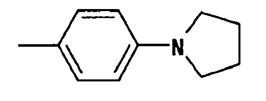 |
| 472 | 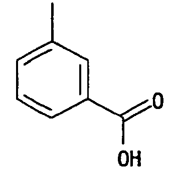 | -CH₃ | -H | 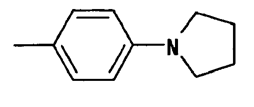 |
| 473 | 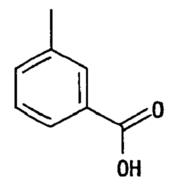 | 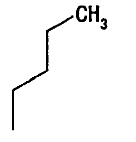 | -H | 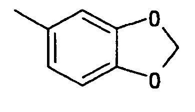 |
| 474 | 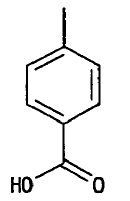 | 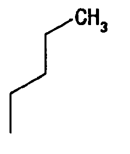 | -H | 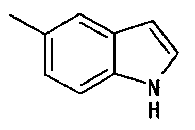 |
| 475 | 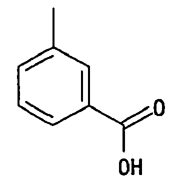 | 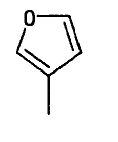 | -H | 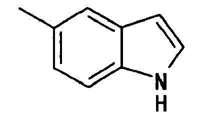 |
| 476 | 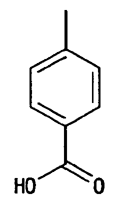 | 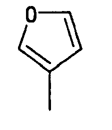 | -H | 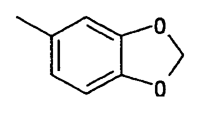 |
| 477 | 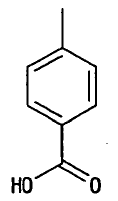 | 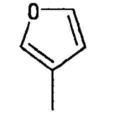 | -H | 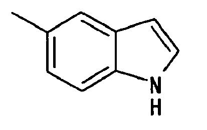 |
| 478 | 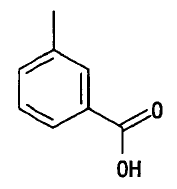 | 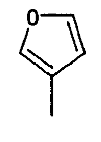 | -H | 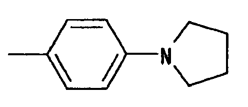 |
| 479 | 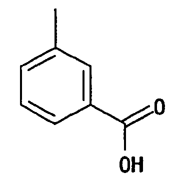 | 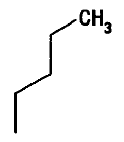 | -H | 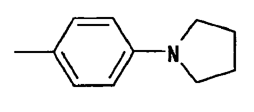 |
| 480 | 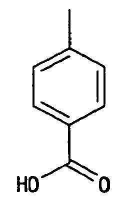 | 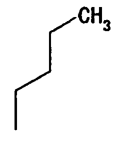 | -H | 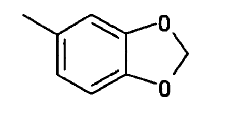 |
| 481 | 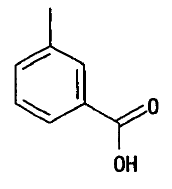 | 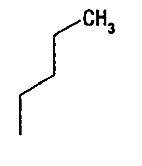 | -H | 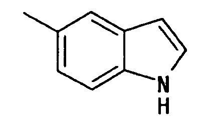 |
| 482 | 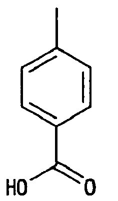 | 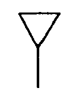 | -H | 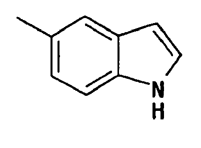 |
| 483 | 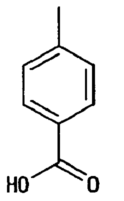 | 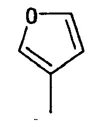 | -H | 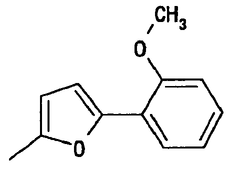 |
| 484 | 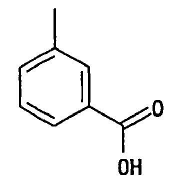 | 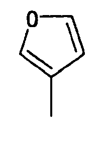 | -H | 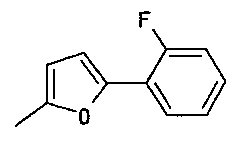 |
| 485 | 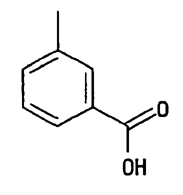 | 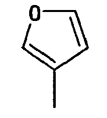 | -H | 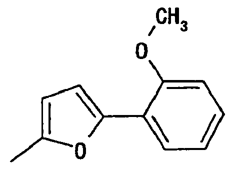 |
| 486 | 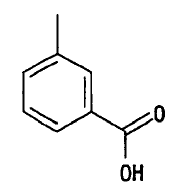 | -CH₃ | -H | 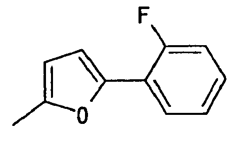 |
| 487 | 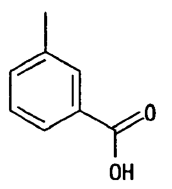 | -CH₃ | -H | 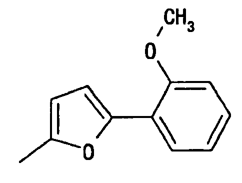 |
| 488 | 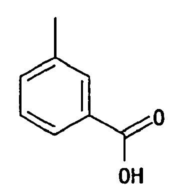 | 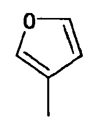 | -H | 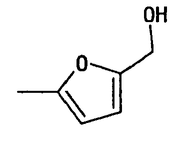 |
| 489 | 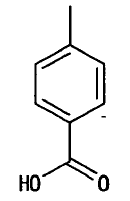 | 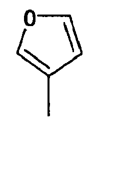 | -H | 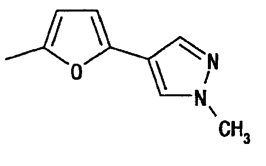 |
| 490 | 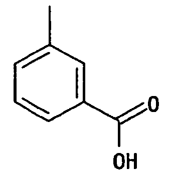 | 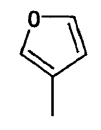 | -H | 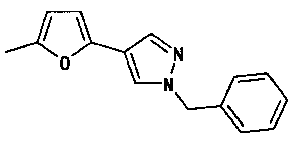 |
| 491 | 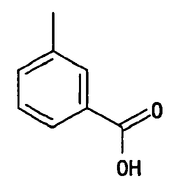 | 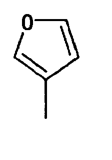 | -H | 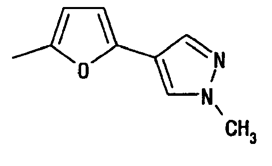 |
| 492 | 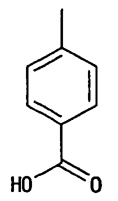 | 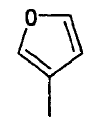 | -H | 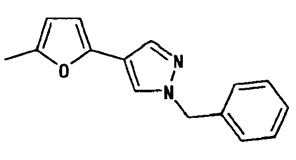 |
| 493 | 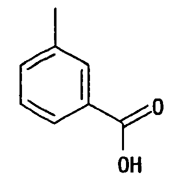 | -CH₃ | -H | 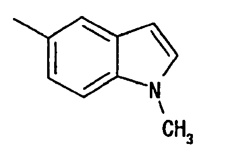 |
| 494 | 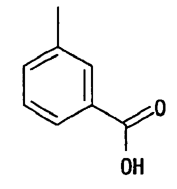 | -CH₃ | -H | 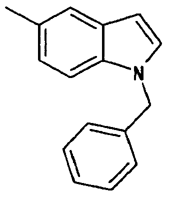 |
| 495 | 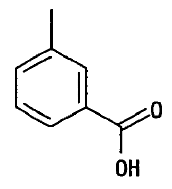 | -CH₃ | -H | 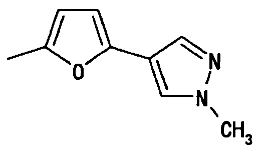 |
| 496 | 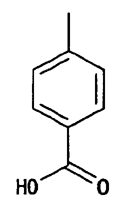 | -CH₃ | -H | 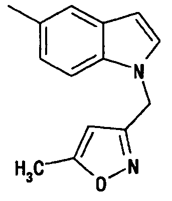 |
| 497 | 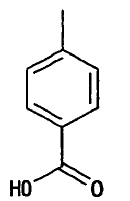 | -CH₃ | -H | 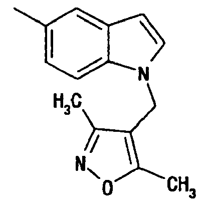 |
| 498 | 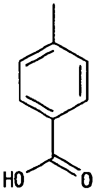 | -CH₃ | -H | 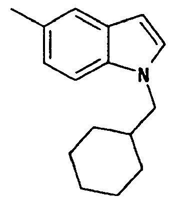 |
| 499 | 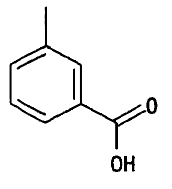 | -CH₃ | -H | 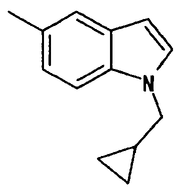 |
| 500 | 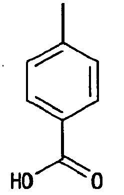 | -CH₃ | -H | 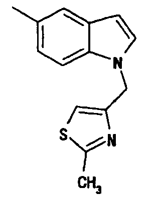 |
| 501 | 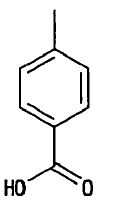 | -CH₃ | -H | 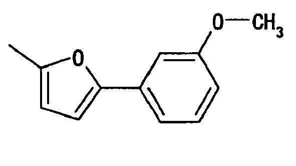 |
| 502 | 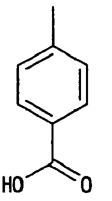 | -CH₃ | -H | 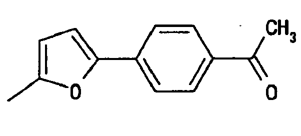 |
| 503 | 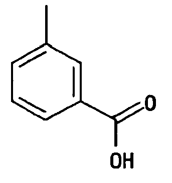 | -CH₃ | -H | 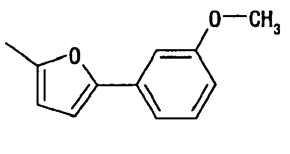 |
| 504 | 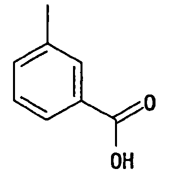 | -CH₃ | -H | 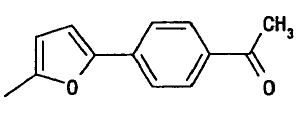 |
| 505 | 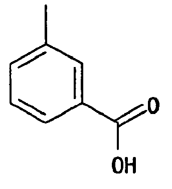 | 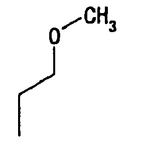 | -H | 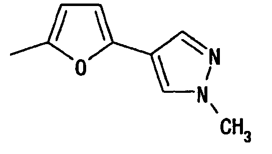 |
| 506 | 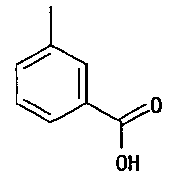 | -CH₃ | -H | 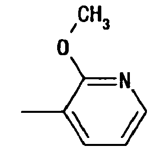 |
| 507 | 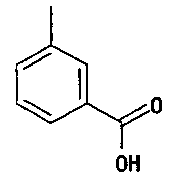 | -CH₃ | -H | 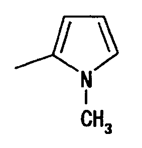 |
| 508 | 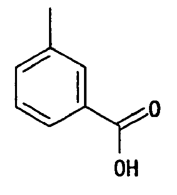 | -CH₃ | -H | 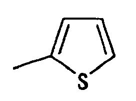 |
| 509 | 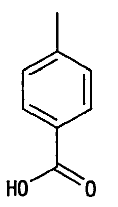 | -CH₃ | -H | 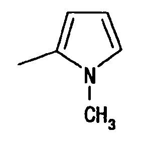 |
| 510 | 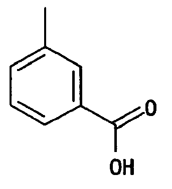 | 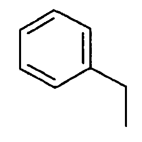 | -H | 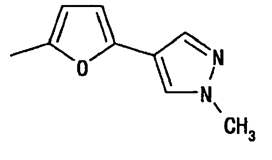 |
| 511 | 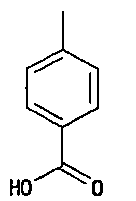 | -CH₃ | -H | 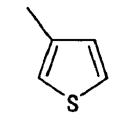 |
| 512 | 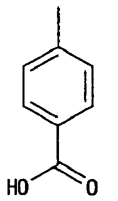 | 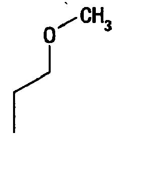 | -H | 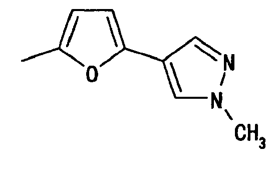 |
| 513 | 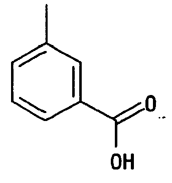 | -CH₃ | -H | 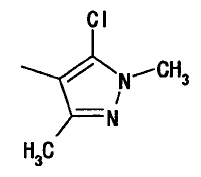 |
| 514 | 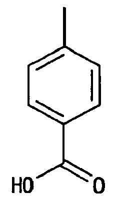 | -CH₃ | -H | 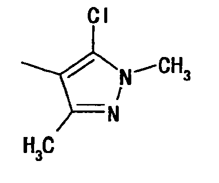 |
| 515 | 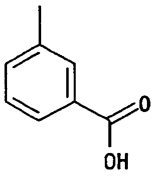 | -CH₃ | -H | 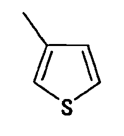 |
| 516 | 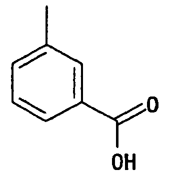 | -CH₃ | -H | 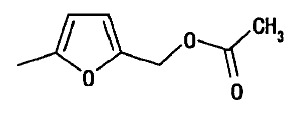 |
| 517 | 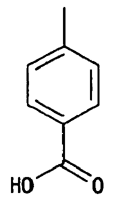 | -CH₃ | -H | 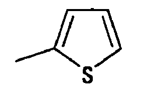 |
| 518 | 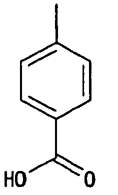 | 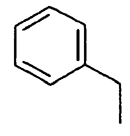 | -H | 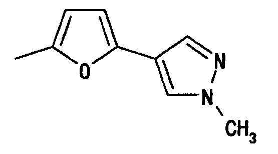 |
| 519 | 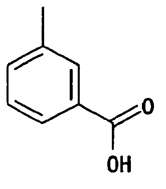 | 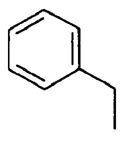 | -H | 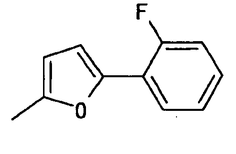 |
| 520 | 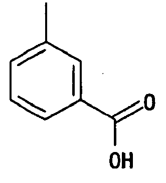 | -CH₃ | -H | 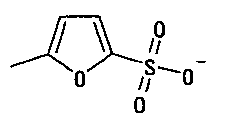 |
| 521 | 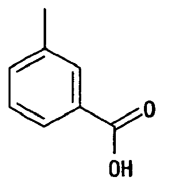 | -CH₃ | -H | 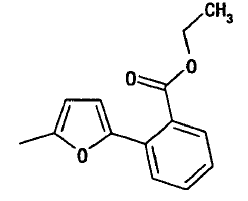 |
| 522 | 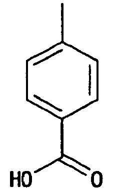 | 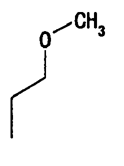 | -H | 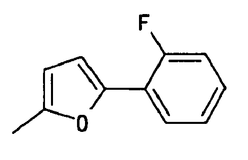 |
| 523 | 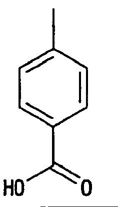 | -CH₃ | -H | 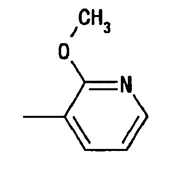 |
| 524 | 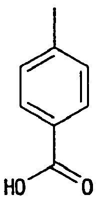 | -CH₃ | -H | 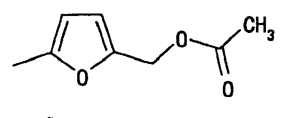 |
| 525 | 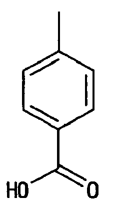 | -CH₃ | -H | 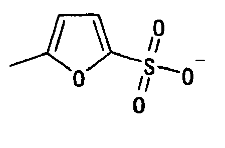 |
| 526 | 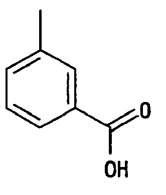 | 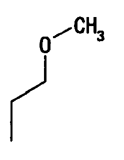 | -H | 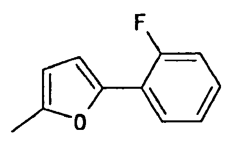 |
| 527 | 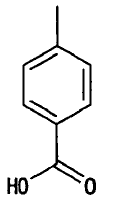 | -CH₃ | -H | 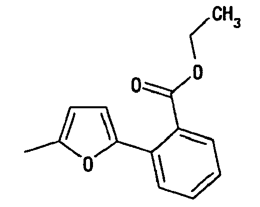 |
| 528 | 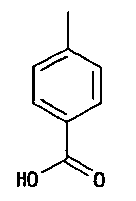 | -CH₃ | -H | 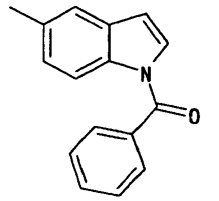 |
| 529 | 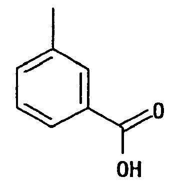 | -CH₃ | -H | 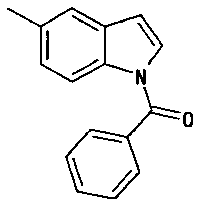 |
| 530 | 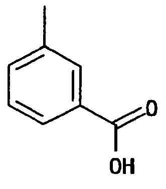 | -CH₃ | -H | 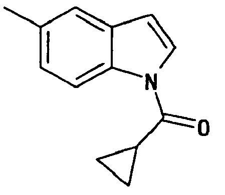 |
| 531 | 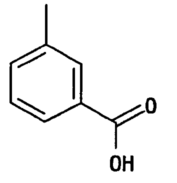 | -CH₃ | -H | 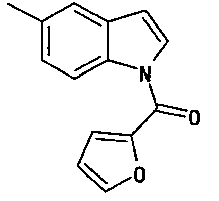 |
| 532 | 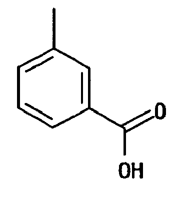 | -CH₃ | -H | 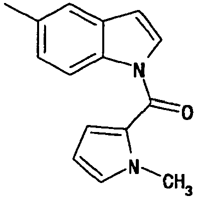 |
| 533 | 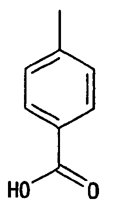 | -CH₃ | -H | 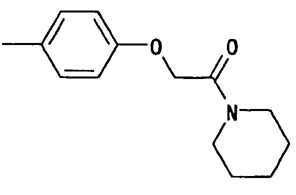 |
| 534 | 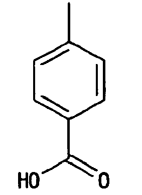 | -CH₃ | -H | 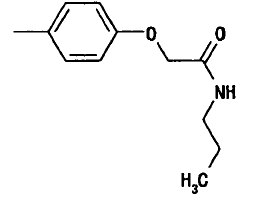 |
| 535 | 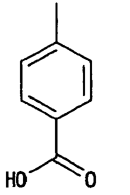 | -CH₃ | -H | 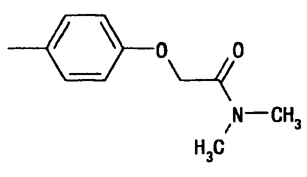 |
| 536 | 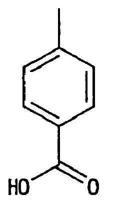 | -CH₃ | -H | 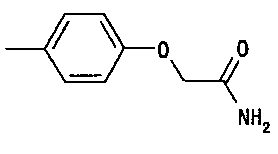 |
| 537 | 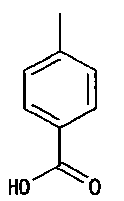 | -CH₃ | -H | 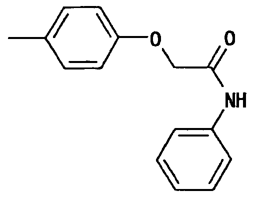 |
| 538 | 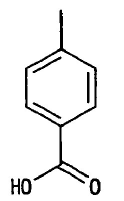 | -CH₃ | -H | 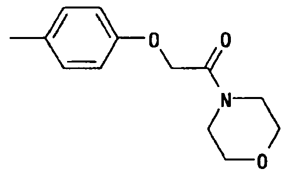 |
| 539 | 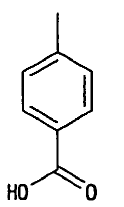 | -CH₃ | -H | 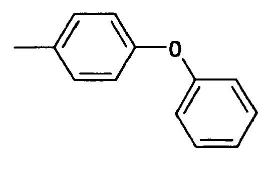 |
| 540 | 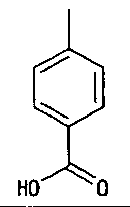 | -CH₃ | -H | 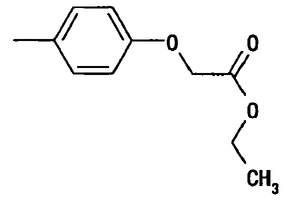 |
| 541 | 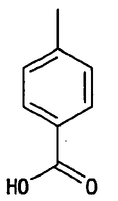 | -CH₃ | -H | 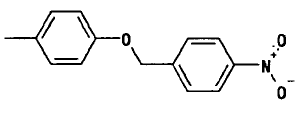 |
| 542 | 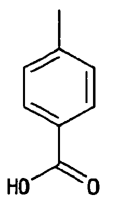 | -CH₃ | -H | 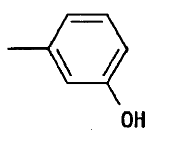 |
| 543 | 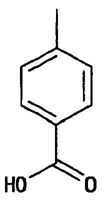 | -CH₃ | -H | 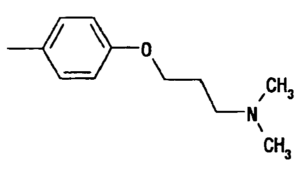 |
| 544 | 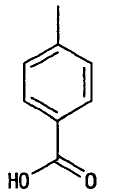 | -CH₃ | -H | 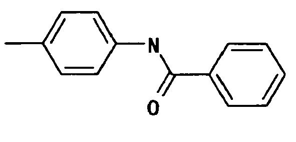 |
| 545 | 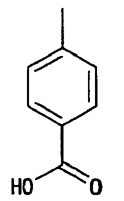 | -CH₃ | -CH₃ | 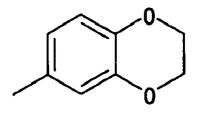 |
| 546 | 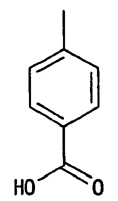 | -CH₃ | -CH₃ | 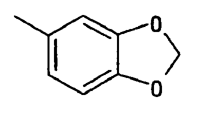 |
| 547 | 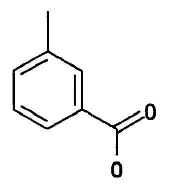 | -CH₃ | -CH₃ | 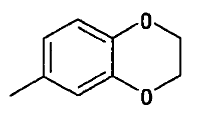 |
| 548 | 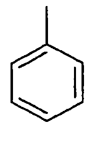 | 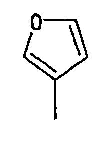 | -H | 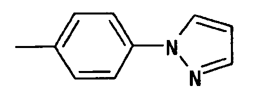 |
| 549 | 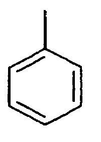 | -CH₃ | -H | 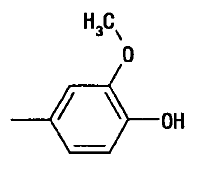 |
| 550 | 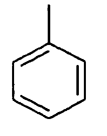 | -CH₃ | -H | 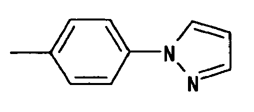 |
| 551 | 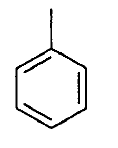 | -CH₃ | -H | 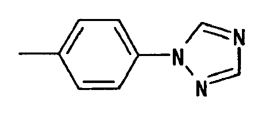 |
| 552 | 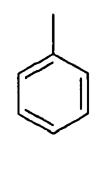 | 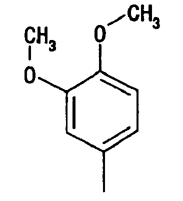 | -H | 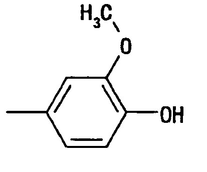 |
| 553 | 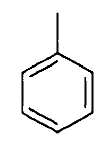 | 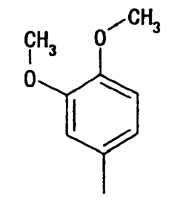 | -H | 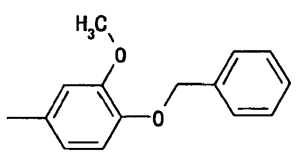 |
| 554 | 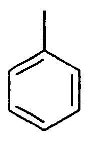 | 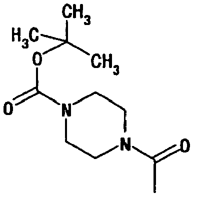 | -H | 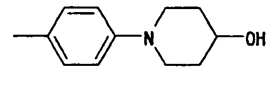 |
| 555 | 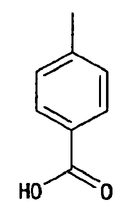 | 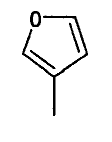 | -H | 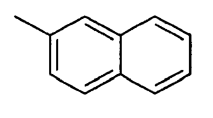 |
| 556 | 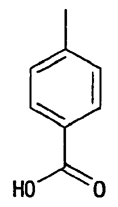 | 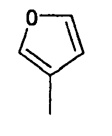 | -H | 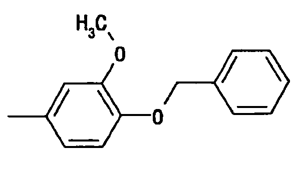 |
| 557 | 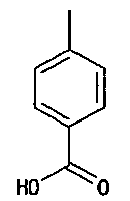 | 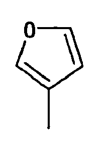 | -H | 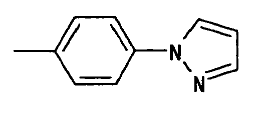 |
| 558 | 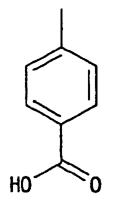 | 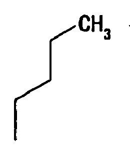 | -H | 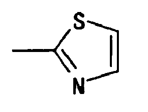 |
| 559 | 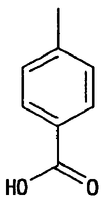 | 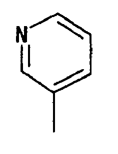 | -H | 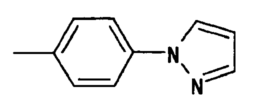 |
| 560 | 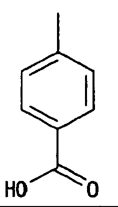 | 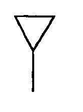 | -H | 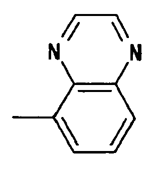 |
| 561 | 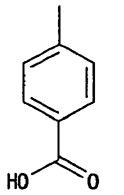 | 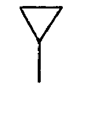 | -H | 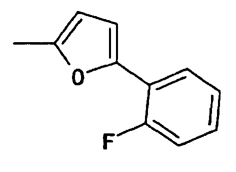 |
| 562 | 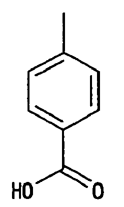 | 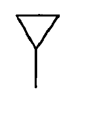 | -H | 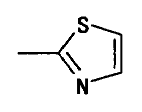 |
| 563 | 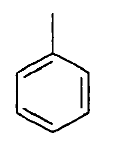 | 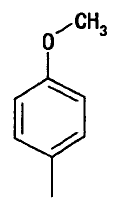 | -H | 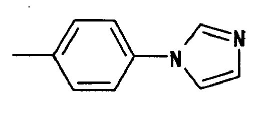 |
| 564 | 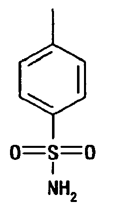 | -CH₃ | -H | 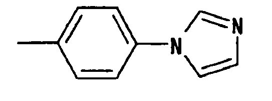 |
| 565 | 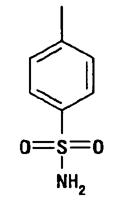 | -CH₃ | -H | 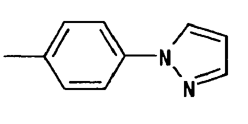 |
| 566 | 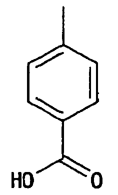 | 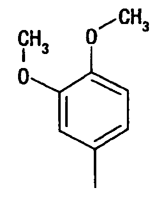 | -H | 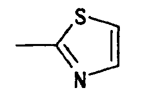 |
| 567 | 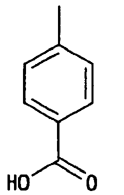 | 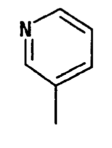 | -H | 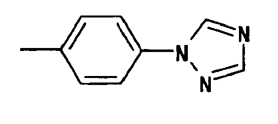 |
| 568 | 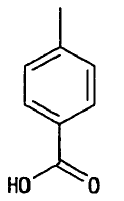 | 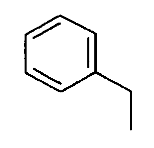 | -H | 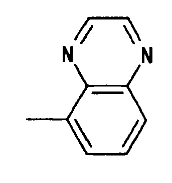 |
| 569 | 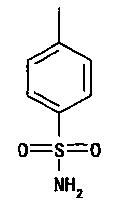 | -CH₃ | -H | 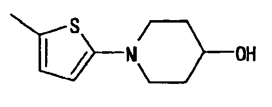 |

Among these exemplary compounds, preferred examples include the above-mentioned exemplary compounds, Nos. 2, 3, 5, 6, 7, 9, 10, 11, 12, 14, 15, 16, 19, 22, 23, 24, 25, 26, 27, 29, 30, 31, 33, 35, 37, 40, 42, 44, 45, 46, 48, 50, 51, 52, 54, 56, 58, 59, 60, 62, 64, 66, 67, 69, 70, 71, 72, 73, 75, 77, 78, 79, 80, 88, 89, 90, 93, 94, 95, 97, 98, 99, 100, 105, 106, 108, 109, 110, 111, 115, 116, 117, 118, 119, 120, 121, 122, 125, 126, 127, 128, 129, 130, 132, 133, 134, 137, 140, 141, 142, 143, 144, 145, 146, 147, 148, 149, 150, 151, 153, 154, 155, 156, 157, 158, 159, 160, 161, 162, 164, 168, 170, 171, 172, 173, 174, 176, 177, 178, 179, 180, 183, 184, 185, 188, 190, 195, 196, 197, 198, 199,200,201,202,203,204,205,206,208,209,210,211,212,213,214, 215,216,217,218,219,220,221,222,223,224,225,226,227,228,229,230,231,232,233, 234,235,236,237,238,239,240,241,242,243,244,245,246,247,248,249,250,251,252, 253,254,255,256,257,258,259,261,262,263,264,265,266,267,268,269,270,271,272, 274,275,276,277,278,279,280,281,282,283,285,286,287,288,289,290,291,292,293, 294,295,296,297,298,300,301,302,303,305,306,307,308,309,310,312,313,314,315, 316,317,318,319,321,322,323,324,325,326,327,328,329,330,331,332,333,334,335, 336,337,339,340,341,342,343,344,345,346,347,349,350,351,352,353,355,356,357, 358,359,360,361,362,363,364,368,369,370,371,372,373,374,375,376,377,378,379, 380,381,383,386,387,390,391,392,393,394,395,397,398,399,400,401,402,404,405, 406, 407, 410, 411, 412, 413, 414, 418, 421, 422, 423, 424, 425, 426, 428, 429, 430, 433, 434, 435, 436, 437, 438, 440, 441, 442, 443, 444, 445, 446, 447, 448, 449, 450, 451, 452, 454, 456, 464,465,470,471,472,473,474,475,476,477,478,479,480,481,482,483,484,485,486, 487,488,489,490,491,492,493,494,495,496,497,498,499,500,501,502,503,504,505, 506,508,510,511,512,513,514,515,516,517,518,519,520,522,524,525,526,527,528, 529, 530, 531, 532, 533, 534, 535, 536, 537, 538, 539, 540, 541, 542, 543, and 544.

More preferably, examples include the above-mentioned exemplary compounds, Nos. 2, 3, 6, 9, 11, 12, 14, 15, 16, 19, 22, 24, 25, 27, 29, 30, 33, 37, 50, 54, 56, 58, 59, 69, 70, 71, 73, 75, 77, 78, 94, 97, 98, 99, 108, 110, 115, 116, 117, 118, 119, 120, 121, 125, 126, 127, 128, 129, 130, 133, 134, 137, 140, 141, 144, 145, 146, 147, 148, 149, 150, 151, 154, 156, 157, 159, 164, 168, 170, 171, 176, 177, 179, 180, 183, 184, 185, 188, 190, 195, 196, 199, 200, 201, 202, 203, 204, 208, 209, 210, 211, 212, 213, 214, 215, 216, 218, 220, 221, 222, 223, 224, 225, 226, 227, 228, 229, 230, 231, 232, 233, 234, 235, 236, 237, 238, 239, 242, 243, 244, 245, 246, 247, 248, 249, 250, 252, 253, 254, 255, 256, 258, 259, 261, 262, 263, 264, 265, 266, 267, 268, 269, 271, 272, 274, 275, 277, 279, 280, 281, 283, 285, 286, 287, 288, 289, 290, 291, 292, 295, 296, 297, 298, 300, 301, 302, 303, 305, 306, 307, 308, 310, 312, 313, 314, 315, 316, 317, 318, 319, 321, 322, 323, 324, 325, 326, 327, 328, 329, 330, 331, 332, 333, 334, 335, 336, 337, 339, 340, 341, 345, 346, 347, 349, 350, 351, 352, 353, 355, 356, 357, 358, 359, 360, 361, 362, 363, 364, 368, 369, 370, 371, 372, 373, 374, 375, 376, 377, 378, 379, 380, 381, 386, 387, 390, 391, 392, 393, 394, 397, 401, 402, 406, 407, 410, 411, 412, 413, 414, 418, 421, 422, 423, 424, 425, 426, 428, 429, 430, 433, 434, 435, 436, 437, 438, 440, 441, 442, 443, 444, 445, 447, 448, 449, 451, 452, 454, 456, 464, 465, 470, 471, 472, 473, 474, 475, 476, 477, 478, 479, 480, 481, 482, 483, 484, 485, 486, 488, 489, 490, 491, 492, 493, 494, 495, 496, 497, 498, 499, 500, 501, 502, 503, 504, 505, 512, 518, 519, 520, 522, 525, 527, 528, 529, 530, 531, 532, 533, 534, 535, 536, 537, 538, 539, 540, 541, 542, 543, 544, and 556.

Even more preferably, examples include the above-mentioned exemplary compounds, Nos. 11, 12, 14, 15, 16, 19, 27, 58, 71, 77, 94, 110, 115, 117, 119, 120, 127, 128, 129, 133, 134, 140, 146, 147, 148, 149, 150, 151, 154, 164, 168, 170, 176, 185, 195, 196, 199, 201, 208, 210, 213, 216, 220, 224, 225, 226, 227, 228, 229, 230, 231, 233, 234, 235, 236, 238, 239, 242, 243, 244, 245, 246, 247, 248, 250, 252, 253, 254, 255, 258, 259, 261, 262, 266, 267, 272, 277, 279, 281, 283, 287, 290, 296, 298, 300, 301, 302, 303, 305, 308, 312, 313, 314, 315, 316, 318, 319, 321, 322, 323, 324, 325, 326, 327, 328, 330, 334, 340, 346, 347, 349, 350, 352, 353, 355, 356, 357, 358, 359, 360, 361, 362, 364, 368, 369, 370, 371, 372, 373, 374, 375, 376, 377, 378, 379, 380, 381, 390, 391, 392, 410, 411, 413, 421, 423, 424, 425, 429, 430, 433, 434, 438, 440, 441, 442, 443, 444, 445, 447, 448, 449, 452, 454, 456, 465, 474, 475, 476, 477, 480, 481, 482, 483, 484, 485, 489, 490, 491, 492, 493, 494, 495, 496, 497, 498, 499, 500, 501, 502, 503, 519, 522, 527, 528, 529, 530, 531, 532, 533, 536, 537, 538, 539, 540, 541, 542, 543, 544, and 556.

### [General synthetic methods]

Representative methods for producing the compounds of the present invention represented by general formula (I) are shown below. As indicated in Scheme 1, compounds of the present invention represented by general formula (I) can be produced by performing an aldol reaction using tetrazol (VI) and aldehyde (VII) in a solvent under the presence of a suitable acid or base, and a subsequent dehydration reaction.

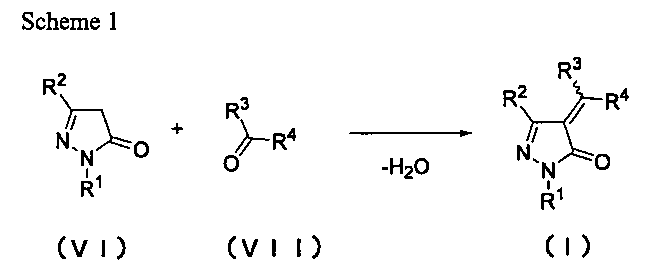

In the formulas, R¹ to R⁴ have the same meanings as described above.

The aldol reaction performed under the presence of an acid or a base, and the subsequent dehydration reaction shown in Scheme 1 can be performed by generally known methods.

Examples of the aforementioned solvent include alcoholic solvents such as methanol, ethanol, and *n*-propanol; etheric solvents such as diethyl ether, tetrahydrofuran, and 1,4-dioxane; and aprotic solvents such as dimethylformamide, dimethylsulfoxide, and acetonitrile. Of them, ethanol, *n*-propanol, and tetrahydrofuran are preferred, and ethanol is more preferred. Sufficiently satisfactory results can be obtained by using ethanol.

Examples of the aforementioned acid include moderate acids such as formic acid, acetic acid, weak acid, and phosphoric acid, and strong acids such as trifluoroacetic acid, hydrochloric acid, and sulfuric acid. Of them, acetic acid is preferred, and sufficiently satisfactory results can be obtained by using acetic acid.

The equivalence of the acid to be used is not particularly limited, but 1 to 50 equivalents, preferably 1 to 20 equivalents, and more preferably 3 to 15 equivalents with respect to the aldehyde are generally used to obtain sufficiently satisfactory results.

The reaction temperature is generally in the range of 20°C to 150°C, preferably in the range of 30°C to 100°C, and more preferably in the range of 50°C to 80°C. The reaction time is generally in the range of 1 to 100 hours, preferably 2 to 50 hours, and more preferably 4 to 24 hours.

Commercially available compounds may be readily used as raw material compounds to be used in these reactions. Compounds that are not commercially available can be produced by applying methods well known to those skilled in the art as described in the patent documents and non-patent patents indicated below.

Pyrazolone derivatives can be produced by applying methods well known to those skilled in the art as disclosed in Synth. Commun., 31, 3361-3376-184, 2001.

Aldehyde derivatives can be produced by applying methods well known to those skilled in the art as disclosed in Acc. Chem. Res, 15, 178, 1982.

Although the above are representative examples of methods for producing compound (I) of the present invention, the raw material compounds and various reagents used in the production of the compounds of the present invention may form salts, hydrates or solvates which may all vary depending on the starting material, solvent used, and such, and are not particularly limited so long as they do not inhibit the reaction. The solvent used may also vary depending on the starting materials, reagents, and such, and needless to say, it is not particularly limited so long as it can dissolve the starting materials to a certain degree without inhibiting the reaction.

When compound (I) of the present invention is obtained in a free form, it can be converted into a prodrug or a salt that may be formed by the aforementioned compound (I), or a hydrate or solvate state thereof according to standard methods. When compound (I) of the present invention is obtained as a prodrug or a salt of compound (I), or a hydrate or solvate of compound (I), it can be converted into the aforementioned free form of compound (I) according to standard methods.

Furthermore, various isomers (for example, geometric isomers, optical isomers based on asymmetric carbon, rotamers, stereoisomers, and tautomers) that can be obtained for compound (I) of the present invention can be purified and isolated by using general separation techniques such as recrystallization, diastereomeric salt method, enzymatic resolution method, and various chromatographies (for example, thin layer chromatography, column chromatography, and gas chromatography).

The above-mentioned compounds of the present invention represented by general formula (I) have an inhibiting activity of IκBα ubiquitination and are useful as active ingredients for inhibitory agents of IκBα ubiquitination. Substances that inhibit the ubiquitination of IκBα inhibit the machinery of IκBα degradation via the ubiquitin proteasome system, suppress the inflammation-associated function of transcription factor NFκB by stabilizing IκBα, and as a result, show anti-inflammatory effects and anticancer effects. Therefore, the above-mentioned inhibitory agents provided by the present invention are useful as pharmaceuticals for preventing and/or treating inflammatory diseases or for treating malignant tumors.

More specifically, compounds of the present invention can be used for preventing and/or treating inflammation or ulcerative diseases such as ulcerative colitis, Crohn's disease, peptic ulcer, and gastritis; arthritis such as chronic rheumatoid arthritis, juvenile rheumatoid arthritis, osteoarthrosis, osteoarthritis, and gout; autoimmune diseases such as systemic lupus erythematosus, scleroderma, polymyositis, dermatomyosis, vasculitis syndrome, Bechet's disease, connective tissue disease, nephrotic syndrome, polymyositis, dermatomyosis, Sjoegren's syndrome, insulin-dependent diabetes mellitus, and multiple sclerosis; allergic diseases such as bronchial asthma, bronchitis, emphysema, allergic rhinitis, atopic dermatitis, and urticaria; ischemic diseases such as angina and myocardial infarction; and inflammatory diseases such as arteriosclerosis. The compounds of the present invention can also be used for treating solid tumors such as gastric cancer, lung cancer, liver cancer, colon cancer, pancreatic cancer, renal cancer, esophageal cancer, and brain tumor, and malignant tumor such as leukemia and malignant lymphoma. However, the application of the inhibitory agents of the present invention is not limited to the specific diseases exemplified above.

Substances selected from the group consisting of compounds represented by formula (I), physiologically acceptable prodrugs or salts thereof, and hydrates thereof and solvates thereof may be used as they are as inhibitory agents of the present invention, but are generally preferred to be formulated in the form of pharmaceutical compositions comprising the above-mentioned substances which are the active ingredients and pharmaceutically acceptable formulation additives. One, two or more each of the above-mentioned substances for active ingredients and formulation additives can be used in combination.

Compounds of the present invention or a prodrug or salt thereof, or a hydrate or solvate thereof can be formulated by commonly used methods into tablets, powders, fine granules, granules, coated tablets, capsules, syrups, troches, inhalants, suppositories, injections, ointments, ophthalmic ointments, eye drops, nasal preparations, ear drops, cataplasms, lotions, or such.

For the formulation, generally used excipients, binders, lubricants, coloring agents, and flavoring agents, and if necessary, stabilizers, emulsifiers, absorbefacients, surfactants, pH regulators, antiseptics, antioxidants, and such may be used. Formulation is carried out using common methods by combining components that are generally used as raw materials for pharmaceutical formulations.

For example, to produce oral formulations, compounds of the present invention or pharmaceutically acceptable prodrug or salt thereof, or hydrate or solvate thereof and excipients are made into powders, fine granules, granules, tablets, coated tablets, capsules, or such using conventional methods; and binders, disintegrators, lubricants, coloring agents, flavoring agents, and such are added if need.

Examples of such components include animal and vegetable oils such as soybean oil, beef tallow, and synthetic glycerides; hydrocarbons such as liquid paraffin, squalane, and solid paraffin; ester oils such as octyldodecyl myristate and isopropyl myristate; higher alcohols such as cetostearyl alcohol and behenyl alcohol; silicone resins; silicone oils; surfactants such as polyoxyethylene fatty acid esters, sorbitan fatty acid esters, glycerin fatty acid esters, polyoxyethylene sorbitan fatty acid esters, polyoxyethylene hydrogenated castor oil, and polyoxyethylene-polyoxypropylene block copolymer; water-soluble polymers such as hydroxyethylcellulose, polyacrylic acid, carboxyvinyl polymer, polyethylene glycol, polyvinylpyrrolidone, and methylcellulose; lower alcohols such as ethanol and isopropanol; polyhydric alcohols such as glycerin, propylene glycol, dipropylene glycol and sorbitol; sugars such as glucose and sucrose; inorganic powders such as silicic anhydride, magnesium aluminum silicate and aluminum silicate; purified water; and the like. Examples of excipients which may be used include lactose, corn starch, white soft sugar, dextrose, mannitol, sorbit, crystalline cellulose, and silicon dioxide; examples of binders which may be used include polyvinyl alcohol, polyvinyl ether, methylcellulose, ethylcellulose, gum arabic, tragacanth, gelatin, shellac, hydroxypropylmethyl cellulose, hydroxypropylcellulose, polyvinylpyrrolidone, polypropylene glycol/polyoxyethylene block polymer, and meglumine; examples of disintegrators which may be used include starch, agar, gelatin powder, crystalline cellulose, calcium carbonate, sodium hydrogencarbonate, calcium citrate, dextrin, pectin, and carboxymethylcellulose calcium; examples of lubricants which may be used include magnesium stearate, talc, polyethylene glycol, silica, and hydrogenated vegetable oils; examples of coloring agents which may be used include those approved to be added to pharmaceuticals; and examples of flavoring agents which may be used include cocoa powder, menthol, aromatic powders, mentha oil, borneol, and cinnamon powder.

These tablets or granules may, of course, be coated with sugar or with other suitable coating, if necessary. When producing a liquid agent such as a syrup or an injection formulation, a common method may be used for formulation by adding to a compound of the present invention or a pharmaceutically acceptable prodrug or salt thereof, a pH adjustor, solubilizer, isotonizing agent, or the like, as well as a solubilizing adjuvant, stabilizer, or such if necessary. There are no particular restrictions on the method of producing an agent for external use, and any common method may be used. More specifically, various raw materials commonly used in drugs, quasi-drugs, cosmetics, and such may be used as base materials in drug formulation. Specifically, the base materials that are used are raw materials such as animal and vegetable oils, mineral oils, ester oils, waxes, higher alcohols, fatty acids, silicone oils, surfactants, phospholipids, alcohols, polyhydric alcohols, water-soluble polymers, clay minerals, purified water and such, and pH adjustors, antioxidants, chelating agents, antiseptic-antifungal agents, coloring agents, aromas and the like may be added if necessary, but the base materials for agents for external use of the present invention are not limited thereto. In addition, if necessary, differentiation-inducing components, or components such as circulation promoting agents, microbicides, antiphlogistic agents, cell activators, vitamins, amino acids, humectants, keratolytic agents, and the like may also be included. The quantity of the above-mentioned base material to be added is an amount that will make a concentration commonly set for production of agents for external use.

When administering a compound of the present invention, or a prodrug or salt thereof, or a hydrate or solvate thereof, the form is not particularly limited, and it may be administered orally or parenterally by conventionally used methods.

Formulations suitable for oral administration include tablets, powders, capsules, granules, fine granules, syrups, and suspending agents. Formulations suitable for parenteral administration include intravenous, intramuscular, or subcutaneous injections, drips, suppositories, agents for external use, eye drops, nasal preparations, ear drops, inhalents, transdermal absorbents, transmucosal absorbents, ointments, creams, and adhesive preparations, but the forms of the formulations are not limited thereto.

Methods for producing these formulations are not particularly limited so long as they are available to those skilled in the art, and those skilled in the art may select and use one, two or more types of suitable formulation additives when producing a formulation. In some cases, the use of pharmaceutical compositions that have active ingredients encapsulated into liposomes, or pharmaceutical compositions that further have antibodies bound to the liposomes, may improve affinity and selectivity against the target organ.

The route of administration and dosage of the pharmaceuticals of the present invention can be appropriately selected according to the severity of symptoms, age, gender, body weight, form of administration/type of salt, specific type of disorder, and such.

The dosage will significantly differ depending on the type of the patient's disorder, severity of symptoms, age of the patient, gender difference, difference in sensitivity to the pharmaceutical agent, and such. For an adult, approximately 0.01 to 1000 mg per day in one or several portions is usually administered. The dosage for injection is usually approximately 1 µg/kg to 3000 µg/kg or so.

Another embodiment of the present invention relates to use of the compound represented by above-mentioned general formula (I) or a physiologically acceptable prodrug or salt thereof, or a hydrate or solvate thereof for producing an inhibitory agent mentioned above.

Furthermore, other embodiments include preventive and/or therapeutic methods for inflammatory diseases. The methods comprise the step of administering to mammals, including humans, a therapeutically and/or preventively effective amount of the above-mentioned compound represented by general formula (I), a physiologically acceptable prodrug or salt thereof, or a hydrate or solvate thereof. Furthermore, methods for treating malignant tumors are also included. The methods comprise the step of administering to mammals, including humans, a therapeutically and/or preventively effective amount of the above-mentioned compound represented by general formula (I) or a physiologically acceptable salt thereof.

All prior art references cited herein are incorporated by reference into this description.

### [Effects of the Invention]

Compounds of the present invention suppress NFκB activation by inhibiting IκBα ubiquitination. Therefore, they are useful as preventive or therapeutic agents for NFκB-associated cancer or inflammatory diseases or the like.

### Best Mode for Carrying Out the Invention

Herein below, the present invention will be specifically described with reference to the Examples. The compounds of the present invention are not to be construed as being limited to the compounds described in the following Examples. Methods for preparing the raw material compounds are also described in the Reference Examples.

### [Reference Example 1]

### Ethyl 3-(3,4-dimethoxyphenyl)-3-oxopropionate

To a solution of 2,2-dimethyl-1,3-dioxane-4,6-dione (14.4 g 10 mmol) in dichloromethane (30 mL), pyridine (0.81 mL, 10 mmol) was added at 0°C or less, and this was stirred at -5°C for 0.5 hours. Subsequently, 3,4-dimethoxybenzoylchloride (2.00 g 10 mmol) was added at 0°C or less, stirred at -5°C for 0.5 hours, 0°C for 0.5 hours, and 25°C for 0.5 hours, and then 1 M hydrochloric acid (30 mL) was added while cooling on ice. This was then extracted with dichloromethane. After drying over magnesium sulfate, dichloromethane was distilled off, this was redissolved in ethanol (30 mL), refluxed for 1.5 hours, stirred at room temperature for 10 hours. It was then stirred at 60°C for 14 hours, ethanol was distilled off, and this was purified by silica gel column chromatography (eluate: hexane : tetrahydrofuran =3:1) which resulted in a colorless oily material (2.27 g, 90% yield).

### [Reference Example 2]

### Ethyl 3-oxo-4-phenylbutyrate

The same procedures of the method for synthesizing ethyl 3-(3,4-dimethoxyphenyl)-3-oxopropionate using 2,2-dimethyl-1,3-dioxane-4,6-dione (14.4 g 10 mmol) and benzoyl chloride (1.16 mL, 10 mmol) was carried out, and this was purified by silica gel column chromatography (eluate: hexane : ethyl acetate =3:1) which resulted in a colorless oily material (1.83 g, 95% yield).

### [Reference Example 3]

4-(3- Furan-3-yl-5-oxo-4,5-dihydropyrazol-1-yl)benzoic acid

4-Hydrazine benzoic acid (2.18 g, 10 mmol) and ethyl 3-furan-3-yl-3-oxo-propionate (1.58 mL, 10 mmol) were dissolved in ethanol (30 mL), stirred at 25°C for 2 hours, and then refluxed for 10 hours. Next, ethanol was distilled off, and solids precipitated from the obtained residue using hexane / ethyl acetate / tetrahydrofuran were collected by filtration. A pale yellow solid (2.37 g, 90% yield) was obtained.

### [Reference Example 4]

### 4-[3-(3,4-Dimethoxyphenyl)-5-oxo-4,5-dihydropyrazol-1-yl]benzoic acid

The same procedures of the method for synthesizing 4-(3-furan-3-yl-5-oxo-4,5-dihydropyrazol-1-yl)benzoic acid were carried out using 4-hydrazine benzoic acid (2.18 g, 10 mmol) and ethyl 3-(3,4-dimethoxyphenyl)-3-oxo-propionate (2.52 g, 10 mmol), and solids precipitated using hexane / ethyl acetate / tetrahydrofuran were collected by filtration which resulted in a pale yellow solid (3.03 g, 89% yield).

### [Reference Example 5]

### 4-(3-Benzyl-5-oxo-4,5-dihydropyrazol-1-yl)benzoic acid

The same procedures of the method for synthesizing 4-(3-furan-3-yl-5-oxo-4,5-dihydropyrazol-1-yl)benzoic acid were carried out using 4-hydrazine benzoic acid (2.18 g, 10 mmol) and ethyl 3-oxo-4-phenylbutyrate (2.06 g, 10 mmol), and solids precipitated using hexane / ethyl acetate / tetrahydrofuran were collected by filtration which resulted in a pale yellow solid (2.53 g, 86% yield).

### [Reference Example 6]

### 4-[3-(2-Methoxyethyl)-5-oxo-4,5-dihydropyrazol-1-yl]benzoic acid

The same procedures of the method for synthesizing 4-(3-furan-3-yl-5-oxo-4,5-dihydropyrazol-1-yl)benzoic acid were carried out using 4-hydrazine benzoic acid (2.18 g, 10 mmol) and methyl 5-methoxy-3-oxo-pentanoate (1.46 ml, 10 mmol), and solids precipitated using hexane / ethyl acetate / tetrahydrofuran were collected by filtration which resulted in a pale yellow solid (2.26 g, 86% yield).

### [Reference Example 7]

### 4-(3-Methoxymethyl-5-oxo-4,5-dihydropyrazol-1-yl)benzoic acid

The same procedures of the method for synthesizing 4-(3-furan-3-yl-5-oxo-4,5-dihydropyrazol-1-yl)benzoic acid were carried out using 4-hydrazine benzoic acid (2.18 g, 10 mmol) and methyl 4-methoxy-3-oxo-butyrate (1.30 mL, 10 mmol), and solids precipitated using hexane / ethyl acetate / tetrahydrofuran were collected by filtration which resulted in a pale yellow solid (1.99 g, 80% yield).

### [Reference Example 8]

### 5-(2-Fluoro-phenyl)-furan-carbaldehyde

Under Ar atmosphere, 5-bromo-furan-2-carbaldehyde (1.75 g, 10 mmol) and 2-fluorobenzene boronic acid (1.40 g, 10 mmol) were dissolved in tetrahydrofuran (30 mL), 1 M aqueous sodium carbonate solution (1 mL) and tetrakistriphenylphophine palladium (29.0 mg, 0.025 mmol) were added, and this was stirred at room temperature for 2 hours and then at 60°C for 14 hours. The reaction mixture solution was filtered through filter paper, and after distilling off the solvent, this was purified by silica gel column chromatography (eluate: hexane : ethyl acetate = 4:1). A pale yellow solid (1.48 g, 78% yield) was obtained.

### [Reference Example 9]

### 5-(1-Methyl-1H-pyrazol-4-yl)-2-furaldehyde

Under Ar atmosphere, the same procedures of the method for synthesizing 5-(2-fluoro-phenyl)-furan-carbaldehyde were carried out using 5-bromo-2-furaldehyde (1.75 g, 10 mmol) and 1-methyl-4-[(4,4,5,5-tetramethyl-2-(1,3,2-dioxaboran))-1H-pyrazole (2.08 g, 10 mmol). Purification was carried out by silica gel column chromatography (eluate: hexane : ethyl acetate = 4:1) which resulted in a pale yellow solid (1.23 g, 70% yield).

### [Reference Example 10]

### 4-Morpholin-1-yl-benzaldehyde

*p*-Fluorobenzaldehyde (1.07 mL, 10 mmol) was dissolved in dimethylformamide (30 mL) where potassium carbonate (2.52 g, 20 mol) and morpholine (814 mL, 10 mmol) were added, and this was stirred at room temperature for 14 hours. The reaction mixture solution was filtered through filter paper, the solvent was distilled off. Purification was carried out by silica gel column chromatography (eluate: hexane : ethyl acetate = 1: 1) which resulted in a pale yellow solid (1.15 g, 60% yield).
¹H NMR (300 MHz, CDCl₃) δ: 9.81 (1H, s), 7.76-7.93 (2H, m), 6.91-6.94 (2H, m), 3.85-3.88 (2H, m), 3.34-3.37 (2H, m).

### [Reference Example 11]

### 4-(4-Phenyl-piperidin-1-yl)benzaldehyde

The same procedures of the method for synthesizing 4-morpholin-1-yl-benzaldehyde were carried out using *p*-fluorobenzaldehyde (1.07 mL, 10 mmol) and 1-phenylpiperidine (1.53 mL, 10 mmol). Purification was carried out by silica gel column chromatography (eluate: hexane : ethyl acetate = 4:1) which resulted in a pale yellow solid (1.86 g, 70% yield).

### [Reference Example 12]

### 1-(4-Formylphenyl)-piperidine-4-carboxylic acid

The same procedures of the method for synthesizing 4-(1-morpholin)benzaldehyde were carried out usingp-fluorobenzaldehyde (1.07 mL, 10 mmol) and isonipecotic acid (1.29 mL, 10 mmol). Purification was carried out by silica gel column chromatography (eluate: hexane: ethyl acetate = 4:1) which resulted in a pale yellow solid (1.51 g, 65% yield).

### [Reference Example 13]

### 5-(4-Hydroxypiperidin-1-yl)-furan-2-carbaldehyde

The same procedures of the method for synthesizing 4-morpholin-1-yl-benzaldehyde were carried out using 5-bromo-furan-2-carbaldehyde (1.75 g, 10 mmol) and 4-hydroxypiperidine (1.02 g, 10 mmol). Purification was carried out by silica gel column chromatography (eluate: hexane : ethyl acetate = 4:1) which resulted in a pale yellow solid (1.56 g, 80% yield).

### [Reference Example 14]

### 2-(4-Formylphenoxy)-N-phenylacetamide

Under anhydrous conditions, bromoacetyl bromide (2.02 g, 10 mM) was dissolved in tetrahydrofuran (30 mL). Then, triethylamine (3.05 mL, 22 mmol) and aniline (913 mL, 10 mmol) were added slowly at 0°C or less, and this was stirred for 1 hour. Subsequently, 4-hydroxybenzaldehyde (1.22 mg, 10 mmol) was added, warmed to room temperature, and then was stirred for 40 hours. Thereafter, while cooling on ice, 1 M hydrochloric acid (15 mL) was added to the reaction mixture solution, this was extracted with ethyl acetate, dried over magnesium sulfate, and then filtered through filter paper. Then, the solvent was distilled off, and purified by silica gel column chromatography (eluate: hexane : ethyl acetate =3:1) which resulted in a pale yellow solid (1.91 g, 75% yield).

### [Reference Example 15]

### 4-Benzyloxy-3-methoxy-benzaldehyde

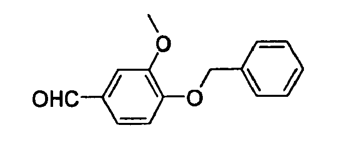

4-Hydroxy-3-methoxybenzaldehyde (1.52 mL, 10 mmol) was dissolved in tetrahydrofuran (30 mL), then triethylamine (1.53 mL, 11 mmol), benzyl bromide (1.19 mL, 10 mmol), and sodium iodide (99 mg, 0.5 mmol) were added at 0°C or less, and this was stirred at room temperature for 14 hours. Thereafter, while cooling on ice, 1 M hydrochloric acid (10 mL) was added to the reaction mixture solution, this was extracted with ethyl acetate, dried over magnesium sulfate, and filtered through filter paper. Then, the solvent was distilled off, and purified by silica gel column chromatography (eluate: hexane : ethyl acetate = 3: 1) which resulted in a pale yellow solid (2.18 g, 90% yield).
¹H NMR (300 MHz, CDCl₃) δ: 9.84 (1H, s), 7.33-7.46 (7H, m), 7.98 (1H, d, J=8.21Hz), 5.25 (2H, s), 8.95 (3H, s).

### [Reference Example 16]

### N-(4-Formyl-phenyl)-benzamide

Methyl 4-aminobenzenecarboxylate (1.51 g, 10 mmol) was dissolved in tetrahydrofuran (30 mL). Then, triethylamine (1.53 mL, 11 mmol) and benzoyl chloride (1.16 mL, 10 mmol) were added at 0°C or less, stirred at 0°C for 1 hour and then at room temperature for 1 hour. While cooling on ice, 1 M hydrochloric acid (10 mL) was added to the reaction mixture solution, this was extracted by adding ethyl acetate, dried over magnesium sulfate, and filtered through filter paper, and then the solvent was distilled off.

Next, the residue was dissolved in diethyl ether (50 mL), lithium aluminum hydride (380 mg, 10 mmol) was added at 0°C, and this was stirred at 20°C for 5 hours. While cooling on ice, 1 M hydrochloric acid (50 mL) was added to the reaction mixture solution, this was extracted with ethyl acetate, dried over magnesium sulfate, filtered through filter paper, and the solvent was distilled off. The residue was dissolved in dichloromethane (30 mL), and then, MS4A (powder, 3.0 g) and *N*-methylmorpholine-*N*-oxide monohydrate (1.35 mg, 10 mmol) were added at 0°C. This was stirred at 0°C for 1 hour, tetrapropylammonium perruthenate (175 mg, 0.5 mmol) was added and stirred at 0°C for 1 hour, and then at room temperature for 1 hour.

The reaction mixture solution was filtered through filter paper, and after the solvent was distilled off, this was purified by silica gel column chromatography (eluate: hexane : ethyl acetate = 3:1) which resulted in a pale yellow solid (1.46 g, 65% yield).

### [Example 1]

### General Synthetic Method

Pyrazolone (0.05 mM) and aldehyde (0.05 mM) were dissolved in ethanol (0.5 mL), and acetic acid (0.4 mL, 0.75 mM) was added. This was stirred at 20°C for 1 hour and at 60°C to 70°C for 14 hours. After distilling off the ethanol and acetic acid, the objective compound was obtained by a purification procedure using recrystallization or HPLC. (Table 1 shows the structures of the obtained compounds, and Table 2 shows the ¹H NMR data, MW, and HPLC data.)

### [Example 2]

### 4-{3-Benzyl-4-[5-(2-fluorophenyl)-furan-2-ylmethylene]-5-oxo-4,5-dihydropyrazol-1-yl} benzoic acid (Compound 267)

The same procedures of the above-mentioned General Synthetic Method of Example 1 were carried out using 4-(3-benzyl-5-oxo-4,5-dihydropyrazol-1-yl) benzoic acid (14.7 mg, 0.05 mM) and 5-(2-fluoro-phenyl)-furan-carbaldehyde (9.5 mg, 0.05 mM). Solids precipitated using *n*-hexane / tetrahydrofuran were collected by filtration which resulted in an orange solid (18.7 mg, 80% yield).
¹H NMR (300 MHz, DMSO-d6) δ: 8.68 (0.9H, d, J=3.85Hz), 8.02-8.12 (5.1 H, m), 7.81-7.83 (1H, m), 7.22-7.67 (9H, m), 4.57 (0.2H, s), 4.19 (1.8H, s). MS (ES⁺, m/z)=467 (M+H)⁺.

### [Example 3]

### 4-[4-(4-Benzyloxy-3-methoxybenzylidine)-3-furan-3-yl-5-oxo-4,5-dihydropyrazol-1-yl] benzoic acid (Compound 556)

The same procedures of the above-mentioned General Synthetic Method of Example 1 were carried out using 4-(3-furan-3-yl-5-oxo-4,5-dihydropyrazol-1-yl) benzoic acid (13.5 mg, 0.05 mM) and 4-benzyloxy-3-methoxy-benzaldehyde (12.1 mg, 0.05 mM), and solids precipitated using *n*-hexane / tetrahydrofuran were collected by filtration which resulted in an orange solid (15.8 mg, 64% yield).
¹H NMR (300 MHz, DMSO-d6) δ: 12.9 (1H, brs), 8.75 (1H, s), 8.53 (1H, s), 8.20-8.22 (1H, m), 8.10 (2H, ABtype, J=74.87, 8.94Hz), 7.93 (2H, s), 7.37-7.50 (5H, m), 7.30 (1H, d, J=8.66 Hz), 7.02 (1H, s), 5.28 (2H, s), 3.90 (3H, s).

### [Example 4]

### 4-[4-(Benzyloxv-3-methoxy-benzylidine)-3-methoxymethyl-5-oxo-4,5-dihydroxy-pyrazol-1-yl] benzoic acid (Compound 234)

The same procedures of the above-mentioned General Synthetic Method of Example 1 were carried out using 4-(3-methoxymethyl-5-oxo-4,5-dihydropyrazol-1-yl) benzoic acid (12.2 mg, 0.05 mM) and 4-benzyloxy-3-methoxy-benzaldehyde (12.1 mg, 0.05 mM). Solids precipitated using *n*-hexane / tetrahydrofuran were collected by filtration which resulted in an orange solid (14.9 mg, 63% yield).
¹H NMR (300 MHz, DMSO-d6) δ: 12.9 (1H, brs), 8.79 (1H, s), 7.92-8.11 (6H, m), 7.29-7.50 (6H, m), 5.27 (2H, s), 4.56 (2H, s), 3.90 (3H, s), 3.38 (3H, s). MS (ES⁺, m/z)=474 (M+H)⁺.

### [Example 5]

### 4-{3-Methyl-4-[4-(1-methyl-1H-pyrazol-4-yl)-furan-2-ylmethylene]5-oxo-4 5-dihydropyrazol-1-yl} benzoic acid (Compound 495)

The same procedures of the above-mentioned General Synthetic Method of Example 1 were carried out using 3-(3-methyl-5-oxo-4,5-dihydro-pyrazol-1-yl) benzoic acid (11.0 mg, 0.05 mM) and 5-(1-methyl-1H-pyrazol-4-yl)-furan-2-carboaldehyde (8.81 mg, 0.05 mM). Solids precipitated using *n*-hexane / tetrahydrofuran were collected by filtration which resulted in an orange solid (15.1 mg, 80% yield).
MS (ES⁺, m/z)=377 (M+H)⁺, 399 (M+Na)⁺.

### [Example 6]

### 3-(3-Furan-3-yl-4-(1H-indol-ylmethylene)-5-oxo-4,5-dihydropyrazol-1-yl) benzoic acid (Compound 475)

The same procedures of the above-mentioned General Synthetic Method of Example 1 were carried out using 4-(3-furan-3-yl-5-oxo-4,5-dihydropyrazol-1-yl) benzoic acid (13.5 mg, 0.05 mM) and 1H-indole-5-carbaldehyde (7.3 mg, 0.05 mM). Solids precipitated using *n*-hexane / tetrahydrofuran were collected by filtration which resulted in an orange solid (15.6 mg, 80% yield). MS (ES⁺, m/z)=376 (M+H)⁺.

Compounds having the structures indicated in Table 1 were obtained by the same methods of the above-mentioned Examples. The results of the mass spectrometry, HPLC analysis, and ¹H NMR (300 MHz, DMSO-d6) analysis for the obtained compounds are shown in Table 4. The compounds in Table 4 can also be purified by fractionation under the HPLC analysis conditions indicated in Table 4 besides recrystallization using suitable solvents indicated in the above-mentioned Examples 2 to 6.

Details of the HPLC analysis conditions shown in Table 4 are described below.

### HPLC analysis conditions 1:

Column: SYNERGI MAX-RP 4 µm 30 x 4.6 mm (phenomenex); solvent A: 90% 0.1% Formic acid in water /10% Methanol; solvent B: 100% MeOH; temperature: 35°C; detection wavelength: 280 nm;

**Table 2**

| gradient 1: | | | |
|---|---|---|---|
| Time (min) | A (%) | B (%) | Flow rate (ml/min) |
| 0 | 70 | 30 | 1 |
| 0.7 | 70 | 100 | 1 |
| 3 | 0 | 100 | 1 |
| 5 | 0 | 100 | 1 |
| 5.3 | 70 | 30 | 1.5 |
| 6 | 70 | 30 | 1.5 |

**Table 3**

| gradient 2: | | | |
|---|---|---|---|
| Time (min) | A (%) | B (%) | Flow rate (ml/min) |
| 0 | 70 | 30 | 1.5 |
| 1.5 | 0 | 100 | 1.5 |
| 2.5 | 0 | 100 | 1.5 |
| 2.6 | 70 | 30 | 1.5 |
| 3 | 70 | 30 | 1.5 |

### HPLC analysis conditions 2:

Column: Xterra MS C18 5 µm 100 x 4.6 mm (Waters); solvent A: MeOH; solvent B: 1% Formic acid in Water; Flow rate: 1 mL/min; column temperature: room temperature; isocratic (mixing ratio of solvent A and solvent B is described in Table 4)

**Table 4**

| compound No. | Molecular weight | m/z (ESI) | | | HPLC | | | | ¹HNMR |
|---|---|---|---|---|---|---|---|---|---|
| | | (M+H)⁺ | (M+Na)⁺ | (M-H)⁻ | Retention time (min) | condition | A (%) | B (%) | |
| 1 | 338 | 339 | | 337 | 3.65 | 1 | gradient 1 | | |
| 2 | 342 | 343 | | | 4.21 | 1 | gradient 1 | | |
| 3 | 428 | 429 | 451 | | 1.96 | 1 | gradient 2 | | |
| 4 | 344 | 345 | | | 3.89 | 1 | gradient 1 | | |
| 5 | 435 | 435 | 457 | | 1.73 | 1 | gradient 2 | | |
| 6 | 376 | 377 | | | 4.2/4.49 | 1 | gradient 1 | | |
| 7 | 312 | | 335 | | 1.58 | 1 | gradient 2 | | |
| 8 | 361 | 362 | 384 | | 1.83 | 1 | gradient 2 | | |
| 9 | 350 | 351 | 373 | | 1.53 | 1 | gradient 2 | | |
| 10 | 358 | 359 | 381 | | 1.53 | 1 | gradient 2 | | |
| 11 | 360 | 361 | 383 | | 1.57 | 1 | gradient 2 | | 9.17(1H, s), 8.02(2H, d, J = 7.59Hz), 7.88(1H, s), 7.62(1 H, s), 7.54-7.61 (1H, m), 7.46(2H, t, J = 7.49Hz), 7.21-7.26(1H, m), 7.02(1 H, d, J = 8.30Hz), 6.85(1H, s), 4.09(3H, s) |
| 12 | 364 | 365 | | | 3.91 | 1 | gradient 1 | | |
| 13 | 366 | 367 | | | 3.85 | 1 | gradient 1 | | |
| 14 | 457 | 457 | 479 | | 1.58 | 1 | gradient 2 | | 8.57(2H, d, J = 9.05Hz), 8.05(2H, d, J = 7.59Hz), 7.86(1H, s), 7.59(2H, d, J = 7.67Hz), 45(2H, t, J = 8.00Hz), 7.21(1H, t, J = 7.40Hz), 6.95(2H, d, J = 9.12Hz), 6.85(1H, s), 3.77(3H, s), 3.67-3-69(4H, m), 3.48-3.52(4H, m) |
| 15 | 398 | 399 | 421 | | 1.81 | 1 | gradient 2 | | 8.94(1H, d, J = 3.94Hz), 7.94-8.08(4H, m), 7.62-7.64(2H, m), 7.36-7.49(3H, m), 7.17-7.31(4H, m), 8.90(1H, s) |
| 16 | 334 | 335 | 357 | | 1.06/1.51 | 1 | gradient 2 | | 8.79(1H, d, J = 3.68Hz), 8.04(2H, d, J = 7.62Hz), 7.90 (1H, s), 7.59-7.61 (2H, m), 7.46(2H, t, J = 7.99Hz), 7.21-7.28(1H, m), 6.87-6.87(1H, m), 6.67(1H, d, J = 3.59Hz), 4.77(2H, s), 2-64(1H, s) |
| 17 | 383 | 384 | 406 | | 1.65 | 1 | gradient 2 | | |
| 18 | 314 | 315 | | | 3.64/3.98 | 1 | gradient 1 | | |
| 19 | 346 | 347 | 369 | | 1.74 | 1 | gradient 2 | | 8.86(1H, d, J = 3.90Hz), 7.98-8.02 (3H, m), 7.29-7.48(5H, m), 7.13-7.27(3H, m), 2.38(3H, s) |
| 20 | 282 | 283 | 305 | | 1.31 | 1 | gradient 2 | | |
| 21 | 367 | 368 | | | 1.19 | 1 | gradient 2 | | |
| 22 | 384 | 385 | 407 | | 1.61 | 1 | gradient 2 | | |
| 23 | 388 | 389 | 411 | | 1.81 | 1 | gradient 2 | | |
| 24 | 475 | 475 | 497 | | 1.78 | 1 | gradient 2 | | |
| 25 | 481 | 481 | 503 | | 1.65 | 1 | gradient 2 | | |
| 26 | 422 | 423 | 445 | | 1.86 | 1 | gradient 2 | | |
| 27 | 358 | 359 | 381 | | 1.57 | 1 | gradient 2 | | 8.68 (1H, d, J = 3.69Hz), 8.01(2H, d, J = 7.62Hz), 7.45(2H, t, J = 7.09Hz), 7.36(3H, d, J = 4.61Hz), 7.22-7.27(4H, m), 6.59(1H, d, J = 3.73Hz), 4.70(2H, s), 4.06(2H, s), 2.39(1H, brs), |
| 28 | 408 | 408 | 430 | | 1.73 | 1 | gradient 2 | | |
| 29 | 396 | 397 | 419 | | 1.71 | 1 | gradient 2 | | 8.24(1H, d, J = 2.09Hz), 8.02-8.05(2H, m), 7.88-7.95(2H, m), 7.19-7.47(8H, m). 6.92(1H, d, J = Hz), 4.27-4.36(4H, m). 4.09(2H, s) |
| 30 | 404 | 405 | 427 | | 1.60/1.70 | 1 | gradient 2 | | |
| 31 | 405 | 406 | | | 4.06 | 1 | gradient 1 | | |
| 32 | 436 | 437 | 459 | | 1.50/1.68 | 1 | gradient 2 | | |
| 33 | 527 | 527 | 549 | | 1.58 | 1 | gradient 2 | | |
| 34 | 468 | 469 | 491 | | 1.68/1.83 | 1 | gradient 2 | | |
| 35 | 404 | 405 | 427 | | 1.51 | 1 | gradient 2 | | |
| 36 | 454 | 454 | | | 1.66 | 1 | gradient 2 | | |
| 37 | 442 | 443 | 465 | | 1.51/1.66 | 1 | gradient 2 | | |
| 38 | 451 | 451 | 473 | | 1.51/1.63 | 1 | gradient 2 | | |
| 39 | 451 | 452 | | | 1.48 | 1 | gradient 2 | | |
| 40 | 371 | 372 | 370 | | 3.74 | 1 | gradient 1 | | |
| 41 | 462 | 462 | | | 1.7 | 1 | gradient 2 | | |
| 42 | 468 | 468 | | | 3.76 | 1 | gradient 1 | | |
| 43 | 409 | 410 | | | 4.05/4.71 | 1 | gradient 1 | | |
| 44 | 345 | 346 | | | 1.31 | 1 | gradient 2 | | |
| 45 | 394 | 395 | | | 4.01 | 1 | gradient 1 | | |
| 46 | 383 | 384 | | | 3.93 | 1 | gradient 1 | | |
| 47 | 409 | 410 | | | 1.91 | 1 | gradient 2 | | |
| 48 | 345 | 346 | 368 | | 1.55 | 1 | gradient 2 | | |
| 49 | 394 | 395 | | | 1.7 | 1 | gradient 2 | | |
| 50 | 350 | 351 | 373 | | 1.66 | 1 | gradient 2 | | |
| 51 | 354 | 355 | 377 | | 1.57/1.83 | 1 | gradient 2 | | |
| 52 | 441 | 441 | 463 | | 1.81 | 1 | gradient 2 | | |
| 53 | 356 | 357 | | | 4.07/4.70 | 1 | gradient 1 | | |
| 54 | 447 | 447 | 469 | | 1.69 | 1 | gradient 2 | | |
| 55 | 388 | 389 | 411 | | 1.9 | 1 | gradient 2 | | |
| 56 | 324 | 325 | 347 | | 1.6 | 1 | gradient 2 | | 8.68 (1H, d, J = 3.67Hz), 7.99(2H, d, J = 7.60Hz), 7.39-7.46(2H, m), 7.32(1H, s), 7.20(1 H, t, J = 7.40Hz), 6.62 (1H, d, J = 3.65Hz), 4.74(2H, s), 2.63-2.89 (2H, m), 2.24(1H, brs), 1.71-1.81(2H, m), 1.43-1.55(2H, m), 1.00(3H, t, J = 7.25Hz), |
| 57 | 374 | 374 | 396 | | 1.78 | 1 | gradient 2 | | |
| 58 | 370 | 371 | | | 4.31 | 1 | gradient 1 | | 8.68(2H, d, J = 8.84Hz). 8.06 (1H, d, J = 2.49Hz), 8.01 (2H, d, J = 1.18Hz), 7.99 (1 H, s), 7.88 (1H, s), 7.85 (1H, s), 7.41-7.47(3H, m), 7.21 (1H, t, J = 7.40Hz), 6.54-6.55 (1H, m), 2-71 (2H, t, J = 7.69Hz), 1.75-1.86 (2H, m), 1.49-1.57(2H, m), 1.03(3H, t, J = 7.32Hz) |
| 59 | 371 | 372 | | | 4.16 | 1 | gradient 1 | | |
| 60 | 371 | 372 | | 370 | 3.7 | 1 | gradient 1 | | |
| 61 | 462 | 462 | | | 3.95/4.21 | 1 | gradient 1 | | |
| 62 | 468 | 468 | | | 3.74 | 1 | gradient 1 | | |
| 63 | 409 | 410 | | | 4.02/4.59 | 1 | gradient 1 | | |
| 64 | 345 | 346 | | | 1.36 | 1 | gradient 2 | | |
| 65 | 394 | 395 | | | 3.96 | 1 | gradient 1 | | |
| 66 | 383 | 384 | | | 3.88 | 1 | gradient 1 | | |
| 67 | 391 | 392 | | | 3.83 | 1 | gradient 1 | | |
| 68 | 392 | 393 | | | 1.05 | 1 | gradient 2 | | |
| 69 | 334 | 335 | 357 | | 1.6 | 1 | gradient 2 | | |
| 70 | 338 | 339 | | | 4.54 | 1 | gradient 1 | | |
| 71 | 425 | 425 | 447 | | 1.76 | 1 | gradient 2 | | 9.00(1H, d, J = 1.99Hz), 7.98(2H, d, J = 7.60Hz), 7.69-7.72(1H, m), 7.59(1H, s), 7.35-7.49(H, m), 7.18(1H, t, J = 7.40Hz), 6.97(1H, d. J = 8.46Hz), 5.29(2H, s), 4.08(3H, s), 1.86-1.92(1H, m). 1.08-1.13(2H, m), 1.00-1.05(2H, m) |
| 72 | 340 | 341 | | | 3.95/4.29 | 1 | gradient 1 | | |
| 73 | 431 | 431 | 453 | | 1.62 | 1 | gradient 2 | | |
| 74 | 372 | 373 | 395 | | 1.85 | 1 | gradient 2 | | |
| 75 | 308 | 309 | 331 | | 1.51 | 1 | gradient 2 | | |
| 76 | 357 | 358 | 380 | | 1.71 | 1 | gradient 2 | | |
| 77 | 346 | 347 | 369 | | 1.67 | 1 | gradient 2 | | 8.41(1H, s), 7.97-8.07(3H, m), 7.56(1H, s), 7.41(2H, t, J = 8.00Hz), 7.17(1H, t, J = 7.39Hz), 6.98(1H, d, J = 8.54Hz), 4.37-4.39(2H, m), 4.30-4.37(2H, m), 1.84-1.93(1H, m), 1.06-1.13(2H, m), 0.98-1.05(2H, m) |
| 78 | 387 | 387 | | | 1.13 | 1 | gradient 2 | | |
| 79 | 354 | 355 | | | 4.06 | 1 | gradient 1 | | |
| 80 | 355 | 356 | | | 3.93 | 1 | gradient 1 | | |
| 81 | 441 | 442 | 464 | | 1.66 | 1 | gradient 2 | | |
| 82 | 357 | 358 | 380 | | 1.32/1.50 | 1 | gradient 2 | | |
| 83 | 448 | 448 | | | 3.59 | 1 | gradient 1 | | |
| 84 | 389 | 390 | 412 | | 1.76 | 1 | gradient 2 | | |
| 85 | 325 | 326 | 348 | | 1.29 | 1 | gradient 2 | | |
| 86 | 374 | 375 | 397 | | 1.51 | 1 | gradient 2 | | |
| 87 | 597 | 597 | | | 4.24 | 1 | gradient 1 | | |
| 8B | 545 | 545 | 567 | | 1.79 | 1 | gradient 2 | | |
| 89 | 530 | 530 | 552 | | 1.61 | 1 | gradient 2 | | |
| 90 | 374 | 375 | 397 | | 1.63 | 1 | gradient 2 | | |
| 91 | 479 | 479 | 501 | | 1.79 | 1 | gradient 2 | | |
| 92 | 424 | 424 | | | 1.76 | 1 | gradient 2 | | |
| 93 | 412 | 413 | | | 1.74 | 1 | gradient 2 | | |
| 94 | 451 | 451 | | | 4.19/4.46 | 1 | gradient 1 | | 8.95(1H, s). 8.03(2H, d, J = 7.58Hz), 7.87(1H, d, J = 0.49Hz), 7.62-7.70(1H, m), 7.61(2H, s), 7.32-7.48(6H, m), 7.21-7.26(2H, m), 6.98(1H, d, J = 8.48Hz), 6.85(1H, s), 5.30(2H, s), 4.08(3H, s) |
| 95 | 404 | 405 | | | 3.74 | 1 | gradient 1 | | |
| 96 | 390 | 391 | | 389 | 3.89/4.55 | 1 | gradient 1 | | |
| 97 | 435 | 435 | | | 4.17/4.54 | 1 | gradient 1 | | |
| 98 | 362 | 363 | | 361 | 4.45 | 1 | gradient 1 | | |
| 99 | 360 | 361 | | | 2.31 | 1 | gradient 1 | | |
| 100 | 437 | 437 | | | 2.9 | 1 | gradient 1 | | |
| 101 | 420 | 420 | | | 3.47 | 1 | gradient 1 | | |
| 102 | 444 | 444 | | | 3.66 | 1 | gradient 1 | | |
| 103 | 431 | 431 | | | 2.88 | 1 | gradient 1 | | |
| 104 | 431 | 431 | | | 3.54 | 1 | gradient 1 | | |
| 105 | 311 | 312 | | 310 | 3.33 | 1 | gradient 1 | | |
| 106 | 403 | 403 | | | 2.96 | 1 | gradient 1 | | |
| 107 | 431 | 431 | | | 3.12 | 1 | gradient 1 | | |
| 108 | 399 | 400 | | | 3.2 | 1 | gradient 1 | | |
| 109 | 408 | 409 | | | 2.85 | 1 | gradient 1 | | |
| 110 | 433 | 434 | | | 3.36 | 1 | gradient 1 | | 9.15(1H, s), 8.72(4H, s), 7.82-8.24(6H, m), 7.37(1H, s), 6.65(2H, s), 2.27-2.31(1H, m), 1.05-1.08(4H, m) |
| 111 | 434 | 435 | | | 3.19 | 1 | gradient 1 | | |
| 112 | 375 | 376 | | 374 | 4.32 | 1 | gradient 1 | | |
| 113 | 375 | 376 | | 374 | 4.26 | 1 | gradient 1 | | |
| 114 | 351 | 352 | | 350 | 3.45 | 1 | gradient 1 | | |
| 115 | 398 | 398 | 420 | | 7.08 | 2 | 70 | 30 | 8.55(2H, d, J = 8.50Hz), 8.13(2H, d, J = 8.79Hz), 7.89(3H, d, J = 8.50Hz), 7.62(2H, d, J = 8.79Hz), 7.35(2H, s), 2.38(3H, s), 1.33(9H, s) |
| 116 | 391 | 392 | | | 4.48 | 2 | 70 | 30 | 9.11(1H, s), 8.77(1H, s), 8.14-8.17(2H, m), 8.01-8.08(3H, m), 7.89-7.93(2H, m). 7.62-7.74(2H, m), 7.36(3H, s), 229(3H, s) |
| 117 | 417 | 418 | 440 | | 6.75 | 2 | 70 | 30 | 8.72(2H, d, J = 8.50Hz), 8.15(2H, d, J = 9.08Hz), 7.83-7.95(6H, m), 7.46-7.56(4H, m), 7.35(2H, s), 2.40(3H, s) |
| 118 | 359 | 360 | 382 | 358 | 5.2 | 2 | 60 | 40 | |
| 119 | 391 | 392 | 414 | 390 | 3.62 | 2 | 70 | 30 | |
| 120 | 536 | 536 | 558 | 534 | 4.83 | 2 | 70 | 30 | 8.78(1H, s), 8.13(2H, d, J = 8.79Hz), 8.01 (2H, d, J = 8.50Hz), 7.89(2H, d, J = 9.08Hz), 7.82(1H, s), 7.62(2H, d, J = 7.91Hz), 7.35(2H, s), 7.28(1H, d, J = 8.50Hz), 6.66(1H, s), 5.37(2H, s), 3.91(3H, s), 3.86(3H, s), 2.36(3H, s) |
| 121 | 502 | 502 | 524 | | 5.4 | 2 | 65 | 35 | 8.15(1 H. d. J = 2.05Hz), 8.11-8.15(3H, m), 7.88-7.91(2H, m), 7.85(1H, s), 7.35(2H, s), 7.07(1 H, d, J = 8.50Hz), 4.86 (2H, s), 3.90(3H, s), 2.36(3H, s), 1.44(9H, s) |
| 122 | 457 | 457 | 479 | 455 | 5.78 | 2 | 80 | 20 | |
| 123 | 452 | 452 | | 450 | 2.7 | 2 | 50 | 50 | |
| 124 | 372 | 373 | | | 8.95 | 2 | 50 | 50 | |
| 125 | 422 | 423 | 445 | | 6.22 | 2 | 75 | 25 | |
| 126 | 425 | 425 | | | 7.67 | 2 | 65 | 35 | 8.12 (2H, d, J = 9.08Hz), 8.00(4H, Abtype, J = 92.89, 8.79Hz), 7.59(1H, s), 7.29 (2H, s), 7.04 (2H, d, J = 9.38Hz), 3.50-3.60(4H, m). 2.30(3H, s), 1.50-1.60(6H, m) |
| 127 | 301 | 302 | 324 | 300 | 5.95 | 2 | 70 | 30 | 11.664(1H, s), 9.16(1 H, s), 8.47-8.50(1H, m), 7.97 (1H, d, J = 7.33Hz), 7.91 (1H, s), 7.50-7.56(2H, m), 7.44(1H, t, J = 7.33Hz), 6.87(2H, s), 6.66(2H, s), 2.18(3H, s) |
| 128 | 338 | 339 | 361 | 337 | 4.38 | 2 | 55 | 45 | |
| 129 | 309 | 310 | | 308 | 7.08 | 2 | 45 | 55 | |
| 130 | 318 | 319 | 341 | 317 | 3.78 | 2 | 50 | 50 | |
| 131 | 304 | 305 | | | 4.08 | 2 | 65 | 35 | |
| 132 | 246 | 247 | 269 | 245 | 3.1 | 2 | 55 | 45 | |
| 133 | 380 | 381 | 403 | 379 | 3.67 | 2 | 65 | 35 | |
| 134 | 380 | 381 | 403 | 379 | 2.27 | 2 | 70 | 30 | |
| 135 | 380 | 381 | | 379 | 2.88 | 2 | 70 | 30 | |
| 136 | 410 | 411 | | 409 | 2.82 | 2 | 70 | 30 | |
| 137 | 380 | 381 | 403 | 379 | 4.2 | 2 | 60 | 40 | |
| 138 | 380 | 381 | 403 | 379 | 3.43 | 2 | 70 | 30 | |
| 139 | 410 | 411 | | 409 | 4.23 | 2 | 65 | 35 | |
| 140 | 380 | 381 | 403 | 379 | 6.63 | 2 | 60 | 40 | |
| 141 | 394 | 395 | 417 | | 5.13 | 2 | 65 | 35 | |
| 142 | 394 | 395 | 417 | | 8.93 | 2 | 60 | 40 | |
| 143 | 381 | 382 | 404 | 380 | 6.77 | 2 | 60 | 40 | |
| 144 | 381 | 382 | 404 | | 7.07 | 2 | 60 | 40 | |
| 145 | 407 | 408 | 431 | 406 | 4.88 | 2 | 60 | 40 | |
| 146 | 415 | 415 | 437 | 413 | 5.85 | 2 | 70 | 30 | 9.79(1 H, s), 8.37(1 H, d, J = 1.76Hz), 8.21(1 H. s), 8.18(2H, d, J = 1.76Hz), 7.86(2H. d, J = 9.08Hz), 7.60(2H. d, J = 8.50Hz), 7.32(1H, d, J = 2.05Hz), 7.29-7.30(2H, m), 2.42(3H, s) |
| 147 | 415 | 415 | 437 | 413 | 5.52 | 2 | 70 | 30 | 9.82(1H, s), 8.53-8.53(1H, m), 8.39(1H, d, J = 2.05Hz), 8.24-8.28(2H, m). 8.21 (1H, s), 7.60-7.66(3H, m), 7.43(2H, s), 7.33(1H, dd, J = 8.50, 1.76Hz), 2.45(3H, s) |
| 148 | 380 | 381 | 403 | 379 | 4 | 2 | 65 | 35 | |
| 149 | 380 | 381 | 403 | 379 | 3.9 | 2 | 65 | 35 | |
| 150 | 407 | 408 | 430 | | 8.03 | 2 | 55 | 45 | 8.79 (2H, d, J = 9.08Hz), 8.72 (1H, d, J = 2.64Hz), 8.08-8.16(4H, m), 7.87-7.93(4H, m), 7.35 (2H, s), 6.66(1H, t, J = 2.20Hz), 2.39 (3H, s) |
| 151 | 380 | 381 | 403 | 379 | 6.88 | 2 | 60 | 40 | |
| 152 | 385 | 386 | 408 | 384 | 4.6 | 2 | 60 | 40 | |
| 153 | 497 | 497 | 519 | | 5.4 | 2 | 60 | 40 | |
| 154 | 540 | 540 | 562 | | 3.33 | 2 | 70 | 30 | 8.75 (1H, m), 8.12-8.19(1H, m), 8.01 (4H, Abtype, J = 73.55, 9.08Hz), 7.84(1H, s), 7.32-7.39 (4H, m), 6.87(1H, d, J = 3.52Hz), 5.32 (2H, s), 4.30 (2H, q, J = 7.03Hz), 3.88 (3H, s), 2.36(3H, s), 1.29(3H, t, J = 7.03Hz) |
| 155 | 540 | 540 | 562 | | 3.27 | 2 | 70 | 30 | |
| 156 | 519 | 519 | 541 | | 6.02 | 2 | 60 | 40 | |
| 157 | 519 | 519 | 541 | | 5.9 | 2 | 60 | 40 | |
| 158 | 497 | 497 | 519 | | 5.48 | 2 | 60 | 40 | |
| 159 | 442 | 442 | 464 | | 5.58 | 2 | 65 | 35 | |
| 160 | 442 | 442 | 464 | | 5.32 | 2 | 65 | 35 | |
| 161 | 484 | 484 | 506 | | 7.37 | 2 | 75 | 25 | |
| 162 | 470 | 470 | 492 | | 6.2 | 2 | 65 | 35 | |
| 163 | 484 | 484 | 506 | | 6.58 | 2 | 65 | 35 | |
| 164 | 381 | 382 | 404 | 380 | 4.18 | 2 | 70 | 30 | |
| 165 | 376 | 377 | 399 | | 5.33/5.85 | 2 | 55 | 45 | |
| 166 | 331 | 332 | 354 | | 6.47/6.98 | 2 | 50 | 50 | |
| 167 | 331 | 332 | 354 | | 6.57 | 2 | 50 | 50 | |
| 168 | 381 | 382 | 404 | | 7.28/8.13 | 2 | 65 | 35 | |
| 169 | 502 | 502 | 524 | | 6.08 | 2 | 70 | 30 | 8.66(2H, d, J = 9.08Hz), 8.02(4H, Abtype, J = 90.54, 9.08Hz), 7.67(1H, s), 7.23-7.34(4H, m), 7.15(2H, d, J = 9.38Hz), 7.00(2H, d, J = 8.50Hz), 6.82-6.87(1H, m), 3.68-3.72(4H, m), 3.34-3.40(4H, m), 2.33(3H, s) |
| 170 | 502 | 502 | 524 | | 5.83 | 2 | 70 | 30 | |
| 171 | 455 | 455 | 477 | | 3.2 | 2 | 65 | 35 | |
| 172 | 441 | 441 | 463 | | 4.82 | 2 | 60 | 40 | |
| 173 | 455 | 455 | 477 | | 4.62 | 2 | 60 | 40 | |
| 174 | 441 | 441 | 463 | | 4.72 | 2 | 60 | 40 | |
| 175 | 427 | 427 | 449 | 425 | 3.4 | 2 | 60 | 40 | |
| 176 | 520 | 520 | 542 | | 5.75 | 2 | 70 | 30 | 8.66(2H, d, J = 9.67Hz), 8.17(4H, Abtype, J = 82.94, 8.79Hz), 7.85(1H, s), 7.31(2H, s), 7.01-7.17(5H, m), 6.87(1H, s), 3.68-3.72(4H, m), 3.24-3.28(4H, m) |
| 177 | 520 | 520 | 542 | | 2.38 | 2 | 80 | 20 | |
| 178 | 427 | 427 | | | 8.83 | 2 | 50 | 50 | |
| 179 | 526 | 526 | 548 | | 4.93 | 2 | 70 | 30 | |
| 180 | 526 | 526 | 548 | | 4.42 | 2 | 70 | 30 | |
| 181 | 330 | 331 | 353 | 329 | 3.4 | 2 | 60 | 40 | |
| 182 | 330 | 331 | 353 | 329 | 3.48 | 2 | 60 | 40 | |
| 183 | 426 | 426 | 448 | | 6.67 | 2 | 35 | 65 | |
| 184 | 426 | 426 | 448 | | 7.3 | 2 | 35 | 65 | |
| 185 | 376 | 377 | 399 | | 5.33/6.55 | 2 | 70 | 30 | |
| 186 | 420 | 421 | 443 | | 5.63/6.93 | 2 | 70 | 30 | |
| 187 | 406 | 407 | 429 | 406 | 3.88 | 2 | 60 | 40 | |
| 188 | 414 | 415 | 437 | | 7.02 | 2 | 60 | 40 | |
| 189 | 345 | 346 | 368 | 344 | 4.9 | 2 | 75 | 25 | |
| 190 | 406 | 407 | 429 | | 4.68/6.62 | 2 | 70 | 30 | |
| 191 | 452 | 453 | 475 | | 3.08/4.55 | 2 | 80 | 20 | |
| 192 | 392 | 393 | 415 | | 4.82 | 2 | 55 | 45 | |
| 193 | 371 | 372 | 394 | | 4.45/5.67 | 2 | 85 | 15 | |
| 194 | 425 | 426 | 448 | 424 | 2.60/2.90 | 2 | 80 | 20 | |
| 195 | 358 | 359 | 381 | 357 | 2.08 | 2 | 80 | 20 | |
| 196 | 364 | 365 | 387 | 363 | 5.12 | 2 | 80 | 20 | |
| 197 | 326 | 327 | 349 | 325 | 2.48 | 2 | 70 | 30 | |
| 198 | 323 | 324 | 346 | 322 | 1.27 | 2 | 70 | 30 | |
| 199 | 314 | 315 | 337 | 313 | 252 | 2 | 80 | 20 | |
| 200 | 346 | 347 | 369 | 345 | 0.98 | 2 | 80 | 20 | |
| 201 | 322 | 323 | 345 | 321 | 1.97 | 2 | 80 | 20 | |
| 202 | 542 | 542 | 564 | | 4.28 | 2 | 70 | 30 | |
| 203 | 542 | 542 | 564 | | 4.15 | 2 | 70 | 30 | |
| 204 | 377 | 378 | 400 | 376 | 5.8 | 2 | 70 | 30 | 8.71(2H, d, J = 8.79Hz), 8.18-820(1H, m), 7.87-7.93(2H, m), 7.78(1H, s), 7.44(2H. t. J = 7.62Hz), 7.13-7.22(3H, m), 4.61-4.64(2H, m), 3.08(2H, q, J = 7.62Hz), 2.34(3H, s), 1.41-1.48(2H, m). 0.82(3H, t, J = 3.81 Hz) |
| 205 | 411 | 412 | | 410 | 4.93 | 2 | 75 | 25 | |
| 206 | 363 | 364 | | 362 | 5.18 | 2 | 65 | 35 | |
| 207 | 483 | 483 | 505 | | 3.95 | 2 | 60 | 40 | |
| 208 | 414 | 415 | 437 | | 4.2 | 2 | 50 | 50 | 8.70(2H. d, J = 8.79Hz), 8.14(2H, d, J = 8.79Hz), 7.84-7.90(3H, m), 7.66(1H, brs), 7.48(1H, brs), 7.35(1 H, s), 7.15(2H, d, J = 9.08Hz). 4.61(2H, s), 2.36(3H, s) |
| 209 | 443 | 443 | | 441 | 5.4 | 2 | 50 | 50 | |
| 210 | 491 | 491 | | 489 | 2.67 | 2 | 70 | 30 | 10.20(1H, s), 8.71(2H, d, J = 9.08Hz). 8.14(2H, d, J = 8.79Hz), 7.84-7.90(3H, m), 7.63(2H, d, J = 8.50Hz), 7.31-7.36(4H, m), 7.21(3H, d, J = 8.79Hz), 7.09(2H, t, J = 7.33Hz), 4.89(2H, s), 2.36(3H, s) |
| 211 | 457 | 457 | 479 | | 3.87 | 2 | 60 | 40 | 8.70(2H, d, J = 9.08Hz), 8.12-8.19(1H, m), 8.02(4H, Abtype, J = 76.05, 8.94Hz), 7.83(1H, s), 7.34(2H, s), 7.15(2H, d. J = 9.08Hz), 4.65(2H, s). 3.10(2H, q, J = 6.60Hz), 2.36(3H, s), 1.45(2H, q, J = 7.33Hz), 0.84(3H, t, J = 7.33Hz) |
| 212 | 354 | 355 | | 353 | 4.49 | 2 | 85 | 15 | |
| 213 | 433 | 434 | 456 | 432 | 273 | 2 | 80 | 20 | 8.69(2H, d, J = 9.08Hz), 8.12(2H, d, J = 9.08Hz), 7.90(1H, s), 7.87-7.89(2H, m), 7.50(2H, t, J = 7.92Hz), 7.27-7.34(3H, m), 7.19(2H, d, J = 7.62Hz), 7.13(2H, d, J = 8.79Hz) |
| 214 | 415 | 416 | 438 | 414 | 6.6 | 2 | 50 | 50 | |
| 215 | 443 | 444 | 466 | | 5 | 2 | 60 | 40 | |
| 216 | 493 | 493 | 515 | | 9.53 | 2 | 65 | 35 | 8.72(2H, d, J = 9.08Hz), 8.29(2H, d, J = 8.79Hz), 8.14(2H, d, J = 8.59Hz), 7.87(3H, t. J = 8.79Hz), 7.77(2H, d, J = 8.21 Hz), 7.34(2H, s), 5.46(2H, s), 3.34(2H, d, J = 0.59Hz), 2.36(3H, s) |
| 217 | 363 | 364 | | | 4.42/5.22 | 2 | 50 | 50 | |
| 218 | 357 | 358 | 380 | 356 | 2.92 | 2 | 60 | 40 | |
| 219 | 357 | 358 | 380 | 356 | 3.3 | 2 | 60 | 40 | |
| 220 | 512 | 512 | 534 | | 3.85/4.33 | 2 | 65 | 35 | |
| 221 | 554 | 554 | 576 | | 8.08 | 2 | 70 | 30 | |
| 222 | 385 | 386 | 408 | 384 | 2.83 | 2 | 60 | 40 | |
| 223 | 385 | 386 | 408 | 384 | 1.73 | 2 | 60 | 40 | |
| 224 | 461 | 461 | 483 | 459 | 2.97 | 2 | 70 | 30 | 10.74(1H, s), 8.70(2H, d, J = 9.08Hz), 8.15(2H, d, J = 8.79Hz), 7.97-8.05(4H, m), 7.90(2H, d, J = 8.79Hz), 7.84(1H, s), 7.55-7.64(3H, m), 7.35(2H, s), 2.38(3H, s) |
| 225 | 479 | 479 | 501 | 477 | 7.45 | 2 | 60 | 40 | 8.70(2H, d, J = 9.38Hz), 8.13-8.16(2H, m), 7.84-7.96(5H, m), 7.62-7.72(3H, m). 7.34-7.43(4H, m), 2.38(3H, s) |
| 226 | 479 | 479 | 501 | 477 | 5.69 | 2 | 65 | 35 | 10.75(1H, s), 8.70(1H, d, J = 9.0BHz), 8.00-8.16(6H, m), 7.84-7.91(3H, m), 7.35-7.44(5H, m) |
| 227 | 511 | 511 | 533 | 509 | 5.67 | 2 | 70 | 30 | 10.93(1H, s), 8.73(2H, d, J = 9.08Hz). 8.64(1H, s), 8.06-8.17(8H, m), 7.85-7.92(3H, m), 7.66-7.69(2H, m), 7.35(2H, s), 2.39(3H, s) |
| 228 | 491 | 491 | 513 | 489 | 5.57 | 2 | 65 | 35 | 10.58(1H, s), 8.69(2H, d, J = 8.79Hz), 8.15(2H, d, J = 8.79Hz), 7.91-8.03(4H, m), 7.88(2H, d, J = 2.05Hz), 7.83(1H, s), 7.35(2H, s), 7.10(2H, d, J = 8.79Hz), 3.86(3H, s), 2.38(3H, s) |
| 229 | 491 | 491 | 513 | 489 | 3.68 | 2 | 70 | 30 | 10.67(1H, s), 8.69(2H, d, J = 9.08Hz), 8.02(4H, Abtype, J = 85.81, 7.91 Hz), 7.95(2H, d, J = 8.79Hz), 7.62-7.65(1H, m), 7.51-7.57(1H, m), 7.34(2H, s), 7.21(1H, d, J = 7.91Hz), 7.09(1H, t, J = 8.06Hz), 3.91(3H, s), 2.38(3H, s) |
| 230 | 487 | 487 | 509 | 485 | 5.23 | 2 | 70 | 30 | 10.76(1H, s), 8.69(2H, d, J = 8.79Hz), 8.15(2H, d, J = 8.79Hz), 7.88-7.93(4H, m), 7.82(1H, s), 7.65-7.70(3H, m), 7.43-7.48(3H, m), 7.35(2H, s), 6.89(1H, d, J = 15.82Hz), 2.37(3H, s) |
| 231 | 450 | 451 | 473 | 449 | 5.02 | 2 | 60 | 40 | 10.67(1H, s), 8.68(2H, d, J = 8.21 Hz), 8.02(4H, Abtype, J = 75.75, 8.94Hz), 7.83(1H, s), 7.44(1H, d, J = 3.81Hz), 7.35(2H, s), 6.75-6.77(1H, m), 2.37(3H, s) |
| 232 | 439 | 439 | 461 | 437 | 7.88 | 2 | 60 | 40 | |
| 233 | 386 | 387 | | | 4 | 1 | gradient 1 | | 12.93(1H, brs), 9.09(1H, s), 8.78(1H, d, J = 8.77Hz), 7.99-8.22(8H, m), 7.62-7.75(2H, m), 4.63(2H, s), 3.42(3H, s) |
| 234 | 473 | 473 | | | 3.96 | 1 | gradient 1 | | 12.90(1H, brs), 8.79(1H, s), 8.02-8.11 (5H, m), 7.92(1H, s), 7.29-7.50(6H, m). 5.27(2H, s), 4.56(2H, s), 3.90(3H, s), 3.38(3H, s) |
| 235 | 479 | 479 | 501 | | 1.11 | 1 | gradient 2 | | 12.85(1H, brs), 8.64(2H, d, J = 9.05Hz), 8.07(4H, Abtype, J = 33.33, 8.97Hz), 7.77(1H, s), 7.08(2H, d. J = 9.87Hz), 4.53(2H, s), 3.64(3H, s). 3.55-3.65(8H, m), 3.35(3H, s) |
| 236 | 405 | 406 | 428 | | 1.23 | 1 | gradient 2 | | 12.82(1H, brs), 8.63-8.67(2H, m), 8.07(4H. Abtype, J = 40.44, 8.90Hz), 7.72(1H, s), 6.74(2H. d, J = 9.12Hz), 4.52(2H, s), 3.33-3.37(4H, m), 3.35(3H, s), 1.98-2.02(4H, m) |
| 237 | 343 | 344 | 366 | | 0.87 | 1 | gradient 2 | | |
| 238 | 402 | 403 | | | 2.47 | 1 | gradient 1 | | 12.89 (1 H, brs), 8.76(2H, d, J = 8.67Hz), 8.53 (1H, s), 7.93-8.21(8H, m). 7.56(1 H, s), 4.59(2H, s), 3.40(3H, s) |
| 239 | 394 | 395 | 417 | | 1.13 | 1 | gradi ent 2 | | 12.91(1H, brs), 8.59 (1H, s), 8.01-8.10(4H, m), 7.87 (1H, s), 7.07 (1H, d, J = 8.53Hz), 4.55 (2H, s), 4.36(4H, dd, J = 20.93, 3.97Hz), 3.37(3H, s) |
| 240 | 442 | 442 | | | 0.75 | 1 | gradi ent 2 | | |
| 241 | 435 | 435 | 457 | | 1.06 | 1 | gradi ent 2 | | |
| 242 | 402 | 403 | | | 3.61 | 1 | gradi ent 1 | | 12.91 (1H, brs), 8.72-8.79(3H, m), 8.01-8.11(7H, m), 7.88(1H, s), 6.66(1H, s), 4.59(2H, s), 3.40(3H, s) |
| 243 | 403 | 404 | | | 3.42 | 1 | gradient 1 | | 12.92(1H, brs), 9.51(1H, s), 8.78(2H, d, J = 8.77Hz), 8.34(1H, s), 8.02-8.11(7H, m), 4.59(2H, s), 3.40(3H, s) |
| 244 | 404 | 405 | | | 3.72 | 1 | gradient 1 | | 12.90(1H, s), 10.75(1H, s), 8.78(1H, s), 8.51(1H, s), 8.11(4H, Abtype, J = 37.07, 8.84Hz), 8.11-8.14(1H, m), 7.93(1H, s, J = Hz), 7.88(1 H, s. J = Hz), 16.95-7.01(2H, m), 3.91(3H, s) |
| 245 | 501 | 501 | | | 1.26 | 1 | gradient 2 | | 12.87(1H, brs), 8.69(2H, d, J = 9.16Hz), 8.48(1H, s), 8.11(4H, Abtype, J = 48.55, 8.96Hz), 7.91(1H, s), 7.78(1H, s), 6.99(2H, d, J = 0.75Hz). 6.99(1H, s), 3.64(3H, s), 3.35-3.63(8H, m) |
| 246 | 442 | 443 | 465 | | 1.54 | 1 | gradient 2 | | 12.90(1H, brs), 8.73(1H, d, J = 3.91 Hz), 8.58(1H, s), 8.13-8.19(1H, m), 8.11(4H, Abtype, J = 35.55, 8.93Hz), 7.95(1H, s), 7.88(1H, s), 7.56(1 H, J = Hz), 7.41-7.48(1H, m), 7.34(2H, t, J = 3.76Hz), 7.04(1H, s) |
| 247 | 378 | 379 | 401 | | 1.01 | 1 | gradient 2 | | 12.90(1H, s), 8.73(1H, d, J = 3.67Hz), 8.55(1H, s). 8.11(4H, Abtype, J = 35.06, 8.90Hz), 7.92(1H, s), 7.77(1H, s), 7.02(1H, s), 6.84(1H, d. J = 3.69Hz), 5.67(1H, brs), 4.60(2H, s) |
| 248 | 427 | 428 | 450 | | 1.39 | 1 | gradient 2 | | 12-84(1H, brs), 8.71(2H, brd, J = 8.64Hz), 8.45(1H, s), 8.11(4H, Abtype, J = 55-86, 8.88Hz), 7.89-7.90(1 H, m). 7.73 (1H, s), 6.98 (1H, d, J = 1.14Hz), 6.74(2H, d, J = 9.19Hz), 3.45-3.50(4H, m), 1.98-2.02(4H, m) |
| 249 | 365 | 366 | 366 | | 3.17/3.79 | 1 | gradient 1 | | |
| 250 | 424 | 425 | | | 2.91/3.05 | 1 | gradient 1 | | 12.91 (1H, brs), 8.75 (2H, d, J = 8.61 Hz), 8.60(1H, s), 8.54(1H, s), 7.71-8.15(9H, m), 7.19(1H, s), 7.05(1H, s) |
| 251 | 464 | 464 | | | 1.69 | 1 | gradient 2 | | |
| 252 | 425 | 426 | | | 3.67 | 1 | gradient 1 | | 12.93(1H, brs), 9.50(1H, s), 8.76(2H, d, J = Hz), 8.60(1H, s), 8.34(1H, s), 8.03-8.15(7H, m), 7.94(1H, s), 7.05(1H, s) |
| 253 | 356 | 357 | | | 4 | 1 | gradient 1 | | 12.88(1H, brs), 9.11 (1H, s), 8.76(1H, dd, J = 8.61, 1.61 Hz), 8.00-8.13(8H, m), 7.62-7.73(2H, m), 2.50(3H, s) |
| 254 | 448 | 449 | 471 | | 1.12 | 1 | gradient 2 | | 12.81 (1H, s), 8.64(2H, d, J = 4.45Hz), 8.05(4H, Abtype. J = 38.34, 8.80Hz). 7.64(1H, s), 7.06(2H, d, J = 9.04Hz). 3.64(3H, s), 3.55(8H, brd, J = 6.07Hz), 232(3H, s) |
| 255 | 326 | 327 | 349 | | 0.69 | 1 | gradient 2 | | 12.85(1H, brs), 8.63(0.7H, d, J = 3.61 Hz), 8.05(4H, Abtype, J = 24.69, 8.96Hz), 8.00-8.11(2H, m), 7.85(0.7H, s), 7.64(0.3H, d, J = 3.61 Hz), 7.55(0.3H, s), 6.80(0.7H, d, J = 3.58Hz). 6.74(0.3H, d, J = 3.58Hz), 5.63(1H, t, J = 5.85Hz), 4.59(2H, t, J = 5.26Hz), 2.33(3H, s) |
| 256 | 375 | 376 | 398 | | 1.25 | 1 | gradient 2 | | |
| 257 | 313 | 314 | 336 | | 0.87 | 1 | gradient 2 | | |
| 258 | 372 | 373 | | | 2.59 | 1 | gradient 1 | | 12.87(1H, brs), 8.77(2H, d, J = 8.69Hz), 8.51(1H, s), 7.91-8.10(8H, m), 7.18(1H, s), 2.38(3H, s) |
| 259 | 364 | 365 | 387 | | 1.12 | 1 | gradient 2 | | 12.85(1H, s), 8.56(1H, s), 7.99-8.10(5H, m), 7.74(1H, s), 7.05(1 H, d, J = 8.47Hz), 4.35(4H, dd, J = 19.25, 3.68Hz), 2.33(3H, s) |
| 260 | 411 | 412 | | | 0.81 | 1 | gradient 2 | | |
| 261 | 372 | 373 | 395 | | 1.1 | 1 | gradient 2 | | 1286(1 H. s), 8.77(2H. d, J = 8.57Hz), 8.70(1H, s), 8.00-8.10 (6H, m), 7.87(2H. s), 6.65 (1H, s). 2.36 (3H, s) |
| 262 | 373 | 374 | | | 3.41 | 1 | gradient 1 | | 1288(1H, brs), 10.04(1H. s). 9.49(1H, s), 8.79(2H, d, J = 8.81 Hz), 8.34(1H, s), 8.00-8.11(5H m). 7.92(1H. s), 2.38(3H. s) |
| 263 | 428 | 429 | 451 | | 1.19 | 1 | gradient 2 | | 12.87(1H, s), 10.71 (1 H, s), 8.83(1 H, s), 8.07(4H. Abtype, J = 24.69, 8.92Hz). 7.83-7.89(2H, m), 7.44(2H. d, J = 7.23Hz). 7.34(2H. t, J = 7.43Hz). 7.23(1 H, t, J = 7.24Hz), 6.95(1 H, d, J = 8.34Hz), 4.15(2H, s), 3.88(3H, s) |
| 264 | 432 | 433 | 455 | | 1.52 | 1 | gradient 2 | | |
| 265 | 519 | 519 | | | 1.52 | 1 | gradient 2 | | 12-88(1H. s), 8.81(1H, s), 8.06(4H. Abtype, J = 18.56, 6.92Hz), 7.96-8.00(1H, m). 7.89(1H. s). 7.15-7.56(11H, m), 5.25(2H, s). 4.16(2H, s), 3.88(3H, s) |
| 266 | 525 | 525 | 547 | | 1.33 | 1 | gradient 2 | | 12.84(1H, brs), 8.58(2H, d, J = 9.12Hz), 8.07(4H, Abtype, J = 36.50, 8.94Hz), 7.71 (1H, s), 7.43(2H, d, J = 7.16Hz), 7.33(2H, t, J = 7.42Hz). 7.22(1H, t, J = 7.26Hz). 7.05(2H, d, J = 9.27Hz), 4.12(2H, m), 3.64(3H, s), 3.53-3.58(8H, m) |
| 267 | 466 | 467 | 489 | | 1.6 | 1 | gradient 2 | | 12.88(1H, s), 8.68(1H, s), 8.02-8.12(5H, m), 7.83(1H, s), 7.52-7.59(1H, m). 7.22-7.45(8H, m), 4.19(2H, s) |
| 268 | 402 | 403 | 425 | | 1.13 | 1 | gradient 2 | | |
| 269 | 452 | 452 | | | 1.46 | 1 | gradient 2 | | |
| 270 | 389 | 390 | | | 3.9 | 1 | gradient 1 | | |
| 271 | 448 | 449 | | | 3.38 | 1 | gradient 1 | | |
| 272 | 440 | 441 | 463 | | 1.38 | 1 | gradient 2 | | 12.88(1H, s), 8.52(1H, s), 8.05(4H. Abtype, J = Hz). 7.98-7.98(1H, m). 7.83(1H, s). 7.44(2H, d, J = 7.29Hz), 7.33(2H, t, J = 7.45Hz), 7.23(1H, t, J = 7.24Hz), 7.04(1H, d, J = 8.54Hz), 4.34(4H, dd, J = 20.62, 3.95Hz), 4.14(2H, s) |
| 273 | 488 | 488 | | | 1.13 | 1 | gradient 2 | | |
| 274 | 481 | 481 | | | 1.33 | 1 | gradient 2 | | |
| 275 | 448 | 449 | 471 | | 1.36 | 1 | gradient 2 | | |
| 276 | 449 | 450 | | | 3.83 | 1 | gradient 1 | | |
| 277 | 565 | 565 | | | 1.45 | 1 | gradient 2 | | 12.90(1H, brs), 8.74(1H, s), 8.04-8.19(5H, m), 7.78(1H, s), 7.36-7.56(6H, m), 7.24-7.28(2H, m), 7.15-7.18(1H, m), 5.27(2H, s), 3.84-3.89(9H, m) |
| 278 | 480 | 481 | | | 3.47 | 1 | gradient 1 | | |
| 279 | 571 | 571 | | | 1.23 | 1 | gradient 2 | | 12.86(1H, s), 8.62(2H, d, J = 9.16Hz), 8.12(4H, Abtype, J = 52.08, 8.94Hz), 7.63(1H, s), 7.22-7.25(2H, m), 7.15(1H, d, J = 8.92Hz), 7.06(2H, d, J = 9.28 Hz), 3.85(3H, s). 3.64 (3H, s), 3.44-3.63(8H, m) |
| 280 | 448 | 449 | 471 | | 1.04 | 1 | gradient 2 | | |
| 281 | 498 | 498 | | | 3.99 | 1 | gradient 1 | | 12.83(1 H, s), 8.62(2H, brd, J = 7.77Hz), 8.12(4H, Abtype, J = 58.90, 8.86Hz), 7.58(1H, s), 7.22(2H, d, J = 7.36Hz), 7.14(1H. d, J = 8.66Hz), 6.71(2H, d, J = 9.12Hz), 3.85(6H, s), 3.46-3.57(4H, m), 1.90-2.00(4H. m) |
| 282 | 495 | 495 | | | 2.79 | 1 | gradient 1 | | |
| 283 | 486 | 487 | | | 1.31 | 1 | gradient 2 | | 12.91 (1H, brs), 8.54(1H, s), 8.10(4H, Abtype, J = 37.88, 8.88Hz), 8.00-8.11 (1H, m), 7.73(1 H. s), 7.26-7.28(2H, m), 7.15(1 H. d, J = 8.78Hz), 7.03(1 H, d, J = 8.54Hz). 4.35(4H, dd. J = 21.23, 3.34Hz), 3.85(6H, s) |
| 284 | 534 | 534 | | | 1.02 | 1 | gradient 2 | | |
| 285 | 527 | 527 | | | 2.65 | 1 | gradient 1 | | |
| 286 | 415 | 416 | | | 0.92 | 1 | gradient 2 | | |
| 287 | 438 | 439 | | | 1.32 | 1 | gradient 2 | | 12.87(1 H, s). 8.77(2H, d, J = 5.57Hz), 8.63-8.67(2H. m), 8.12(4H, Abtype, J = 52.73, 8.90Hz). 7.72(2H, d, J = 5.98Hz), 7.62(1 H, s), 6.73(2H, d, J = 9.21 Hz), 3.46-3.50(4H, m), 1.98-2.02(4H, m) |
| 288 | 427 | 428 | | | 3.75 | 1 | gradient 1 | | |
| 289 | 506 | 506 | | | 4.33 | 1 | gradient 1 | | |
| 290 | 453 | 454 | | | 1.64 | 1 | gradient 2 | | |
| 291 | 438 | 439 | | | 1.39 | 1 | gradient 2 | | |
| 292 | 427 | 428 | | | 4.04 | 1 | gradient 1 | | |
| 293 | 475 | 475 | | | 1.04 | 1 | gradient 2 | | |
| 294 | 435 | 436 | | | 3.91 | 1 | gradient 1 | | |
| 295 | 394 | 395 | | | 1.27 | 1 | gradient 2 | | 12.86(1H. brs), 10.65(1H, brs), 8.80(1H, s), 8.06(4H, Abtype, J = 27.29, 8.93Hz). 8.00-8.06 (1 H, m), 7.77(1 H, s), 6.96(1H, d, J = 8.37Hz), 2.74(2H, t. J = 7.57Hz), 2.50(3H, s), 1.66-1.76(2H, m), 1.38-1.51 (2H, m), 0.96(3H, t. J = 7.32Hz) |
| 296 | 485 | 485 | 507 | | 1.56 | 1 | gradient 2 | | 12.86(1H. s). 8.79(1H, s), 7.94-8.15(5H. m), 7.81 (1H. s). 7.34-7.50(5H, m), 7.28(1H, d, J = 8.66Hz), 5.25(2H, s), 3.88(3H, s). 2.74(2H, t. J = 7.59Hz). 1.66-1.76(2H, m), 1.39-1.51(2H, m). 0.96(3H, t, J = 7.32Hz) |
| 297 | 400 | 401 | | | 1.48 | 1 | gradient 2 | | |
| 298 | 491 | 491 | 513 | | 1.39 | 1 | gradient 2 | | 12.82 (1H, s), 8.65 (2H, d, J = 9.13Hz), 8.06 (4H, Abtype, J = 37.60, 8.88Hz), 7.67 (1H, s), 7.07 (2H, d, J = 9.13Hz), 3.64 (3H, s), 3.55(8H, d, J = 6.29Hz). 2.72(2H, t. J = 7.56Hz), 2.51 (3H, s). 1.64-1.74 (2H, m), 1.38-1.50 (2H, m), 0.95 (3H, t. J = 7.32Hz) |
| 299 | 432 | 433 | 455 | | 1.64 | 1 | gradient 2 | | |
| 300 | 368 | 369 | 391 | | 1.14 | 1 | gradient 2 | | 1 2-85(1H, brs), 8.64(1 H, d, J = 3.59Hz). 8.00-8.11(4H, m), 7.74(0.7H, s). 7.65(0.3H, d, J = 3.59Hz), 7.55(0.3H, s), 6.79(0.7H, d, J = 3.59Hz), 6.75(0.3H, d, J = 3.59Hz). 5.62-5.64(1 H, m). 4.59(2H, d, J = Hz), 2.74(2H, t, J = Hz). 2.50(3H, s), 1.62-1.73(2H. m), 1.37-1.49(2H, m). 0.94(3H, t, J = 7.32Hz) |
| 301 | 418 | 418 | 440 | | 1.52 | 1 | gradient 2 | | 12.79(1H, s), 8.65(2H, d, J = 8.39Hz). 8.06(4H, Abtype, J = 44.48, 8.95Hz), 7.59(1H, s), 6.7(2H, d, J = 9.17Hz), 3.44(3H, m), 2.69 (2H, t, J = 7.58Hz), 1.97-2.01(4H, m), 1.63-1.74(2H, m), 1.37-1.49(2H, m), 0.95(3H, t, J = 7.31 Hz) |
| 302 | 414 | 415 | | | 1.31 | 1 | gradient 2 | | 12.88(1H, brs), 8.76(2H, d, J = 8.80Hz), 8.49-8.51 (1 H, m), 8.05(4H, Abtype, J = 19.94, 8.98Hz). 7.90-7.95(4H, m). 7.18(1H. s). 2.75(2H, t, J = 7.59Hz), 1.67-1.78(2H, m), 1.40-1.52(2H, m), 0.96(3H, t, J = 7.32Hz) |
| 303 | 406 | 407 | 429 | | 1.44 | 1 | gradient 2 | | 12.86(1H, s), 8.57(1H, s, J=), 8.02-8.10(3H, m), 7.77(1H, s), 7.05(1H, d, J = 8.55Hz), 4.38-4.39(2H, m), 4.33-4.38(2H, m), 2.72(2H, t, J = 7.55Hz), 1.65-1.72(2H, m), 1.4-1.47(2H, m), 0.95(3H, t, J = 7.30Hz) |
| 304 | 454 | 454 | | | 1.16 | 1 | gradient 2 | | |
| 305 | 414 | 415 | 437 | | 1.38 | 1 | gradient 2 | | 12.87(1H, brs), 8.78(2H, d, J = 8.90Hz), 8.70(2H. s), 7.91-8.11(7H, m). 7.86(2H, d, J = 1.57Hz). 6.65 (1H, s), 2.76(2H. t, J = 7.60Hz). 1.67-1.78(2H. m), 1.40-1.52(2H, m), 0.96 (3H, t, J = 7.32Hz) |
| 306 | 415 | 416 | | | 3.9 | 1 | gradient 1 | | |
| 307 | 512 | 512 | | | 3.59 | 1 | gradient 1 | | |
| 308 | 389 | 390 | 412 | | 0.84 | 1 | gradient 2 | | |
| 309 | 438 | 439 | | | 1.26 | 1 | gradient 2 | | |
| 310 | 427 | 428 | | | 1.17 | 1 | gradient 2 | | |
| 311 | 475 | 475 | | | 0.87 | 1 | gradient 2 | | |
| 312 | 378 | 379 | 401 | | 1.11 | 1 | gradient 2 | | 12.85(1H, brs), 10.66(1H, s), 8.83(1H, s), 8.04(4H, Abtype, J = 24.37, 8.84Hz). 7.98-8.09(2H, m), 6.98(1H, d, J = 8.35Hz), 3.90(3H, s), 2.22-2.24(1H, m), 1.01-1.03(4H, m) |
| 313 | 382 | 383 | 405 | | 1.45 | 1 | gradient 2 | | 12.88(1H, brs), 9.12(1H, s), 8.77(1H, d, J = 8.72Hz), 8.28(1H, s). 8.00-8.10(7H, m), 7.61-7.73(2H, m), 2.29-2.33(1H, m). 0.95-1.08(4H, m) |
| 314 | 469 | 469 | 491 | | 1.45 | 1 | gradient 2 | | 12-85(1H, s), 8.81(1H, d, J = 1.85Hz). 7.96-8.15(6H, m), 7.34-7.60(5H, m). 7.30(1H, d. J = 8.65Hz), 5.26(2H, s), 3.89(3H, s), 2.20-2.31(1H, m), 1.02-1.04(4H, m) |
| 315 | 475 | 475 | 497 | | 1.36 | 1 | gradient 2 | | 12.81 (1H, s), 8.67(2H, d, J = 9.10Hz), 8.04(4H, Abtype, J = 34.39, 8.86Hz), 7.90(1H, s), 7.08(2H, d, J = 9.10Hz), 3.64(3H, s). 3.55-3.57(8H, m). 2-22-2.31(1H, m), 0.99-1.00(4H, m) |
| 316 | 352 | 353 | 375 | | 1.13 | 1 | gradient 2 | | 12.85(1H, s), 8.65(1H, d, J = 3.68Hz). 8.04-8.08(4H, m). 8.01 (1H, s), 6.80(0.75H. d, J = 3.68Hz), 6.75(0.25H. d, J = 3.68Hz). 5.61-5.66(1 H, m). 4.59(1H, d, J = 5.51 Hz), 2.21-2.31 (1H, m), 1.00-1.04(4H, m) |
| 317 | 401 | 402 | | | 1.47 | 1 | gradient 2 | | |
| 318 | 398 | 399 | | | 1.26 | 1 | gradient 2 | | 12.878(1H, s), 8.79 (2H, d, J = 8.83Hz), 8.52(1H, s), 8.17(1H, s), 7.93-8.10(7H, m), 7.19(1H, s), 2.26-2.30(1 H, m), 1.05-1.07(4H, m) |
| 319 | 390 | 391 | | | 1.39 | 1 | gradient 2 | | 12.85(1H, s). 8.60(1 H, s). 7.98-8.05(6H, m), 7.07(1H d, J=8.53Hz), 4.36(4H, d, J = 16.97Hz), 2.22-2.25(1H, m), 0.90-1.10(4H, m), 2.22-2.25(1H, m) |
| 320 | 438 | 438 | | | 1.15 | 1 | gradient 2 | | |
| 321 | 431 | 431 | 453 | | 1.34 | 1 | gradient 2 | | 8.65(2H. d, J = 9.05Hz). 8.04(4H. Abtype, J = 35.08. 8.91 Hz). 7.89(1 H, s). 7.03-7.11 (2H, m), 3.19-3.53 (3H, m), 2.45(4H, brs), 2.22-2.24(5H, m), 0.98-1.00(4H, m) |
| 322 | 398 | 399 | 421 | | 1.36 | 1 | gradient 2 | | 12.86(1 H, s). 8.79(2H, d, J = 8.71 Hz). 8.71(1H. s). 7.98-8.13(7H. m). 7.87(1H, s). 6.65(1 H, s). 2.24-2.26(1 H, m). 0.83-1.06(4H, m) |
| 323 | 399 | 400 | | | 3.7 | 1 | gradient 1 | | 12-88(1H, brs), 9.50(1H, s). 8.80(2H, d, J = 8.84Hz). 8.34(1 H, s), 7.99-8.17(7H, m), 2.23-2.30(1H. m), 1.05-1.07(4H, m) |
| 324 | 444 | 445 | | | 1.32 | 1 | gradient 2 | | 12.82(1H, brs), 10.68(1H, brs), 8.73(1H, s), 8.17(2H, d, J = 8.88Hz). 8.04(2H, d, J = 8.88Hz), 7.97-8.00(1H, m), 7.67(3H, t. J = 4.36Hz), 7.15(2H, d, J = 8.78Hz), 6.95(1H, d, J = 8.39Hz), 3.90(3H, s), 3.86(3H, s) |
| 325 | 448 | 449 | | | 4.28/4.57 | 1 | gradient 1 | | 12.86(1H, brs), 9.00(1H, s), 8.72(1H, d, J = 8.84Hz), 8.18(2H, d, J = 8.84Hz), 7.97-8.07(4H, m), 7.60-7.92(8H, m), 7.18(2H, d, J = 8.76Hz), 3.88(3H, s) |
| 326 | 523 | 523 | 545 | | 1.58 | 1 | gradient 2 | | 12.86(1H, brs), 8.15(1H, s). 8.03-8.10(5H, m), 7.78-7.83(2H, m), 7.72(1H, s), 7.36-7.49(1H, m). 7.25-7.28(1 H, m). 5.27(2H, s), 3.88(3H, s) |
| 327 | 388 | 389 | | 387 | 3.65 | 1 | gradient 1 | | 12.94(1H, brs), 9.60(1H, d, J = 7.32Hz), 9.27(1H, s), 8.08-9.13(2H, m), 8.32-8.42(1H, m), 8.05-8.09(5H, m), 4.67(2H, s), 3.44(3H, s) |
| 328 | 356 | 357 | | 355 | 2.81/3.11 | 1 | gradient 1 | | 12.91(1H, brs), 8.67(1H, d, J = 3.62Hz), 8.06(4H, q, J = 9.04Hz), 7.80(1H, s), 6.82(1H, d, J = 3.61 Hz), 4.59(2H, s), 4.56(2H, s), 3.36(3H, s) |
| 329 | 410 | 411 | | 409 | 4.06 | 1 | gradient 1 | | 12.93(1H, s), 9.39(1H, d, J = 7.57Hz), 9.24(1H, s), 9.09-9.10(2H, m), 8.54(1H, s), 8.40(1H, d, J = 7.31 Hz), 7.99-8.14(6H, m), 7.08(1H, s) |
| 330 | 416 | 417 | | 415 | 3.95 | 1 | gradient 1 | | 12.91 (1H, s), 8.53-8.57(2H, m), 8.11-8.13(1H, m), 8.09(4H, ABtype, J = 25.28, 8.86Hz), 7.91(2H, d, J = 11.43Hz), 7.06(1H, d, J = 8.56Hz), 7.01(1H, d, J = 1.23Hz), 4.39(4H, dd, J = 21.7, 3.22Hz) |
| 331 | 358 | 359 | | 357 | 3.79 | 1 | gradient 1 | | |
| 332 | 474 | 475 | | 473 | 3.63 | 1 | gradient 1 | | |
| 333 | 479 | 479 | | | 3.93 | 1 | gradient 1 | | 12.93(1H, s), 9.03 (1H, s), 8.74(1H, d, J = 8.74Hz), 8.19(2H, d, J = 9.01 Hz), 8.01-8.08(6H. m), 7.61-7.73(2H, m), 7.35-7.37(2H, m), 7.19(1 H, d, J = 8.91 Hz), 3.88(6H, s) |
| 334 | 389 | 390 | | 388 | 3.13 | 1 | gradient 1 | | |
| 335 | 512 | 512 | | 510 | 3.84 | 1 | gradient 1 | | |
| 336 | 398 | 399 | | 397 | 4.51/4.80 | 1 | gradient 1 | | |
| 337 | 415 | 416 | | 414 | 3.44 | 1 | gradient 1 | | |
| 338 | 506 | 506 | | 504 | 3.99 | 1 | gradient 1 | | |
| 339 | 453 | 454 | | 452 | 3.78/4.34 | 1 | gradient 1 | | |
| 340 | 447 | 447 | | 445 | 2.81 | 1 | gradient 1 | | |
| 341 | 453 | 454 | | 452 | 3.81/4.45 | 1 | gradient 1 | | |
| 342 | 493 | 495 | | | 3.63/3.84 | 1 | gradi ent 1 | | |
| 343 | 494 | 496 | | | 3.40/3.68 | 1 | gradient 1 | | |
| 344 | 510 | 512 | | 510 | 3.61 | 1 | gradient 1 | | |
| 345 | 417 | 420 | | | 4 | 1 | gradient 1 | | |
| 346 | 467 | 467 | 489 | | 8.53 | 2 | 70 | 30 | |
| 347 | 501 | 501 | 523 | 499 | 4.17 | 2 | 80 | 20 | 8.81-8.81(1H, m), 8.00-8.11(8H. m), 7.81 (1H, s), 7.62(2H, d. J = 8.21 Hz). 7.27(1H, d, J = 8.79Hz), 5.37(2H, s), 3.91(3H, s), 3.86(3H, s), 2.35(3H, s) |
| 348 | 416 | 417 | | 415 | 7.35 | 2 | 50 | 50 | |
| 349 | 345 | 346 | 368 | 344 | 5.1 | 2 | 70 | 30 | |
| 350 | 389 | 390 | | 388 | 6.98 | 2 | 75 | 25 | 8.61(2H, d, J = 9.08Hz). 8.05(4H, Abtype, J = 80.95, 7.77Hz). 7.60(1H, s), 7.05(2H, d, J = 9.38Hz), 2.50-2.52 (4H, m), 2-31(3H, s), 1.58-1.62(6H, m) |
| 351 | 324 | 325 | 347 | 323 | 5.53 | 2 | 70 | 30 | |
| 352 | 457 | 457 | 479 | 455 | 8.65 | 2 | 60 | 40 | |
| 353 | 490 | 490 | | 488 | 5.8 | 2 | 75 | 25 | |
| 354 | 375 | 376 | 398 | 374 | 4.73 | 2 | 70 | 30 | |
| 355 | 345 | 346 | 368 | 344 | 4.03 | 2 | 70 | 30 | |
| 356 | 345 | 346 | 368 | 344 | 5.75 | 2 | 70 | 30 | |
| 357 | 414 | 415 | 437 | 413 | 3.07 | 2 | 70 | 30 | |
| 358 | 413 | 413 | 435 | 411 | 6.57 | 2 | 70 | 30 | |
| 359 | 359 | 360 | 382 | 358 | 7.77 | 2 | 70 | 30 | |
| 360 | 359 | 360 | 382 | 358 | 5.57 | 2 | 75 | 25 | 8.98(1H, s), 8.09(4H, Abtype, J = 35.93, 8.79Hz), 7.79-7.83(2H, m), 7.63(1H, d, J = 7.62Hz), 7.39-7.45(1H, m), 7.15(1H, t, J = 7.47Hz). 4.04(3H, s), 5.45(3H, s) |
| 361 | 443 | 443 | 465 | 441 | 7.63 | 2 | 60 | 40 | |
| 362 | 429 | 429 | 451 | 427 | 9.28 | 2 | 60 | 40 | |
| 363 | 372 | 373 | 395 | 371 | 8.13 | 2 | 60 | 40 | |
| 364 | 345 | 346 | 368 | 344 | 4.5 | 2 | 70 | 30 | |
| 365 | 345 | 346 | | 344 | 7.33 | 2 | 70 | 30 | |
| 366 | 375 | 376 | | 374 | 6.15 | 2 | 70 | 30 | |
| 367 | 345 | 346 | 368 | 344 | 6.2 | 2 | 70 | 30 | |
| 368 | 346 | 347 | 369 | 345 | 6.63 | 2 | 70 | 30 | 9.23-9.24(1H, m), 8.53-8.61(2H, m), 8.16-8.20(2H, m), 8.01 (1H, s), 7.76-7.83(2H, m), 7.58(1H, t, J = 7.91Hz), 7.21-7.21(1H, m), 2.40(3H, s) |
| 369 | 413 | 413 | 435 | 411 | 6.32 | 2 | 70 | 30 | |
| 370 | 346 | 347 | 369 | 345 | 7.88 | 2 | 70 | 30 | 9.21 (1H, s), 8.54(1H, dd, J = 8.79, 1.76Hz). 8.16-8.17(1H, m), 8.12-8.13(1H, m). 8.10-8.11(1H, m), 8.01-8.04(3H. m). 7.82(1H, d, J = 8.79Hz). 7.19-7.20(1H, m), 3.40(3H, s) |
| 371 | 443 | 443 | 465 | 441 | 7.4 | 2 | 60 | 40 | |
| 372 | 429 | 429 | 451 | 427 | 8.95 | 2 | 60 | 40 | |
| 373 | 429 | 429 | 451 | 427 | 6.03 | 2 | 60 | 40 | |
| 374 | 457 | 457 | 479 | 455 | 8.27 | 2 | 60 | 40 | |
| 375 | 506 | 506 | 528 | 504 | 6.8 | 2 | 60 | 40 | |
| 376 | 457 | 457 | 479 | 455 | 9.17 | 2 | 60 | 40 | |
| 377 | 380 | 380 | 402 | 378 | 4.22 | 2 | 80 | 20 | 9.84(1H, s), 8.64-8.66(1H, m), 8.39(1H, d, J = 1.76Hz), 8.23-8.24(1H, m), 8.19(1H, s), 7.72-7.75(1H, m), 7.62(1H, d, J = 8.79Hz), 7.52-7.57(1H, m), 7.31-7.34(1H, m), 2.44(3H, s) |
| 378 | 380 | 380 | 402 | 378 | 4.87 | 2 | 80 | 20 | 9.81 (1H, s). 8.39-8.40(1H, m), 8.19(2H, d, J = 5.28Hz). 8.15(1H, s), 7.99-8.02(2H, m), 7.61(1 H, d, J = 8.50Hz), 7.33(1H, dd, J = 8.65, 1.91 Hz). 2.44(3H, s) |
| 379 | 372 | 373 | 395 | 371 | 4.77 | 2 | 70 | 30 | 8.80(2H, d, J = 8.79Hz), 8.70(1H, d, J = 2.64Hz), 8.56(1H, s), 8.19(1H, d, J = 7.03Hz), 8.09(2H, d, J = 8.79Hz), 7.87-7.90(2H, m), 7.77(1H, d, J=7.62Hz), 7.58(1H, t, J = 7.62Hz), 6.65-6.66(1H, m), 238(3H, s) |
| 380 | 345 | 346 | 368 | 344 | 5.88 | 2 | 70 | 30 | |
| 381 | 345 | 346 | 368 | 344 | 5.32 | 2 | 70 | 30 | |
| 382 | 432 | 432 | 454 | | 7.1 | 2 | 70 | 30 | |
| 383 | 462 | 462 | 484 | | 8.1 | 2 | 60 | 40 | |
| 384 | 429 | 430 | 452 | | 6.57 | 2 | 70 | 30 | |
| 385 | 432 | 432 | 454 | | 7.15 | 2 | 70 | 30 | |
| 386 | 433 | 434 | 456 | | 8.03 | 2 | 60 | 40 | |
| 387 | 462 | 462 | 484 | | 7.97 | 2 | 60 | 40 | |
| 388 | 429 | 430 | | | 6.57 | 2 | 70 | 30 | |
| 389 | 391 | 392 | 414 | | 8.52 | 2 | 60 | 40 | |
| 390 | 406 | 407 | 429 | | 9.23 | 2 | 70 | 30 | 8.79(1H, d. J = 1.76Hz), 8.05(4H, Abtype, J = 24.91, 8.79Hz), 7.80(1H, s). 7.15(1H, d, J = 8.79Hz). 3.97(2H, d, J = 7.03Hz), 3.90(3H, s), 2.35(3H, s), 1.35(1H, s), 0.60-0.63(2H, m), 0.35-0.36(2H, m) |
| 391 | 461 | 462 | 484 | | 5.38 | 2 | 70 | 30 | 8.78(1H, d, J = 2.05Hz), 8.15(1H, dd, J = 8.79, 2.05Hz), 8.06(4H, Abtype, J = 24.32, 8.79Hz), 7.82(1H, s), 7.33(1H, d, J = 8.50Hz), 5.09(2H, s), 3.87(3H, s), 2.43(3H, s), 2.36(3H, s), 2.23(3H, s) |
| 392 | 505 | 505 | 527 | | 8.22 | 2 | 70 | 30 | 8.77(1H, s), 8.14-8.17(1H, m), 8.05(4H, Abtype, J = 23.94, 8.79Hz), 7.83(1H, s), 7.37(1H, d, J = 8.79Hz), 7.32(1H, d, J = 3.52Hz), 6.87-6.88(1H, m), 5.32(2H, s), 4.45(2H, q, J = 7.33Hz), 3.88(3H, s), 2.36(3H, s), 1.29(3H, t, J =7.33Hz) |
| 393 | 406 | 407 | 429 | | 7.68 | 2 | 70 | 30 | |
| 394 | 461 | 462 | 484 | | 4.87 | 2 | 70 | 30 | |
| 395 | 505 | 505 | 527 | | 7.13 | 2 | 70 | 30 | |
| 396 | 448 | 448 | 470 | | 6.52 | 2 | 60 | 40 | |
| 397 | 498 | 498 | 520 | | 6.65 | 2 | 70 | 30 | |
| 398 | 421 | 422 | 444 | | 7.3 | 2 | 60 | 40 | |
| 399 | 421 | 422 | 444 | | 6.95 | 2 | 60 | 40 | |
| 400 | 421 | 422 | 444 | | 7.27 | 2 | 60 | 40 | |
| 401 | 421 | 422 | 444 | | 6.87 | 2 | 60 | 40 | |
| 402 | 364 | 365 | 387 | 363 | 4.7 | 2 | 70 | 30 | |
| 403 | 436 | 437 | 459 | | 5.97 | 2 | 70 | 30 | |
| 404 | 417 | 418 | 440 | | 8.8 | 2 | 65 | 35 | |
| 405 | 421 | 422 | 444 | | 8.73 | 2 | 60 | 40 | |
| 406 | 449 | 449 | 471 | | 5.42 | 2 | 85 | 15 | |
| 407 | 449 | 449 | 471 | | 4.68 | 2 | 85 | 15 | |
| 408 | 363 | 364 | 386 | 362 | 4.12 | 2 | 65 | 35 | |
| 409 | 391 | 392 | 414 | | 8.62 | 2 | 60 | 40 | |
| 410 | 448 | 448 | 470 | | 5.77 | 2 | 65 | 35 | |
| 411 | 433 | 434 | 456 | | 8.42 | 2 | 60 | 40 | 8.58(1H, d, J = 2.05Hz), 7.98-8.08(3H, m), 7.74(1H, s), 8.51(1H, t, J = 8.21 Hz), 7.20-7.23(1H, m), 7.05(1 H, d, J = 8.50Hz), 4.38-4.39(2H, m), 4.31-4.33(2H, m), 3.39-3.62(8H, m), 2.33(3H, s) |
| 412 | 421 | 422 | 444 | | 5.42 | 2 | 60 | 40 | 8.57(1 H, d, J = 2.05Hz), 8.03-8.06(1H, m), 7.94-7.97(2H, m), 7.72(1H, s), 7.47(2H, d, J = 8.79Hz), 7.04(1 H, d, J = 8.50Hz), 4.15-4.37(4H, m), 3.47-3.59(4H, m), 2.96(3H, s), 2.31(3H, s) |
| 413 | 433 | 434 | 456 | | 5.75 | 2 | 60 | 40 | |
| 414 | 421 | 422 | 444 | | 5.38 | 2 | 60 | 40 | |
| 415 | 433 | 434 | 456 | | 5.73 | 2 | 60 | 40 | |
| 416 | 447 | 447 | | | 5.45 | 2 | 45 | 55 | |
| 417 | 296 | 297 | 319 | | 5.47 | 2 | 65 | 35 | |
| 418 | 296 | 297 | 319 | 295 | 4.68 | 2 | 65 | 35 | |
| 419 | 447 | 447 | | | 5.45 | 2 | 45 | 55 | |
| 420 | 477 | 477 | 499 | | 5.45 | 2 | 45 | 55 | |
| 421 | 419 | 420 | | | 4.07 | 2 | 70 | 30 | |
| 422 | 405 | 406 | 428 | | 4.05 | 2 | 70 | 30 | |
| 423 | 467 | 467 | | | 4.27 | 2 | 80 | 20 | 8.67(2H, d, J = 9.08Hz), 8.59-8.61(1 H, m), 8.20-8.22(1H, m), 7.72-7.74(1 H. m), 7.65(1H, s), 7.54(1H, t, J = 7.91Hz), 7.23-7.28(2H, m), 7.14(2H, d, J = 9.38Hz). 7.00(2H, d, J = 8.21 Hz). 6.84(1 H. t, J = 7.33Hz). 3.68-3.69(4H. m), 3.30-3.35(4H, m), 2.33(3H, s) |
| 424 | 433 | 434 | 456 | 432 | 6.83 | 2 | 65 | 35 | |
| 425 | 341 | 342 | 364 | 340 | 6.6 | 2 | 65 | 35 | |
| 426 | 419 | 420 | | | 4.75 | 2 | 70 | 30 | |
| 427 | 405 | 406 | 428 | | 4.78 | 2 | 70 | 30 | |
| 428 | 346 | 347 | 369 | 345 | 4.18/4.58 | 2 | 80 | 20 | |
| 429 | 346 | 347 | 369 | | 4.92/5.47 | 2 | 80 | 20 | |
| 430 | 467 | 467 | 489 | | 5.05 | 2 | 80 | 20 | |
| 431 | 391 | 392 | 414 | 390 | 9.07 | 2 | 60 | 40 | |
| 432 | 391 | 392 | 414 | 390 | 7.68 | 2 | 60 | 40 | |
| 433 | 519 | 519 | | | 3.95 | 2 | 85 | 15 | 8.71 (2H, d, J = 9.38Hz), 8.48-8.49(1H. m). 8.11 (4H, Abtype, J = 50.11, 9.08Hz), 7.91(1H, t, J = 1.76Hz), 7.79(1H, s), 7.23-7.28(2H, m). 7.15(2H, d, J = 9.67Hz). 8.98-7.01(3H, m), 6.79-6.87(2H, m), 3.72-3.74(8H, m) |
| 434 | 509 | 509 | | | 5.95 | 2 | 85 | 15 | 8.68(2H. d, J = 9.08Hz), 8.11(4H, Abtype, J = 38.38, 9.08Hz), 7.68(1H, s), 7.25(2H, t, J = 8.79Hz), 7.14(2H, d, J = 9.67Hz), 7.00(2H, d, J = 7.91 Hz), 6.87 (1H, s), 6.82(1H, t, J = 7.33Hz), 3.68-3.72(4H, m), 3.30-3.35(4H, m), 2.73(2H, t, J = 7.33Hz), 1.68-1.73(2H, m), 1.41-1.48(2H, m), 0.96(3H, t, J = 7.33Hz) |
| 435 | 459 | 459 | | | 4.82 | 2 | 50 | 50 | |
| 436 | 491 | 491 | 513 | | 6.7 | 2 | 75 | 25 | |
| 437 | 491 | 491 | 513 | | 2.25 | 2 | 75 | 25 | |
| 438 | 472 | 472 | 494 | | 8.8 | 2 | 70 | 30 | 8.67(2H, d, J = 9.38Hz), 8.46(1H, s), 8.11(4H, Abtype, J = 42.16, 9.08Hz), 7.90(1H, t, J = 1.76Hz). 7.74(1H, s), 7.08(2H, d, J = 9.67Hz), 6.98-6.99(1H, m), 4.52-4.54(1H, m), 4.23(1H, s), 4.19(1H, s), 3.25-3.30(2H, m), 2.99-3.09(2H, m), 1.75-1.80(3H, m), 1.14-1.19(2H, m) |
| 439 | 295 | 296 | 318 | 294 | 3.7 | 2 | 70 | 30 | |
| 440 | 495 | 495 | 517 | | 4.85 | 2 | 70 | 30 | |
| 441 | 391 | 392 | 414 | 390 | 6.08 | 2 | 65 | 35 | 8.66(2H, d, J = 9.08Hz), 8.59(1H, t, J = 1.76Hz), 8.21-8.22(0.5H, m), 8.1 9-8.19(0.5H, m), 7.73-7.74(0.5H, m), 7.71-7.72(0.5H, m), 7.65(1H, s), 7.53(1H, t, J = 7.91 Hz), 7.94(2H, d, J = 9.38Hz), 3.73-3.76(4H, m), 3.46-3.49(4H, m), 2.33(3H, s) |
| 442 | 432 | 433 | 455 | 431 | 4.22 | 2 | 65 | 35 | |
| 443 | 462 | 462 | 484 | | 4.78 | 2 | 80 | 20 | 8.63(2H, d, J = 9.08Hz), 8.05(4H, Abtype, J = 40.73, 9.08Hz), 7.62(1H, s), 7.06(2H, d, J = 9.38Hz), 4.5(1H, brs), 4.19(1H, s), 4.14(1H, s), 3.25-3.30(2H, m), 3.01 (2H, t, J = 7.03Hz). 2.71(2H, t. J = 7.03Hz), 1.64-1.78(5H, m), 1.35-1.47(2H, m). 1.17-1.23(2H, m), 0.93(3H, t, J = 7.03Hz) |
| 444 | 485 | 485 | 507 | | 4.38 | 2 | 70 | 30 | |
| 445 | 486 | 486 | 508 | | 5.38 | 2 | 65 | 35 | |
| 446 | 477 | 477 | 499 | | 6.22 | 2 | 50 | 50 | |
| 447 | 469 | 469 | 491 | | 4.55 | 2 | 65 | 35 | |
| 448 | 485 | 485 | 507 | | 8.5 | 2 | 75 | 25 | 8.66(2H. d, J = 9.08Hz), 8.06(4H, Abtype, J = 38.97, 9.08Hz), 7.66(1H, s). 7.03-7.16(6H, m), 3.39-3.69(4H, m), 3.24-3.27(4H, m), 2.33(3H, s) |
| 449 | 485 | 485 | 507 | | 7.23 | 2 | 75 | 25 | |
| 450 | 459 | 459 | | | 5.1 | 2 | 50 | 50 | |
| 451 | 390 | 391 | 413 | | 6.07 | 2 | 45 | 55 | |
| 452 | 390 | 391 | 413 | | 5.95 | 2 | 45 | 55 | |
| 453 | 379 | 380 | 402 | | 4.7 | 2 | 60 | 40 | |
| 454 | 407 | 407 | 429 | | 8.07 | 2 | 80 | 20 | 8.64 (1H, d, J = 4.10Hz), 7.99-8.09(4H, m), 7.93-7.96(1H, m), 7.81-7.92(1H, m), 7.80(1H, s), 7.55-7.61(3H, m). 2.36(3H, s) |
| 455 | 379 | 380 | 402 | 378 | 3.75 | 2 | 60 | 40 | |
| 456 | 407 | 407 | 429 | | 5.82/7.77 | 2 | 80 | 20 | 8.67(1H, d, J = 3.81 Hz), 8.00-8.10(6H, m), 7.81 (1H, s), 7.48-7.66(5H, m), 3.30-3.36(8H, m), 2.37(3H, s) |
| 457 | 381 | 382 | | | 5.88 | 2 | 50 | 50 | |
| 458 | 475 | 475 | | | 4.13 | 2 | 70 | 30 | |
| 459 | 514 | 515 | 537 | | 3.58 | 2 | 70 | 30 | |
| 460 | 457 | 457 | | | 5.93 | 2 | 65 | 35 | |
| 461 | 475 | 475 | | | 6.18 | 2 | 65 | 35 | |
| 462 | 514 | 515 | 537 | | 5.58 | 2 | 65 | 35 | |
| 463 | 457 | 457 | | | 4.42 | 2 | 70 | 30 | |
| 464 | 380 | 381 | 403 | | 5.65 | 2 | 70 | 30 | |
| 465 | 375 | 376 | 398 | 374 | 4.87 | 2 | 70 | 30 | 12.88(1H, brs), 11.74(1H, brs), 9.16 (1H, s), 8.46(1H, dd, J = 8.79, 1.76Hz), 8.03-8.33(5H, m), 7.53-7.55(2H, m), 6.66-6.69(1H, m), 4.60(2H, s), 3.40(3H, s) |
| 466 | 475 | 475 | 497 | | 2.4 | 2 | 70 | 30 | |
| 467 | 475 | 475 | | | 5.12 | 2 | 70 | 30 | |
| 468 | 394 | 395 | 417 | 393 | 4.75 | 2 | 70 | 30 | |
| 469 | 380 | 381 | 403 | 379 | 4.62 | 2 | 70 | 30 | |
| 470 | 406 | 407 | 429 | 405 | 8.87 | 2 | 75 | 25 | |
| 471 | 405 | 406 | 428 | 404 | 4.07 | 2 | 75 | 25 | |
| 472 | 375 | 376 | 398 | | 7.1 | 2 | 70 | 30 | |
| 473 | 392 | 393 | 415 | | 8.45 | 2 | 75 | 25 | |
| 474 | 387 | 388 | 410 | 386 | 6.70/9.22 | 2 | 75 | 25 | |
| 475 | 397 | 398 | 420 | 396 | 7.2 | 2 | 70 | 30 | |
| 476 | 402 | 403 | 425 | | 6.13 | 2 | 75 | 25 | |
| 477 | 397 | 398 | 420 | 396 | 5.42 | 2 | 75 | 25 | |
| 478 | 427 | 428 | 450 | | 7.13 | 2 | 75 | 25 | |
| 479 | 418 | 418 | 440 | | 6.37 | 2 | 80 | 20 | 8.67(2H, d, J = 8.21Hz). 8.62(1H, t, J = 1.76Hz). 8.22(1 H, d, J = 9.38Hz), 7.72(1H, d, J = 7.62Hz), 7.61(1H, s), 7.53(1H, t, J = 7.62Hz), 6.71(2H. d, J = 9.08Hz), 3.10-3.48(2H, m), 3.28-3.36(2H. m), 2.71(2H, t, J = 7.47Hz), 2.00-2.18(4H, m), 1.64-1.76(2H, m), 1.38-1.48(2H, m), 0.95(3H, t, J = 7.33Hz) |
| 480 | 392 | 393 | 415 | 391 | 5.65 | 2 | 80 | 20 | 8.71(1H, d, J = 1.47Hz), 8.00-8.08(1H, m), 8.06(4H, Abtype, J = 24.32, 9.08Hz), 7.81(1H, s), 7.17(1H, d, J = 8.21Hz), 6.21(2H, s), 2.74(2H, t, J = 7.62Hz), 1.69-1.74(2H, m), 1.41-1.49(2H, m), 0.96(3H, t, J = 7.33Hz) |
| 481 | 387 | 388 | 410 | 386 | 5.97/7.8 | 2 | 75 | 25 | |
| 482 | 371 | 372 | 394 | 370 | 5.33 | 2 | 75 | 25 | |
| 483 | 454 | 455 | 477 | | 6.7 | 2 | 85 | 15 | |
| 484 | 442 | 443 | 465 | 441 | 5.45 | 2 | 85 | 15 | |
| 485 | 454 | 455 | 477 | | 5.45 | 2 | 85 | 15 | |
| 486 | 390 | 391 | 413 | | 4.83/5.67 | 2 | 80 | 20 | |
| 487 | 402 | 403 | 425 | | 5.03/5.40 | 2 | 80 | 20 | |
| 488 | 378 | 379 | 401 | 377 | 5.58 | 2 | 65 | 35 | |
| 489 | 428 | 429 | 451 | 427 | 5.05 | 2 | 75 | 25 | |
| 490 | 505 | 505 | 527 | 503 | 4.65 | 2 | 80 | 20 | |
| 491 | 428 | 429 | 451 | 427 | 4.33 | 2 | 75 | 25 | |
| 492 | 505 | 505 | 527 | 503 | 5.28 | 2 | 80 | 20 | |
| 493 | 359 | 360 | 382 | | 3.72 | 2 | 75 | 25 | |
| 494 | 435 | 436 | 458 | | 6.63 | 2 | 75 | 25 | |
| 495 | 376 | 377 | 399 | | 5.42/6.13 | 2 | 65 | 35 | |
| 496 | 440 | 441 | 463 | | 5.95 | 2 | 70 | 30 | |
| 497 | 454 | 455 | 477 | | 5.97 | 2 | 70 | 30 | |
| 498 | 442 | 442 | 464 | | 9.07 | 2 | 80 | 20 | |
| 499 | 399 | 400 | 422 | | 5.62 | 2 | 75 | 25 | |
| 500 | 457 | 457 | 479 | | 6.8 | 2 | 70 | 30 | |
| 501 | 402 | 403 | 425 | | 5.2/5.72 | 2 | 80 | 20 | |
| 502 | 414 | 415 | 437 | | 3.40/3.70 | 2 | 80 | 20 | |
| 503 | 402 | 403 | 425 | 401 | 4.82 | 2 | 80 | 20 | |
| 504 | 414 | 415 | 437 | 413 | 4.81/5.27 | 2 | 75 | 25 | |
| 505 | 420 | 421 | 443 | 419 | 4.2 | 2 | 70 | 30 | |
| 506 | 337 | 338 | 360 | | 7.92 | 2 | 65 | 35 | |
| 507 | 309 | 310 | 332 | 308 | 4.5 | 2 | 60 | 40 | |
| 508 | 312 | 313 | 335 | 311 | 7.2 | 2 | 60 | 40 | |
| 509 | 309 | 310 | 332 | | 5.13 | 2 | 60 | 40 | |
| 510 | 452 | 453 | 475 | | 3.30/5.23 | 2 | 75 | 25 | |
| 511 | 312 | 313 | 335 | 311 | 3.27 | 2 | 75 | 25 | |
| 512 | 420 | 421 | 443 | | 6.83 | 2 | 65 | 35 | |
| 513 | 359 | 359 | 381 | | 3.58 | 2 | 65 | 35 | |
| 514 | 359 | 359 | 381 | | 3.87 | 2 | 65 | 35 | |
| 515 | 312 | 313 | 335 | | 2.93 | 2 | 75 | 25 | |
| 516 | 368 | 369 | 390 | | 4.80/5.57 | 2 | 65 | 35 | |
| 517 | 312 | 313 | 335 | 311 | 5.52 | 2 | 65 | 35 | |
| 518 | 452 | 453 | 475 | 451 | 2.55/3.83 | 2 | 80 | 20 | |
| 519 | 466 | 467 | 489 | 465 | 3.67/5.83 | 2 | 85 | 15 | |
| 520 | 375 | 377 | 399 | 375 | 3.22/3.55 | 2 | 70 | 30 | |
| 521 | 444 | 445 | 467 | 443 | 4.15/5.25 | 2 | 75 | 25 | |
| 522 | 434 | 435 | 457 | | 5.15/6.53 | 2 | 80 | 20 | |
| 523 | 337 | 338 | 360 | 336 | 5.7 | 2 | 70 | 30 | |
| 524 | 368 | 369 | 391 | 366 | 5.33/6.40 | 2 | 65 | 35 | |
| 525 | 375 | 377 | 399 | 375 | 3.38/4.00 | 2 | 65 | 35 | |
| 526 | 434 | 435 | 461 | 433 | 3.27/3.75 | 2 | 85 | 15 | |
| 527 | 444 | 445 | 467 | 443 | 4.95/6.65 | 2 | 75 | 25 | |
| 528 | 449 | 450 | 472 | | 5.35 | 2 | 80 | 20 | |
| 529 | 449 | 450 | 472 | | 8.08 | 2 | 75 | 25 | |
| 530 | 413 | 414 | 436 | 412 | 9.23 | 2 | 70 | 30 | |
| 531 | 439 | 440 | 462 | 438 | 5.78 | 2 | 75 | 25 | |
| 532 | 452 | 453 | 475 | | 7.2 | 2 | 75 | 25 | |
| 533 | 448 | 448 | 470 | 446 | 3.13 | 2 | 75 | 25 | 8.67(2H, d, J = 9.08Hz), 8.05(4H, Abtype, J = 19.49, 1.91 Hz), 7.82(1H, s), 7.11(2H, d, J = 9.08Hz), 5.01(2H, s), 3.35-3.45(4H, m), 2.36(3H, s), 1.50-1.65(4H, m), 1.40-1.49(2H, m) |
| 534 | 421 | 422 | 444 | 420 | 4.52 | 2 | 70 | 30 | |
| 535 | 407 | 408 | | 406 | 4.03 | 2 | 65 | 35 | |
| 536 | 379 | 380 | | 379 | 2.63 | 2 | 70 | 30 | 8.69(2H, d, J = 9.08Hz), 7.87-8.10(4H, m), 7.82(1 H, s), 7.64(1 H, brs); 7.46(1H, brs), 7.14(2H, d, J = 9.08Hz), 4.61(2H, s), 2.35(3H, s) |
| 537 | 455 | 456 | 478 | 454 | 3.57 | 2 | 75 | 25 | |
| 538 | 449 | 450 | 472 | 448 | 2.87 | 2 | 70 | 30 | 8.68(2H, d, J = 9.08Hz), 8.05(4H, Abtype, J = 26.38, 9.08), 7.82(1 H, s), 7.13(2H, d, J = 9.08Hz), 5.05(2H, s), 3.55-3.68(4H, m), 3.42-3.50(4H, m), 2.36(3H, s) |
| 539 | 398 | 399 | 421 | 397 | 5.7 | 2 | 80 | 20 | |
| 540 | 408 | 409 | 431 | 407 | 5.08 | 2 | 70 | 30 | 8.05(4H, Abtype, J = 26.08, 9.08Hz), 7.83(1H, s), 7.15(2H, d, J = 8.79Hz), 7.97(2H, s). 4.19(2H, q, J = 7.03Hz). 2.36(3H, s), 1.23(3H, t, J = 7.18Hz) |
| 541 | 457 | 458 | 480 | 456 | 7.58 | 2 | 75 | 25 | 8.71(2H. d. J = 9.08Hz), 8.32(2H, d. J = 8.79Hz), 8.05(4H, Abtype, J = 25.64, 8.93Hz). 7.83(1H, s), 7.77(2H, d, J = 8.50Hz), 7.26(2H, d, J = 9.08Hz), 5.45(2H, s), 2.36(3H, s) |
| 542 | 322 | 323 | 345 | 321 | 6.93 | 2 | 60 | 40 | 9.84(1H, s), 8.16(1 H, t, J = 1.901 Hz), 7.97-8.10 (4H, m), 7.91 (1H, brd, J = 7.91 Hz), 7.78 (1H, s), 7.38 (1H, t, J = 7.91 Hz), 7.05 (1H, dd, J = 7.32, 2.34Hz), 2.36 (3H. s) |
| 543 | 407 | 408 | | | 3.63/4.25 | 2 | 50 | 50 | |
| 544 | 425 | 426 | 448 | 424 | 7.68 | 2 | 70 | 30 | |
| 545 | 378 | 379 | 401 | 377 | 6.92 | 2 | 70 | 30 | |
| 546 | 364 | 365 | 387 | 363 | 6.08 | 2 | 70 | 30 | |
| 547 | 378 | 379 | 401 | 377 | 5.72 | 2 | 70 | 30 | |

By using the methods indicated below, it can be easily confirmed that the above-mentioned compounds represented by general formula (I) has an activity to inhibit ubiquitination of IκBα.

### Method 1: Measurement of ubiquitinating enzyme activity

### (1) Materials

A recombinant vector His-UBCH5b containing a polyhistidine tag (His) and UBCH5b was prepared. *E. coli* was transformed with the vector, the protein was expressed, and affinity purification was carried out using Ni-NTA agarose (Qiagen). Four types of recombinant baculoviruses (ROC1, Cullin1, Skp1, and His-TrCP1) were prepared for the SCF complex protein, insect cells were infected in quadruplicate for expression, and affinity purification was carried out using Ni-NTA agarose. Recombinant baculoviruses His-IKKβ, His-E1 were prepared. The insect cells were infected with each of those for expression, and affinity purification was carried out using Ni-NTA agarose. A recombinant vector GST-IκBα was prepared by fusing glutathione-S-transferase tag (GST) and IκBα, *E. coli* was transformed with the vector for the protein expression, and affinity purification was carried out using glutathione sepharose (Amersham Biosciences). Genetic transformants were prepared by PCR amplification followed by recombination, all of which were based on published human-derived sequences.

### (2) Assay methods

Phosphorylation reaction of IκBα was performed by incubating the above-mentioned assay buffer (50 mM Tris-HCl (pH7.4), 2 mMATP, 5 mM MgCl₂, 0.5 mM DTT, 0.1% BSA) containing IκBα and IKKβ at room temperature for 1 hour. Ubiquitination reactions were carried out as follows. Compound samples at various concentrations, ubiquitin, Eu (K)-labeled ubiquitin, E1, UbcH5b, and SCF complex were added to phosphorylated IκBα, and then this was incubated at room temperature for 30 minutes. The reaction was terminated by adding EDTA, the above-mentioned revelation buffer (100 mM phosphate buffer (pH7.0), 0.5 M KF, 0.1% BSA) containing XL665-labeled anti-GST antibody was added and this was left to stand at room temperature for 2 hours. Measurements were made using ARVO-HTS (Perkin Elmer). The ratio of fluorescence intensities at 620 nm and 665 nm (Em 665 nm/Em 620 nm x 10,000) with excitation light at 337 nm was measured.

The ubiquitination level without the addition of a compound sample was used as the control value, and the level in the absence of SCF complex was used as the blank value for the assays. Inhibitory activities of the compounds were indicated as % inhibition = 100 - (value with a compound sample added - blank value) / (control value - blank value) x 100. IC₅₀ was indicated as the concentration of the compound showing 50% inhibition. Eu (K)-labeled ubiquitin, XL665-labeled anti-GST antibody, and XL665-labeled anti-His antibody were purchased from CIS bio international. Costar solid black plates (96 wells) were used for the assays.

### Method 2: Measurement of ICAM-1 expressing activity

2.0 x 10⁴ HT-29 cells (ATCC) were plated onto a 96-well plate, and the cells were cultured for 48 hours using a McCoy' 5A medium (GIBCO) containing 10% inactivated fetal calf serum. The culture medium was removed, a culture medium containing a compound to be evaluated (final DMSO concentration of 0.5%) was added, and this was incubated at 37°C for 30 minutes. Thirty ng/mL of human TNFα (GIBCO) was added, and this was incubated at 37°C for 4 hours. After washing once with a washing solution (PBS containing 0.05% Tween 20), ice-cooled methanol was added, and this was left on ice for 10 minutes and then fixed. After removing methanol, PBS containing 1% BSA was added, and blocking was performed by letting this stand at room temperature for 30 minutes. Anti-ICAM-1 antibody (Santa Cruz Biotechnology) was added, and this was left to stand at room temperature for 1 hour. After washing with the washing solution, POD-labeled secondary antibody was added, and this was left to stand at room temperature for 30 minutes. This was washed, 100 µL of 100 mM citric acid buffer (pH4.2) containing 1 mM TMBZ and 0.03% H₂O₂ was added, and this was reacted at room temperature for 15 minutes. The reaction was terminated with 100 µL of 2 N H₂SO₄, and the absorbance at 450 nm was measured using ARVO-HTS (Perkin Elmer).

When performing the above-mentioned cell evaluation tests, cytotoxicity tests were carried out at the same time. The cultured HT-29 cells were treated with the compound and TNFα in the same manner as mentioned above, WST reagent (Dojindo Laboratories) was added to the culture medium, and were incubation at 37°C for 1 hour. Then, the absorbance at 450 nm was measured.

When cells are stimulated with TNFα, IκBα is degraded after ubiquitination resulting in the activation of NFκB and the promotion of the expression of adhesion factor ICAM-1. Therefore, the suppression of ICAM-1 expression described above serves as an indicator to determine the effectiveness of the compound in the cells. Furthermore, the cytotoxicity tests carried out at the same time can show that the compound is not cytotoxic.

### Method 3: Measurement of intracellular proteolytic activity

2.0 x 10⁵ HeLa cells (ATCC) were plated onto a 12-well plate, and the cells were cultured overnight using a Modified Eagle's Medium (MEM) (GIBCO) containing 10% inactivated fetal calf serum. A compound was added and treated at 37°C for 30 minutes. Thirty ng/mL of human TNFα was added, incubated at 37°C for 30 minutes, and the cells were collected. Cell lysis solution (10 mM HEPES (pH7.4), 60 mM KCI, 1 mM EDTA, 0.1% NP-40, 1 mM DTT, Protease Inhibitor Cocktail (Boehringer Manheim)) was added to this, and 5 µg of protein was applied to an SDS-PAGE gel. Electrophoresis was performed, the protein was transferred to a nitrocellulose membrane, and intracellular IκBα was detected by Western blotting using an anti-IκBα antibody.

Measurement of p53 degradation activity was performed by adding a compound to pre-cultured HeLa cells as in the IκBα measurements, incubated at 37°C for 6 hours, and the culture medium was removed where cells were collected. A pellet was obtained by centrifugation at 5,000 rpm for 5 minutes, to which 100 µL of PBS was added, and after sonication for 15 seconds, 5 µg of protein was applied to a 4-10% SDS-PAGE gel. Electrophoresis was perfomed, the protein was transferred to a nitrocellulose membrane, and intracellular p53 was detected by Western blotting using anti-p53 antibody (Santa Cruz Biotechnology).

When cells are stimulated with TNFα, IκBα is degraded by proteasomes after ubiquitination. Therefore, by examining the suppression of intracellular degradation of the above-mentioned IκBα protein, effectiveness of the compound in the cells can be determined. Furthermore, whether the degradation-inhibiting activity of the compound is IκBα-specific can be determined by detecting a p53 protein degradation-inhibiting activity in which degradation takes place because of another ubiquitin ligase (E3).

### Method 4: Measurement of LPS-induced TNFα production in cells

1.0 x 10⁵ human monocytic THP-1 cell line (ATCC) was cultured on a 96-well plate using RPMI 1640 (GIBCO) containing 10% inactivated fetal calf serum. A culture medium containing a compound to be evaluated (final DMSO concentration of 0.5%) was added, and this was incubated at 37°C for 30 minutes. Next, lipopolysaccharide (LPS) (Sigma-Aldrich) was added at 1 µg/mL, and this was incubated at 37°C for 8 hours. The culture supernatant was collected, measurements were made using the Human TNFα ELISA Assay Kit (Amersham Bioscience). Cytotoxicity tests were carried out as follows. The cells were treated in the same manner with the compound and LPS, cultured for 8 hours, which was then treated with the compound and LPS. Then, WST reagent were added to the culture medium, incubated at 37°C for 4 hours, and then the absorbance was measured at 450 nm.

### Method 5: Measurement of LPS-induced TNFα production in mice

The compounds to be administered were prepared at the time of use by suspending the compound in a 0.5% (w/v) carmelose-sodium (CMC-Na) solvant. Eight-week old Balb/c mice (male) were used to intraperitoneally administer the solvent (control group) or a compound of the present invention (compound-administered group), 30 minutes before lipopolysaccharide (LPS) administration. Ninety minutes after LPS administration (2.5 µg/500 µL in PBS), blood was collected from the mouse axillary vein. The obtained blood was transferred to a blood-collection tube containing a serum/plasma separating agent, and then centrifuged at 12,000 rpm at 4°C for 10 minutes to collect the plasma. TNFα in the obtained mouse plasma was measured using BD OptEIA^{™} ELISA Set (BD Biosciences).

IκBα becomes ubiquitinated and degraded by proteasome upon LPS stimulation of cells or animals. Degradation of IκBα promotes expression of inflammatory cytokine TNFα via activation of NFκB. Therefore, the effectiveness of the compounds in the cells and animals can be determined by examining the suppressive effects of the production of inflammatory cytokine TNFα in the above-mentioned cells and animals.

### Industrial Application

Compounds of the present invention suppress NFκB activation by inhibiting IκBα ubiquitination. Therefore, they are useful as preventive or therapeutic agents for NFκB-associated cancer or inflammatory diseases or the like.

## Claims

1. A compound represented by formula (I), or a pharmaceutically acceptable prodrug or salt thereof, or a hydrate or solvate thereof, 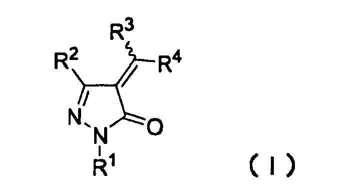 (wherein,
R¹ represents a C₁₋₆ alkyl group, a C₃₋₈ cycloalkyl group, a C₁₋₆ alkoxycarbonyl group, a C₁₋₆ alkoxycarbonyl C₁₋₃ alkyl group, a C₆₋₁₀ aryl group, a C₆₋₁₀ aryl C₁₋₆ alkyl group, or a 5- to 10-membered heteroaryl group;
the aforementioned C₁₋₆ alkyl group may comprise a substituent selected from a halogen atom, a carboxyl group, a C₃₋₈ cycloalkyl group, a C₁₋₆ alkoxycarbonyl group, a 5- to 10-membered heteroaryl group, a 5- to 8-membered heterocyclic group, and -NR^{1d}R^{2d} (wherein R^{1d} and R^{2d} each independently represent a hydrogen atom or a C₁₋₆ alkyl group);
the aforementioned aryl group and the aryl moiety of the C₆₋₁₀ aryl C₁₋₆ alkyl group may each independently comprise 1 to 3 substituents selected from the group consisting of a halogen atom, a hydroxy group, a cyano group, a nitro group, a carboxyl group, a C₁₋₆ alkyl group, a C₁₋₆ alkoxy group, -NR^{1a}R^{2a} (wherein R^{1a} and R^{2a} each independently represent a hydrogen atom or a C₁₋₆ alkyl group), and -SO₂R^{b} (wherein R^{b} represents a hydroxy group, a piperidyl group, a hydroxypiperidyl group, or -NR^{1b}R^{2b} (wherein, R^{1b} and R^{2b} each independently represent a hydrogen atom or a C₁₋₆ alkyl group));
alternatively, R¹ represents a group represented by formula (II), 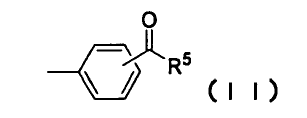 (wherein,
R⁵ represents a 5- to 10-membered heteroaryl group, a 5- to 8-membered heterocyclic group, or a group represented by -NR^{1c}R^{2c} (wherein, R^{1c} and R^{2c} each independently represent a hydrogen atom, or a C₁₋₆ alkyl group or benzodioxoylmethyl group that may comprise substituents selected from Group A described below; and the 5- to 10-membered heteroaryl group and the 5- to 8-membered heterocyclic group may each independently comprise 1 to 3 substituents selected from the group consisting of a hydroxy group, a carboxyl group, a C₁₋₆ alkyl group, a hydroxy C₁₋₆ alkyl group, a C₆₋₁₀ aryl group, and a hydroxy C₆₋₁₀ aryl group;
Group A: a halogen atom, a hydroxy group, a carboxyl group, a C₁₋₆ alkoxy group, a C₃₋₈ cycloalkyl group, a C₁₋₆ alkoxycarbonyl group, a C₁₋₆ alkylcarbonyloxy group, a 5- to 10-membered heteroaryl group, a 5- to 8-membered heterocyclic group, and -NR^{1d}R^{2d} (wherein, R^{1d} and R^{2d} each independently represent a hydrogen atom or a C₁₋₆ alkyl group);
R² represents a C₁₋₆ alkyl group, C₃₋₈ cycloalkyl group, R^{e}CO-that may comprise substituents selected from Group A mentioned above (wherein, R^{e} represents a heterocyclic group or -NR^{1e}R^{2e} (wherein, R^{1e} and R^{2e} each independently represent a hydrogen atom or a C₁₋₆ alkyl group), and the heterocyclic group may comprise a C₁₋₆ alkoxycarbonyl group as a substituent), a 5- to 10-membered heteroaryl group, a C₆₋₁₀ aryl group, or a C₆₋₁₀ aryl C₁₋₆ alkyl group (the aforementioned aryl group and the aryl moiety of the C₆₋₁₀ aryl C₁₋₆ alkyl group may comprise 1 to 3 substituents selected from the group consisting of a halogen, a hydroxy group, and a C₁₋₆ alkoxy group);
R³ represents a hydrogen atom or a C₁₋₆ alkyl group, R³ and R⁴ may be either an E or Z isomer;
R⁴ represents a C₆₋₁₀ aryl group, a 5- to 10-membered heteroaryl group, or a 5- to 8-membered heterocyclic group, and said C₆₋₁₀ aryl group, 5- to 10-membered heteroaryl group, and 5- to 8-membered heterocyclic group may each independently comprise 1 to 3 substituents selected from Group B described below;
Group B: a halogen atom, a nitro group, a C₁₋₆ alkoxy group, a carboxyl group, an oxo group, a C₁₋₆ alkyl group which may comprise a substituent selected from Group A described above (when said alkyl group comprises an isoxazolyl group as a substituent, the isoxazolyl group may comprise a C₁₋₃ alkyl group as a substituent), a C₁₋₆ alkylenedioxy group, C₆₋₁₀ aryl group, C₆₋₁₀ aryl C₁₋₆ alkyl group, -SO₃H, -SO₂NR^{1f}R^{2f} (wherein, R^{1f} and R^{2f} each independently represent a hydrogen atom or a C₁₋₆ alkyl group), a C₃₋₈ cycloalkylcarbonyl group, a C₆₋₁₀ arylcarbonyl group, a 5- to 10-membered heteroarylcarbonyl group, a 5- to 10-membered heteroaryl group, and a 5- to 8-membered heterocyclic group;
In Group B mentioned above, the C₆₋₁₀ aryl group, 5- to 10-membered heteroaryl group, and 5- to 8-membered heterocyclic group may each independently comprise 1 to 4 substituents selected from Group C described below;
Group C: a halogen atom, a C₁₋₆ alkyl group that may comprise a substituent selected from Group A described above, a C₁₋₆ alkoxy group, a hydroxy group, a carboxyl group, a C₁₋₆ alkylcarbonyl group, a C₁₋₆ alkoxycarbonyl group, a C₆₋₁₀ arylcarbonyl group, a C₆₋₁₀ aryl C₁₋₃ alkoxycarbonyl group, a C₆₋₁₀ aryl group, a C₆₋₁₀aryl C₁₋₆ alkyl group, a halogenated C₆₋₁₀ aryl group, a methanesulfonyl group, a 5- to 8-membered heterocyclic group, and a 5- to 10-membered heteroarylcarbonyl group;
alternatively, R⁴ may represent a group represented by formula (III),
-Ar¹-O-R⁶ (III)
(wherein,
Ar¹ represents a phenyl group or a C₁₋₃ alkoxyphenyl group;
R⁶ represents a hydrogen atom, a C₁₋₆ alkyl group that may comprise a substituent selected from Group A described above (when said alkyl group comprises a 5- to 10-membered heteroaryl group as a substituent, said 5- to 10-membered heteroaryl group may comprise 1 to 3 substituents selected from the group consisting of a C₁₋₆ alkyl group and a C₁₋₆ alkoxycarbonyl group), a methanesulfonyl group, a phenylsulfonyl group, a *p*-toluenesulfonyl group, a *p*-(*t*-butyl)phenylsulfonyl group, a C₆₋₁₀ aryl group, a C₆₋₁₀ aryl C₁₋₆ alkyl group (in R⁶, the aforementioned aryl group and the aryl moiety of the C₆₋₁₀ aryl C₁₋₆ alkyl group may comprise 1 to 3 substituents selected from a nitro group, a C₁₋₆ alkyl group, and a C₁₋₃ alkoxycarbonyl group), and R^{g}CO-CH₂- (wherein, R^{g} represents a heterocyclic group or -NR^{1g}R^{2g} (wherein, R^{1g} and R^{2g} each independently represent a hydrogen atom, a C₁₋₆ alkyl group, or a C₆₋₁₀ aryl group));
alternatively, R⁴ may represent a group represented by formula (IV); 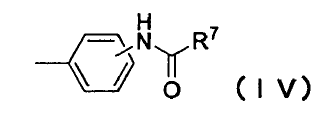 (wherein,
R⁷ represents a C₂₋₆ alkenyl group, a C₆₋₁₀ aryl group, a 5- to 10-membered heteroaryl group, or a phenylethylene group;
the aforementioned C₆₋₁₀ aryl group, 5- to 10-membered heteroaryl group, and phenylethylene group may each independently comprise 1 to 3 substituents selected from the group consisting of a halogen atom, a hydroxy group, a C₁₋₃ alkoxycarbonyl group, and a C₁₋₃ alkoxy group)).

2. The compound of claim 1, or a pharmaceutically acceptable prodrug or salt thereof, or a hydrate or solvate thereof, wherein the aforementioned R¹ represents a C₁₋₃ alkyl group, a C₃₋₈ cycloalkyl group, a C₁₋₆ alkoxycarbonyl C₁₋₃ alkyl group, a C₆₋₁₀ aryl C₁₋₃ alkyl group, a C₆₋₁₀ aryl group, a C₁₋₆ alkoxycarbonyl group, or a pyridyl group;
the aforementioned aryl group and the aryl moiety of the C₆₋₁₀ aryl C₁₋₃ alkyl group may each independently comprise 1 to 3 substituents selected from the group consisting of a halogen atom, a hydroxy group, a cyano group, a nitro group, a carboxyl group, a C₁₋₆ alkyl group, a C₁₋₆ alkoxy group, -SO₂R^{a} (wherein, R^{a} represents a hydroxy group, -NH₂, a piperidyl group, or a hydroxypiperidyl group), and -NR^{1b}R^{2b} (wherein, R^{1b} and R^{2b} each independently represent a hydrogen atom or a C₁₋₆ alkyl group);
alternatively, the aforementioned R¹ represents a group represented by formula (II) 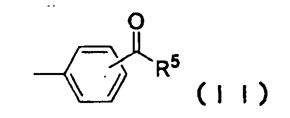 (wherein,
R⁵ represents -NH₂, a benzodioxoylmethylamino group, a mono C₁₋₆ alkylamino group, a di C₁₋₆ alkylamino group, a mono (C₃₋₈ cycloalkyl C₁₋₃ alkyl) amino group, a hydroxy C₁₋₆ alkylamino group, an *N*-(hydroxy C₁₋₆ alkyl)-*N*-C₁₋₆ alkylamino group, a C₁₋₆ alkoxy C₁₋₆ alkylamino group, a pyrrolidinyl group, a piperidyl group, a piperazinyl group, a tetrahydropyridyl group, or a morpholinyl group;
said pyrrolidinyl group, piperidyl group, piperazinyl group, and tetrahydropyridyl group may each independently comprise 1 to 3 substituents selected from the group consisting of a hydroxy group, a carboxyl group, a C₁₋₆ alkyl group, a hydroxy C₁₋₆ alkyl group, a C₆₋₁₀ aryl group, and a hydroxy C₆₋₁₀ aryl group).

3. The compound of claim 1, or a pharmaceutically acceptable prodrug or salt thereof, or a hydrate or solvate thereof, wherein the aforementioned R¹ represents a C₆₋₁₀ aryl C₁₋₃ alkyl group, a C₆₋₁₀ aryl group, a cyclohexyl group, or a pyridyl group;
the aforementioned C₆₋₁₀ aryl group and the aryl moiety of the C₆₋₁₀ aryl C₁₋₃ alkyl group may each independently comprise 1 to 3 substituents selected from the group consisting of a halogen atom, a hydroxy group, a cyano group, a nitro group, a carboxyl group, a C₁₋₆ alkyl group, a C₁₋₆ alkoxy group, -SO₂R^{a} (wherein, R⁸ represents a hydroxy group, -NH₂, a piperidyl group, or a hydroxypiperidyl group), and -NR^{1b}R^{2b} (wherein, R^{1b} and R^{2b} each independently represent a hydrogen atom or a C₁₋₆ alkyl group);
alternatively, the aforementioned R¹ represents a group represented by formula (II) 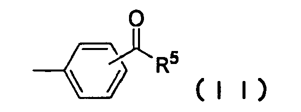 (wherein,
R⁵ represents -NH₂, a benzodioxoylmethylamino group, a mono C₁₋₆ alkylamino group, a di C₁₋₆ alkylamino group, a mono (C₃₋₈ cycloalkyl C₁₋₃ alkyl) amino group, a hydroxy C₁₋₆ alkylamino group, an *N*-(hydroxy C₁₋₆ alkyl)-*N*-C₁₋₆ alkylamino group, a C₁₋₆ alkoxy C₁₋₆ alkylamino group, a pyrrolidinyl group, a piperidyl group, a piperazinyl group, a tetrahydropyridyl group, or a morpholinyl group;
said pyrrolidinyl group, piperidyl group, piperazinyl group, and tetrahydropyridyl group may each independently comprise 1 to 3 substituents selected from the group consisting of a hydroxy group, a carboxyl group, a C₁₋₆ alkyl group, a hydroxy C₁₋₆ alkyl group, a C₆₋₁₀ aryl group, and a hydroxy C₆₋₁₀ aryl group).

4. The compound of any one of claims 1 to 3, or a pharmaceutically acceptable prodrug or salt thereof, or a hydrate or solvate thereof, wherein R² represents a C₁₋₆ alkyl group, a C₃₋₈ cycloalkyl group, a halogenated C₁₋₆ alkyl group, a C₁₋₆ alkoxy C₁₋₆ alkyl group, R^{e}CO- (wherein R^{e} represents a heterocyclic group or -NR^{1e}R^{2e} (wherein, R^{1e} and R^{2e} each independently represent a hydrogen atom or a C₁₋₆ alkyl group), and the heterocyclic group may comprise a C₁₋₆ alkoxycarbonyl group as a substituent), a furyl group, a pyridyl group, a C₆₋₁₀ aryl group, or a C₆₋₁₀ aryl C₁₋₆ alkyl group (the aforementioned aryl group and the aryl moiety of the C₆₋₁₀ aryl C₁₋₆ alkyl group may comprise 1 to 3 substituents selected from the group consisting of a halogen, a hydroxy group, and a C₁₋₆ alkoxy group).

5. The compound of any one of claims 1 to 4, or a pharmaceutically acceptable prodrug or salt thereof, or a hydrate or solvate thereof, wherein R³ is a hydrogen atom.

6. The compound of any one of claims 1 to 5, or a pharmaceutically acceptable prodrug or salt thereof, or a hydrate or solvate thereof, wherein
R⁴ represents a phenyl group, a naphthyl group, an indolyl group, a furyl group, a thienyl group, a thiazolyl group, a pyrrolyl group, a benzofuranyl group, a dimethylpyrrolyl group, a pyridyl group, a quinoxalinyl group, a pyrazolinyl group, or a pyrazolyl group;
said phenyl group, naphthyl group, indolyl group, furyl group, thienyl group, thiazolyl group, pyrrolyl group, benzofuranyl group, dimethylpyrrolyl group, pyridyl group, quinoxalinyl group, pyrazolinyl group, and pyrazolyl group may each independently comprise 1 to 3 substituents selected from Group D described below;
Group D: a halogen atom, a nitro group, a C₁₋₃ alkoxy group, a carboxyl group, an oxo group, a C₁₋₆ alkyl group, a C₃₋₈ cycloalkyl C₁₋₃ alkyl group, a hydroxy C₁₋₃ alkyl group, a C₆₋₁₀ aryl C₁₋₃ alkyl group, an isoxazolyl C₁₋₃ alkyl group (the aforementioned isoxazolyl ring may comprise a C₁₋₃ alkyl group as a substituent), a C₁₋₃ alkylcarbonyloxy C₁₋₃ alkyl group, a C₁₋₆ alkylenedioxy group, a phenyl group, -SO₃H, a C₆₋₁₀ arylcarbonyl group, a furylcarbonyl group, a C₃₋₈ cycloalkylcarbonyl group, an *N*-methylpyrrolylcarbonyl group, a morpholinyl group, a pyrrolyl group, a triazolyl group, a thiazolyl group, an imidazolyl group, a pyrazolyl group, a pyrrolidinyl group, a piperidyl group, and a piperazinyl group;
in the aforementioned Group D, a phenyl group, a morpholinyl group, a pyrrolyl group, a triazolyl group, a thiazolyl group, an imidazolyl group, a pyrazolyl group, a pyrrolidinyl group, a piperidyl group, and a piperazinyl group may each independently comprise 1 to 4 substituents selected from Group E described below;
Group E: a halogen atom, a C₁₋₃ alkyl group, a hydroxy C₁₋₃ alkyl group, a C₁₋₃ alkoxy group, a C₁₋₆ alkoxycarbonyl C₁₋₆ alkyl group, a hydroxy group, a carboxyl group, a C₁₋₆ alkylcarbonyl group, a C₁₋₆ alkoxycarbonyl group, a C₆₋₁₀ arylcarbonyl group, a C₆₋₁₀ aryl C₁₋₃ alkoxycarbonyl group, a phenyl group, a halophenyl group, a benzyl group, a methanesulfonyl group, a pyrrolidinyl group, a furylcarbonyl group, and an isoxazolylcarbonyl group;
alternatively, R⁴ may represent a group represented by formula (III);
-Ar¹-O-R⁶ (III)
(wherein,
Ar¹ represents a phenyl group or a C₁₋₃ alkoxyphenyl group;
R⁶ represents a hydrogen atom, a C₁₋₆ alkyl group (said alkyl group may comprise an isoxazolyl group, a furyl group, or a benzotriazole group as a substituent, and said isoxazolyl group, furyl group, and benzotriazole group may each independently comprise 1 to 3 substituents selected from the group consisting of a C₁₋₃ alkyl group and a C₁₋₃ alkoxycarbonyl group), a C₃₋₈ cycloalkylmethyl group, a hydroxycarbonyl C₁₋₃ alkyl group, a C₁₋₆ alkoxycarbonyl C₁₋₃ alkyl group, a tetrahydrofuranylmethyl group, an amino C₁₋₆ alkyl group, a mono C₁₋₃ alkylamino C₁₋₆ alkyl group, a di C₁₋₃ alkylamino C₁₋₆ alkyl group, a methanesulfonyl group, a phenylsulfonyl group, a *p*-toluenesulfonyl group, ap-(t-butyl)phenylsulfonyl group, a C₆₋₁₀ aryl group, a C₆₋₁₀ aryl C₁₋₆ alkyl group (said C₆₋₁₀ aryl group and the aryl moiety of the C₆₋₁₀ aryl C₁₋₆ alkyl group may be substituted with 1 to 3 groups selected from a nitro group, a C₁₋₆ alkyl group, a carboxyl group, and a C₁₋₃ alkoxycarbonyl group); or
R^{g}CO-CH₂- (wherein, R^{g} represents a piperidyl group, a morpholinyl group, or -NR^{1g}R^{2g} (wherein, R^{1g} and R^{2g} each independently represent a hydrogen atom, a C₁₋₆ alkyl group, or a C₆₋₁₀ aryl group)); and
alternatively, R⁴ may represent a group represented by formula (IV); 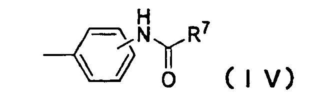 (wherein, R⁷ represents a C₂₋₆ alkenyl group, a phenyl group, a furyl group, a naphthyl group, or a phenylethylene group;
the aforementioned phenyl group, furyl group, naphthyl group, phenylethylene group may each independently comprise 1 to 3 substituents selected from the group consisting of a halogen atom, a hydroxy group, a C₁₋₃ alkoxycarbonyl group, and a C₁₋₃ alkoxy group)).

7. The compound of any one of claims 1 to 5, or a pharmaceutically acceptable prodrug or salt thereof, or a hydrate or solvate thereof, wherein
R⁴ represents a phenyl group, a naphthyl group, a quinoxalinyl group, a thienyl group, a thiazolyl group, a benzofuranyl group, an indolyl group, or a furyl group;
the aforementioned phenyl group may be substituted with a group selected from a halogen atom, a nitro group, a carboxyl group, a morpholinyl group, a pyrrolyl group, a pyrazolyl group, a pyrazolinyl group, a triazolyl group, an imidazolyl group, a thiazolyl group, a phenyl group, a C₁₋₆ alkyl group, a C₁₋₆ alkylenedioxy group, a C₁₋₃ alkylimidazolyl group, a C₆₋₁₀ aryl C₁₋₃ alkylimidazolyl group, -SO₂NR^{1f}R^{2f} (wherein, R^{1f} and R^{2f} each independently represent a hydrogen atom or a C₁₋₆ alkyl group), a pyrrolidinyl group (the pyrrolidinyl group may comprise a hydroxy group or a hydroxymethyl group as a substituent), a piperidyl group (the piperidyl group may be substituted with a group selected from a hydroxy group, a pyrrolidinyl group, a carboxyl group, a fluorophenyl group, and a hydroxymethyl group), and a piperazinyl group (the piperazinyl group may be substituted with a group selected from a phenyl group, a fluorophenyl group, a C₁₋₃ alkyl group, a C₁₋₆ alkylcarbonyl group, a furylcarbonyl group, an isoxazolylcarbonyl group, a C₁₋₆ alkoxycarbonyl group, a C₆₋₁₀ arylcarbonyl group, and a methanesulfonyl group);
the aforementioned indolyl group may be substituted with a group selected from a halogen atom, a benzyl group, a C₁₋₃ alkyl group, a C₃₋₈ cycloalkylmethyl group, a methylisoxazolylmethyl group, a dimethylisoxazolylmethyl group, a methylthiazolylmethyl group, a C₃₋₈ cycloalkylcarbonyl group, a furylcarbonyl group, a methylpyrrolylcarbonyl group, and a C₆₋₁₀ arylcarbonyl group (the aryl moiety may be substituted with a C₁₋₆ alkyl group);
the aforementioned furyl group may be substituted with a group selected from a hydroxymethyl group, a nitro group, a hydroxysulfonyl group, an *N*-methylpyrazolyl group, an *N*-benzylpyrazolyl group, and a phenyl group (the phenyl group may comprise a substituent selected from a halogen atom, a C₁₋₃ alkoxy group, a C₁₋₃ alkoxycarbonyl group, and a C₁₋₃ alkylcarbonyl group);
alternatively, the aforementioned R⁴ represents formula (III);
-Ar¹-O-R⁶ (III)
(wherein,
Ar¹ represents a phenyl group, a methoxyphenyl group, or an ethoxyphenyl group;
R⁶ represents a hydrogen atom, a methyl group, a cyclopropylmethyl group, a cyclohexylmethyl group, an ethoxycarbonylmethyl group, a *t*-butoxycarbonylmethyl group, an ethoxycarbonylfurylmethyl group, -CH₂CH₂CH₂N(CH₃)₂, a benzotriazolmethyl group, a *p*-(*t*-butylphenyl)sulfonyl group, a *p*-toluenesulfonyl group, -CH₂CO₂H, R^{g}CO-CH₂- (wherein, R^{g} represents -NH₂, -N(CH₃)₂, an *n*-propylamino group, a phenylamino group, a piperidyl group, or a morpholinyl group), a phenyl group, a benzyl group, an isoxazolylmethyl group, an oxazolyl group, a triazolyl group, or a furyl group; said phenyl group, benzyl group, isoxazolylmethyl group, oxazolyl group, triazolyl group, and furyl group may comprise 1 to 3 substituents selected from a methyl group, an ethyl group, a nitro group, -CO₂Me, and -CO₂Et); or
alternatively, the aforementioned R⁴ represents formula (IV) 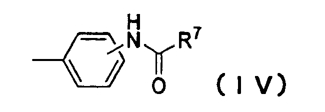 (wherein, R⁷ represents a -CH=C(CH₃)₂, -CH=CHPh, a furyl group, a naphthyl group, or a phenyl group;
said phenyl group may comprise 1 to 3 substituents selected from a halogen atom, a hydroxy group, and a C₁₋₃ alkoxy group.

8. The compound of any one of claims 1 to 5, or a pharmaceutically acceptable prodrug or salt thereof, or a hydrate or solvate thereof, wherein
R⁴ represents a naphthyl group, a quinoxalinyl group, a thienyl group, a thiazolyl group, a benzofuranyl group, a 4-chloroindol-3-yl group, a 1-benzylindol-5-yl group, a 1-(3,5-dimethylisoxazol-4-ylmethyl)indol-5-yl group, a 1-benzylindol-5-yl group, a 1-methylindol-5-yl group, a 1-methylindol-2-yl group, a 1-cyclopropylmethylindol-5-yl group, a 1-cyclohexylmethylindol-5-yl group, a 1-cyclopropylcarbonylindol-5-yl group, a 1-benzoylindol-5-yl group, a 1-(furan-2-carbonyl)indol-5-yl group, a 1-(1-methylpyrrol-2-carbonyl)indol-5-yl group, a 5-chloroindol-3-yl group, an indol-4-yl group, an indol-5-yl group, an indol-6-yl group, an indol-7-yl group, a 1-methylindol-2-yl group, a 1-(5-methylisoxazol-3-ylmethyl)indol-5-yl group, a 1-(2-methylthiazol-4-ylmethyl)indol-5-yl group, a phenyl group, or a furyl group;
the aforementioned phenyl group may be substituted with a group selected from a fluorine atom, a nitro group, a carboxyl group, a morpholinyl group, a pyrrolyl group, a pyrazolyl group, a pyrazolinyl group, a triazolyl group, a thiazolyl group, a phenyl group, a *t*-butyl group, a C₁₋₆ alkylenedioxy group, an imidazolyl group, a C₁₋₃ alkylimidazolyl group, a C₆₋₁₀ aryl C₁₋₃ alkylimidazolyl group, -SO₂NHCH₃, -SO₂N(CH₃)₂, -SO₂NHPh, or SO₂NPh₂,
a pyrrolidinyl group (the pyrrolidinyl group may comprise a hydroxy group or a hydroxymethyl group as a substituent),
a piperidyl group (said piperidyl group may comprise as a substituent a group selected from a hydroxy group, a hydroxymethyl group, a pyrrolidinyl group, a carboxyl group, and a fluorophenyl group), and
a piperazinyl group (said piperazinyl group may comprise as a substituent a group selected from a methyl group, a methoxycarbonyl group, an ethoxycarbonyl group, a t-butoxycarbonyl, an acetyl group, a phenyl group, a fluorophenyl group, a benzoyl group, a furan-3-carbonyl group, an isoxazol-4-carbonyl group, a methylsulfonyl group, and a tosyl group);
the aforementioned furyl group may comprise a substituent selected from a nitro group,
a hydroxymethyl group, a hydroxysulfonyl group, a phenyl group (said phenyl group may comprise as a substituent a group selected from a fluorine atom, a chlorine atom, a methoxy group, a methoxycarbonyl group, an ethoxycarbonyl group, and a methylcarbonyl group), an *N*-methylpyrazolyl group, and an *N*-benzylpyrazolyl group;
alternatively, the aforementioned R⁴ represents formula (III);
-Ar¹-O-R⁶ (III)
(wherein,
Ar¹ represents a phenyl group, a methoxyphenyl group, or an ethoxyphenyl group;
R⁶ represents a hydrogen atom, a methyl group, a cyclopropylmethyl group, a cyclohexylmethyl group, an ethoxycarbonylmethyl group, a *t*-butoxycarbonylmethyl group, an ethoxycarbonylfurylmethyl group, -CH₂CH₂CH₂N(CH₃)₂, a benzotriazolmethyl group, a *p*-(*t*-butylphenyl)sulfonyl group, a *p*-toluenesulfonyl group, -CH₂CO₂H, R^{g}CO-CH₂- (wherein, R^{g} represents -NH₂, -N(CH₃)₂, an *n*-propylamino group, a phenylamino group, a piperidyl group, or a morpholinyl group), a phenyl group, a benzyl group, an isoxazolylmethyl group, an oxazolyl group, a triazolyl group, or a furyl group, said phenyl group, benzyl group, isoxazolylmethyl group, oxazolyl group, triazolyl group, and furyl group may comprise 1 to 3 substituents selected from a methyl group, an ethyl group, a nitro group, -CO₂Me, and -CO₂Et); or
alternatively, the aforementioned R⁴ represents formula (IV) 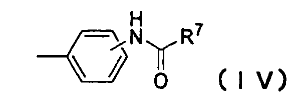 (wherein, R⁷ represents -CH=C(CH₃)₂, -CH=CHPh, a furyl group, a naphthyl group, or a phenyl group;
said phenyl group may comprise 1 to 3 substituents selected from a halogen atom, a hydroxy group, and a C₁₋₃ alkoxy group.
